# EUROPEAN PATENT APPLICATION

(11) **EP 2 253 618 A1**
(43) Date of publication of application: **24.11.2010**
(21) Application number: 09715685.5
(22) Date of filing: 27.02.2009
(51) Int. Cl.: C07D 213/64, A61K 31/44, A61K 31/4433, A61K 31/4439, A61K 31/444, A61K 31/505, A61K 31/506, A61K 31/5377, A61P 25/18, A61P 43/00, C07D 213/65, C07D 213/68, C07D 213/70, C07D 213/74, C07D 213/75, C07D 239/34, C07D 239/42, C07D 295/12, C07D 401/12, C07D 403/04, C07D 403/12

(54) **COMPOUND HAVING 6-MEMBERED AROMATIC RING**

(30) Priority: 27.02.2008 JP 2008046757
(71) Applicant: Takeda Pharmaceutical Company Limited, Osaka 540-8645 (JP)
(72) Inventor: SETOH, Masaki, Osaka-shi Osaka 532-8686 (JP); KOBAYASHI, Toshitake, Tsukuba-shi Ibaraki 300-4293 (JP); TANAKA, Toshio, Tsukuba-shi Ibaraki 300-4293 (JP); BABA, Atsuo, Tsukuba-shi Ibaraki 300-4293 (JP)
(74) Representative: Held, Stephan
(86) International application number: PCT/JP2009/000882
(87) International publication number: WO 2009/107391

(57) **Abstract**

An object of the present invention is to provide an agent for preventing or treating schizophrenia or the like, wherein the compound of the present invention has GPR52 agonist activity.

[Means]

A compound represented by the following formula (I) or salt thereof:
wherein
A represents -(CH²)ₙ-CO-NR^{a}- (n is an integer of 0 to 3) or -NR^{a}-CO-,
B represents a hydrogen atom, halogen atom, cyano group, hydroxy group, or the like, X¹, X², X³, and X⁴ represent the same or different -CR^{x}=, or -N=,
Y represents -O-, -S-, -S(O)-, -S(O)₂-, or NR^{y}-,
Z represents a bond, methylene, or ethylene,
Ar¹ represents a five- to ten-membered aromatic ring (except for thiazole) which may be substituted with one or more substituents selected from halogen atoms, optionally halogenated C₁₋₆ alkyl groups, and the like,
Ar² represents a five- to six-membered aromatic ring which may be substituted with one or more substituents selected from halogen atoms, optionally halogenated C₁₋₆ alkyl groups, and the like, and which may be condensed with an optionally substituted five-to six-membered ring, and
R^{a}, R^{b}, R^{x}, and R^{y} represent the same or different hydrogen atom, halogen atoms, or the like].

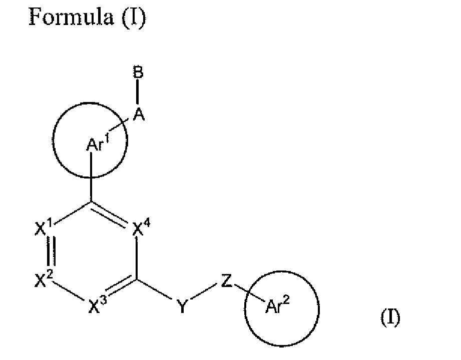

## Description

### [Technical Field]

The present invention relates to a novel compound containing a six-membered aromatic ring, particularly a pyridine derivative, a method for manufacturing the same, and a pharmaceutical composition containing the same. More specifically, the present invention relates to a compound having an agonistic effect on GPR52, which is effective as a pharmaceutical agent for preventing and treating mental disorders such as schizophrenia.

### [Background of the Invention]

Schizophrenia is a disease that occurs in people from adolescence to adulthood and shows characteristic thinking disturbances, disturbances of ego, and behavioral abnormalities associated therewith. The disease reportedly develops in about 1% of the entire population but is chronic in most cases, and is associated with a decrease in initiative, interpersonal contact, or the like, which leads to considerable social hardship. The core symptoms of schizophrenia are broadly classified into (1) positive symptoms such as delusions and hallucination, (2) negative symptoms such as hypesthesia, social withdrawal, and loss of motivation or concentration, and (3) cognitive dysfunction In these core symptoms, hyperactivity of the dopamine nervous system in the mesolimbic system is believed to be intimately involved in the development of positive symptoms, whereas depression of the nervous system such as the glutamic acid nervous system in the cortex of the frontal lobe is believed to be intimately involved in the development of negative symptoms or impaired cognitive function.
Typical antipsychotic drugs with dopamine D2 receptor antagonist action such as chlorpromazine have shown effect in improving positive symptoms. On the other hand, multi-acting receptor targeted agents such as clozapine and olanzapine show consistent effects toward negative symptoms or cognitive impairment, but it is known that many patients respond poorly to these drugs. Other problems with typical antipsychotics are the side effects, which include the development of akathisia, dystonias, and extrapyramidal symptoms such as Parkinson-like movement disorders, as well as hyperprolactinemia. Granulocytopenia is also a serious side effect of clozapine, and side effects such as weight gain, lipidosis, oversedation, and prolonged QT interval on electrocardiogram are problems associated with atypical antipsychotics such as olanzapine.
Human GPR52 (Sawzdargo et al., Molecular Brain Research, 64: 193-198, 1999) is a G protein-coupled receptor (GPCR). GPR52 agonists, ligands, and the like were recently found to increase intracellular cAMP levels in nerve cells expressing GPR52 or so on, and are thus believed to be capable of improving positive symptoms of schizophrenia by suppressing mesolimbic dopamine pathway hyperactivity, which is thought to be one of the causes of positive symptoms in schizophrenia. These were also found to be capable of improving cognitive disorders and negative symptoms in schizophrenia by improving decreased function of NMDA receptors in the cerebral cortex, which is thought to be one of the causes of such troubles (WO 2006/098520).
There is thus a need to develop a compound that would have an agonistic effect on GPR52 and that would be useful as a pharmaceutical agent for preventing and treating mental disorders such as schizophrenia.

International Publication WO 2005/066139 pamphlet discloses compounds represented by the following general formula, which include compounds containing six-membered aromatic rings: 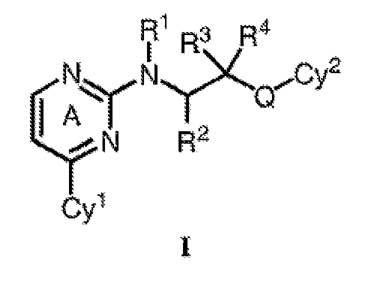 International Publication WO 2004/096797 pamphlet discloses compounds represented by the following general formula, which include compounds containing six-membered aromatic rings: 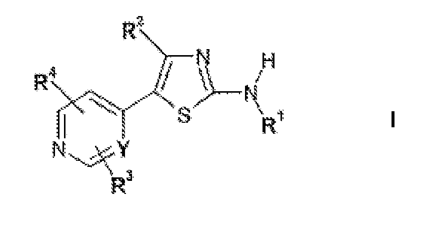 NPL 1 below describes 1-{2-[(3-chlorobenzyl)oxy]-6-methylpyridin-4-yl}-2-methyl-1H-imidazo le-4-carboxamide.
NPL 2 below describes tert-butyl {5-[6-(3-chlorophenoxy)pyrazin-2-yl]pyridin-3-yl}carbamate.
International Publication WO 2004/096797 pamphlet discloses compounds represented by the following general formula, which include compounds containing six-membered aromatic rings: 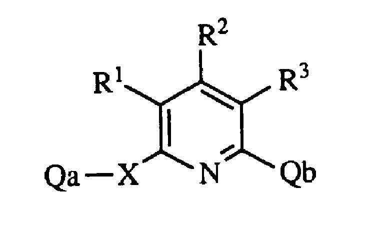 [PTL 1]
International Publication WO 2006/098520 Pamphlet
[PTL 2]
International Publication WO 2005/066139 Pamphlet
[PTL 3]
International Publication WO 2004/096797 Pamphlet
[NPL 1]
Sawzdargo et al., Molecular Brain Research, 64: pp. 193-198, 1999
[NPL 2]
Kuo, Gee-Hong et al, Journal of Medicinal Chemistry, 48(15): pp. 4892-4909, 2005

### [Summary of Invention]

### [Technical Problem]

An object of the present invention is to provide a compound that has an agonistic effect on GPR52 and that is useful as a pharmaceutical agent for preventing and treating mental disorders such as schizophrenia.

### [Solution to Problem]

The present inventors found that compounds represented by formula (I) or salts thereof (herein also referred to as compounds (I)) have an agonistic effect on GPR52, and the present invention was perfected upon further investigation.

Specifically, the present invention is intended to provide the following [1] to [16] and the like.
[1] a compound represented by formula (I): 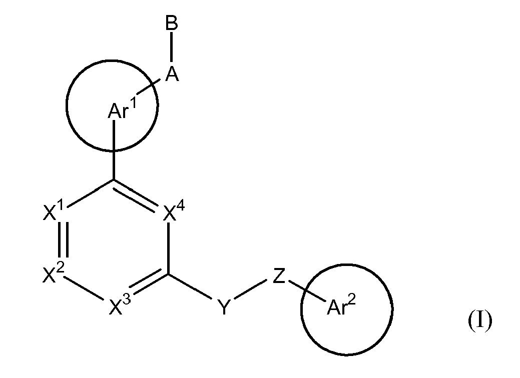 wherein
   A represents -(CH₂)n-CO-NR^{a}- (n is an integer of 0 to 3) or -NR^{a}-CO-,
   B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
   X¹, X², X³, and X⁴ represent the same or different -Ck^{x}=, or -N=,
   Y represents -O-, -S-, -S(O)-, -S(O)₂-, or -NR^{y}-,
   Z represents a bond, methylene, or ethylene,
   Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
   Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
   R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, providing that 1-{2-[(3-chlorobenzyl)oxy]-6-methylpyridin-4-yl}-2-methyl-1H-imidazole-4-carboxamide, tert-butyl {5-[6-(3-chlorophenoxy)pyrazin-2-yl]pyridin-3-yl}carbamate, 5-{6-[3-(trifluoromethyl)phenoxy]pyridin-2-yl}-1,3,4-oxadiazole-2-carboxamide, and N-hydroxy-5-{6-[methyl(2-phenylethyl)amino]pyridin-2-yl}thiophene-2-carboxamide are excluded,
   or a salt thereof.
[2] the compound according to [1] above, wherein A is -CO-NH- or - NH-CO-;
[3] the compound according to [1], wherein
   B is a
      (1) hydrogen atom,
      (2) C₁₋₆ alkyl group which may be substituted with one or more substituents selected from
         (a) halogen atoms,
         (b) hydroxy group,
         (c) cyano group,
         (d) amino group which may be mono- or di-substituted with substituents selected from optionally hydroxy-substituted C₁₋₆ alkyl groups, C₆₋₁₀ aryl groups, C₁₋₆ alkoxy-carbonyl groups, C₁₋₆ alkyl-carbonyl groups, and carbamoyl groups,
         (e) C₆₋₁₀ aryl groups which may be substituted with 1 to 3 substituents selected from halogen atoms, hydroxy group, and amino group,
         (f) C₁₋₆ alkylsulfanyl groups,
         (g) C₁₋₆ alkylsulfinyl groups,
         (h) C₁₋₆ alkylsulfonyl groups,
         (i) C₁₋₆ alkoxy groups,
         (j) C₆₋₁₀ aryloxy groups,
         (k) C₇₋₁₃ aralkyloxy groups,
         (l) five- to ten-membered heterocyclic groups which may be substituted with one or more substituents selected from C₁₋₆ alkyl groups, C₁₋₆ alkyl-carbonyl groups, and oxo group, and
         (m) carbamoyl,
      (3) C₂₋₆ alkenyl group which may be substituted with a five- to six-membered heterocyclic group,
      (4) C₃₋₁₀ cycloalkyl group,
      (5) C₆₋₁₀ aryl group which may be substituted with one or more substituents selected from
         (a) hydroxy group,
         (b) cyano group,
         (c) C₁₋₆ alkoxy groups,
         (d) mono- or di-C₁₋₆ alkyl-amino groups, and
         (e) C₁₋₆ alkyl-carbonylamino groups,
      (6) amino group which may be mono- or di-substituted with substituents selected from
         (a) C₁₋₆ alkyl groups which may be substituted with mono- or di-C₁₋₆ alkylamino groups, and
         (b) five- to ten-membered heterocyclic groups which may be substituted with one or more substituents selected from halogen atoms and oxo group, or
      (7) five- to ten-membered heterocyclic group which may be substituted with one or more substituents selected from
         (a) halogen atoms,
         (b) C₁₋₆ alkyl groups which may be substituted with one or more substituents selected from hydroxy group and mono- or di-C₁₋₆ alkylamino groups,
         (c) C₁₋₆ alkoxy groups,
         (d) C₁₋₆ alkyl-carbonyl groups,
         (e) C₁₋₆ alkoxy-carbonyl groups, and
         (f) carbamoyl groups;
[4] the compound according to [1] above, wherein one or two of X¹, X², X³, and X⁴ are -N=;
[5] the compound according to [1] above, wherein Y is -O-;
[6] the compound according to [1] above, wherein Ar¹ is a benzene ring or indole ring which may be substituted with one or more substituents selected from halogen atoms and optionally halogenated C₁₋₆ alkyl groups;
[7] the compound according to [1] above, wherein R^{x} is a hydrogen atom or optionally halogenated C₁₋₆ alkyl group;
[8] the compound according to [1] above, wherein
   A is -CO-NH- or -NH-CO-,
   B is a
      (1) hydrogen atom,
      (2) C₁₋₆ alkyl group which may be substituted with one or more substituents selected from
         (a) halogen atoms,
         (b) hydroxy group,
         (c) cyano group,
         (d) amino group which may be mono- or di-substituted with substituents selected from optionally hydroxy-substituted C₁₋₆ alkyl groups, C₆₋₁₀ aryl groups, C₁₋₆ alkoxy-carbonyl groups, C₁₋₆ alkyl-carbonyl groups, and carbamoyl groups,
         (e) C₆₋₁₀ aryl groups which may be substituted with 1 to 3 substituents selected from halogen atoms, hydroxy group, and amino group,
         (f) C₁₋₆ alkylsulfanyl groups,
         (g) C₁₋₆ alkylsulfinyl groups,
         (h) C₁₋₆ alkylsulfonyl groups,
         (i) C₁₋₆ alkoxy groups,
         (j) C₆₋₁₀ aryloxy groups,
         (k) C₇₋₁₃ aralkyloxy groups,
         (l) five- to ten-membered heterocyclic groups which may be substituted with one or more substituents selected from C₁₋₆ alkyl groups, C₁₋₆ alkyl-carbonyl groups, and oxo group, and
         (m) carbamoyl,
      (3) C₂₋₆ alkenyl group which may be substituted with a five- to six-membered heterocyclic group(s),
      (4) C₃₋₁₀ cycloalkyl group,
      (5) C₆₋₁₀ aryl group which may be substituted with one or more substituents selected from
         (a) hydroxy group,
         (b) cyano group,
         (c) C₁₋₆ alkoxy groups,
         (d) mono- or di-C₁₋₆ alkyl-amino groups, and
         (e) C₁₋₆ alkyl-carbonylamino groups,
      (6) amino group which may be mono- or di-substituted with substituents selected from
         (a) C₁₋₆ alkyl groups which may be substituted with mono- or di-C₁₋₆ alkylamino groups, and
         (b) five- to ten-membered heterocyclic groups which may be substituted with one or more substituents selected from halogen atoms and oxo group, or
      (7) five- to ten-membered heterocyclic group which may be substituted with one or more substituents selected from
         (a) halogen atoms,
         (b) C₁₋₆ alkyl groups which may be substituted with one or more substituents selected from hydroxy group and mono- or di-C₁₋₆ alkylamino groups,
         (c) C₁₋₆ alkoxy groups,
         (d) C₁₋₆ alkyl-carbonyl groups,
         (e) C₁₋₆ alkoxy-carbonyl groups, and
         (f) carbamoyl groups;
   X¹, X², X³, and X⁴ are the same or different -CR^{x}=, or -N=, and one or two of X¹, X², X³, and X⁴ are -N=,
   Y is -O-,
   Ar¹ is a benzene ring or indole ring which may be substituted with one or more substituents selected from halogen atoms and optionally halogenated C₁₋₆ alkyl groups, Ar² is a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
   R^{x} is a hydrogen atom or optionally halogenated C₁₋₆ alkyl group.
[9] a compound according to [1] above, selected from:
   4-amino-N-(3-(2-((2-(3,4-dimethoxyphenyl)ethyl)amino)pyrimidin-4-yl)phenyl)butanamide hydrochloride,
   N-(3-(2-((2-(3,4-dimethoxyphenyl)ethyl)amino)pyrimidin-4-yl)phenyl)-N³,N³-dimethyl-β-alaninamide hydrochloride,
   3-(6-(2-(3,4-dimethoxyphenyl)ethoxy)pyridin-2-yl)-N-(2-pyrrolidin-1-ylethyl)benzamide,
   N-(2-hydroxyethyl)-3-(6-(2-(3-(trifluoromethyl)phenyl)ethoxy)pyridin-2-yl)benzamide,
   N-(2-hydroxyethyl)-3-(6-(2-(3-methoxyphenyl)ethoxy)pyridin-2-yl)benzamide,
   3-(6-(3,5-dichlorophenoxy)pyridin-2-yl)-N-(2-hydroxyethyl)benzamide,
   N-(2-aminoethyl)-3-(6-(2-(3-methoxyphenyl)ethoxy)pyridin-2-yl)benzamide hydrochloride,
   3-(6-(2,4-dichlorophenoxy)pyridin-2-yl)-N-(2-pyrrolidin-1-ylethyl)benzamide,
   3-(6-((2,4-dichlorophenyl)thio)pyridin-2-yl)-N-(2-hydroxyethyl)benzamide,
   N-(2-pyrrolidin-1-ylethyl)-3-[2-({2-[3-(trifluoromethyl)phenyl]ethyl}amino)pyrimidin-4-yl]benzamide,
   N-cyclopropyl-3-{6-[2-(3,4-dimethoxyphenyl)ethoxy]pyridin-2-yl}benzamide,
   N-(3-(6-(2-(3,4-dimethoxyphenyl)ethoxy)pyridin-2-yl)phenyl)acetamide,
   3-(6-(2-(3,4-dimethoxyphenyl)ethoxy)pyridin-2-yl)-N-(pyridin-3-ylmethyl)benzamide,
   3-(6-(3,5-dichlorophenoxy)pyridin-2-yl)-N-(2-(methylsulfinyl)ethyl)benzamide, and
   3-(6-((2,4-dichlorophenyl)amino)pyridin-2-yl)-N-(2-hydroxyethyl)benzamide;
[10] a prodrug of the compound according [1] above;
[11] a pharmaceutical agent comprising the compound according to [1] above or the prodrug according to [10] above;
[12] a GPR52 activating agent comprising a compound represented by formula (I): 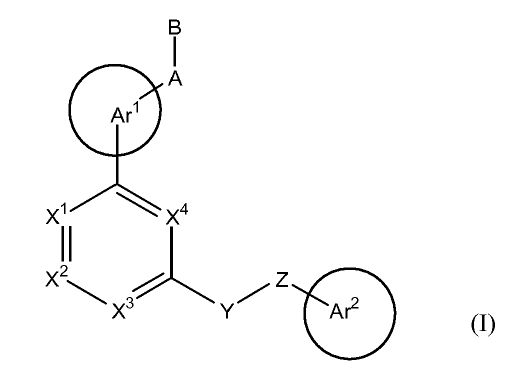 wherein
   A represents -(CH₂)n-CO-NR^{a}- (n is an integer of 0 to 3) or -NR^{a}-CO-,
   B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
   X¹, X², X³, and X⁴ represent the same or different -CR^{x}=, or -N=,
   Y represents -O-, -S-, -S(O)-, -S(O)₂-, or -NR^{y}-,
   Z represents a bond, methylene, or ethylene,
   Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
   Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
   R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, , or a salt or prodrug thereof;
[13] the GPR52 activating agent according to [12] above, which is an agent for preventing or treating schizophrenia.
[14] a method for preventing or treating diseases involving GPR52 activity in a mammal, comprising administering a compound represented by formula (I): 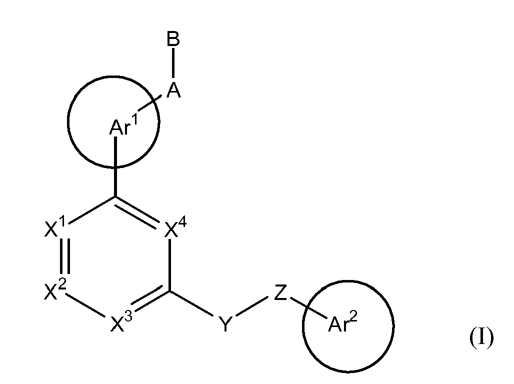 wherein
   A represents -(CH₂)ₙ-CO-NR^{a}- (n is an integer of 0 to 3) or -NR^{a}-CO-,
   B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
   X¹, X², X³, and X⁴ represent the same or different -Ck^{x}=, or -N=,
   Y represents -O-, -S-, -S(O)-, -S(O)₂-, or -NR^{y}-,
   Z represents a bond, methylene, or ethylene,
   Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
   Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
   R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, or a salt or prodrug thereof, to the mammal;
[15] the method according to [14] above, wherein the disease involving GPR52 activity is schizophrenia;
[16] the use of a compound represented by formula (I): 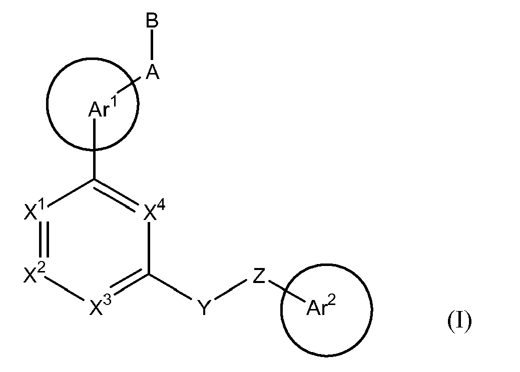 wherein
   A represents -(CH₂)n-CO-NR^{a}- (n is an integer of 0 to 3) or -NR^{a}-CO-,
   B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
   X¹, X², X³, and X⁴ represent the same or different -Ck^{x}=, or -N=,
   Y represents -O-, -S-, -S(O)-, -S(O)₂-, or -NR^{y}-,
   Z represents a bond, methylene, or ethylene,
   Ar¹ represents a five- to ten-membered aromatic ring (except for thiazole) which may be substituted with one or more substituents selected from halogen atoms, optionally halogenated C₁₋₆ alkyl groups, and optionally halogenated C₁-₆ alkoxy groups,
   Ar² represents a five- to six-membered aromatic ring which may be substituted with one or more substituents selected from halogen atoms, optionally halogenated C₁-₆ alkyl groups, and optionally halogenated C₁₋₆ alkoxy groups, and which may be condensed with an optionally substituted five- to six-membered ring, and
   R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, or a salt or prodrug thereof, for the manufacture of a GPR52 activating agent; and
[17] the use according to [16] above, wherein the GPR52 activating agent is as an agent for preventing or treating schizophrenia.

### [Advantageous Effects of Invention]

The compound of the present invention has an agonistic effect on GPR52 and is useful as a pharmaceutical agent for preventing and treating mental disorders such as schizophrenia.

### [Description of Embodiments]

The present invention will be described in detail below.

Unless otherwise noted, examples of the "halogen atoms" used herein include fluorine, chlorine, bromine, and iodine.
Unless otherwise noted, examples of the "C₁₋₆ alkyl groups" and "C₁₋₆ alkyl" in substituents used herein include methyl, ethyl, propyl, isopropyl, butyl, isobutyl, sec-butyl, tert-butyl, pentyl, isopentyl, neopentyl, 1-ethylpropyl, and hexyl. Of these, "C₁₋₄ alkyl (groups)" are preferred. Unless otherwise noted, examples of the "optionally halogenated C₁₋₆ alkyl groups" used herein include C₁₋₆ alkyl groups which may be substituted with one or more (preferably one to three) halogen atoms selected from fluorine, chlorine, bromine, and iodine atoms.
Unless otherwise noted, examples of the "C₁₋₆ alkoxy groups" and "C₁₋₆ alkoxy" in substituents used herein include methoxy, ethoxy, propoxy, isopropoxy, butoxy, isobutoxy, sec-butoxy, tert-butoxy, pentyloxy, and hexyloxy. Of these, "C₁₋₄ alkoxy (groups)" are preferred. Examples of the "optionally halogenated C₁₋₆ alkoxy groups" used herein include C₁₋₆ alkoxy groups which may be substituted with one or more (preferably one to three) halogen atoms selected from fluorine, chlorine, bromine, and iodine atoms.

Unless otherwise specified, examples of the "C₁₋₁₄ hydrocarbon groups" in "optionally substituted C₁₋₁₄ hydrocarbon groups" include C₁₋₁₀ alkyl groups, C₂₋₁₀ alkenyl groups, C₂₋₁₀ alkynyl groups, C₃₋₁₀ cycloalkyl groups, C₃₋₁₀ cycloalkenyl groups, C₄₋₁₀ cycloalkadienyl groups, C₆₋₁₄ aryl groups, C₇₋₁₃ aralkyl groups, C₈₋₁₃ arylalkenyl groups, and C₃₋₁₀ cycloalkyl-C₁₋₆ alkyl groups.

Here, examples of C₁₋₁₀ alkyl groups include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, and decyl.

Unless otherwise noted, examples of C₂₋₁₀ alkenyl groups used herein include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, and 1-octenyl. Unless otherwise noted, the C₂₋₆ alkenyl groups used herein include the ones above that have a carbon number of 2 to 6.

Unless otherwise noted, the C₂₋₁₀ alkynyl groups and "C₂₋₁₀ alkynyl" in substituents used herein include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, and 1-octynyl.

Unless otherwise noted, examples of the C₃₋₁₀ cycloalkyl groups and "C₃₋₁₀ cycloalkyl" in substituents used herein include cyclopropyl, cyclobutyl, cyclopentyl, cyclohexyl, cycloheptyl, and cyclooctyl.

Unless otherwise noted, examples of the C₃₋₁₀ cycloalkenyl groups used herein include 2-cyclopenten-1-yl, 3-cyclopenten-1-yl, 2-cyclohexen-1-yl, and 3-cyclohexen-1-yl.

Unless otherwise noted, examples of the C₄₋₁₀ cycloalkadienyl groups used herein include 2,4-cyclopentadien-1-yl, 2,4-cyclohexadien-1-yl, and 2,5-cyclohexadien-1-yl.

The above C₃₋₁₀ cycloalkyl groups, C₃₋₁₀ cycloalkenyl groups, and C₄₋₁₀ cycloalkadienyl groups may each be condensed with a benzene ring, wherein examples of such condensed cyclic groups include indanyl, dihydronaphthyl, tetrahydronaphthyl, and fluorenyl. Examples of the above hydrocarbon groups also include crosslinked hydrocarbons such as bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantly, and norbornanyl.

Unless otherwise noted, examples of the C₆₋₁₄ aryl groups used herein include phenyl, naphthyl, anthryl, phenanthryl, acenaphthylenyl, and biphenylyl. Examples of C₆₋₁₀ aryl groups and "C₆₋₁₀ aryl" in substituents used herein include the ones above that have a carbon number of 6 to 10 (such as phenyl and naphthyl).

Unless otherwise noted, examples of C₇₋₂₀ aralkyl groups and "C₇₋₂₀ aralkyl" in substituents used herein include C₁₋₆ alkyl groups substituted with one to three C₆₋₁₀ aryl groups, more specific examples of which include benzyl, phenethyl, naphthylmethyl, biphenylylmethyl, and trityl. Examples of C₇₋₁₃ aralkyl groups and "C₇₋₁₃ aralkyl" in substituents used herein include the ones above that have a carbon number of 7 to 13.
Examples of C₇₋₁₃ aralkyloxy groups include C₁₋₆ alkoxy groups substituted with one to three C₆₋₁₀ aryl groups.

Examples of C₈₋₁₃ arylalkenyl groups include styryl or the like.
Examples of C₃₋₁₀ cycloalkyl-C₁₋₆ alkyl groups include cyclohexylmethyl or the like.

The C₁₋₁₀ alkyl groups, C₂₋₁₀ alkenyl groups, and C₂₋₁₀ alkynyl groups given above as examples of"C₁₋₁₄ hydrocarbon groups" may have one or more (preferably one to three) substituents in substitutable positions.
Examples of such substituents include:
(1) C₃₋₁₀ cycloalkyl groups (such as cyclopropyl and cyclohexyl);
(2) C₆₋₁₄ aryl groups (such as phenyl and naphthyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups,
   (d) halogen atoms, and
   (e) amino group;
(3) aromatic heterocyclic groups (such as thienyl, furyl, pyridyl, imidazolyl, pyrazolyl, oxazolyl, thiazolyl, tetrazolyl, oxadiazolyl, pyrazinyl, quinolyl, indolyl, and benzoisoxazolyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups, and
   (d) halogen atoms;
(4) non-aromatic heterocyclic groups (such as tetrahydrofuryl, morpholinyl, piperidyl, pyrrolidinyl, piperazinyl, tetrahydropyranyl, and tetrahydroquinolinyl) which may be substituted with one or more (preferably one to three) substituents selected from
   (a) C₁₋₆ alkyl groups (such as methyl and isopropyl) which may be substituted with one or more (preferably one to three) halogen atoms,
   (b) hydroxy group,
   (c) C₁₋₆ alkoxy groups,
   (d) oxo group,
   (e) halogen atoms, and
   (f) C₇₋₂₀ aralkylthio groups (such as benzylthio and tritylthio);
(5) amino group which may be mono- or di-substituted with substituents selected from
   (a) optionally hydroxyl-substituted C₁₋₆ alkyl groups (such as methyl and hydroxymethyl),
   (b) C₆₋₁₀ aryl groups (such as phenyl),
   (c) C₁₋₆ alkyl-carbonyl groups (such as acetyl),
   (d) C₁₋₆ alkoxy-carbonyl groups (such as tert-butoxycarbonyl),
   (e) C₆₋₁₄ aryl-carbonyl groups (such as benzoyl),
   (f) C₇₋₁₃ aralkyl-carbonyl groups (such as benzylcarbonyl and phenethylcarbonyl),
   (g) carbamoyl groups (such as carbamoyl, methylcarbamoyl, benzylcarbamoyl, and dimethylcarbamoyl) which may be mono- or di-substituted with substituents selected from C₁₋₆ alkyl groups, C₆₋₁₄ aryl groups, and C₇₋₁₃ aralkyl groups;
   (h) C₁₋₆ alkylsulfonyl groups (such as methylsulfonyl, ethylsulfonyl, and isopropylsulfonyl),
   (i) C₆₋₁₄ arylsulfonyl groups (such as benzenesulfonyl, toluenesulfonyl, 1-naphthalenesulfonyl, and 2-naphthalenesulfonyl), and
   (j) C₇₋₁₃ aralkylsulfonyl groups such as (benzylsulfonyl);
(6) amidino group;
(7) C₁₋₆ alkyl-carbonyl groups which may be substituted with one or more (preferably one to three) halogen atoms;
(8) C₁₋₆ alkoxy-carbonyl groups which may be substituted with one or more (preferably one to three) halogen atoms;
(9) aromatic heterocyclic-carbonyl groups (such as thienylcarbonyl and indolylcarbonyl) which may be substituted with one or more (preferably one to three) amino groups (the amino groups may be mono- or di-substituted with substituents selected from C₁₋₆ alkyl groups and aromatic heterocyclic-sulfonyl groups (such as thienylsulfonyl));

(10) non-aromatic heterocyclic-carbonyl groups (such as morpholinylcarbonyl);
(11) C₁₋₆ alkylsulfonyl groups (such as methylsulfonyl) which may be substituted with one or more (preferably one to three) halogen atoms;
(12) carbamoyl group which may be mono- or di-substituted with substituents selected from
   (a) C₁₋₆ alkyl groups which may be substituted with one or more (preferably one to three) halogen atoms,
   (b) C₆₋₁₄ aryl groups (such as phenyl),
   (c) C₇₋₁₃ aralkyl groups (such as benzyl), and
   (d) aromatic heterocyclic-C₁₋₆ alkyl groups (such as furfuryl);
(13) thiocarbamoyl group which may be mono- or di-substituted with C₁₋₆ alkyl groups optionally substituted with one or more (preferably one to three) halogen atoms;
(14) sulfamoyl groups which may be mono- or di-substituted with C₁₋₆ alkyl groups optionally substituted with one or more (preferably one to three) halogen atoms;
(15) carboxy groups;
(16) hydroxy group;
(17) C₁₋₆ alkoxy groups (such as methoxy) which may be substituted with one or more (preferably one to three) substituents selected from
   (a) halogen atoms,
   (b) carboxy groups,
   (c) C₁₋₆ alkoxy groups, and
   (d) C₁₋₆ alkoxy-carbonyl groups;
(18) C₂₋₆ alkenyloxy groups (such as ethenyloxy) which may be substituted with one or more (preferably one to three) halogen atoms;
(19) C₃₋₁₀ cycloalkyloxy groups (such as cyclohexyloxy);
(20) C₇₋₁₃ aralkyloxy groups (such as benzyloxy) which may be substituted with one or more (preferably one to three) halogen atoms;

(21) C₆₋₁₄ aryloxy groups (such as phenyloxy and naphthyloxy);
(22) C₁₋₆ alkyl-carbonyloxy groups (such as acetyloxy and tert-butylcarbonyloxy);
(23) mercapto groups;
(24) C₁₋₆ alkylthio groups (such as methylthio and ethylthio) which may be substituted with one to three substituents selected from halogen atoms and C₆₋₁₄ aryl groups;
(25) C₆₋₁₄ arylthio groups (such as phenylthio and naphthylthio);
(26) aromatic heterocyclic thio groups (such as tetrazolylthio) which may be substituted with one or more (preferably one to three) C₁₋₆ alkyl groups;
(27) sulfo groups;
(28) cyano group;
(29) azide groups;
(30) nitro groups;
(31) nitroso groups;
(32) halogen atoms;
(33) C₁₋₆ alkylsulfinyl groups (such as methylsulfinyl);
(34) oxo group;
(35) C₃₋₁₀ cycloalkyl-C₁₋₆ alkyloxy groups (such as cyclopropylmethyloxy);
(36) C₁₋₃ alkylenedioxy groups;
(37) aromatic heterocyclic-carbonylthio groups (such as indolylcarbonylthio) which may be substituted with one or more (preferably one to three) amino group (the amino group may be mono- or di-substituted with substituents selected from C₁₋₆ alkyl groups and aromatic heterocyclic-sulfonyl groups (such as thienylsulfonyl)); and
(38) formyl groups.

The C₃₋₁₀ cycloalkyl groups, C₃₋₁₀ cycloalkenyl groups, C₄₋₁₀ cycloalkadienyl groups, C₆₋₁₄ aryl groups, C₇₋₁₃ aralkyl groups, C₈₋₁₃ arylalkenyl groups, and C₃₋₁₀ cycloalkyl-C₁₋₆ alkyl groups given above as examples of "hydrocarbon groups" may have one or more (preferably one to three) substituents in substitutable positions.
Examples of such substituents include:
(1) C₃₋₁₀ cycloalkyl groups (such as cyclopropyl and cyclohexyl);
(2) C₆₋₁₄ aryl groups (such as phenyl and naphthyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups, and
   (d) halogen atoms;
(3) aromatic heterocyclic groups (such as thienyl, furyl, pyridyl, pyrimidinyl, pyridazinyl, oxazolyl, thiazolyl, tetrazolyl, oxadiazolyl, pyrazinyl, quinolyl, and indolyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups, and
   (d) halogen atoms;
(4) non-aromatic heterocyclic groups (such as tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl, and thiazolinyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups,
   (d) C₁₋₆ alkyl-carbonyl groups,
   (e) C₁₋₆ alkyl-sulfonyl groups,
   (f) oxo group, and
   (g) halogen atoms;
(5) amino group which may be mono- or di-substituted with substituents selected from
   (a) C₁₋₆ alkyl groups,
   (b) C₁₋₆ alkyl-carbonyl groups,
   (c) C₁₋₆ alkoxy-carbonyl groups,
   (d) C₆₋₁₄ aryl-carbonyl groups (such as benzoyl),
   (e) C₇₋₁₃ aralkyl-carbonyl groups (such as benzylcarbonyl and phenethylcarbonyl),
   (f) carbamoyl groups (such as carbamoyl, methylcarbamoyl, benzylcarbamoyl, and dimethylcarbamoyl) which may be mono- or di-substituted with substituents selected from C₁₋₆ alkyl groups, C₆₋₁₄ aryl groups, and C₇₋₁₃ aralkyl groups;
   (g) C₁₋₆ alkylsulfonyl groups (such as methylsulfonyl, ethylsulfonyl, and isopropylsulfonyl),
   (h) C₆₋₁₄ arylsulfonyl groups (such as benzenesulfonyl, toluenesulfonyl, 1-naphthalenesulfonyl, and 2-naphthalenesulfonyl), and
   (i) C₇₋₁₃ aralkylsulfonyl groups (such as benzylsulfonyl);
(6) amidino group;

(7) C₁₋₆ alkyl-carbonyl groups which may be substituted with one or more (preferably one to three) halogen atoms;
(8) C₁₋₆ alkoxy-carbonyl groups which may be substituted with one or more (preferably one to three) halogen atoms;
(9) aromatic heterocyclic-carbonyl groups (such as thienylcarbonyl and indolylcarbonyl) which may be substituted with one or more (preferably one to three) amino groups (the amino groups may be mono- or di-substituted with substituents selected from C₁₋₆ alkyl groups and aromatic heterocyclic-sulfonyl groups (such as thienylsulfonyl));
(10) non-aromatic heterocyclic-carbonyl groups (such as morpholinylcarbonyl and pyrrolidinocarbonyl);
(11) C₁₋₆ alkylsulfonyl groups (such as methylsulfonyl and ethylsulfonyl) which may be substituted with one or more (preferably one to three) halogen atoms;
(12) carbamoyl groups which may be mono- or di-substituted with substituents selected from
   (a) C₁₋₆ alkyl groups which may be substituted with one or more (preferably one to three) halogen atoms,
   (b) C₆₋₁₄ aryl groups (such as phenyl),
   (c) C₇₋₁₃ aralkyl groups (such as benzyl), and
   (d) aromatic heterocyclic-C₁₋₆ alkyl groups (such as furfuryl);
(13) thiocarbamoyl groups which may be mono- or di-substituted with C₁₋₆ alkyl groups optionally substituted with one or more (preferably one to three) halogen atoms;
(14) sulfamoyl groups which may be mono- or di-substituted with C₁₋₆ alkyl groups optionally substituted with one or more (preferably one to three) halogen atoms;
(15) carboxy groups;
(16) hydroxy group;
(17) C₁₋₆ alkoxy groups which may be substituted with one or more (preferably one to three) substituents selected from
   (a) halogen atoms,
   (b) carboxy groups,
   (c) C₁₋₆ alkoxy groups, and
   (d) C₁₋₆ alkoxy-carbonyl groups;
(18) C₂₋₆ alkenyloxy groups (such as ethenyloxy) which may be substituted with one or more (preferably one to three) halogen atoms;
(19) C₃₋₁₀ cycloalkyloxy groups (such as cyclohexyloxy);
(20) C₇₋₁₃ aralkyloxy groups (such as benzyloxy) which may be substituted with one or more (preferably one to three) halogen atoms;
(21) C₆₋₁₄ aryloxy groups (such as phenyloxy and naphthyloxy);
(22) C₁₋₆ alkyl-carbonyloxy groups (such as acetyloxy and tert-butylcarbonyloxy);

(23) mercapto groups;
(24) C₁₋₆ alkylthio groups (such as methylthio and ethylthio) which may be substituted with one or more (preferably one to three) substituents selected from
   (a) halogen atoms, and
   (b) C₆₋₁₄ aryl groups;
(25) C₆₋₁₄ arylthio groups (such as phenylthio and naphthylthio);
(26) aromatic heterocyclic thio groups (such as tetrazolylthio) which may be substituted with one or more (preferably one to three) C₁₋₆ alkyl groups;
(27) sulfo groups;
(28) cyano group;
(29) azide groups;
(30) nitro groups;
(31) nitroso groups;
(32) halogen atoms;
(33) C₁₋₆ alkylsulfinyl groups (such as methylsulfinyl);
(34) oxo group;
(35) C₃₋₁₀ cycloalkyl-C₁₋₆ alkyloxy groups (such as cyclopropylmethyloxy);
(36) C₁₋₃ alkylenedioxy groups;
(37) aromatic heterocyclic-carbonylthio groups (such as indolylcarbonylthio) which may be substituted with one or more (preferably one to three) amino group [the amino group may be mono- or di-substituted with substituents selected from C₁₋₆ alkyl groups and aromatic heterocyclic-sulfonyl groups (such as thienylsulfonyl)];
(38) formyl groups;
(39) aromatic heterocyclic-oxo groups (such as pyrimidyloxy and pyrazinyloxy);

(40) C₁₋₆ alkyl groups which may be substituted with one or more (preferably one to three) substituents selected from
   (a) halogen atoms,
   (b) carboxy groups,
   (c) hydroxy group,
   (d) C₁₋₆ alkoxy groups which may be substituted with one or more (preferably one to three) substituents selected from carboxy groups and C₁-₆ alkoxy-carbonyl groups,
   (e) C₁₋₆ alkoxy-carbonyl groups,
   (f) C₁₋₆ alkyl-carbonyloxy groups (such as acetyloxy and tert-butylcarbonyloxy),
   (g) carbamoyl groups which may be mono- or di-substituted with substituents selected from C₁₋₆ alkyl, C₁₋₆ alkylsulfonyl groups, and amino group,
   (h) aromatic heterocyclic groups (such as thienyl, tetrazolyl, and imidazolyl) which may be substituted with one or more (preferably one to three) C₁₋₆ alkyl groups,
   (i) non-aromatic heterocyclic groups (such as tetrahydrofuranyl, piperidino, piperazinyl, morpholinyl, dihydrooxadiazolyl, and hexahydropyrazinooxazinyl (such as hexahydropyrazino[2,1-c][1,4]oxazinyl)) which may be substituted with one to three substituents selected from C₁₋₆ alkyl-carbonyl groups and oxo group,
   (j) amino group which may be mono- or di-substituted with C₁₋₆ alkyl groups (the C₁₋₆ alkyl groups may be substituted with one or more (preferably one to three) substituents selected from non-aromatic heterocyclic groups (such as morpholinyl), C₁₋₆ alkoxy groups, and C₁₋₆ alkylsulfonyl groups),
   (k) C₁₋₆ alkylsulfonyl groups which may be substituted with one or more (preferably one to three) carboxy groups,
   (l) C₁₋₆ alkylthio groups which may be substituted with one or more (preferably one to three) substituents selected from carboxy groups, C₁₋₆ alkoxy-carbonyl groups, hydroxy group, and carbamoyl groups,
   (m) phosphono groups which may be mono- or di-substituted with C₁₋₆ alkyl groups,
   (n) non-aromatic heterocyclic-carbonyl groups (such as morpholinylcarbonyl),
   (o) cyano group, and
   (p) C₆₋₁₄ aryloxy groups which may be substituted with one to three substituents selected from carboxy groups and C₁₋₆ alkoxy-carbonyl groups;
(41) C₂₋₆ alkenyl groups (such as ethenyl and 1-propenyl) which may be substituted with one to three substituents selected from
   (a) halogen atoms,
   (b) carboxy groups,
   (c) C₁₋₆ alkoxy-carbonyl groups, and
   (d) carbamoyl groups; and
(42) C₇₋₁₃ aralkyl groups (such as benzyl) which may be substituted with one or more (preferably one to three) substituents selected from
   (a) C₁₋₆ alkyl groups which may be substituted with one or more (preferably one to three) halogen atoms,
   (b) hydroxy group,
   (c) C₁₋₆ alkoxy groups, and
   (d) halogen atoms.

Unless otherwise noted, examples of the "heterocyclic groups" (and "heterocyclic-"in substituents) of the "optionally substituted heterocyclic groups" used herein include five- to twelve-membered (preferably five- to eight-membered) aromatic heterocyclic groups (heteroaryl groups) or saturated or unsaturated non-aromatic heterocyclic groups (aliphatic heterocyclic groups) having one or more (preferably one to four, and even more preferably one or two) hetero atoms selected from oxygen atom, optionally oxidized sulfur atoms, and nitrogen atom or the like (preferably oxygen atom, sulfur atom, and nitrogen atom or the like) as the atoms (ring atoms) forming the ring system.
Unless otherwise noted, examples of the "five- to ten-membered heterocyclic groups" or "five-to ten-membered heterocyclic-" in substituents used herein include such "heterocyclic groups" that have five to ten members.
Unless otherwise noted, examples of the "five- to six-membered heterocycles" or "five-to six-membered heterocyclic-" in substituents used herein include such "heterocyclic groups" that have five to six members.

Unless otherwise noted, examples of the "aromatic heterocyclic groups" (and "aromatic heterocyclic-" in substituents) used herein are five- to twelve-membered (preferably five- to eight-membered) and have one or more (preferably one to four, and more preferably one or two) hetero atoms selected from oxygen atom, optionally oxidized sulfur atoms, and nitrogen atom or the like (preferably oxygen atom, sulfur atom, and nitrogen atom or the like) as the atoms (ring atoms) forming the ring system. Examples thereof include monocyclic aromatic heterocyclic groups (examples include five- or six-membered monocyclic aromatic heterocyclic groups such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl) and fused aromatic heterocyclic groups (examples include eight- to twelve-membered fused aromatic heterocyclic groups such as benzofuranyl, isobenzofuranyl, benzothienyl, indolyl, isoindolyl, 1H-indazolyl, benzindazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cinnolinyl, quinazolinyl, quinoxazolinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl and 1,2,4-triazolo[4,3-b]pyridazinyl. Preferred examples of fused aromatic heterocyclic groups include heterocycles in which a five- or six-membered monocyclic aromatic heterocyclic group noted above is condensed with a benzene ring, or heterocycles in which two of the same or different five- or six-membered monocyclic aromatic heterocyclic groups noted above are condensed.

Unless otherwise noted, examples of the "non-aromatic heterocyclic groups (aliphatic heterocyclic groups)" (and "non-aromatic heterocyclic-" in substituents) used herein are five- to twelve-membered (preferably five- to eight-membered) and have one or more (preferably one to four, and even more preferably one or two) hetero atoms selected from oxygen atom, optionally oxidized sulfur atoms, and nitrogen atom or the like (preferably oxygen atom, sulfur atom, and nitrogen atom or the like) as the atoms (ring atoms) forming the ring system. Examples thereof include three- to eight-membered (preferably five- or six-membered) saturated or unsaturated (preferably saturated) non-aromatic heterocyclic groups (aliphatic heterocyclic groups) such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, and dihydro-1,2,4-oxadiazolyl.
Such "heterocyclic groups" and "five- or six-membered heterocyclic groups" may have one or more (preferably one to three) substituents in substitutable positions.
Examples of such substituents include the substituents given as examples for "C₃-10 cycloalkyl groups, C₃₋₁₀ cycloalkenyl groups, C₄₋₁₀ cycloalkadienyl groups, C₆₋₁₄ aryl groups, C₇₋₁₃ aralkyl groups, C₈₋₁₃ arylalkenyl groups, and C₃₋₁₀ cycloalkyl-C₁₋₆ alkyl groups" noted above.

Unless otherwise noted, examples of such "substituents" for "optionally substituted amino group" include the substituents given as examples for "C₃₋₁₀ cycloalkyl groups, C₃₋₁₀ cycloalkenyl groups, C₄₋₁₀ cycloalkadienyl groups, C₆₋₁₄ aryl groups, C₇₋₁₃ aralkyl groups, C₈₋₁₃ arylalkenyl groups, and C₃₋₁₀ cycloalkyl-C₁₋₆ alkyl groups" noted above. The "amino groups" may have one or two such substituents in substitutable positions (that is, may be mono- or di-substituted).

Unless otherwise noted, examples of the "acyl groups" used herein include optionally substituted hydrocarbon-carbonyl groups, optionally substituted heterocyclic-carbonyl groups, optionally substituted hydrocarbon-sulfonyl groups, and optionally substituted heterocyclic-sulfonyl groups.
Examples of the "optionally substituted hydrocarbon (groups)" in the "optionally substituted hydrocarbon-carbonyl groups" and "optionally substituted hydrocarbon-sulfonyl groups" include the groups given as examples of "optionally substituted C₁₋₁₄ hydrocarbon groups" above.

The symbols in formula (I) above will be described.

In formula (I), A represents -(CH₂)ₙ-CO-NR^{a}- (n is an integer of 0 to 3) or -NR^{a}-CO-.
Here, R^{a} represents a hydrogen atom, halogen atom, optionally halogenated C₁₋₆ alkyl group, or optionally halogenated C₁₋₆ alkoxy group.
A is preferably -CO-NH- or -NH-CO-.

In formula (I), B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group (preferably an optionally substituted five- or six-membered heterocyclic group), optionally substituted amino group, or acyl group.
Here, R^{b} represents a hydrogen atom, halogen atom, optionally halogenated C₁₋₆ alkyl group, or optionally halogenated C₁₋₆ alkoxy group.
B is preferably
   (1) a hydrogen atom,
   (2) a C₁₋₆ alkyl group (such as methyl, ethyl, n-propyl, or i-propyl) which may be substituted with one or more (preferably one to three) substituents selected from
      (a) halogen atoms (such as bromine and fluorine atoms),
      (b) hydroxy group,
      (c) cyano group,
      (d) amino group which may be mono- or di-substituted with one or more substituents (preferably one to three) selected from optionally hydroxy-substituted C₁₋₆ alkyl groups (such as methyl and hydroxymethyl), C₆₋₁₀ aryls (such as phenyl), C₁₋₆ alkyl-carbonyls (such as acetyl), C₁₋₆ alkoxy-carbonyls (such as tert-butoxycarbonyl), and carbamoyl groups,
      (e) C₆₋₁₀ aryl groups (such as phenyl) which may be substituted with one or more (preferably one to three) substituents selected from halogen atoms (such as chlorine atom), hydroxy group, and amino group,
      (f) C₁₋₆ alkylsulfanyl groups (such as methylsulfanyl),
      (g) C₁₋₆ alkylsulfinyl groups (such as methylsulfinyl),
      (h) C₁₋₆ alkylsulfonyl groups (such as methylsulfonyl),
      (i) C₁₋₆ alkoxy groups (such as methoxy, ethoxy, and isopropoxy),
      (j) C₆₋₁₀ aryloxy groups (such as phenoxy),
      (k) C₇₋₁₃ aralkyloxy groups (such as benzyloxy),
      (l) five- to ten-membered heterocyclic groups (such as pyrrolidinyl, pyrazolyl, imidazolyl, tetrazolyl, furyl, tetrahydrofuryl, thienyl, piperidyl, piperazinyl, tetrahydropyranyl, pyridyl, morpholinyl, pyrazinyl, tetrahydroquinolinyl, and benzoisoxazolyl) which may be substituted with one or more (preferably one to three) substituents selected from C₁₋₆ alkyl groups (such as methyl and isopropyl), C₁₋₆ alkyl-carbonyl groups (such as acetyl), and oxo group, and
      (m) carbamoyl groups,
   (3) a C₂₋₆ alkenyl group (such as vinyl) which may be substituted with a five- or six-membered heterocyclic group (such as imidazolyl, furyl, and pyridyl),
   (4) a C₃₋₁₀ cycloalkyl group (such as cyclopropyl),
   (5) a C₆₋₁₀ aryl group (such as phenyl) which may be substituted with one or more (preferably one to three) substituents selected from
      (a) hydroxy group,
      (b) cyano group,
      (c) C₁₋₆ alkoxy groups (such as methoxy),
      (d) mono- or di-C₁₋₆ alkyl-amino (such as dimethylamino), and
      (e) C₁₋₆ alkyl-carbonylamino (such as acetylamino),
   (6) an amino group which may be mono- or di-substituted with substituents selected from
      (a) C₁₋₆ alkyl groups (such as methyl and dimethylaminoethyl) which may be substituted with mono- or di-C₁₋₆ alkylamino groups, and
      (b) five- to ten-membered heterocyclic groups (such as pyrrolidinyl, thiazole, thiazolidinyl, tetrahydrofuryl, pyridyl, morpholinyl, quinolinyl) which may be substituted with one or more (preferably one to three) substituents selected from halogen atoms (such as fluorine atom) and oxo group, or
   (7) a five- to ten-membered heterocyclic groups (such as pyrazolyl, piperidyl, imidazolyl, furyl, tetrahydrofuryl, isoxazolyl, thiazolyl, isothiazolyl, thiadiazolyl, furazanyl, piperidyl, pyridyl, tetrahydropyranyl, pyridazinyl, tetrahydroindolyl, isoindolyl, indazolyl, dihydrobenzofuranyl, benzothiophenyl, tetrahydroquinolinyl, and tetrahydrobenzoisoxazolyl) which may be substituted with one or more (preferably one to three) substituents selected from
      (a) halogen atoms (such as fluorine atom),
      (b) C₁₋₆ alkyl groups (such as methyl and isopropyl) which may be substituted with one or more (preferably one to three) substituents selected from hydroxy group and mono- or di-C₁₋₆ alkylamino groups (such as dimethylamino),
      (c) C₁₋₆ alkoxy groups (such as methoxy),
      (d) C₁₋₆ alkyl-carbonyl groups (such as acetyl),
      (e) C₁₋₆ alkoxy-carbonyl groups (such as tert-butoxycarbonyl), and
      (f) carbamoyl.

B is also preferably:
(1) a hydrogen atom,
(2) a C₁₋₆ alkyl group (such as methyl, ethyl, n-propyl, or i-propyl) which may be substituted with one or more (preferably one to three) substituents selected from
   (a) halogen atoms (such as bromine and fluorine atoms),
   (b) hydroxy group,
   (c) cyano group,
   (d) amino group which may be mono- or di-substituted with substituents selected from optionally hydroxy-substituted C₁₋₆ alkyl groups (such as methyl and hydroxyethyl), C₆₋₁₀ aryls (such as phenyl), C₁₋₆ alkoxy-carbonyls (such as tert-butoxycarbonyl), and C₁₋₆ alkyl-carbonyls (such as acetyl), and carbamoyl groups,
   (e) C₆₋₁₀ aryl groups (such as phenyl) which may be substituted with one to three substituents selected from halogen atoms (such as chlorine atom), hydroxy group, and mono- or di-C₁₋₆ alkylamino groups,
   (f) C₁₋₆ alkylsulfanyl groups (such as methylsulfanyl),
   (g) C₁₋₆ alkylsulfinyl groups (such as methylsulfinyl),
   (h) C₁₋₆ alkoxy groups (such as methoxy, ethoxy, and isopropoxy),
   (i) C₆₋₁₀ aryloxy groups (such as phenoxy),
   (j) C₇₋₁₃ aralkyloxy groups (such as benzyloxy),
   (k) five- to ten-membered (preferably five- or six-membered) heterocyclic groups (such as pyrrolidinyl, pyrazolyl, imidazolyl, tetrazolyl, furyl, tetrahydrofuryl, thienyl, piperidyl, piperazinyl, tetrahydropyranyl, pyridyl, morpholinyl, pyrazinyl, tetrahydroquinolinyl, and benzoisoxazolyl) which may be substituted with one or more substituents selected from C₁₋₆ alkyl groups (such as methyl and isopropyl), C₁₋₆ alkyl-carbonyl groups (such as acetyl), and oxo group, and
   (l) carbamoyl,
(3) a C₂₋₆ alkenyl group (such as vinyl) which may be substituted with a five- or six-membered heterocyclic group (such as imidazolyl, furyl, and pyridyl),
(4) a C₃₋₁₀ cycloalkyl group (such as cyclopropyl) which may be substituted with a C₁₋₆ alkoxy group (such as methoxy),
(5) a C₆₋₁₀ aryl group (such as phenyl) which may be substituted with one or more (preferably one to three) substituents selected from
   (a) hydroxy group,
   (b) cyano group,
   (c) C₁₋₆ alkoxy groups (such as methoxy),
   (d) mono- or di-C₁₋₆ alkyl-amino (such as dimethylamino), and
   (e) C₁₋₆ alkyl-carbonylamino (such as acetylamino),
(6) an amino group which may be mono- or di-substituted with substituents selected from
   (a) C₁₋₆ alkyl groups (such as methyl and dimethylaminoethyl) which may be substituted with mono- or di-C₁₋₆ alkylamino groups, and
   (b) five- to ten-membered heterocyclic groups (such as pyrrolidinyl, thiazole, thiazolidinyl, tetrahydrofuryl, pyridyl, morpholinyl, quinolinyl) which may be substituted with one or more (preferably one to three) substituents selected from halogen atoms (such as fluorine atom) and oxo group, or
(7) a five- to ten-membered heterocyclic groups (such as pyrazolyl, piperidyl, imidazolyl, furyl, tetrahydrofuryl, isoxazolyl, thiazolyl, isothiazolyl, thiadiazolyl, furazanyl, piperidyl, pyridyl, tetrahydropyranyl, pyridazinyl, tetrahydroindolyl, isoindolyl, indazolyl, dihydrobenzofuranyl, benzothiophenyl, tetrahydroquinolinyl, and tetrahydrobenzoisoxazolyl) which may be substituted with one or more (preferably one to three) substituents selected from
   (a) halogen atoms (such as fluorine atom),
   (b) C₁₋₆ alkyl groups (such as methyl and isopropyl) which may be substituted with one or more (preferably one to three) substituents selected from hydroxy group and mono- or di-C₁₋₆ alkylamino groups (such as dimethylamino),
   (c) C₁₋₆ alkoxy groups (such as methoxy),
   (d) C₁₋₆ alkyl-carbonyl groups (such as acetyl),
   (e) C₁₋₆ alkoxy-carbonyl groups (such as tert-butoxycarbonyl),
   (f) carbamoyl,
   (g) five- or six-membered heterocyclic groups (such as morpholinyl), and
   (h) oxo.

In formula (I), X¹, X², X³, and X⁴ represent the same or different -CR^{x}= or -N=.
Here, R^{x} represents a hydrogen atom, halogen atom, optionally halogenated C₁₋₆ alkyl group, or optionally halogenated C₁₋₆ alkoxy group.
One or two of X¹, X², X³, and X⁴ are preferably -N=.
The moiety represented by the following in formula (I) 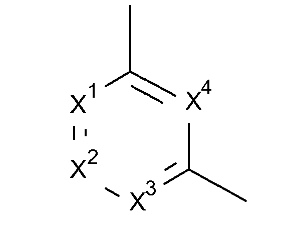 is

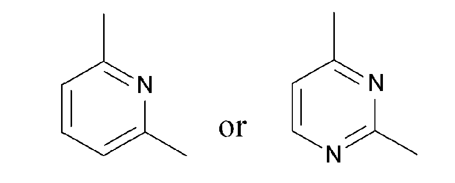

In formula (I), Y is -O-, -S-, -S(O)-, -S(O)₂-, or -NR^{y}-.
Here, R^{y} represents a hydrogen atom, halogen atom, optionally halogenated C₁₋₆ alkyl group, or optionally halogenated C₁₋₆ alkoxy group. R^{y} is preferably a hydrogen atom.
Y is preferably -O-, -S-, or -NR^{y}- (where R^{y} is a hydrogen atom or C₁₋₆ alkyl group), more preferably -O-, -S-, or -NH-, and even more preferably -O-.

In formula (I), Z is a bond, methylene, or ethylene (dimethylene). -Y-Z- is preferably not -NR^{y}-(CH₂)₂-.

In formula (I), Ar¹ is a five- to ten-membered aromatic ring (except for thiazole) which may be substituted with one or more (preferably one to three) substituents selected from the same or different halogen atoms, optionally halogenated C₁₋₆ alkyl groups, and optionally halogenated C₁₋₆ alkoxy groups.

Examples of such "five- to ten-membered aromatic rings" include:
(1) C₆₋₁₀ aromatic carbon rings (such as benzene and naphthalene),
(2) five- or six-membered monocyclic aromatic heterocycles (including five-membered rings having one to four hetero atoms selected from nitrogen, oxygen, and sulfur atoms, such as thiophene, furan, oxazole, isoxazole, isothiazole, imidazole, pyrazole, triazole, and tetrazole; or six-membered rings having one to four hetero atoms selected from nitrogen, oxygen, and sulfur atoms other than carbon atoms, such as pyridine, pyrimidine, triazine, pyridazine, and pyrazine), and
(3) seven- to ten-membered bicyclic aromatic heterocycles (including heterocycles in which a five- to six-membered monocyclic aromatic heterocycle noted above is condensed with a benzene ring, such as benzofuran, isobenzofuran, indole, isoindole, indazole, quinoline, and isoquinoline; or heterocycles in which two of the same or different five- or six-membered nitrogen-containing monocyclic aromatic heterocycles noted above are condensed, such as indolizine and naphthyridine).
The "five- to ten-membered aromatic ring" is preferably benzene, for example.
The "five- to ten-membered aromatic ring" is preferably not substituted or has one to three (more preferably one) of the substituents noted above. The substituents are preferably a halogen atom such as fluorine, for example.

In formula (I), Ar² is a five- or six-membered aromatic ring which may be substituted with one or more substituents selected from the same or different halogen atoms, optionally halogenated C₁₋₆ alkyl groups, and optionally halogenated C₁₋₆ alkoxy groups, and may be condensed with an optionally substituted five- or six-membered ring.

Preferred examples of "halogen atoms" as the substituent include chlorine and fluorine atoms.
Preferred examples of an "optionally halogenated C₁₋₆ alkyl group" as the substituent include trifluoromethyl.
Preferred examples of an "optionally halogenated C₁₋₆ alkoxy" as the substituent include methoxy.
Examples of such "five- or six-membered aromatic rings" include:
(1) benzene, and
(2) five- or six-membered monocyclic aromatic heterocycles (including five-membered rings having one to four hetero atoms selected from nitrogen, oxygen, and sulfur atoms, such as thiophene, furan, oxazole, isoxazole, thiazole, isothiazole, imidazole, pyrazole, triazole, and tetrazole; or six-membered rings having one to four hetero atoms selected from nitrogen, oxygen, and sulfur atoms other than carbon atoms, such as pyridine, pyrimidine, triazine, pyridazine, and pyrazine).
Preferred examples of the "five- or six-membered aromatic ring" include furan, thiophene, benzene, and pyridine.
The "five- or six-membered aromatic ring" is preferably not substituted or has one to three (more preferably one or two) of the substituents noted above.
Examples of "optionally substituted five- or six-membered rings" with which the above "five- or six-membered aromatic ring" may be condensed include five- or six-membered saturated or unsaturated carbon rings or heterocycles (such as cyclopentene and pyrrole). The "five- or six-membered ring" may have one to three substituents, and examples of such substituents include ones that are the same as the substituents for the "optionally substituted five- or six-membered heterocyclic groups."

Compound (I) is preferably a compound in which
A is -CO-NH- or -NH-CO-;
B is
   (1) a hydrogen atom,
   (2) a C₁₋₆ alkyl group (such as methyl, ethyl, n-propyl, or i-propyl) which may be substituted with one or more (preferably one to three) substituents selected from
      (a) halogen atoms (such as bromine and fluorine atoms),
      (b) hydroxy group,
      (c) cyano group,
      (d) amino group which may be mono- or di-substituted with substituents selected from optionally hydroxy-substituted C₁₋₆ alkyls (such as methyl and hydroxymethyl), C₆₋₁₀ aryls (such as phenyl), C₁₋₆ alkoxy-carbonyls (such as tert-butoxycarbonyl), and C₁₋₆ alkyl-carbonyls (such as acetyl), and carbamoyl groups,
      (e) C₆₋₁₀ aryl groups (such as phenyl) which may be substituted with one to three substituents selected from halogen atoms (such as chlorine atom), hydroxy group, and amino group,
      (f) C₁₋₆ alkylsulfanyl groups (such as methylsulfanyl),
      (g) C₁₋₆ alkylsulfinyl groups (such as methylsulfinyl),
      (h) C₁₋₆ alkoxy groups (such as methoxy, ethoxy, and isopropoxy),
      (i) C₆₋₁₀ aryloxy groups (such as phenoxy),
      (j) C₇₋₁₃ aralkyloxy groups (such as benzyloxy),
      (k) five- to ten-membered (preferably five- or six-membered) heterocyclic groups (such as pyrrolidinyl, pyrazolyl, imidazolyl, tetrazolyl, furyl, tetrahydrofuryl, thienyl, piperidyl, piperazinyl, piperazinyl, tetrahydropyranyl, pyridyl, morpholinyl, pyrazinyl, tetrahydroquinolinyl, and benzoisoxazolyl) which may be substituted with one or more substituents selected from C₁₋₆ alkyl groups (such as methyl and isopropyl), C₁₋₆ alkyl-carbonyl groups (such as acetyl), and oxo group, and
      (l) carbamoyl,
   (3) a C₂₋₆ alkenyl group (such as vinyl) which may be substituted with a five- or six-membered heterocyclic group(s) (such as imidazolyl, furyl, and pyridyl),
   (4) a C₃₋₁₀ cycloalkyl group (such as cyclopropyl),
   (5) a C₆₋₁₀ aryl group (such as phenyl) which may be substituted with one or more (preferably one to three) substituents selected from
      (a) hydroxy group,
      (b) cyano group,
      (c) C₁₋₆ alkoxy groups (such as methoxy),
      (d) mono- or di-C₁₋₆ alkyl-amino (such as dimethylamino), and
      (e) C₁₋₆ alkyl-carbonylamino (such as acetylamino),
   (6) an amino group which may be mono- or di-substituted with substituents selected from
      (a) C₁₋₆ alkyl groups (such as methyl and dimethylaminoethyl) which may be substituted with mono- or di-C₁₋₆ alkylamino groups, and
      (b) five- to ten-membered heterocyclic groups (such as pyrrolidinyl, thiazole, thiazolidinyl, tetrahydrofuryl, pyridyl, morpholinyl, quinolinyl) which may be substituted with one or more (preferably one to three) substituents selected from halogen atoms (such as fluorine atom) and oxo group, or
   (7) a five- to ten-membered heterocyclic groups (such as pyrazolyl, piperidyl, imidazolyl, furyl, tetrahydrofuryl, isoxazolyl, thiazolyl, isothiazolyl, thiadiazolyl, furazanyl, piperidyl, pyridyl, tetrahydropyranyl, pyridazinyl, tetrahydroindolyl, isoindolyl, indazolyl, dihydrobenzofuranyl, benzothiophenyl, tetrahydroquinolinyl, and tetrahydrobenzoisoxazolyl) which may be substituted with one or more (preferably one to three) substituents selected from
      (a) halogen atoms (such as fluorine atom),
      (b) C₁₋₆ alkyl groups (such as methyl and isopropyl) which may be substituted with one or more (preferably one to three) substituents selected from hydroxy group and mono- or di-C₁₋₆ alkylamino groups (such as dimethylamino),
      (c) C₁₋₆ alkoxy groups (such as methoxy),
      (d) C₁₋₆ alkyl-carbonyl groups (such as acetyl),
      (e) C₁₋₆ alkoxy-carbonyl groups (such as tert-butoxycarbonyl), and
      (f) carbamoyl;
X¹, X², X³, and X⁴ are the same or different -CR^{X}= or -N=, and one or two of X¹, X², X³, and X⁴ are -N=;
Y is -O-;
Ar¹ is a benzene ring or indole ring which may be substituted with one or more (preferably one to three) substituents selected from halogen atoms and optionally halogenated C₁₋₆ alkyl groups;
Ar² is a five-or six-membered aromatic ring (such as furan, thiophene, benzene, and pyridine) which may be substituted with one or more (preferably one to three) substituents selected from the same or different halogen atoms, optionally halogenated C₁₋₆ alkyl groups, and optionally halogenated C₁₋₆ alkoxy groups, and which may be condensed with a five- or six-membered ring (such as cyclopentene and pyrrole); and R^{X} is a hydrogen atom or optionally halogenated C₁₋₆ alkyl group (such as trifluoromethyl).

Compound (I) is also preferably a compound in which
A is -CO-NH- or -NH-CO-;
B is preferably:
   (1) a hydrogen atom,
   (2) a C₁₋₆ alkyl group (such as methyl, ethyl, n-propyl, or i-propyl) which may be substituted with one or more (preferably one to three) substituents selected from
      (a) halogen atoms (such as bromine and fluorine atoms),
      (b) hydroxy group,
      (c) cyano group,
      (d) amino group which may be mono- or di-substituted with substituents selected from optionally hydroxy-substituted C₁₋₆ alkyl groups (such as methyl and hydroxyethyl), C₆₋₁₀ aryls (such as phenyl), C₁₋₆ alkoxy-carbonyls (such as tert-butoxycarbonyl), and C₁₋₆ alkyl-carbonyls (such as acetyl), and carbamoyl groups,
      (e) C₆₋₁₀ aryl groups (such as phenyl) which may be substituted with one to three substituents selected from halogen atoms (such as chlorine atom), hydroxy group, and mono- or di-C₁₋₆ alkylamino groups,
      (f) C₁₋₆ alkylsulfanyl groups (such as methylsulfanyl),
      (g) C₁₋₆ alkylsulfinyl groups (such as methylsulfinyl),
      (h) C₁₋₆ alkoxy groups (such as methoxy, ethoxy, and isopropoxy),
      (i) C₆₋₁₀ aryloxy groups (such as phenoxy),
      (j) C₇₋₁₃ aralkyloxy groups (such as benzyloxy),
      (k) five- to ten-membered (preferably five- or six-membered) heterocyclic groups (such as pyrrolidinyl, pyrazolyl, imidazolyl, tetrazolyl, furyl, tetrahydrofuryl, thienyl, piperidyl, piperazinyl, tetrahydropyranyl, pyridyl, morpholinyl, pyrazinyl, tetrahydroquinolinyl, and benzoisoxazolyl) which may be substituted with one or more substituents selected from C₁₋₆ alkyl groups (such as methyl and isopropyl), C₁₋₆ alkyl-carbonyl groups (such as acetyl), and oxo group, and
      (l) carbamoyl,
   (3) a C₂₋₆ alkenyl group (such as vinyl) which may be substituted with a five- or six-membered heterocyclic group (such as imidazolyl, furyl, and pyridyl),
   (4) a C₃₋₁₀ cycloalkyl group (such as cyclopropyl) which may be substituted with a C₁₋₆ alkoxy group (such as methoxy),
   (5) a C₆₋₁₀ aryl group (such as phenyl) which may be substituted with one or more (preferably one to three) substituents selected from
      (a) hydroxy group,
      (b) cyano group,
      (c) C₁₋₆ alkoxy groups (such as methoxy),
      (d) mono- or di-C₁₋₆ alkyl-amino (such as dimethylamino), and
      (e) C₁₋₆ alkyl-carbonylamino (such as acetylamino),
   (6) an amino group which may be mono- or di-substituted with substituents selected from
      (a) C₁₋₆ alkyl groups (such as methyl and dimethylaminoethyl) which may be substituted with mono- or di-C₁₋₆ alkylamino groups, and
      (b) five- to ten-membered heterocyclic groups (such as pyrrolidinyl, thiazole, thiazolidinyl, tetrahydrofuryl, pyridyl, morpholinyl, quinolinyl) which may be substituted with one or more (preferably one to three) substituents selected from halogen atoms (such as fluorine atom) and oxo group, or
   (7) a five- to ten-membered heterocyclic groups (such as pyrazolyl, piperidyl, imidazolyl, furyl, tetrahydrofuryl, isoxazolyl, thiazolyl, isothiazolyl, thiadiazolyl, furazanyl, piperidyl, pyridyl, tetrahydropyranyl, pyridazinyl, tetrahydroindolyl, isoindolyl, indazolyl, dihydrobenzofuranyl, benzothiophenyl, tetrahydroquinolinyl, and tetrahydrobenzoisoxazolyl) which may be substituted with one or more (preferably one to three) substituents selected from
      (a) halogen atoms (such as fluorine atom),
      (b) C₁₋₆ alkyl groups (such as methyl and isopropyl) which may be substituted with one or more (preferably one to three) substituents selected from hydroxy group and mono- or di-C₁₋₆ alkylamino groups (such as dimethylamino),
      (c) C₁₋₆ alkoxy groups (such as methoxy),
      (d) C₁₋₆ alkyl-carbonyl groups (such as acetyl),
      (e) C₁₋₆ alkoxy-carbonyl groups (such as tert-butoxycarbonyl), and
      (f) carbamoyl,
      (g) five- or six-membered heterocyclic groups (such as morpholinyl), and
      (h) oxo;
X¹, X², X³, and X⁴ are the same or different -CR^{x}= or -N=, and one or two of X¹, X², X³, and X⁴ are -N=;
Y is -O-, -S-, or -NH-;
Ar¹ is a benzene ring or indole ring which may be substituted with one or more (preferably one to three) substituents selected from halogen atoms and optionally halogenated C₁₋₆ alkyl groups;
Ar² is a five-or six-membered aromatic ring (such as furan, thiophene, benzene, and pyridine) which may be substituted with one or more (preferably one to three) substituents selected from the same or different halogen atoms, optionally halogenated C₁₋₆ alkyl groups, and optionally halogenated C₁₋₆ alkoxy groups, and which may be condensed with a five- or six-membered ring (such as cyclopentene and pyrrole); and R^{x} is a hydrogen atom or optionally halogenated C₁₋₆ alkyl group (such as trifluoromethyl).

Compound (I) is, in particular, preferably
4-amino-N-(3-(2-((2-(3,4-dimethoxyphenyl)ethyl)amino)pyrimidin-4-yl)phenyl)butanamide hydrochloride (Working Example 33),
N-(3-(2-((2-(3,4-dimethoxyphenyl)ethyl)amino)pyrimidin-4-yl)phenyl)-N3,N3-dimethyl-β-alaninamide hydrochloride (Working Example 30),
3-(6-(2-(3,4-dimethoxyphenyl)ethoxy)pyridin-2-yl)-N-(2-pyrrolidin-1-ylethyl)benzamide (Working Example 39),
N-(2-hydroxyethyl)-3-(6-(2-(3-(trifluoromethyl)phenyl)ethoxy)pyridin-2-yl)benzamide (Working Example 65),
N-(2-hydroxyethyl)-3-(6-(2-(3-methoxyphenyl)ethoxy)pyridin-2-yl)benzamide (Working Example 69),
3-(6-(3,5-dichlorophenoxy)pyridin-2-yl)-N-(2-hydroxyethyl)benzamide (Working Example 87),
N-(2-aminoethyl)-3-(6-(2-(3-methoxyphenyl)ethoxy)pyridin-2-yl)benzamide hydrochloride (Working Example 81),
3-(6-(2,4-dichlorophenoxy)pyridin-2-yl)-N-(2-pyrrolidin-1-ylethyl)benzamide (Working Example 85),
3-(6-((2,4-dichlorophenyl)thio)pyridin-2-yl)-N-(2-hydroxyethyl)benzamide (Working Example 101),
N-(2-pyrrolidin-1-ylethyl)-3-[2-({2-[3-(trifluoromethyl)phenyl]ethyl}amino)pyrimidin-4-yl]benzamide (Working Example 146),
N-cyclopropyl-3-{6-[2-(3,4-dimethoxyphenyl)ethoxy]pyridin-2-yl}benzamide (Working Example 168),
N-(3-(6-(2-(3,4-dimethoxyphenyl)ethoxy)pyridin-2-yl)phenyl)acetamide (Working Example 234),
3-(6-(2-(3,4-dimethoxyphenyl)ethoxy)pyridin-2-yl)-N-(pyridin-3-ylmethyl)benzamide (Working Example 195),
3-(6-(3,5-dichlorophenoxy)pyridin-2-yl)-N-(2-(methylsulfmyl)ethyl)benzamide (Working Example 96), and
3-(6-((2,4-dichlorophenyl)amino)pyridin-2-yl)-N-(2-hydroxyethyl)benzamide (Working Example 104).

Examples of salts for when compound (I) is in the form of a salt include salts with inorganic bases, salts with organic bases, salts with inorganic acids, salts with organic acids, and salts with basic or acidic amino acids.
Preferable examples of salts with inorganic bases include salts with alkali metals such as sodium and potassium salts; salts with alkaline earth metals such as calcium and magnesium salts; aluminum salts; and ammonium salts.
Preferable examples of salts with organic bases include salts with trimethylamine, triethylamine, pyridine, picoline, ethanolamine, diethanolamine, triethanolamine, dicyclohexylamine, and N,N-dibenzylethylenediamine.
Preferable examples of salts with inorganic acids include salts with hydrochloric acid, hydrobromic acid, nitric acid, sulfuric acid, and phosphoric acid.
Preferable examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.
Preferable examples of salts with basic amino acids include salts with arginine, lysine, and ornithine.
Preferable examples of salts with acidic amino acids include salts with aspartic acid and glutamic acid.

A prodrug of compound (I) is a compound that is converted to compound (I) by a reaction involving an enzyme, gastric acid, or the like under the physiological conditions in the body; that is, a compound that is converted to compound (I) by enzymatic oxidation, reduction, hydrolysis, or the like, or a compound that is converted to compound (I) by hydrolysis or the like involving gastric acid or the like. Examples of prodrugs of compound (I) include compounds in which an amino group of compound (I) is acylated, alkylated, or phosphorylated (such as compounds in which an amino group of compound (I) is eicosanoylated, alanylated, pentylaminocarbonylated, (5-methyl-2-oxo-1,3-dioxolen-4-yl) methoxycarbonylated, tetrahydrofuranylated, pyrrolidylmethylated, pivaloyloxymethylated, or tert-butylated); compounds in which a hydroxy group of compound (I) is acylated, alkylated, phosphorylated, or borated (such as compounds in which a hydroxy group of compound (I) is acetylated, palmitoylated, propanoylated, pivaloylated, succinylated, fumarylated, alanylated, or dimethylaminomethylcarbonylated); compounds in which a carboxyl group of compound (I) is esterified or amidated (such as compounds in which a carboxyl group of compound (I) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, or methylamidated). These compounds can be produced from compound (I) by a method that is well known per se.
A prodrug of compound (I) may also be a compound that is converted to compound (I) under physiological conditions as described in Development of Pharmaceutical Products, Vol. 7, Molecular Design, pp. 163-198, Hirokawa Shoten (1990).
Compound (I) may also be labeled with an isotope (such as ³H, ¹⁴C, ³⁵S, and ¹²⁵I) or the like.
Compound (I) may furthermore be an anhydride, a hydrate, or a solvate.

Methods for producing compound (I) will be described. Compound (I) can be produced by reaction formulas 1 through 10 below or a method based thereon. The compounds in the reaction formulas may also be in the form of salts. Examples of such salts include the same ones noted above for salts of compound (I). 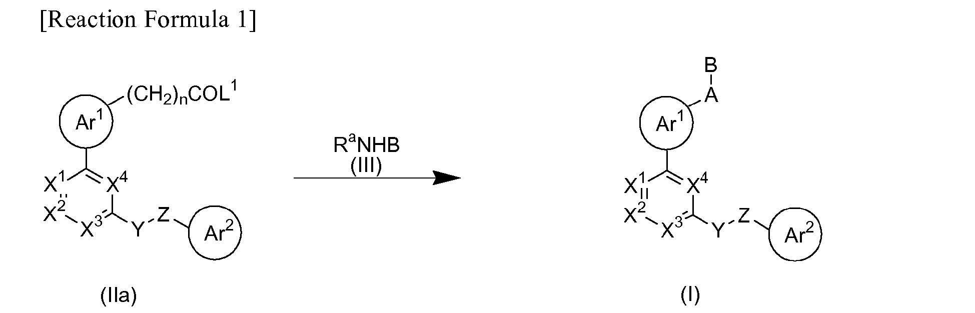 (In the formula, L¹ represents a leaving group, and the other symbols are synonymous with the above.)
Compound (I) can be produced by reacting compound (IIa) and compound (III) in the presence of a base or acid as needed.
Compound (III) is commercially available, and can be produced according to a method that is well known per se or a method based thereon.
Examples of the "leaving group" represented by L¹ include hydroxy group, halogen atoms (such as fluorine, chlorine, bromine, or iodine), optionally halogenated C₁₋₅ alkylsulfonyloxy groups (such as methanesulfonyloxy, ethanesulfonyloxy, or trichloromethanesulfonyloxy), optionally substituted C₆₋₁₀ arylsulfonyloxy groups, optionally substituted phenyloxy groups, and optionally substituted benzothiazol-2-yl thio groups.
Examples of "optionally substituted C₆₋₁₀ arylsulfonyloxy groups" include C₆₋₁₀ arylsulfonyloxy groups (such as phenylsulfonyloxy and naphthylsulfonyloxy) which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include phenylsulfonyloxy, m-nitrophenylsulfonyloxy, and p-toluenesulfonyloxy.
Examples of "optionally substituted phenyloxy groups" include phenyloxy groups which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include phenyloxy and 4-nitrophenoxy.
Examples of optionally substituted benzothiazol-2-yl groups include benzothiazol-2-yl groups which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include benzothiazol-2-yl.
Compound (III) is used in an amount of about 1 to 10 mol, and preferably about 1 to 2 mol, per mol compound (IIa).
Examples of "bases" include basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tributoxide.
The "base" is normally used in an amount of about 0.1 to 10 equivalents, and preferably 0.8 to 2 equivalents, relative to compound (IIa).
Examples of "acids" include methanesulfonic acid, p-toluenesulfonic acid, and camphorsulfonic acid.
The "acid" is normally used in an amount of about 0.1 to 10 equivalents, and preferably 0.8 to 3 equivalents, relative to compound (IIa).
It is advantageous to carry out the reaction without a solvent or with a solvent that is inert to the reaction. Examples of such solvents include, but are not particularly limited to, the following solvents or mixtures thereof, as long as the reaction can progress: water; ethers such as diethyl ether, tetrahydrofuran, 1,4-dioxane, and 1,2-dimethoxyethane; hydrocarbons such as benzene, toluene, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and nitrogen-containing aromatic hydrocarbons such as pyridine, lutidine, and quinoline.
The reaction temperature is generally in the range of about -40 to 150°C, and preferably 0 to 100°C.
The reaction time is generally in the range of 5 minutes to 24 hours, and preferably 10 minutes to 5 hours.
When L¹ is OH, compound (IIa) may be reacted with compound (III) in the presence of an appropriate condensation agent as an alternative method.
Compound (III) is generally used in an amount of about 0.8 to about 10 mol, and preferably about 0.8 to about 2 mol, per mol compound (IIa).
Examples of "condensation agents" include N,N'-carbodiimides such as N,N'-dicyclohexyl carbodiimide and 1-ethyl-3-(3-dimethylaminopropyl)carbodiimide hydrochloride (WSC); azolides such as N,N'-carbonylimidazole; 2-halogenopyridinium salts such as 2-chloro-1-methyl pyridinium iodide and 2-fluoro-1-methyl pyridinium iodide; and other compounds such as N-ethoxycarbonyl-2-ethoxy-1,2-dihydroquinoline, diethyl cyanophosphate, phosphorus oxychloride, and acetic anhydride.
The "condensation agent" is generally used in an amount of about 0.8 to about 5 mol, and preferably about 1 to about 3 mol, per mol compound (IIa).
The reaction may also be carried out in the presence of a base as needed. Examples of "bases" include basic salts such as potassium acetate and sodium acetate; and tertiary amines such as triethylamine, tripropylamine, tributylamine, cyclohexyl dimethylamine, 4-dimethylaminopyridine, N-methylpiperidine, N-methylpyrrolidine, and N-methylmorpholine. The reaction may also be carried out in the presence of a condensation accelerator such as 1-hydroxy-1H-benzotriazole (HOBt) monohydrate as needed.
The "base" is generally used in an amount of about 0.5 to about 5 mol, and preferably about 2 to about 3 mol, per mol compound (IIa).
It is advantageous to carry out the reaction using a solvent that is inert to the reaction. Examples of such a solvent include the following solvents or mixtures thereof: alcohols such as methanol, ethanol, and propanol; hydrocarbons such as hexane, cyclohexane, benzene, toluene, and xylene; ethers such as diethyl ether, diisopropyl ether, tetrahydrofuran, 1,4-dioxane, and 1,2-dimethoxy ethane; amides such as N,N-dimethyl formamide, N,N-dimethyl acetamide, hexamethyl phosphoric triamide, and 1-methyl pyrrolidin-2-one; sulfoxides such as dimethyl sulfoxide; halogenated hydrocarbons such as dichloromethane, chloroform, carbon tetrachloride, and 1,2-dichloroethane; nitriles such as acetonitrile and propionitrile; and acid anhydrides such as acetic anhydride.
The reaction time is generally about 10 min to about 48 hours, and preferably about 30 min to about 24 hours.
The reaction temperature is generally in the range of about -20 to about 150°C, and preferably about 0 to about 100°C.
The reaction time can be shortened using a microwave reactor or the like.
The compound (I) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography.

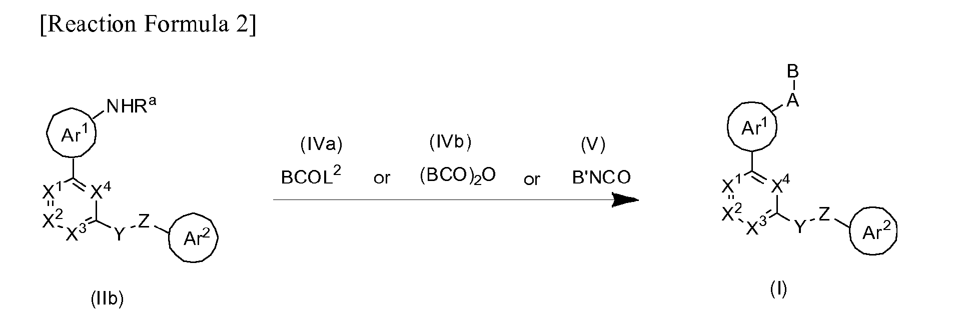

(In the formula, L² represents a leaving group, B' represents a group derived by the removal of an amino group from B when B is an optionally substituted amino group, and the other symbols are synonymous with the above.)
Compound (I) can be produced by allowing compound (IIb) to react with compound (IVa), compound (IVb), or compound (V) in the presence of a base or acid as needed.
Compound (IVa), compound (IVb), or compound (V) is commercially available, and can be produced according to a method that is well known per se or a method based thereon.
Examples of the "leaving group" represented by L² include hydroxy group, halogen atoms (such as fluorine, chlorine, bromine, or iodine), optionally halogenated C₁₋₆ alkoxy groups (such as methoxy, ethoxy, propoxy, isopropoxy, butoxy, isobutoxy, sec-butoxy, pentyloxy, and hexyloxy), optionally halogenated C₁₋₅ alkylsulfonyloxy groups (such as methanesulfonyloxy, ethanesulfonyloxy, or trichloromethanesulfonyloxy), optionally substituted C₆₋₁₀ arylsulfonyloxy groups, optionally substituted phenyloxy groups, and optionally substituted benzothiazol-2-yl thio groups.
Examples of "optionally substituted C₆₋₁₀ arylsulfonyloxy groups" include C₆₋₁₀ arylsulfonyloxy groups (such as phenylsulfonyloxy and naphthylsulfonyloxy) which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include phenylsulfonyloxy, m-nitrophenylsulfonyloxy, and p-toluenesulfonyloxy.
Examples of "optionally substituted phenyloxy groups" include phenyloxy groups which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include phenyloxy and 4-nitrophenoxy.
Examples of optionally substituted benzothiazol-2-ylthio groups include benzothiazol-2-ylthio groups which may have one to three substituents selected from C₁₋₆ alkyls (such as methyl, ethyl, propyl, isopropyl, butyl, isobutyl, sec-butyl, tert-butyl, pentyl, and hexyl), C₁₋₆ alkoxy groups (such as methoxy, ethoxy, propoxy, isopropoxy, butoxy, isobutoxy, sec-butoxy, pentyloxy, and hexyloxy), and nitro; specific examples thereof include benzothiazol-2-ylthio.
Compound (IVa), compound (IVb), or compound (V) is used in an amount of about 1 to 10 mol, and preferably about 1 to 2 mol, per mol compound (IIb).
Examples of "bases" include basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tributoxide.
The "base" is normally used in an amount of about 0.1 to 10 equivalents, and preferably 0.8 to 2 equivalents, relative to compound (IIb).
Examples of "acids" include methanesulfonic acid, p-toluenesulfonic acid, and camphorsulfonic acid.
The "acid" is normally used in an amount of about 0.1 to 10 equivalents, and preferably 0.8 to 3 equivalents, relative to compound (IIb).
It is advantageous to carry out the reaction without a solvent or with a solvent that is inert to the reaction. Examples of such solvents include, but are not particularly limited to, the following solvents or mixtures thereof, as long as the reaction can progress: water; ethers such as diethyl ether, tetrahydrofuran, 1,4-dioxane, and 1,2-dimethoxyethane; hydrocarbons such as benzene, toluene, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and nitrogen-containing aromatic hydrocarbons such as pyridine, lutidine, and quinoline.
The reaction temperature is generally in the range of about -40 to 150°C, and preferably 0 to 100°C.
The reaction time is generally in the range of 5 minutes to 24 hours, and preferably 10 minutes to 5 hours.
Compound (II) may be reacted with BCOOH in the presence of an appropriate condensation agent as an alternative method.
BCOOH is generally used in an amount of about 0.8 to about 10 mol, and preferably about 0.8 to about 2 mol, per mol compound (IIb).
Examples of "condensation agents" include N,N'-carbodiimides such as N,N'-dicyclohexyl carbodiimide and 1-ethyl-3-(3-dimethylaminopropyl)carbodiimide hydrochloride (WSC); azolides such as N,N'-carbonylimidazole; 2-halogenopyridinium salts such as 2-chloro-1-methyl pyridinium iodide and 2-fluoro-1-methyl pyridinium iodide; and other compounds such as N-ethoxycarbonyl-2-ethoxy-1,2-dihydroquinoline, diethyl cyanophosphate, phosphorus oxychloride, and acetic anhydride.
The "condensation agent" is generally used in an amount of about 0.8 to about 5 mol, and preferably about 1 to about 3 mol, per mol compound (IIb).
The reaction may also be carried out in the presence of a base as needed. Examples of "bases" include basic salts such as such as potassium acetate and sodium acetate; and tertiary amines such as triethylamine, tripropylamine, tributylamine, cyclohexyl dimethylamine, 4-dimethylaminopyridine, N-methylpiperidine, N-methylpyrrolidine, and N-methylmorpholine. The reaction may also be carried out in the presence of a condensation accelerator such as 1-hydroxy-1H-benzotriazole (HOBt) monohydrate as needed.
The "base" is generally used in an amount of about 0.5 to about 5 mol, and preferably about 2 to about 3 mol, per mol compound (IIb).
It is advantageous to carry out the reaction using a solvent that is inert to the reaction. Examples of such a solvent include the following solvents or mixtures thereof: alcohols such as methanol, ethanol, and propanol; hydrocarbons such as hexane, cyclohexane, benzene, toluene, and xylene; ethers such as diethyl ether, diisopropyl ether, tetrahydrofuran, 1,4-dioxane, and 1,2-dimethoxy ethane; amides such as N,N-dimethyl formamide, N,N-dimethyl acetamide, hexamethyl phosphoric triamide, and 1-methyl pyrrolidin-2-one; sulfoxides such as dimethyl sulfoxide; halogenated hydrocarbons such as dichloromethane, chloroform, carbon tetrachloride, and 1,2-dichloroethane; nitriles such as acetonitrile and propionitrile; and acid anhydrides such as acetic anhydride.
The reaction time is generally about 10 min to about 48 hours, and preferably about 30 min to about 24 hours.
The reaction temperature is generally in the range of about -20 to about 150°C, and preferably about 0 to about 100°C.
The reaction time can be shortened using a microwave reactor or the like.
The compound (I) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography.

### [Reaction Formula 3]

Compounds (I) in which B is -NHB' can also be produced by the process represented in the following reaction formula. In other words, compound (IIb) can be 2,2,2-trichloroethoxycarbonylated with 2,2,2-trichloroethyl chloroformate to synthesize compound (I'), which can then be reacted with compound (VI) to obtain compound (I). 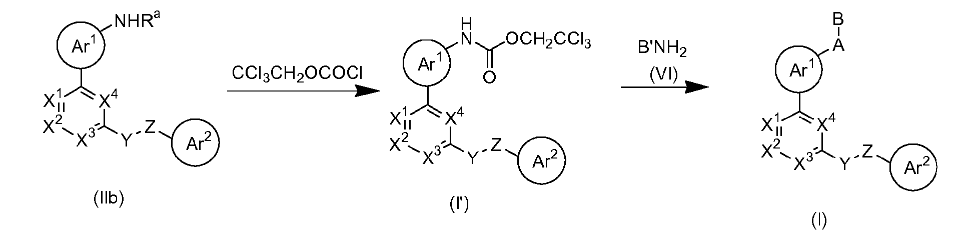 (In the formula the symbols are synonymous with the above.)
Compound (I') can be produced from compound (IIb) by the same method for producing compound (I) from compound (IIb) in reaction formula 2.
Compound (I) can be produced by reacting compound (I') with compound (VI) under basic conditions in a solvent that does not affect the reaction.
Compound (VI) is commercially available, and can also be produced according to a method that is well known per se or a method based thereon.
Compound (VI) is generally used in an amount of about 2 to 10 mol, and preferably about 2 to 5 mol, per mol compound (I').
Examples of the "base" include pyridine, triethylamine, diisopropylethylamine, potassium carbonate, sodium carbonate, sodium hydride, and potassium hydride.
The "base" is generally used in an amount of about 2 to 10 mol, and preferably about 2 to 5 mol, per mol compound (I').
Examples of solvents that do not affect the reaction include: ethers such as tetrahydrofuran; halogenated hydrocarbons such as chloroform; aromatic hydrocarbons such as toluene; amides such as N,N-dimethyl formamide; and sulfoxides such as dimethyl sulfoxide. These solvents may be used in the form of mixtures of two or more in suitable proportions.
The reaction temperature is generally in the range of about -50 to 200°C.
The reaction time is generally in the range of about 0.5 minutes to about 36 hours.
The compound (I) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography.

### [Reaction Formula 4]

Compound (I) can be produced by the method represented in the following reaction formula, that is, Suzuki coupling. 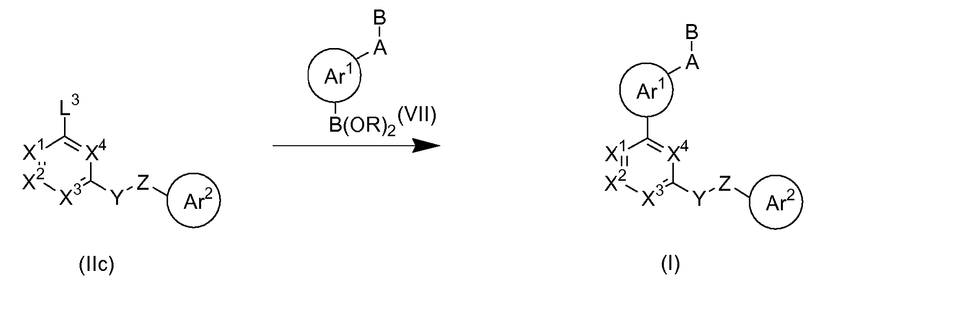 (In the formula, L³ represents a leaving group, and the other symbols are synonymous with the above.)
Compound (IIc) is reacted with a boronic acid (VII) such as a substituted boronic acid or substituted boronic ester in a solvent under basic conditions in the presence of a transition metal catalyst.
Compound (VII) can be procured in the form of a commercially available product, and can also be produced according to a method that is well known per se or a method based thereon.
Examples of the "leaving group" represented by L³ include halogen atoms (such as chlorine, bromine, and iodine), and optionally halogenated C₁₋₅ alkylsulfonyloxy groups (such as trifluoromethanesulfonyloxy).
The "boronic acid" is generally used in an amount of about 0.5 to about 10 mol, and preferably about 0.9 to about 3 mol, per mol compound (IIc).
Examples of "bases" include basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; and metal alkoxides such as sodium methoxide, sodium ethoxide, sodium, tributoxide, and potassium tributoxide.
Examples of "transition metal catalysts" include palladium catalysts such as palladium acetate, palladium chloride, tetrakis(triphenylphosphine)palladium, 1,1-bis-(diphenylphosphino)ferrocene dichloropalladium, and dichlorobis(triphenylphosphine)palladium. The transition metal catalyst is generally used in an amount of about 0.001 to about 3 mol, and preferably about 0.02 to about 0.2 mol, per mol compound (IIc).
Examples of solvent include the following solvents or mixtures thereof: ethers such as diethyl ether, diisopropyl ether, tetrahydrofuran, dioxane, and 1,2-dimethoxyethane; alcohols such as methanol, ethanol, and propanol; hydrocarbons such as benzene, toluene, carbon disulfide, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; sulfoxides such as dimethyl sulfoxide; and water.
The reaction temperature is generally in the range of 0 to 250°C, and preferably 50 to 150°C. The reaction time is generally about 5 min to about 48 hours, and preferably about 30 min to about 24 hours.
The reaction time can be shortened using a microwave reactor or the like.
The product can be used as is, in the form of the reaction solution or in the form of a crude product, in subsequent reactions, but can be isolated from the reaction mixture by a conventional method and easily purified by a common separation technique (such as recrystallization, distillation, or chromatography).

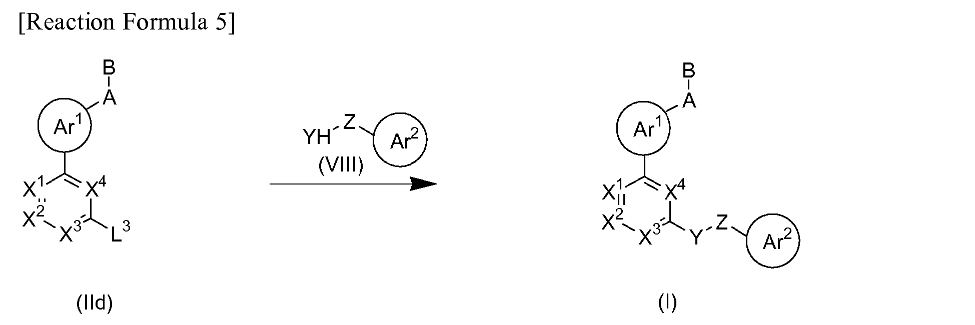

(In the formula, the symbols are synonymous with the above.)
Compound (I) can be produced by reacting compound (IId) and compound (VIII) in the presence of a base as needed. A copper catalyst such as copper or a copper salt may also be used as needed. Alternatively, the compound can also be produced by the Buchwald cross-coupling reaction.

Compound (VIII) can be easily procured in the form of a commercially available product, and can also be produced according to a method that is well known per se or a method based thereon.

Compound (VIII) is generally used in an amount of about 0.8 to about 10 mol, and preferably about 1 to about 5 mol, per mol compound (IId).
Examples of "bases" include basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, sodium tributoxide, and potassium tributoxide.
The "base" is generally used in an amount of about 0.8 to about 10 mol, and preferably about 1 to about 5 mol, per mol compound (IId).

It is advantageous to carry out the reaction using a solvent that is inert to the reaction. Examples of such solvents include, but are not particularly limited to, the following solvents or mixtures thereof, as long as the reaction can progress: alcohols such as methanol, ethanol, and propanol; ethers such as diethyl ether, tetrahydrofuran, 1,4-dioxane, and 1,2-dimethoxyethane; hydrocarbons such as benzene, toluene, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; and sulfoxides such as dimethyl sulfoxide.

Examples of "copper catalysts" include copper, copper halides (such as CuI, CuBr, and CuCl), and copper oxide (CuO).
The "copper catalyst" is generally used in an amount of about 0.1 to about 10 mol, and preferably about 0.5 to about 2 mol, per mol compound (IId).

Examples of palladium catalysts for when compound (I) is synthesized by the Buchwald reaction include palladium acetate, palladium chloride, tetrakis(triphenylphosphine)palladium, bis(dibenzylideneacetone)palladium, and tris(dibenzylideneacetone)palladium, and examples of "ligands" include phosphines, preferably trialkylphosphines, triarylphosphines, and trialkoxyphosphines.

The palladium catalyst is generally used in an amount of about 0.001 to about 5 mol, and preferably about 0.01 to about 0.5 mol, per mol compound (IId). The "phosphine" is generally used in an amount of about 0.001 to about 10 mol, and preferably about 0.01 to about 1 mol, per mol compound (IId).

The reaction time is generally about 30 min to about 72 hours, and preferably about 1 hour to about 48 hours.
The reaction temperature is generally in the range of about -20 to about 200°C, and preferably about 0 to about 150°C.

The reaction time can be shortened using a microwave reactor or the like.

The product can be used as is, in the form of a reaction solution or in the form of a crude product, in subsequent reactions, but can be isolated from the reaction mixture by a conventional method and easily purified by a common separation technique (such as recrystallization, distillation, or chromatography).

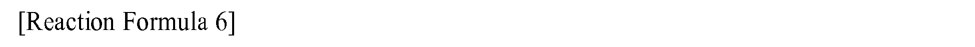

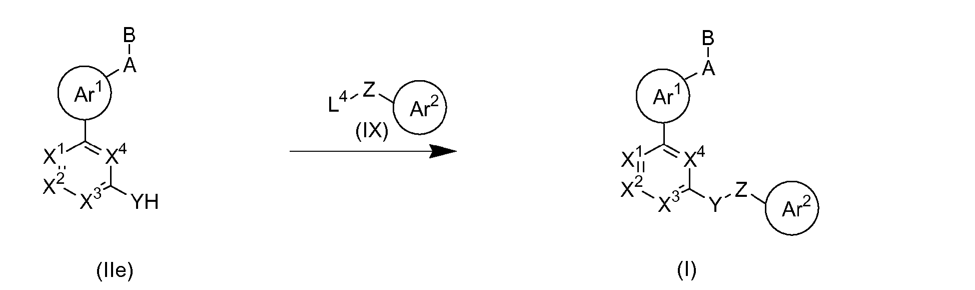

Compound (I) can be produced by reacting compound (IIe) and compound (IX) in the presence of a base as needed.
Examples of the "leaving group" represented by L⁴ include hydroxy group, halogen atoms (such as fluorine, chlorine, bromine, and iodine), optionally halogenated C₁₋₅ alkylsulfonyloxy groups (such as methanesulfonyloxy, ethanesulfonyloxy, and trichloromethanesulfonyloxy), and C₆₋₁₀ arylsulfonyloxy groups which may have substituents. Examples of "optionally substituted C₆₋₁₀ arylsulfonyloxy groups" include C₆₋₁₀ arylsulfonyloxy groups (such as phenylsulfonyloxy and naphthylsulfonyloxy) which may have one to three substituents selected from C₁₋₆ alkyl groups, C₁₋₆ alkoxy groups, and nitro; specific examples thereof include phenylsulfonyloxy, m-nitrophenylsulfonyloxy, and p-toluenesulfonyloxy.
Compound (IX) is generally used in an amount of about 1.0 to 5.0 mol, and preferably about 1.0 to 2.0 mol, per mol compound (IIe).
Examples of "bases" include basic salts such as sodium carbonate, potassium carbonate, cesium carbonate, and sodium bicarbonate; aromatic amines such as pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, and N-methylmorpholine; alkali metal hydrides such as sodium hydride and potassium hydride; metal amides such as sodium amide, lithium diisopropylamide, and lithium hexamethyldisilazide; and metal alkoxides such as sodium methoxide, sodium ethoxide, and potassium tributoxide. The base is generally used in an amount of about 1.0 to 5.0 mol, and preferably about 1.0 to 2.0 mol, per mol compound (IIe).
It is advantageous to carry out the reaction using a solvent that is inert to the reaction. Examples of such solvents include, but are not particularly limited to, the following solvents or mixtures thereof, as long as the reaction can progress: alcohols such as methanol, ethanol, and propanol; ethers such as diethyl ether, tetrahydrofuran, dioxane, and 1,2-dimethoxyethane; hydrocarbons such as benzene, toluene, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; and sulfoxides such as dimethyl sulfoxide.
The reaction time is generally in the range of 30 minutes to 48 hours, and preferably 1 hour to 24 hours. The reaction temperature is generally in the range of -20 to 200°C, and preferably 0 to 150°C.
The Mitsunobu reaction (Synthesis, 1981, pages 1-27) can be used instead of the above reaction.
In this reaction, compound (IIe) is reacted with compound (IX) in which L⁴ is OH in the presence of an azodicarboxylate (such as diethyl azodicarboxylate) and a phosphine (such as triphenylphosphine or tributylphosphine).
Compound (IX) in which L⁴ is OH is generally used in an amount of about 1.0 to 5.0 mol, and preferably about 1.0 to 2.0 mol, per mol compound (IIe).
The "azodicarboxylate" and "phosphine" are each generally used in an amount of about 1.0 to 5.0 mol, and preferably about 1.0 to 2.0 mol, per mol compound (IIe).
It is advantageous to carry out the reaction using a solvent that is inert to the reaction. Examples of such solvents include, but are not particularly limited to, the following solvents or mixtures thereof, as long as the reaction can progress: ethers such as diethyl ether, tetrahydrofuran, dioxane, and 1,2-dimethoxyethane; hydrocarbons such as benzene, toluene, cyclohexane, and hexane; amides such as N,N-dimethyl formamide and N,N-dimethyl acetamide; halogenated hydrocarbons such as dichloromethane, chloroform, carbon tetrachloride, and 1,2-dichloroethane; nitriles such as acetonitrile and propionitrile; and sulfoxides such as dimethyl sulfoxide.
The reaction time is generally in the range of 5 minutes to 48 hours, and preferably 30 minutes to 24 hours. The reaction temperature is generally in the range of -20 to 200°C, and preferably 0 to 100°C.

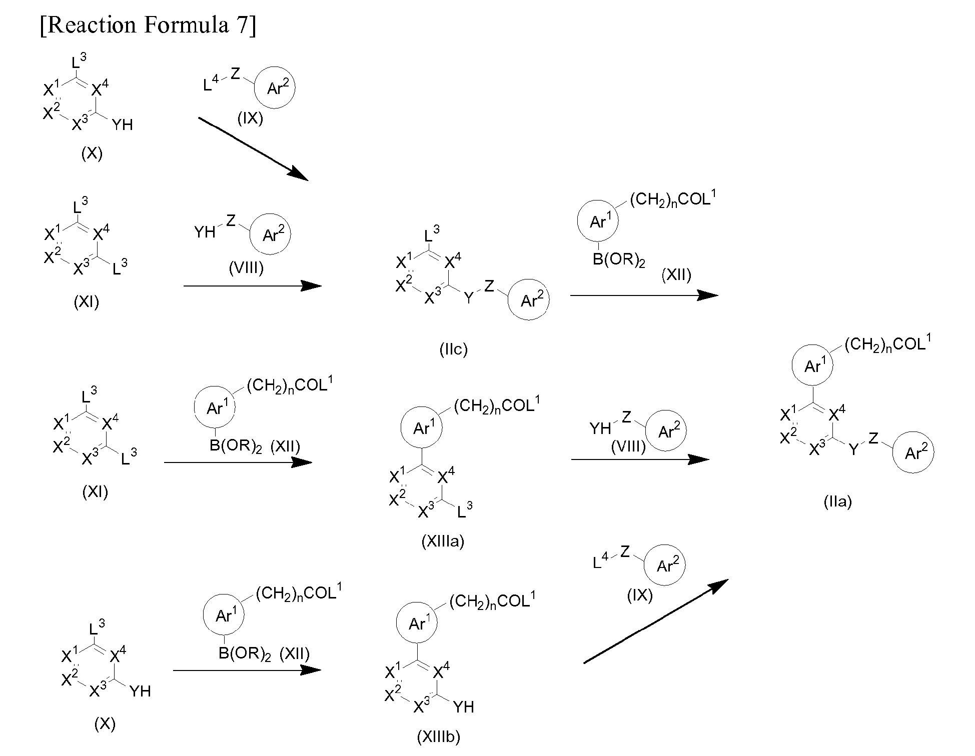

Compound (IIc) can be produced from compound (X) by the same method for producing compound (I) from compound (IIe) in reaction formula 6, or from compound (XI) by the same method for producing compound (I) from compound (IId) in reaction formula 5.
Compound (XIIIa) can be produced from compound (XI) by the same method for producing compound (I) from compound (IIc) in reaction formula 4.
Compound (XIIIb) can be produced from compound (X) by the same method for producing compound (I) from compound (IIc) in reaction formula 4.
Compound (IIa) can be produced from compound (IIc) by the same method for producing compound (I) from compound (IIc) in reaction formula 4, from compound (XIIIa) by the same method for producing compound (I) from compound (IId) in reaction formula 5, or from compound (XIIIb) by the same method for producing compound (I) from compound (IIe) in reaction formula 6.

The compound (IIa) or (IIc) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography. 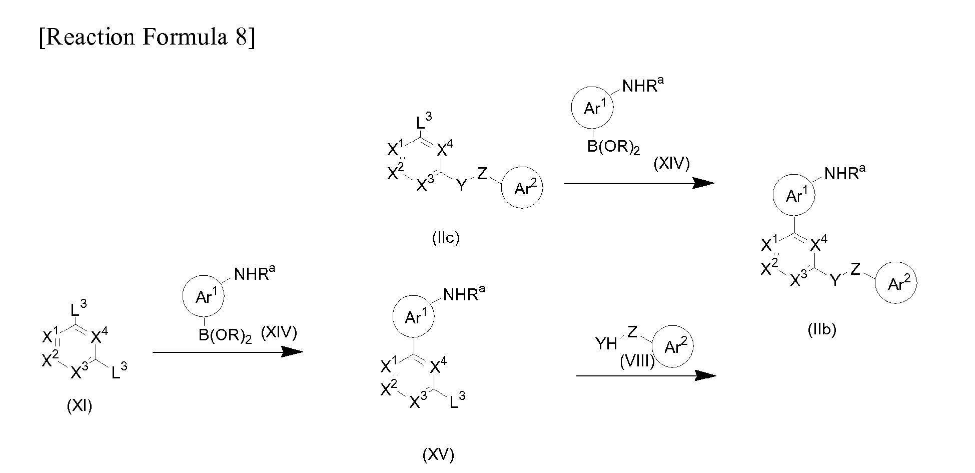 Compound (XV) can be produced from compound (XI) by the same method for producing compound (I) from compound (IIc) in reaction formula 4.
Compound (IIb) can be produced from compound (IIc) by the same method for producing compound (I) from compound (IIc) in reaction formula 4, or from compound (XV) by the same method for producing compound (I) from compound (IId) in reaction formula 5.

The compound (IIb) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography. 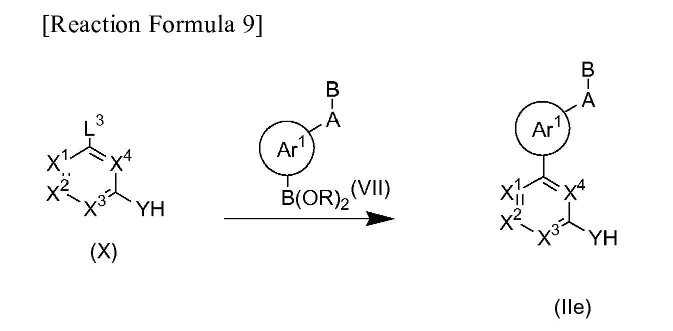 Compound (IIe) can be produced from compound (X) by the same method for producing compound (I) from compound (IIc) in reaction formula 4.
The compound (IIe) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography. 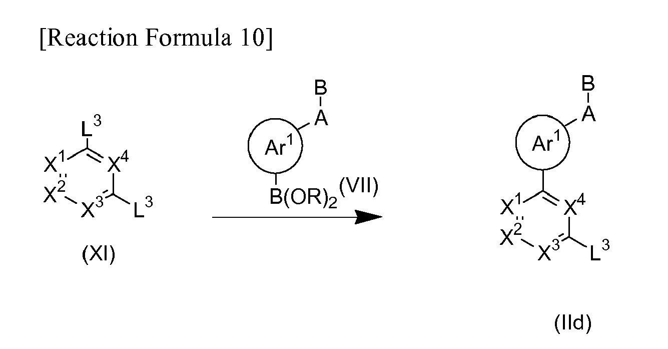 Compound (IId) can be produced from compound (XI) by the same method for producing compound (I) from compound (IIc) in reaction formula 4.
The compound (IId) thus obtained can be isolated from the reaction mixture by a conventional method, and can be isolated and purified by a well-known technique of separation and purification such as concentration, vacuum concentration, solvent extraction, crystallization, recrystallization, transfer dissolution, or chromatography.

When isomerization has occurred, configurational isomers (E and Z isomers) of compound (I) can be isolated and purified by a conventional technique of separation such as extraction, recrystallization, distillation, or chromatography to produce a pure compound. The isomerization of double bonds can be allowed to progress to obtain the corresponding pure isomers through the use of heat, acid catalysts, transition metal complexes, metal catalysts, radical catalysts, exposure to light, strongly basic catalysts, or the like according to the method in the New Course in Experimental Chemistry 14 (Ed. by The Chemical Society of Japan), pages 251-253 and Course in Experimental Chemistry 19, 4th Edition (Ed. by The Chemical Society of Japan), pages 273-274, or methods based thereon.
Stereoisomers of compound (I) may also be produced, depending on the type of substituent, but such isomers are encompassed by the present invention, both individually and in combination.
Compound (I) may or may not be in the form of a hydrate.
In either case, compound (I) can be synthesized through the following additional reactions as needed, either individually or in any combination: deprotection, acylation, alkylation, hydrogenation, oxidation, reduction, carbon chain extension, and substituent replacement.
Target substances that are obtained in free form as a result of the above reactions can be converted to salts by a conventional method, and those that are obtained in the form of salts can also be converted to the free form or another salt by a conventional method. The compound (I) thus obtained can be isolated and purified from the reaction mixture by a well-known technique such as transfer dissolution, concentration, solvent extraction, fractional distillation, crystallization, recrystallization, or chromatography.
When compound (I) is in the form of a configurational isomer, diastereoisomer, conformer, or the like, it can also be isolated by the above separation and purification techniques as needed. When compound (I) is in the form of a racemic mixture, it can be separated into the d and 1 forms by a common technique of optical resolution.

Compound (I) has excellent GPR52 agonist activity, and can be used as an agent for preventing or treating various diseases in mammals (such as humans, cows, horses, dogs, cats, monkeys, mice, and rats, but particularly humans). The compound of the present invention has low toxicity (such as acute toxicity, chronic toxicity, cardiac toxicity, carcinogenicity, or genotoxicity) and few side effects.

Compound (I) or a prodrug thereof (hereinafter, also simply referred to as compound of the invention) can be used as a pharmaceutical agent for preventing or treating schizophrenia.
The compound of the present invention has excellent GPR52 agonist activity and is useful for the prevention or treatment of diseases in mammals (including humans, mice, rats, rabbits, dogs, cats, cows, horses, swine, and monkeys) such as mental disorders (including schizophrenia, depression, anxiety, bipolar disorders or PTSD, anxiety neuroses, and obsessive-compulsive neuroses), neurodegenerative diseases (such as Alzheimer's disease, mild cognitive impairment (MCI), Parkinson's disease, amyotrophic lateral sclerosis (ALS), Huntington's disease, spinocerebellar degeneration, multiple sclerosis (MS), and Pick disease). The compound of the present invention is particularly useful for improving symptoms of schizophrenia, including (1) positive symptoms such as delusion and hallucination, (2) negative symptoms such as hypesthesia, social withdrawal, and diminished motivation/loss of concentration, and (3) cognitive dysfunction.

The compound of the present invention has low toxicity (such as acute toxicity, chronic toxicity, genotoxicity, reproductive toxicity, cardiac toxicity, drug interactions, and carcinogenicity), and can therefore be safely administered, as such or while mixed with a pharmaceutically acceptable carrier or the like, in the form of the pharmaceutical composition of the present invention to mammals (such as humans, mice, rats, rabbits, dogs, cats, cows, horses, swine, and monkeys), and can be used as an agent for preventing or treating the diseases.

A variety of organic or inorganic substances commonly used as formulation materials may be used as the pharmaceutically acceptable carrier herein, and may be compounded in the form of excipients, lubricants, binders, and disintegrants in solid formulations, and in the form of solvents, dissolution aids, suspending agents, isotonizing agents, buffers, and soothing agents or the like in liquid formulations. Additives such as preservatives, antioxidants, colorants, and sweeteners can also be used as needed.

Preferred examples of excipients include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate, and magnesium aluminometasilicate.
Preferred examples of lubricants lubricant include magnesium stearate, calcium stearate, talc, and colloidal silica.

Preferred examples of binders include pregelatinized starch, saccharose, gelatin, gum arabic, methylcellulose, carboxymethyl cellulose, sodium carboxymethyl cellulose, crystalline cellulose, sucrose, D-mannitol, trehalose, dextrin, pullulan, hydroxypropyl cellulose, hydroxypropyl methyl cellulose, and polyvinyl pyrrolidone.

Preferred examples of disintegrants include lactose, sucrose, starch, carboxymethyl cellulose, calcium carboxymethyl cellulose, croscarmellose sodium, sodium carboxymethyl starch, light anhydrous silicic acid, and low-substituted hydroxypropylcellulose.

Preferred examples of solvents include water for injection, physiological saline, Ringer's solution, alcohol, propylene glycol, polyethylene glycol, sesame oil, corn oil, olive oil, and cottonseed oil.

Preferred examples of dissolution aids include polyethylene glycol, propylene glycol, D-mannitol, trehalose, benzyl benzoate, ethanol, trisaminomethane, cholesterol, triethanolamine, sodium carbonate, sodium citrate, sodium salicylate, and sodium acetate.

Preferred examples of suspending agents include surfactants such as stearyl triethanolamine, sodium lauryl sulfate, lauryl aminopropionic acid, lecithin, benzalkonium chloride, benzethonium chloride, and glycerol monostearate; hydrophilic polymers such as polyvinyl alcohol, polyvinyl pyrrolidone, sodium carboxymethyl cellulose, methylcellulose, hydroxymethyl cellulose, hydroxyethyl cellulose, and hydroxypropyl cellulose; and polysorbates and polyoxyethylene hydrogenated castor oil.

Preferred examples of isotonizing agents include sodium chloride, glycerin, D-mannitol, D-sorbitol, and glucose.
Preferred examples of buffers include phosphate, acetate, carbonate, and citrate buffers.
Preferred examples of soothing agents include benzyl alcohol.
Preferred examples of preservatives include p-hydroxybenzoic acid esters, chlorobutanol, benzyl alcohol, phenethyl alcohol, dehydroacetic acid, and sorbic acid.
Preferred examples of antioxidants include sulfites and ascorbates.

Preferred examples of colorants include water-soluble edible tar pigments (including food coloring such as Food Color Red Nos. 2 and 3, Food Color Yellow Nos. 4 and 5, and Food Color Blue Nos. 1 and 2), water-insoluble lake pigments (including aluminum salts of the aforementioned water-soluble edible tar pigments), and natural pigments (such as β-carotene, chlorophyll, and red oxide).
Preferred examples of sweeteners include saccharin sodium, dipotassium glycyrrhizinate, aspartame, and stevia.

Examples of dosage forms for the aforementioned pharmaceutical composition include oral agents such as tablets (including sugar-coated tablets, film-coated tablets, sublingual tablets, and orally disintegrable tablets), capsules (including soft capsules and micro capsules), granules, powders, troches, syrups, emulsions, suspensions, and films (such as orally disintegrable films); and parenteral agents such as injections (including subcutaneous injections, intravenous injections, intramuscular injections, intraperitoneal injections, and drip infusions), topical agents (such as transdermal formulations and ointments), suppositories (such as rectal suppositories and vaginal suppositories), pellets, nasal agents, pulmonary formulations (inhalants), and ophthalmic solutions. These can be can be safely administered orally or parenterally (such as locally, rectally, or intravenously).
These formulations may also be controlled-release formulations such as rapid-release formulations and sustained-release formulations (such as sustained-release microcapsules).

The pharmaceutical composition of the present invention can be produced by a method commonly used in the technical field of drug formulations, such as the methods described in the Japan Pharmacopoeia. Specific methods for producing formulations are described below.
The content of the compound of the present invention in the pharmaceutical composition of the present invention will vary depending on the dosage form, the dose of the compound of the present invention, and the like, but is in the range of about 0.01 to 100% by weight, for example.

The dosage of the compound of the present invention will vary depending on the subject of treatment, route of administration, treated disease, symptoms, and the like, but when given orally to adults patients with schizophrenia, for example, the normal single dose is about 0.1 to about 20 mg/kg body weight, preferably about 0.2 to about 10 mg/kg body weight, and even more preferably about 0.5 to about 10 mg/kg, at a time, and is preferably given once or several times a day.

The compound of the present invention may be used in combination with other active ingredients. Examples of such active ingredients include:
atypical antipsychotic agents (such as clozapine, olanzapine, risperidone, aripiprazole,
iloperidone, asenapine, ziprasidone, quetiapine, and zotepine);
typical antipsychotic agents (such as haloperidol and chlorpromazine);
selective serotonin reuptake inhibitors (such as paroxetine, sertraline, fluvoxamine, and fluoxetine), selective serotonin-noradrenaline reuptake inhibitors (such as milnacipran and venlafaxine),
and selective noradrenaline-dopamine reuptake inhibitors (e.g., bupropion);
tetracyclic antidepressants (such as amoxapine and clomipramine);
tricyclic antidepressants (such as imipramine and amitriptyline);
other antidepressant agents (such as NS-2359, Lu AA21004, and DOV21947);
α7-nicotinic receptor partial modulators (such as SSR-180711 and PNU-120596);
NK2 antagonists;
NK3 antagonists;
glycine transporter 1 inhibitors (such as ALX5407 and SSR504734);
metabolic glutamate receptor modulators (such as CDPPB and MPEP);
anxiolytics [benzodiazepine-based agents (such as diazepam and etizolam) and serotonin 5-HT_{1A} agonists (such as tandospirone)];
sleep inducing agents [benzodiazepine-based agents (e.g.., estazolam and triazolam); non-benzodiazepine-based agents (such as zolpidem), and
melatonin receptor agonists (such as ramelteon)];
β-amyloid vaccines;
β-amyloid-degrading enzymes and the like;
brain function activators (such as aniracetam and nicergoline);
anti-Parkinson agents [such as dopamine receptor agonists (including L-DOPA, bromocriptine, pergolide, talipexole, pramipexole, cabergoline, and amantadine), monoamine oxidase (MAO) inhibitors (such as deprenyl, selegiline, remacemide, and riluzole), anticholinergic agents (such as trihexyphenidyl and biperiden); and COMT inhibitors (such as entacapone)];
therapeutic agents for amyotrophic lateral sclerosis (including neurotrophic factors and riluzo le);
antihyperlipidemic drugs such as cholesterol-lowering drugs [statins (such as pravastatin sodium, atorvastatin, simvastatin, and rosuvastatin), fibrates (such as clofibrate), and squalene synthase inhibitors];
therapeutic agents for abnormal behaviors/wandering symptoms associated with dementia (such as sedatives and anxiolytics);
apoptosis inhibitors (such as CPI-1189, IDN-6556, and CEP-1347);
neuronal differentiation/regeneration accelerators (such as leteprinim and xaliproden (SR-57746-A), and SB-216763);
antihypertensive agents;
therapeutic agents for diabetes mellitus;
nonsteroidal anti-inflammatory agents (such as meloxicam, tenoxicam, indomethacin, ibuprofen, celecoxib, rofecoxib, aspirin, and indomethacin);
disease-modifying anti-rheumatic drugs (DMARDs);
anticytokine agents (such as TNF inhibitors and MAP kinase inhibitors);
steroid agents (such as dexamethasone, hexestrol, and cortisone acetate); sex hormones or the derivatives thereof (such as progesterone, estradiol, and estradiol benzoate); parathyroid hormone (PTH); and
calcium receptor blockers (hereinafter, also simply referred to as concomitant drugs).
The compound of the present invention can in particular preferably be used in combination with various drugs having action on the central nervous system and drugs for the treatment of diseases that tend to develop with schizophrenia (such as drugs for the treatment of diabetes).
The compound of the present invention can in particular preferably be used in combination with various active ingredients having no action on GPR52.
The time of administration of the compound of the present invention and concomitant drugs is not limited, and they may be given at the same or different times to the subjects of treatment. The compound of the present invention and a concomitant drug may furthermore be given in the form of two formulations containing their respective active ingredients or in the form of a single formulation containing both active ingredients.
The aforementioned concomitant drugs may be used in combinations of two or more in suitable proportions.
The dosage of concomitant drugs can be suitably selected based on the clinically used dose. The compounding ratio of the compound of the present invention and concomitant drugs can be suitably selected depending on the subject of treatment, route of administration, disease being treated, symptoms, combination, and the like. When the subject of treatment is human, for example, the concomitant drug may be used in an amount of 0.01 to 100 parts by weigh per part by weight of the compound of the present invention.
When the compound of the present invention is used in combination with a concomitant drug, the amounts of the respective drugs can be reduced to within a safe range in consideration of their counter-effects.

### [Working Examples]

The present invention will be illustrated in further detail by the following reference examples, working examples, preparation example, and test example, but these examples, which are merely embodiments, do not limit the present invention and may be modified without departing from the scope of the invention.
In the following reference examples and working examples, "room temperature" ordinarily indicates a temperature from about 10°C to about 35°C. Percentages for yield indicate mol/mol% and percentages for solvent used in chromatography indicate percent by volume, but otherwise indicate percent by weight. Broad peaks such as OH and NH protons that could not be confirmed in the proton NMR spectra are not included in the data.
Other abbreviations used in this document are defined below.
s: singlet
d: doublet
dd: doublet of doublets
dt: doublet of triplets
t: triplet
tt: triplet of triplets
td: triplet of doublets
q: quartet
septet
m: multiplet
br: broad
J: coupling constant
Hz: Hertz
CDCl₃ : deuterated chloroform
DMSO-d₆ : deuterated dimethyl sulfoxide
¹ H-NMR: proton nuclear magnetic resonance
HPLC: high performance liquid chromatography
THF: tetrahydrofuran
DMF: N,N-dimethyl formamide
DMSO: dimethyl sulfoxide
NMP: N-methyl pyrrolidone
HOBt: 1-hydroxybenzotriazole
WSC: 1-ethyl-3-(3-dimethylaminopropyl)carbodiimide hydrochloride
HATU: 2-(7-aza-1H-benzotriazol-1-yl)-1,1,3,3-tetramethyluronium hexafluorophosphate
DMTMM: 4-(4,6-dimethoxy-1,3,5-triazin-2-yl)-4-methylmorpholinium chloride n-hydrate
DBU: 1,8-diazabicyclo[5.4.0]-7-undecene
LC-MS: liquid chromatography-mass spectrometry
ESI: electrospray ionization

### Reference Example 1

### 2-Chloro-4-(3-nitrophenyl)pyrimidine

3-Nitrophenylboronic acid (2.2 g, 13.2 mmol), tetrakis(triphenylphosphine)palladium (0.77 g, 0.67 mmol), and 2 N sodium carbonate aqueous solution (8 mL) were added to a dimethoxyethane (100 mL) solution of 2,4-dichloropyrimidine (2.0 g, 13.4 mmol), and the mixture was heated to reflux for 13 hours in an argon atmosphere. Ethyl acetate was added to the reaction solution, and the solution was washed with water and with saturated brine, dried, and concentrated. The residue was purified by silica gel column chromatography (THF) and then recrystallized from ethyl acetate to give 1.2 g of the titled compound (yield: 39%). ¹ H-NMR (CDCl₃ ) δ : 7.72 - 7.77 (2H, m), 8.39 - 8.43 (1H, m), 8.47 - 8.51 (1H, m), 8.77 (1H, d, J = 5.1 Hz), 8.93 (1H, t, J = 2.1 Hz).

### Reference Example 2

### (4-(3-Nitrophenyl)pyrimidin-2-yl)-(2-(3,4-dimethoxyphenyl)ethyl)amine

2-(3,4-Dimethoxyphenyl)ethylamine (1.3 g, 7.2 mmol) and ethyl diisopropylamine (1.7 mL, 9.5 mmol) were added to an n-butanol (15 mL) solution of 2-chloro-4-(3-nitrophenyl)pyrimidine (1.1 g, 4.7 mmol) synthesized in Reference Example 1, and the mixture was heated for 30 min to 130°C while irradiated with microwaves. The reaction solution was dissolved in ethyl acetate, and the solution was washed with water and with saturated brine, dried, and was concentrated. The residue was purified by silica gel column chromatography (ethyl acetate) and recrystallized from ethyl acetate-hexane to give 1.5 g of the titled compound (yield: 84%). ¹ H-NMR (CDCl₃ ) δ : 2.93 (2H, t, J = 6.9 Hz), 3.75 - 3.82 (2H, m), 3.87 (3H, s), 3.88 (3H, s), 5.30 (1H, br t, J = 5.7 Hz), 6.78 (1H, s), 6.84 (2H, d, J = 0.6 Hz), 7.03 (1H, d, J = 5.4 Hz), 7.65 (1H, t, J = 8.1 Hz), 8.30 - 8.37 (2H, m), 8.41 (1H, d, J = 5.1 Hz), 8.92 (1H, br s).

### Reference Example 3

### 4-(3-Aminophenyl)-N-(2-(3,4-dimethoxyphenyl)ethyl)pyrimidine-2-amine

10%-Palladium carbon (0.13 g) was added to a THF-ethanol (1:1, 40 mL) solution of (4-(3-nitrophenyl)pyrimidin-2-yl)-(2-(3,4-dimethoxyphenyl)ethyl)amine (1.3 g, 3.4 mmol) synthesized in Reference Example 2, and the mixture was stirred for 1 day at room temperature in a hydrogen atmosphere at ordinary pressure. The reaction solution was filtered and concentrated. The residue was purified by basic silica gel column chromatography (hexane-ethyl acetate = 10:0 to 0:10) and was recrystallized from ethyl acetate-hexane to give 1.2 g (quantitative) of the titled compound.
¹H-NMR (CDCl₃ ) δ : 2.91 (2H, t, J = 6.9 Hz), 3.72 - 3.82 (4H, m), 3.87 (6H, s), 5.21 (1H, br t, J = 5.7 Hz), 6.78-6.84 (4H, m), 6.93 (1H, d, J = 5.1 Hz), 7.22- 7.27 (1H, m), 7.36- 7.39 (2H, m), 8.31 (1H, d, J = 5.1 Hz).

### Reference Example 4

### Ethyl 3-(2-chloropyrimidin-4-yl)benzoate

The titled compound was obtained from 2,4-dichloropyrimidine and 3-ethoxycarbonylphenylboronic acid in the same manner as in Reference Example 1.
Yield: 58%.
¹H-NMR (CDCl₃ ) δ : 1.44 (3H, t, J = 7.2 Hz), 4.45 (2H, q, J = 7.2 Hz), 7.62 (1H, t, J = 7.5 Hz), 7.74 (1H, d, J = 5.1 Hz), 8.20-8.24 (1H, m), 8.33 - 8.37 (1H, m), 8.69 - 8.70 (2H, m).

### Reference Example 5

### Ethyl 3-(2-(2-(3,4-dimethoxyphenyl)ethylamino)pyrimidin-4-yl)benzoate

The titled compound was obtained from ethyl 3-(2-chloropyrimidin-4-yl)benzoate synthesized in Reference Example 4 and 2-(3,4-dimethoxyphenyl)ethylamine in the same manner as in Reference Example 2. Yield: 78%.
¹H-NMR (CDCl₃ ) δ : 1.42 (3H, t, J = 7.2 Hz), 2.92 (2H, t, J = 6.9 Hz), 3.78 (2H, q, J = 6.9 Hz), 3.87 (6H, s), 4.42 (2H, q, J = 7.2 Hz), 5.26 (1H, t, J = 5.4 Hz), 6.78 - 6.83 (3H, m), 7.03 (1H, d, J = 5.1 Hz), 7.55 (1H, t, J = 7.8 Hz), 8.13 - 8.16 (1H, m), 8.26 (1H, brd, J = 7.5 Hz), 8.37 (1H, d, J = 5.1 Hz), 8.67 (1H, s).

### Reference Example 6

### 3-(2-(2-(3,4-dimethoxyphenyl)ethylamino)pyrimidin-4-yl)benzoic acid

Lithium hydroxide monohydrate (296 mg, 7.1 mmol) was added to a THF-ethanol-water (1:1:1, 60 ml) solution of ethyl 3-(2-(2-(3,4-dimethoxyphenyl)ethylamino)pyrimidin-4-yl)benzoate (1.9 g, 4.7 mmol) synthesized in Reference Example 5, and the mixture was stirred for 20 min at room temperature. The reaction solution was concentrated at reduced pressure and was made acidic with the addition of diluted hydrochloric acid, aqueous ammonia and saturated ammonium chloride aqueous solution were then added, and the product was extracted with ethyl acetate. The extract was washed with water and saturated brine, dried, and concentrated, and the residue was recrystallized from ethyl acetate-diisopropyl ether to give 1.5 g of the titled compound (yield: 84%).
¹H-NMR (DMSO-d₆) δ : 2.96 (2H, t, J = 7.2 Hz), 3.83 - 3.86 (8H, m), 6.79 - 6.88 (4H, m), 7.06 (1H, d, J = 5.4 Hz), 7.60 (1H, t, J = 7.8 Hz), 8.22 - 8.33 (3H, m), 8.78 (1H, s).

### Reference Example 7

### 2-Chloro-6-(2-(3,4-dimethoxyphenyl)ethoxy)pyridine

A tetrahydrofuran (30 mL) solution of 6-chloropyridin-2-ol (1.0 g, 7.72 mmol), 2-(3,4-dimethoxyphenyl)ethanol (1.55 g, 8.44 mmol), triphenylphosphine (2.23 g, 8.44 mmol), and diethyl azodicarboxylate (1.61 g, 8.44 mmol) was stirred for 1 hour at room temperature. The reaction mixture was concentrated at reduced pressure, and the residue was chromatographed on a silica gel column (hexane-ethyl acetate 80:20) to give 1.76 g of the titled compound (yield: 78%). Oily substance.
¹H-NMR (CDCl₃ ) δ : 3.01 (2H, t, J = 6.9 Hz), 3.86 (3H, s), 3.88 (3H, s), 4.49 (2H, t, J = 6.9 Hz), 6.60 - 6.89 (5H, m), 7.45- 7.92 (1H, m).

### Reference Example 8

### 4-(2-(3-Bromophenoxy)ethyl)-1,2-dimethoxybenzene

The titled compound was obtained using 3-bromophenol in the same manner as in Reference Example 7. Yield: 56%. Oily substance.
¹H-NMR (CDCl₃ ) δ : 3.03 (2H, t, J = 6.9 Hz), 3.86 (3H, s), 3.87 (3H, s), 4.12 (2H, t, J = 6.9 Hz), 6.78 - 6.85 (4H, m), 7.02 - 7.16 (3H, m).

### Reference Example 9

### 2-Chloro-6-(2-(3-(trifluoromethyl)phenyl)ethoxy)pyridine

The titled compound was obtained using 2-(3-(trifluoromethyl)phenyl)ethanol in the same manner as in Reference Example 7. Yield: 83%. Oily substance.
¹H-NMR (CDCl₃ ) δ : 3.13 (2H, t, J = 6.9 Hz), 4.52 (2H, t, J = 6.9 Hz), 6.62 (1H, t, J = 8.4 Hz), 6.88 (1H, t, J = 7.5 Hz), 7.38 - 7.53 (5H, m).

### Reference Example 10

### 2-Chloro-6-(2-(3-fluorophenyl)ethoxy)pyridine

The titled compound was obtained using 2-(3-fluorophenyl)ethanol in the same manner as in Reference Example 7. Yield: 78%. Oily substance.
¹H-NMR (CDCl₃ ) δ : 3.06 (2H, t, J = 6.9 Hz), 4.50 (2H, t, J = 6.9 Hz), 6.62 (1H, d, J = 8.1 Hz), 6.84 - 7.06 (4H, m), 7.20 - 7.29 (1H, m), 7.48 (1H, dd, J = 8.4, 7.5 Hz).

### Reference Example 11

### 2-Chloro-6-(2-(3-methylphenyl)ethoxy)pyridine

The titled compound was obtained using 2-(3-methylphenyl)ethanol in the same manner as in Reference Example 7. Yield: 97%. Oily substance.
¹H-NMR (CDCl₃ ) δ : 2.33 (3H, s), 3.03 (2H, t, J = 7.2 Hz), 4.49 (2H, t, J = 7.2 Hz), 6.62 (1H, d, J = 8.1 Hz), 6.86 (1H, d, J = 7.5 Hz), 7.00-7.20 (4H, m), 7.47 (1H, t, J = 8.1 Hz).

### Reference Example 12

### 2-Chloro-6-(2-(2,4-dichlorophenyl)ethoxy)pyridine

The titled compound was obtained using 2-(2,4-dichlorophenyl)ethanol in the same manner as in Reference Example 7. Yield: 67%. Oily substance.
¹H-NMR (CDCl₃ ) δ : 3.18 (2H, t, J = 6.9 Hz), 4.50 (2H, t, J = 6.9 Hz), 6.61 (1H, d, J = 8.1 Hz), 6.88 (1H, d, J = 7.5 Hz), 7.17 (1H, dd, J = 8.4, 2.1 Hz), 7.22 (1H, s), 7.37 (1H, t, J = 2.1 Hz), 7.49 (1H, t, J = 7.8 Hz).

### Reference Example 13

### 2-Chloro-6-(2-(3-methoxyphenyl)ethoxy)pyridine

The titled compound was obtained using 2-(3-methoxyphenyl)ethanol in the same manner as in Reference Example 7. Yield: 67%. Oily substance.
¹H-NMR (CDCl₃ ) δ : 3.05 (2H, t, J = 6.9 Hz), 3.80 (3H, s), 4.50 (2H, t, J = 6.9 Hz), 6.62 (1H, d, J = 8.1 Hz), 6.74 - 6.89 (4H, m), 7.21 (1H, t, J = 8.1 Hz), 7.47 (1H, t, J = 7.5 Hz).

### Reference Example 14

### 2-Chloro-6-(2-((2,4-dichlorobenzyl)oxy)pyridine

The titled compound was obtained using (2,4-dichlorophenyl)methanol in the same manner as in Reference Example 7. Yield: 51 %. Oily substance.
¹H-NMR (CDCl₃ ) δ : 5.42 (2H, s), 6.72 (1H, d, J = 8.1 Hz), 6.93 (1H, d, J = 7.5 Hz), 7.22 - 7.28 (1H, m), 7.41 (1H, d, J = 2.7 Hz), 7.47 (1H, d, J = 8.4 Hz), 7.54 (1H, t, J = 7.8 Hz).

### Reference Example 15

### 3-Chloro-5-(2-(3,4-dimethoxyphenyl)ethoxy)pyridine

The titled compound was obtained using 5-chloropyridin-3-ol in the same manner as in Reference Example 7. Yield: 78%. Melting point: 101 - 102°C. (Ethyl acetate-hexane).
¹H-NMR (CDCl₃ ) δ : 3.06 (2H, t, J = 6.9 Hz), 3.87 (3H, s), 3.88 (3H, s), 4.19 (2H, t, J = 6.9 Hz), 6.77 - 6.82 (3H, m), 7.16- 7.20 (1H, m), 8.15-8.20 (2H, m).

### Reference Example 16

### 2-Chloro-4-(2-(3,4-dimethoxyphenyl)ethoxy)pyridine

A DMF (10 mL) suspension of 2-chloro-4-nitropyridine (1.10 g, 6.31 mmol) and 2-(3,4-dimethoxyphenyl)ethanol (1.23 g, 6.75 mmol) was added at 0°C to a DMF (30 mL) suspension of sodium hydride (60% liquid paraffin dispersion, 504 mg, 12.6 mmol), and the mixture was stirred for 1 hour at room temperature. Water was added to the reaction solution, and the product was extracted with ethyl acetate. The combined extract was washed with water, dried over magnesium sulfate, and then concentrated at reduced pressure, and the resulting residue was purified by silica gel column chromatography (hexane-ethyl acetate 1:1) to give 1.87 g of the titled compound (yield: quantitative). Melting point: 116 - 117°C. (Ethyl acetate-hexane).
¹H-NMR (CDCl₃ ) δ : 3.05 (2H, t, J = 6.9 Hz), 3.87 (3H, s), 3.88 (3H, s), 4.19 (2H, t, J = 6.9 Hz), 6.70 - 6.83 (5H, m), 8.17 (1H, d, J = 5.7 Hz).

### Reference Example 17

### 2-(2-(3,4-Dimethoxyphenyl)ethoxy)-4-iodopyridine

Sodium hydride (60% liquid paraffin dispersion, 358 mg, 8.96 mmol) was added at 0°C to a DMF (3 mL) solution of 2-fluoro-4-iodopyridine (1.0 g, 4.48 mmol) and 2-(3,4-dimethoxyphenyl)ethanol (0.9 g, 5.0 mmol), and the mixture was stirred for 16 hours at room temperature. Water was added to the reaction solution, and the product was extracted with ethyl acetate. The combined extract was washed with water, dried over magnesium sulfate, and then concentrated at reduced pressure, and the resulting residue was purified by silica gel column chromatography (hexane-ethyl acetate 1:1) to give 0.88 g of the titled compound (yield: 51 %). Oily substance.
¹H-NMR (CDCl₃ ) δ : 3.00 (2H, t, J = 6.9 Hz), 3.86 (3H, s), 3.87 (3H, s), 4.46 (2H, t, J = 6.9 Hz), 6.77 - 6.80 (3H, m), 7.13 - 7.21 (2H, m), 7.79 (1H, d, J = 5.4 Hz).

### Reference Example 18

### 2-(2-(3,4-Dimethoxyphenyl)ethoxy)-6-(3-nitrophenyl)pyridine

The titled compound was obtained using 2-chloro-6-(2-(3,4-dimethoxyphenyl)ethoxy)pyridine obtained in Reference Example 7 in the same manner as in Reference Example 2. Yield: 92%.
¹H-NMR (CDCl₃ ) δ : 3.10 (2H, t, J = 7.2 Hz), 3.86 (3H, s), 3.88 (3H, s), 4.64 (2H, t, J = 7.2 Hz), 6.75 (1H, d, J = 8.1 Hz), 6.83 - 6.91 (3H, m), 7.39 (1H, d, J = 7.5 Hz), 7.60 (1H, t, J = 7.5 Hz), 7.67 (1H, t, J = 7.5 Hz), 8.19 - 8.24 (1H, m), 8.30 - 8.36 (1H, m), 8.88 (1H, s).

### Reference Example 19

### 3-(6-(2-(3,4-Dimethoxyphenyl)ethoxy)pyridin-2-yl)aniline

The titled compound was obtained using 2-(2-(3,4-dimethoxyphenyl)ethoxy)-6-(3-nitrophenyl)pyridine obtained in Reference Example 18 in the same manner as in Reference Example 3. Yield: 80%. Melting point: 103 - 104°C.
¹H-NMR (CDCl₃ ) δ : 3.08 (2H, t, J = 7.2 Hz), 3.74 (2H, br s), 3.86 (3H, s), 3.87 (3H, s), 4.61(2H, t, J = 7.2 Hz), 6.67 (1H, d, J = 7.8 Hz), 6.67-6.72 (1H, m), 6.79 - 6.87 (3H, m), 7.17 - 7.29 (2H, m), 7.35 - 7.58 (2H, m), 7.51 (1H, t, J = 7.5 Hz).

### Reference Example 20

### Ethyl 3-(2-(2-(3,4-dimethoxyphenyl)ethoxy)pyrimidin-4-yl)benzoate and ethyl 3-(4-(2-(3,4-dimethoxyphenyl)ethoxy)pyrimidin-2-yl)benzoate

Sodium hydride (60% liquid paraffin dispersion, 324 mg, 8.12 mmol) was added at 0°C to a DMF (50 mL) solution of 2,4-dichloropyrimidine (1.0 g, 6.71 mmol) and 2-(3,4-dimethoxyphenyl)ethanol (1.35 g, 7.38 mmol), and the mixture was stirred for 3 hours at 60°C. Water was added to the reaction solution, and the product was extracted with ethyl acetate. An ether mixture (1.06 g) was obtained. A 2 N sodium carbonate aqueous solution (20 mL)-1,2-dimethoxyethane (20 mL) mixture of tetrakis(triphenylphosphine)palladium (0) (125 mg, 0.11 mmol), (3-(ethoxycarbonyl)phenyl)boronic acid (837 mg, 4.32 mmol), and the above mixture was reacted for 16 hours at 90°C in a nitrogen atmosphere. Water was added to the reaction solution, and the product was extracted with ethyl acetate. The combined organic layers were washed with saturated brine, then dried over anhydrous sodium sulfate, and then concentrated at reduced pressure, and the residue was purified by silica gel column chromatography (ethyl acetate-hexane 1:1) to give 840 mg (yield: 31%) of ethyl 3-(4-(2-(3,4-dimethoxyphenyl)ethoxy)pyrimidin-2-yl)benzoate and 240 mg (yield: 9%) of ethyl 3-(2-(2-(3,4-dimethoxyphenyl)ethoxy)pyrimidin-4-yl)benzoate.

### Ethyl 3-(4-(2-(3,4-dimethoxyphenyl)ethoxy)pyrimidin-2-yl)benzoate

Melting point: 101 - 102 °C. (Ethyl acetate-hexane).
¹H-NMR (CDCl₃ ) δ : 1.41 (3H, t, J = 7.2 Hz), 3.10 (2H, t, J = 7.2 Hz), 3.86(3H, s), 3.88 (3H, s), 4.42 (2H, q, J = 7.2 Hz), 4.71 (2H, t, J = 7.2 Hz), 6.64 (1H, d, J = 5.4 Hz), 6.79 - 6.90 (3H, m), 7.54 (1H, t, J = 7.5 Hz), 8.15 (1H, d, J = 7.5 Hz), 8.52 (1H, d, J = 5.7 Hz), 8.60 (1H, d, J = 8.1 Hz), 9.07 (1H, s).

### Ethyl 3-(2-(2-(3,4-dimethoxyphenyl)ethoxy)pyrimidin-4-yl)benzoate

Melting point: 94 - 95 °C. (Ethyl acetate-hexane).
¹H-NMR (CDCl₃ ) δ : 1.42 (3H, t, J = 7.2 Hz), 3.14 (2H, t, J = 6.9 Hz), 3.86(3H, s), 3.88 (3H, s), 4.42 (2H, q, J = 7.2 Hz), 4.66 (2H, t, J = 6.9 Hz), 6.79 - 6.90 (3H, m), 7.41 (1H, d, J = 5.1 Hz), 7.56 (1H, t, J = 7.8 Hz), 8.17 (1H, d, J = 7.8 Hz), 8.32 (1H, d, J = 7.8 Hz), 8.57 (1H, d, J = 5.1 Hz), 8.69 (1H, s).

### Reference Example 21

3-(2-(2-(3,4-dimethoxyphenyl)ethoxy)pyrimidin-4-yl)benzoic acid 1 N sodium hydroxide aqueous solution (5 mL, 5 mmol) was added at room temperature to an ethanol (30 mL) solution of ethyl 3-(2-(2-(3,4-dimethoxyphenyl)ethoxy)pyrimidin-4-yl)benzoate (240 mg, 0.588 mmol) obtained in Reference Example 20, and the mixture was stirred for 2 hours at 60°C and then concentrated at reduced pressure. Water and hydrochloric acid were added to the reaction solution to make the aqueous layer acidic, and the product was extracted with ethyl acetate. The organic layer was washed with water and saturated brine, and then dried over anhydrous sodium sulfate. The solvent was distilled off at reduced pressure, and the resulting residue was crystallized from ethyl acetate-hexane to give 200 mg of the titled compound (yield: 89%). Melting point: 171 - 172°C (Ethyl acetate-hexane). ¹H-NMR (CDCl₃ ) δ : 3.15 (2H, t, J = 6.9 Hz), 3.85(3H, s), 3.89 (3H, s), 4.68 (2H, t, J = 6.9 Hz), 6.80 - 6.92 (3H, m), 7.43 (1H, d, J = 5.4 Hz), 7.62 (1H, t, J = 7.8 Hz), 8.25 (1H, d, J = 7.8 Hz), 8.38 (1H, d, J = 7.8 Hz), 8.62 (1H, d, J = 5.4 Hz), 8.79 (1H, s), 1H unconfirmed.

### Reference Example 22

### 3-(4-(2-(3,4-dimethoxyphenyl)ethoxy)pyrimidin-2-yl)benzoic acid

The titled compound was obtained using ethyl 3-(4-(2-(3,4-dimethoxyphenyl)ethoxy)pyrimidin-2-yl)benzoate obtained in Reference Example 20 in the same manner as in Reference Example 21. Yield: 84%. Melting point: 193 - 194°C. (Ethyl acetate-hexane).
¹H-NMR (CDCl₃ ) δ : 3.12 (2H, t, J = 7.2 Hz), 3.87(3H, s), 3.89 (3H, s), 4.74 (2H, t, J = 7.2 Hz), 6.69 (1H, d, J = 5.7 Hz), 6.82 - 6.90 (3H, m), 7.59 (1H, t, J = 7.8 Hz), 8.23 (1H, d, J = 7.5 Hz), 8.63 (1H, d, J = 5.7 Hz), 8.66 (1H, d, J = 8.1 Hz), 9.26 (1H, s), 1H unconfirmed.

### Reference Example 23

### 3-(6-(2-(3,4-dimethoxyphenyl)ethoxy)pyridin-2-yl)benzoic acid

A mixture of 2-chloro-6-(2-(3,4-dimethoxyphenyl)ethoxy)pyridine (1.76 g, 5.99 mmol) obtained in Reference Example 7, (3-(ethoxycarbonyl)phenyl)boronic acid (1.28 g, 6.59 mmol), and tetrakis(triphenylphosphine)palladium (0) (207 mg, 0.18 mmol) in 2 N sodium carbonate aqueous solution (20 mL)-1,2-dimethoxyethane (20 mL) was reacted for 16 hours at 90°C in a nitrogen atmosphere. Water was added to the reaction solution, and the product was extracted with ethyl acetate. The combined organic layers were washed with saturated brine, then dried over anhydrous sodium sulfate, and then concentrated at reduced pressure, and the residue was purified by silica gel column chromatography (ethyl acetate-hexane 2:3) to give 1.36 g of ethyl 3-(6-(2-(3,4-dimethoxyphenyl)ethoxy)pyridin-2-yl)benzoate. 1 N sodium hydroxide aqueous solution (10 mL, 10 mmol) was added at room temperature to an ethanol (50 mL) solution of this compound, and the mixture was stirred for 2 hours at 60°C and was then concentrated at reduced pressure. Water and hydrochloric acid were added to the reaction solution to make the aqueous layer acidic, and the product was extracted with ethyl acetate. The organic layer was washed with water and saturated brine, and then dried over anhydrous sodium sulfate. The solvent was distilled off at reduced pressure, and the resulting residue was crystallized from ethyl acetate-hexane to give 820 mg of the titled compound (yield: 36%). Melting point: 147 - 148°C.
¹H-NMR (CDCl₃ ) δ : 3.11 (2H, t, J = 7.2 Hz), 3.86 (3H, s), 3.88 (3H, s), 4.66 (2H, t, J = 7.2 Hz), 6.72 (1H, d, J = 8.1 Hz), 6.82 - 6.91 (3H, m), 7.41 (1H, d, J = 7.5 Hz), 7.57 (1H, t, J = 7.5 Hz), 7.66 (1H, t, J = 7.5 Hz), 8.14 (1H, dd, J = 7.5, 1.2 Hz).8.30 (1H, d, J = 6.6 Hz), 8.76 (1H, s), 1H unconfirmed.

### Reference Example 24

### 3'-(2-(3,4-dimethoxyphenyl)ethoxy)biphenyl-3-carboxylic acid

The titled compound was obtained using 4-(2-(3-bromophenoxy)ethyl)-1,2-dimethoxybenzene obtained in Reference Example 8 was used in the same manner as in Reference Example 23. Yield: 62%. Melting point: 100 - 102°C. (Ethyl acetate-hexane).
¹H-NMR (CDCl₃ ) δ : 3.08 (2H, t, J = 7.2 Hz), 3.86 (3H, s), 3.89 (3H, s), 4.23 (2H, t, J = 7.2 Hz), 6.79 - 7.00 (4H, m), 7.05 - 7.25 (2H, m), 7.37 (1H, t, J = 8.1 Hz), 7.53 (2H, t, J = 7.5 Hz), 7.82 (1H, dd, J = 7.5, 1.2 Hz), 8.06 - 8.12 (1H, m), 8.53 (1H, m).

### Reference Example 25

### 3-(6-(2-(3-(trifluoromethyl)phenyl)ethoxy)pyridin-2-yl)benzoic acid

The titled compound was obtained using 2-chloro-6-(2-(3-(trifluoromethyl)phenyl)ethoxy)pyridine obtained in Reference Example 9 in the same manner as in Reference Example 23. Yield: 45%. Melting point: 135 - 136°C. (Ethyl acetate-hexane).
¹H-NMR (CDCl₃ ) δ : 3.21 (2H, t, J = 6.9 Hz), 4.69 (2H, t, J = 6.9 Hz), 6.71 (1H, d, J = 8.4 Hz), 7.40 - 7.72 (7H, m), 8.14 (1H, d, J = 7.5 Hz), 8.29 (1H, d, J = 8.4 Hz), 8.75 (1H, s), 1H unconfirmed.

### Reference Example 26

### 3-(6-(2-(3-fluorophenyl)ethoxy)pyridin-2-yl)benzoic acid

The titled compound was obtained using 2-chloro-6-(2-(3-fluorophenyl)ethoxy)pyridine obtained in Reference Example 10 in the same manner as in Reference Example 23. Yield: 46%. Melting point: 142 - 143°C. (Ethyl acetate-hexane).
¹H-NMR (CDCl₃ ) δ : 3.16 (2H, t, J = 6.9 Hz), 4.67 (2H, t, J = 6.9 Hz), 6.72 (1H, d, J = 7.8 Hz), 6.88 - 6.96 (1H, m), 7.05 (1H, d, J = 10.2 Hz), 7.12 (1H, d, J = 7.8 Hz), 7.23 - 7.32 (1H, m), 7.41 (1H, d, J = 7.2 Hz), 7.57 (1H, t, J = 7.8 Hz), 7.66 (1H, t, J = 7.5 Hz), 8.14 (1H, d, J = 7.8 Hz), 8.29 (1H, d, J = 8.1 Hz), 8.76 (1H, s), 1H unconfirmed.

### Reference Example 27

### 3-(6-(2-(3-methylphenyl)ethoxy)pyridin-2-yl)benzoic acid

The titled compound was obtained using 2-chloro-6-(2-(3-methylphenyl)ethoxy)pyridine obtained in Reference Example 11 in the same manner as in Reference Example 23. Yield: 27%. Melting point: 125 - 126°C. (Ethyl acetate-hexane).
¹H-NMR (CDCl₃ ) δ : 2.35 (3H, s), 3.13 (2H, t, J = 7.2 Hz), 4.66 (2H, t, J = 7.2 Hz), 6.72 (1H, d, J = 8.1 Hz), 7.05 (1H, d, J = 6.3 Hz), 7.14 - 7.25 (3H, m), 7.40 (1H, d, J = 7.8 Hz), 7.57 (1H, t, J = 8.1 Hz), 7.66 (1H, t, J = 7.5 Hz), 8.14 (1H, d, J = 7.5 Hz), 8.32 (1H, d, J = 7.8 Hz), 8.76 (1H, s), 1H unconfirmed.

### Reference Example 28

### 3-(6-(2-(2,4-dichlorophenyl)ethoxy)pyridin-2-yl)benzoic acid

The titled compound was obtained using 2-chloro-6-(2-(2,4-dichlorophenyl)ethoxy)pyridine obtained in Reference Example 12 in the same manner as in Reference Example 23. Yield: 40%. Melting point: 199 - 200°C. (Ethyl acetate-hexane).
¹H-NMR (CDCl₃ ) δ : 3.27 (2H, t, J = 6.9 Hz), 4.68 (2H, t, J = 6.9 Hz), 6.70 (1H, d, J = 8.1 Hz), 7.18 - 7.26 (1H, m), 7.30- 7.43 (3H, m), 7.57 (1H, t, J = 7.8 Hz), 7.66 (1H, t, J = 7.8 Hz), 8.15 (1H, d, J = 7.8 Hz), 8.29 (1H, d, J = 7.8 Hz), 8.77 (1H, s), 1H unconfirmed.

### Reference Example 29

### 3-(6-(2-(3-methoxyphenyl)ethoxy)pyridin-2-yl)benzoic acid

The titled compound was obtained using 2-chloro-6-(2-(3-methoxyphenyl)ethoxy)pyridine obtained in Reference Example 13 in the same manner as in Reference Example 23. Yield: 40%. Melting point: 156 - 157°C. (Ethyl acetate-hexane).
¹H-NMR (CDCl₃ ) δ : 3.14 (2H, t, J = 7.2 Hz), 3.80 (3H, s), 4.67 (2H, t, J = 7.2 Hz), 6.72 (1H, d, J = 7.2 Hz), 6.75 - 6.90 (1H, m), 6.87 - 6.90 (1H, m), 6.94 (1H, t, J = 7.8 Hz), 7.25 (1H, t, J = 7.8 Hz), 7.40 (1H, d, J = 7.5 Hz), 7.57 (1H, t, J = 7.8 Hz), 7.65 (1H, t, J = 7.5 Hz), 8.10 - 8.16 (1H, m), 8.28 - 8.33 (1H, m), 8.75 (1H, t, J = 1.5 Hz), 1H unconfirmed.

### Reference Example 30

### 3-(6-((2,4-dichlorobenzyl)oxy)pyridin-2-yl)benzoic acid

The titled compound was obtained using 2-chloro-6-(2-((2,4-dichlorobenzyl)oxy)pyridine obtained in Reference Example 14 in the same manner as in Reference Example 23. Yield: 49%. Melting point: 186 - 187°C. (Ethyl acetate-hexane).
¹H-NMR (CDCl₃ ) δ : 5.60 (2H, s), 6.82 (1H, d, J = 8.4 Hz), 7.22 - 7.27 (1H, m), 7.42 - 7.61 (4H, m), 7.71 (1H, d, J = 8.1 Hz), 8.14 (1H, t, J = 7.8 Hz), 8.28 (1H, d, J = 8.1 Hz).8.71 (1H, t, J = 1.8 Hz), 1H unconfirmed.

### Reference Example 31

### 3-(4-(2-(3,4-dimethoxyphenyl)ethoxy)pyridin-2-yl)benzoic acid

The titled compound was obtained using 2-chloro-4-(2-(3,4-dimethoxyphenyl)ethoxy)pyridine obtained in Reference Example 16 in the same manner as in Reference Example 23. Yield: 67%. Melting point: 194 - 195°C. (Ethyl acetate-hexane).
¹H-NMR (CDCl₃ ) δ : 3.11 (2H, t, J = 6.9 Hz), 3.87(3H, s), 3.90 (3H, s), 4.31 (2H, t, J = 6.9 Hz), 6.70 -6.90 (4H, m), 7.29 (1H, d, J = 2.4 Hz), 7.56 (1H, t, J = 7.5 Hz), 8.04 (1H, d, J = 7.8 Hz), 8.19 (1H, d, J = 7.5 Hz).8.81 (1H, d, J = 6.0 Hz), 9.01 (1H, s), 1H unconfirmed.

### Reference Example 32

### 3-(5-(2-(3,4-dimethoxyphenyl)ethoxy)pyridin-3-yl)benzoic acid

The titled compound was obtained using 3-chloro-5-(2-(3,4-dimethoxyphenyl)ethoxy)pyridine obtained in Reference Example 15 in the same manner as in Reference Example 23. Yield: 47%. Melting point: 181 - 182°C. (Ethyl acetate-hexane).
¹H-NMR (CDCl₃ ) δ : 3.11 (2H, t, J = 6.6 Hz), 3.86(3H, s), 3.89 (3H, s), 4.31 (2H, t, J = 6.6 Hz), 6.81 - 6.85 (3H, m), 7.47 (1H, s), 7.58 (1H, t, J = 7.8 Hz), 7.79 (1H, d, J = 7.2 Hz), 8.16 (1H, d, J = 7.8 Hz), 8.37 (1H, s), 8.41 (1H, s), 8.57 (1H, s), 1H unconfirmed.

### Reference Example 33

### 3-(2-(2-(3,4-dimethoxyphenyl)ethoxy)pyridin-4-yl)benzoic acid

The titled compound was obtained using 2-(2-(3,4-dimethoxyphenyl)ethoxy)-4-iodopyridine obtained in Reference Example 17 in the same manner as in Reference Example 23. Yield: 58%. Melting point: 148 - 149°C. (Ethyl acetate-hexane).
¹H-NMR (CDCl₃ ) δ : 3.08 (2H, t, J = 6.9 Hz), 3.86(3H, s), 3.88 (3H, s), 4.57 (2H, t, J = 6.9 Hz), 6.80 - 6.87 (3H, m), 6.99 (1H, s), 7.14 (1H, dd, J = 5.4, 1.8 Hz), 7.59 (1H, t, J = 7.8 Hz), 7.85 (1H, dd, J = 5.4, 1.8 Hz), 8.17 (1H, dd, J = 7.8, 1.2 Hz), 8.24 (1H, d, J = 5.1 Hz), 8.36 (1H, s), 1H unconfirmed.

### Reference Example 34

### Methyl 3-(6-(2,4-dichlorophenoxy)pyridin-2-yl)benzoate

A DMF (138 mL) mixture of 2,4-dichlorophenol (5.0 g, 30.7 mmol), 2,6-dichloropyridine (4.1 g, 27.9 mmol), and potassium carbonate (4.2 g, 30.7 mmol) was added at room temperature, the mixture was stirred for 16 hours at 30°C, cold water (300 mL) was then poured in, and the product was extracted with ethyl acetate. The organic layer was washed with saturated brine and dried over sodium sulfate. The solvent was distilled off at reduced pressure, and the residue was purified by silica gel column chromatography (ethyl acetate-hexane 3:7) to give a crude product of 2-chloro-6-(2,4-dichlorophenoxy)pyridine. A 2 N sodium carbonate aqueous solution (20 mL)-1,2-dimethoxyethane (20 mL) mixture of the above compound (3.9 g), (3-(methoxycarbonyl)phenyl)boronic acid (2.8 g, 15.6 mmol), and tetrakis(triphenylphosphine)palladium (0) (492 mg, 0.43 mmol) was reacted for 16 hours at 90°C in a nitrogen atmosphere. Water was added to the reaction solution, and the product was extracted with ethyl acetate. The combined organic layers were washed with saturated brine, then dried over anhydrous sodium sulfate, and then concentrated at reduced pressure, and the residue was purified by silica gel column chromatography (ethyl acetate-hexane 2:3) to give 2.4 g of the titled compound (yield 23%). Melting point: 112 - 113°C. (Ethyl acetate-hexane).
¹H-NMR (CDCl₃ ) δ : 3.93 (3H, s), 6.93 (1H, d, J = 8.4 Hz), 7.21 - 7.33 (2H, m), 7.45 (1H, t, J = 7.8 Hz), 7.51 (1H, d, J = 2.4 Hz), 7.55 (1H, t, J = 7.5 Hz), 7.80 (1H, t, J = 7.5 Hz), 8.01 (2H, dt, J = 7.5, 1.5 Hz), 8.48 (1H, t, J = 1.5 Hz).

### Reference Example 35

### Methyl 3 -(6-(3,5-dichlorophenoxy)pyridin-2-yl)benzoate

The titled compound was obtained using 3,5-dichlorophenol in the same manner as in Reference Example 34. Yield: 38%. Melting point: 125 - 126°C. (Ethyl acetate-hexane).
¹H-NMR (CDCl₃ ) δ : 3.94 (3H, s), 6.90 (1H, d, J = 7.8 Hz), 7.18 (2H, d, J = 1.8 Hz), 7.22 (1H, d, J = 1.8 Hz), 7.50 (1H, t, J = 7.8 Hz), 7.60 (1H, d, J = 7.8 Hz), 7.82 (1H, t, J = 7.8 Hz), 8.03-8.12 (2H, m), 8.55 (1H, s).

### Reference Example 36

### Methyl 3-(6-(2,3-dichlorophenoxy)pyridin-2-yl)benzoate

The titled compound was obtained using 2,3-dichlorophenol in the same manner as in Reference Example 34. Yield: 28%. Melting point: 105 - 106°C. (Ethyl acetate-hexane).
¹H-NMR (CDCl₃ ) δ : 3.93 (3H, s), 6.93 (1H, d, J = 8.1 Hz), 7.23- 7.30 (2H, m), 7.38 (1H, dd, J = 7.5, 1.8 Hz), 7.44 (1H, t, J = 7.8 Hz), 7.55 (1H, d, J = 7.8 Hz), 7.80 (1H, t, J = 7.8 Hz), 7.97-8.03 (2H, m), 8.55 (1H, t, J = 1.5 Hz).

### Reference Example 37

### Methyl 3-(6-(3,4-dichlorophenoxy)pyridin-2-yl)benzoate

The titled compound was obtained using 3,4-dichlorophenol in the same manner as in Reference Example 34. Yield: 16%. Melting point: 133 - 134°C. (Ethyl acetate-hexane).
¹H-NMR (CDCl₃ ) δ : 3.94 (3H, s), 6.89 (1H, d, J = 7.8 Hz), 7.11 (1H, dd, J = 8.7, 3.0 Hz), 7.40 (1H, d, J = 3.0 Hz), 7.45- 7.53 (2H, m), 7.58 (1H, d, J = 7.2 Hz), 7.80 (1H, t, J = 7.8 Hz), 8.01 - 8.10 (2H, m), 8.53 (1H, s).

### Reference Example 38

### 3-(6-(2,4-dichlorophenoxy)pyridin-2-yl)benzoic acid

The titled compound was obtained using methyl 3-(6-(2,4-dichlorophenoxy)pyridin-2-yl)benzoate obtained in Reference Example 32 in the same manner as in Reference Example 21. Yield: 78%. Melting point: 244 - 245°C. (Ethyl acetate-hexane).
¹H-NMR (DMSO-d₆) δ : 7.11 (1H, d, J = 8.1 Hz), 7.42- 7.59 (3H, m), 7.72- 7.82 (2H, m), 7.91 - 8.07 (3H, m), 8.38 (1H, s), 1H unconfirmed.

### Reference Example 39

### 3-(6-(3,5-dichlorophenoxy)pyridin-2-yl)benzoic acid

The titled compound was obtained using methyl 3-(6-(3,5-dichlorophenoxy)pyridin-2-yl)benzoate obtained in Reference Example 33 in the same manner as in Reference Example 21. Yield: 77%. Melting point: 234 - 235°C. (Ethyl acetate-hexane).
¹H-NMR (DMSO-d₆ ) δ: 7.10 (1H, d, J = 8.1 Hz), 7.41 (2H, d, J = 1.8 Hz), 7.49 (1H, d, J = 1.8 Hz), 7.58 (1H, t, J = 7.8 Hz), 7.85 (1H, d, J = 7.8 Hz), 7.96 (1H, d, J = 8.1 Hz), 8.01 (1H, t, J = 8.1 Hz), 8.13 (1H, d, J = 8.1 Hz), 8.46 (1H, s), 13.0 (1H, br s).

### Reference Example 40

### 3-(6-(2,3-dichlorophenoxy)pyridin-2-yl)benzoic acid

The titled compound was obtained using methyl 3-(6-(2,3-dichlorophenoxy)pyridin-2-yl)benzoate obtained in Reference Example 34 in the same manner as in Reference Example 21. Yield: 90%. Melting point: 216 - 217°C. (Ethyl acetate-hexane).
¹H-NMR (CDCl₃ ) δ : 6.94 (1H, d, J = 7.8 Hz), 7.20 - 7.31 (2H, m), 7.39 (1H, dd, J = 7.5, 1.2 Hz), 7.48 (1H, t, J = 7.8 Hz), 7.57 (1H, d, J = 7.8 Hz), 7.82 (1H, t, J = 7.8 Hz), 8.07 (2H, t, J = 7.8 Hz), 8.55 (1H, s), 1H unconfirmed.

### Reference Example 41

### 3-(6-(3,4-Dichlorophenoxy)pyridin-2-yl)benzoic acid

The titled compound was obtained using methyl 3-(6-(3,4-dichlorophenoxy)pyridin-2-yl)benzoate obtained in Reference Example 35 in the same manner as in Reference Example 21. Yield: 91 %. Melting point: 230 - 231°C. (Ethyl acetate-hexane).
¹H-NMR (DMSO-d₆) δ : 7.09 (1H, d, J = 8.1 Hz), 7.29 (1H, dd, J = 8.7, 2.7 Hz), 7.58 (1H, t, J = 7.8 Hz), 7.64 (1H, d, J = 2.7 Hz), 7.70 (1H, t, J = 9.0 Hz), 7.83 (1H, d, J = 7.8 Hz), 7.93-8.04 (2H, m), 8.13 (1H, d, J = 7.8 Hz), 8.46 (1H, s), 1H unconfirmed.

### Reference Example 42

### 3-Bromo-N-(2-cyanoethyl)benzamide

WSC (5.73 g, 29.9 mmol) and HOBt (4.04 g, 29.9 mmol) were added to a DMF (25 mL) solution of 3-bromobenzoic acid (5.00 g, 24.9 mmol) and 3-aminopropanenitrile (2.21 mL, 29.9 mmol), and the mixture was stirred for 15 hours at room temperature. The addition of saturated aqueous sodium bicarbonate to the reaction solution was followed by extraction with ethyl acetate. The extract was washed with water and dried over anhydrous magnesium sulfate, and the solvent was then distilled off at reduced pressure. Diethyl ether was added to the residue, and the crystals were filtered off to give 5.66 g of the titled compound (yield: 90%). Melting point: 100 - 101°C (ethyl acetate).
¹H-NMR (CDCl₃ ):δ 2.76 (2H, t, J = 6.2 Hz), 3.71 (2H, q, J = 6.2 Hz), 6.82 (1H, br s), 7.32 (1H, t, J = 7.9 Hz), 7.58- 7.74 (2H, m), 7.94 (1H, t, J = 1.8 Hz).

### Reference Example 43

### N-(2-Cyanoethyl)-3-(4,4,5,5-tetramethyl-1,3,2-dioxaborolan-2-yl)benzamide

(1,1-Bis(diphenylphosphino)ferrocene)dichloropalladium (II) complex with dichloromethane (327 mg, 0.40 mmol) was added to a mixture of 3-bromo-N-(2-cyanoethyl)benzamide (2.00 g, 7.90 mmol) obtained in Working Example 42, bis(pinacolato)diboron (2.41 g, 9.48 mmol), potassium acetate (930 mg, 9.48 mmol), and DMSO (45 mL), and the mixture was heated and stirred for 15 hours at 90°C. The reaction solution was poured into ethyl acetate and was washed with 1 M magnesium sulfate aqueous solution and saturated brine. The solution was then dried over anhydrous magnesium sulfate, and the solvent was then distilled off at reduced pressure. The residue was purified by silica gel column chromatography to give 1.60 g of the titled compound (yield: 67%) in the form of an oily substance.
¹H-NMR (DMSO- d₆ ):δ 1.36 (12H, s), 2.72-2.80 (2H, m), 3.71 (2H, q, J = 6.4 Hz), 6.85 (1H, br s), 7.46 (1H, t, J = 7.6 Hz), 7.96 (2H, dd, J = 7.6, 1.9 Hz), 8.12 (1H, s).

### Reference Example 44

### 2-Chloro-6-(2,4-dimethylphenoxy)pyridine

2,4-dimethylphenol (0.326 mL, 2.70 mmol) was added to a mixture of 2,6-dichloropyridine (400 mg, 2.70 mmol) and potassium carbonate (373 mg, 2.70 mmol), and the mixture was heated and stirred for 6 hours at 130°C. The addition of water to the reaction solution was followed by extraction with ethyl acetate. The extract was washed with water and dried over anhydrous magnesium sulfate, and the solvent was then distilled off at reduced pressure. The residue was purified by silica gel column chromatography to give 510 mg of the titled compound (yield: 81 %) in the form of an oily substance.
¹H-NMR (CDCl₃ ):δ 2.14 (3H, s), 2.33 (3H, s), 6.59 (1H, d, J = 8.1 Hz), 6.90 - 6.95 (1H, m), 6.98 (1H, d, J = 7.5 Hz), 7.00 - 7.04 (1H, m), 7.07 (1H, s), 7.56 (1H, t, J = 7.8 Hz).

### Reference Example 45

### 2-Chloro-6-(2,4-difluorophenoxy)pyridine

The titled compound was synthesized using 2,4-difluorophenol in the same manner as in Reference Example 44. Yield: 93%.
¹H-NMR (CDCl₃ ):δ 6.84 - 6.99 (3H, m), 7.04 (1H, d, J = 7.6 Hz), 7.14-7.24 (1H, m), 7.61 - 7.68 (1H, m)

### Reference Example 46

### 2-Chloro-6-(4-chlorophenoxy)pyridine

The titled compound was synthesized using 4-chlorophenol in the same manner as in Reference Example 44. Yield: 96%.
¹H-NMR (CDCl₃ ):δ 6.79 (1H, d, J = 8.1 Hz), 7.05 (1H, d, J = 7.5 Hz), 7.07- 7.13 (2H, m), 7.32- 7.40 (2H, m), 7.63 (1H, t, J = 7.8 Hz).

### Reference Example 47

### 2-Chloro-6-(3-chlorophenoxy)pyridine

The titled compound was synthesized using 3-chlorophenol in the same manner as in Reference Example 44. Yield: 90%.
¹H-NMR (CDCl₃ ):δ 6.80 (1H, d, J = 8.1 Hz), 7.01 - 7.11 (2H, m), 7.14-7.24 (2H, m), 7.32 (1H, t, J = 8.1 Hz), 7.65 (1H, t, J = 7.8 Hz).

### Reference Example 48

### 2-Chloro-6-(2-chlorophenoxy)pyridine

The titled compound was synthesized using 2-chlorophenol in the same manner as in Reference Example 44. Yield: 97%.
¹H-NMR (CDCl₃ ):δ 6.80 (1H, d, J = 8.0 Hz), 7.03 (1H, d, J = 7.6 Hz), 7.15 - 7.24 (2H, m), 7.27 - 7.35 (1H, m), 7.46 (1H, dd, J = 7.8, 1.3 Hz), 7.63 (1H, t, J = 8.0 Hz)

### Reference Example 49

### 2-Chloro-6-(3-methoxyphenoxy)pyridine

The titled compound was synthesized using 2-chlorophenol in the same manner as in Reference Example 44. Yield: 75%.
¹H-NMR (CDCl₃ ):δ 3.80 (3H, s), 6.67 - 6.80 (4H, m), 7.03 (1H, d, J = 7.6 Hz), 7.25 - 7.33 (1H, m), 7.61 (1H, t, J = 8.0 Hz).

### Reference Example 50

### 2-Chloro-6-(cyclohexyloxy)pyridine

After all of a toluene solution of diethyl azodicarboxylate (2.2 mol/l, 2.10 mL, 4.63 mmol) had been added in the form of drops at 0°C to a THF (10 mL) solution of 6-chloropyridin-2-ol (500 mg, 3.86 mmol), cyclohexanol (0.449 mL, 4.25 mmol), and triphenylphosphine (1.11g, 4.25 mmol), the mixture was stirred for 15 hours at room temperature. The solvent was distilled off at reduced pressure, and he residue was purified by silica gel column chromatography to give 225 mg of the titled compound (yield: 28%) in the form of an oily substance.
¹H-NMR (CDCl₃ ):δ 1.20 - 1.66 (6H, m), 1.71 - 1.85 (2H, m), 1.93 - 2.05 (2H, m), 4.95 - 5.08 (1H, m), 6.59 (1H, dd, J = 8.2, 0.7 Hz), 6.83 (1H, d, J = 7.5 Hz), 7.42- 7.52 (1H, m).

### Reference Example 51

### 5-((6-chloropyridin-2-yl)oxy)-1H-indole

The titled compound was synthesized using 1H-indol-5-ol in the same manner as in Reference Example 44. Yield: 26%. Melting point: 117 - 118°C (ethyl acetate).
¹H-NMR (CDCl₃ ):δ 6.53 (1H, t, J = 2.7 Hz), 6.64 (1H, d, J = 8.3 Hz), 6.93- 7.02 (2H, m), 7.22- 7.28 (1H, m), 7.37 (1H, d, J = 5.7 Hz), 7.39 (1H, s), 7.54 (1H, t, J = 8.0 Hz), 8.31 (1H, br s).

### Reference Example 52

### 2-Chloro-6-(2,3-dihydro-1H-inden-5-yloxy)pyridine

The titled compound was synthesized using indan-5-ol in the same manner as in Reference Example 44. Yield: 83%.
¹H-NMR (CDCl₃ ):δ 2.03-2.17 (2H, m), 2.84-2.97 (4H, m), 6.69 (1H, d, J = 8.3 Hz), 6.88 (1H, dd, J = 8.0, 2.3 Hz), 6.94- 7.04 (2H, m), 7.21 (1H, d, J = 8.0 Hz), 7.53- 7.65 (1H, m).

### Reference Example 53

### 1-(3-((6-Chloropyridin-2-yl)oxy)phenyl)ethanone

The titled compound was synthesized using 1-(3-hydroxyphenyl)ethanone in the same manner as in Reference Example 44. Yield: 80%. Melting point: 82 - 83°C (ethyl acetate).
¹H-NMR (CDCl₃ ):δ 2.61 (3H, s), 6.83 (1H, d, J = 8.1 Hz), 7.07 (1H, d, J = 7.2 Hz), 7.33- 7.40 (1H, m), 7.50 (1H, t, J = 7.9 Hz), 7.66 (1H, t, J = 7.9 Hz), 7.71 - 7.74 (1H, m), 7.78- 7.84 (1H, m).

### Reference Example 54

### 3-((6-chloropyridin-2-yl)oxy)-N,N-dimethylaniline

The titled compound was synthesized using 3-(dimethylamino)phenol in the same manner as in Reference Example 42. Yield: 43%. Melting point: 71 - 72°C (ethyl acetate).
¹H-NMR (CDCl₃ ):δ 2.95 (6H, s), 6.43-6.50 (2H, m), 6.54-6.60 (1H, m), 6.68 (1H, d, J = 8.3 Hz), 7.01 (1H, d, J = 7.5 Hz), 7.19- 7.27 (1H, m), 7.53- 7.60 (1H, m).

### Reference Example 55

### 2-Chloro-6-((5-chloropyridin-3-yl)oxy)pyridine

The titled compound was synthesized using 5-chloropyridin-3-ol in the same manner as in Reference Example 44. Yield: 67%. Melting point: 46 - 47°C (ethyl acetate).
¹H-NMR (CDCl₃ ):δ 6.92 (1H, d, J = 8.1 Hz), 7.11 (1H, d, J = 7.5 Hz), 7.58 (1H, t, J = 2.3 Hz), 7.70 (1H, t, J = 7.8 Hz), 8.41 (1H, d, J = 2.4 Hz), 8.44 (1H, d, J = 2.1 Hz).

### Reference Example 56

### 2-Chloro-6-(2,5-dichlorophenoxy)pyridine

The titled compound was synthesized using 2,5-dichlorophenol in the same manner as in Reference Example 44. Yield: 81 %. Melting point: 82 - 84°C (ethyl acetate).
¹H-NMR (CDCl₃ ):δ 6.87 (1H, d, J = 8.1 Hz), 7.07 (1H, d, J = 7.7 Hz), 7.14- 7.20 (1H, m), 7.23 (1H, d, J = 2.4 Hz), 7.39 (1H, d, J = 8.7 Hz), 7.63- 7.70 (1H, m).

### Reference Example 57

### 2-Chloro-6-(2,4-dichlorophenoxy)pyridine

A DMF (45 mL) solution of 2,6-dichloropyridine (3.00 g, 20.3 mmol), 2,4-dichlorophenol (3.47 g, 21.3 mmol), and potassium carbonate (3.08 g, 22.3 mmol) was stirred at 120°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:9) to give 5.10 g of the titled compound (yield 92%) in the form of an oily substance.
¹H-NMR (CDCl₃ ) δ: 6.86 (1H, d, J = 8.1 Hz), 7.04 (1H, d, J = 7.5 Hz ), 7.12 - 7.18 (1H, m), 7.23 - 7.31 (1H, m), 7.47 (1H, d, J = 2.4 Hz ), 7.61 - 7.68 (1H, m).

### Reference Example 58

### 2-Chloro-6-((2,4-dichlorophenyl)thio)pyridine

A DMF (30 mL) solution of 2,4-dichloropyridine (2.00 g, 13.5 mmol), 2,4-dichlorothiophenol (1.76 mL, 14.2 mmol), and potassium carbonate (2.05 g, 14.9 mmol) was stirred at 120°C for 5 hours. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:9) to give 1.68 g of the titled compound (yield 43%) in the form of an oily substance.
¹H-NMR (CDCl₃ ) δ: 6.73 - 6.80 (1H, m), 7.03 - 7.10 (1H, m), 7.28 - 7.34 (1H, m), 7.40 - 7.49 (1H, m), 7.54 - 7.66 (2H, m).

### Reference Example 59

### 2-Chloro-6-(2,4-dichlorophenoxy)-4-(trifluoromethyl)pyridine

The titled compound was obtained in the form of an oily substance using 2,6-dichloro-4-(trifluoromethyl)pyridine in the same manner as in Reference Example 1. Yield: 82%.
¹H-NMR (CDCl₃ ) δ: 7.07 - 7.20 (2H, m), 7.22 - 7.36 (2H, m), 7.50 (1H, d, J = 2.4 Hz).

### Reference Example 60

### 2,2,2-Trichloroethyl (6-fluoropyridin-3-yl)carbamate

2,2,2-Trichloroethyl chlorocarbonate (0.406 mL, 2.95 mmol) was added in the form of drops at 0°C to a THF (10 mL) solution of 6-fluoropyridin-3-amine (300 mg, 2.68 mmol) and triethylamine (0.213 mL, 2.95 mmol), and the mixture was then stirred for 1 hour at 0°C. The addition of water to the reaction solution was followed by extraction with ethyl acetate. The extract was dried over anhydrous magnesium sulfate, and the solvent was then distilled off at reduced pressure. Hexane was added to the resulting crystals, which were filtered off to give 710 mg of the titled compound (yield: 92%). Melting point: 114 - 115°C (ethyl acetate).
¹H-NMR (CDCl₃ ):δ 4.84 (2H, s), 6.96 (1H, dd, J = 8.9, 3.2 Hz), 7.05 (1H, br s), 8.08 (1H, br s), 8.19(1H, s).

### Reference Example 61

### Ethyl 3-bromo-5-fluorobenzoate

An ethanol (40 mL) solution of 3-bromo-5-fluorobenzoic acid (2.00 g, 9.13 mmol) and concentrated sulfuric acid(2.0 ml) was heated to reflux over night. Water was poured into the reaction solution, and the product was extracted with ethyl acetate. The extract was washed with water and dried over anhydrous magnesium sulfate, and the solvent was distilled off at reduced pressure to give 1.89 g of the titled compound (yield: 84%) in the form of an oily substance.
¹H-NMR (CDCl₃ ) δ: 1.40 (3H, t, J = 7.2 Hz), 4.39 (2H, q, J = 7.2 Hz), 7.40 - 7.47 (1H, m), 7.64 - 7.70 (1H, m), 7.96 - 8.00 (1H, m).

### Reference Example 62

### Ethyl 3-bromo-4-fluorobenzoate

The titled compound was obtained in the form of an oily substance using 3-bromo-4-fluorobenzoic acid in the same manner as in Reference Example 61. Yield: 88%.
¹H-NMR (CDCl₃ ) δ: 1.40 (3H, t, J = 7.2 Hz), 4.38 (2H, q, J = 7.2 Hz), 7.13 - 7.21 (1H, m), 7.94 - 8.04 (1H, m), 8.26 (1H, dd, J = 7.2, 2.1 Hz).

### Reference Example 63

### Ethyl 3-bromo-2-fluorobenzoate

The titled compound was obtained in the form of an oily substance using 3-bromo-2-fluorobenzoic acid in the same manner as in Reference Example 61. Yield: 91 %.
¹H-NMR (CDCl₃ ) δ: 1.36 - 1.44 (3H, m), 4.36 - 4.46 (2H, m), 7.05 - 7.14 (1H, m), 7.69 - 7.77 (1H, m), 7.83 - 7.91 (1H, m).

### Reference Example 64

### Ethyl 5-bromo-2-fluorobenzoate

The titled compound was obtained in the form of an oily substance using 5-bromo-2-fluorobenzoic acid in the same manner as in Reference Example 61. Yield: 92%.
¹H-NMR (CDCl₃ ) δ: 1.40 (3H, t, J = 7.2 Hz), 4.40 (2H, q, J = 7.2 Hz), 7.04 (1H, dd, J = 10.2, 8.9 Hz), 7.56 - 7.64 (1H, m), 8.05 (1H, dd, J = 8.9, 2.6 Hz).

### Reference Example 65

### Methyl 4-methyl-3-(4,4,5,5-tetramethyl-1,3,2-dioxaborolan-2-yl)benzoate

The titled compound was obtained in solid form using methyl 3-bromo-4-methylbenzoate acid in the same manner as in Reference Example 43.
Yield: 65%.
¹H-NMR (CDCl₃ ) δ: 1.35 (12H, s), 2.58 (3H, s), 3.90 (3H, s), 7.23 (1H, d, J = 7.9 Hz), 7.97 (1H, dd, J = 7.9, 2.0 Hz ), 8.40 (1H, d, J = 2.0 Hz).

### Reference Example 66

### Ethyl 3-(6-((2,4-dichlorophenyl)thio)pyridin-2-yl)benzoate

Tetrakis(triphenylphosphine)palladium (0) (802 mg, 0.694 mmol) was added at room temperature in a nitrogen atmosphere to a 1,2-dimethoxyethane (35 mL) solution of2-chloro-6-((2,4-dichlorophenyl)thio)pyridine (1.68 g, 5.78 mmol) obtained in Reference Example 58, (3-(ethoxycarbonyl)-phenyl)boronic acid (1.35 g, 6.94 mmol), and 2 N sodium carbonate aqueous solution (11.6 mL), and the mixture was stirred at 100°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:9) to give 1.13 g of the titled compound (yield 48%) in solid form.
¹H-NMR (CDCl₃ ) δ: 1.36 - 1.46 (3H, m), 4.42 (2H, q, J = 7.2 Hz), 6.91 - 6.99 (1H, m), 7.28 - 7.36 (2H, m), 7.44 - 7.76 (4H, m), 8.01 - 8.14 (2H, m), 8.50 - 8.56 (1H, m).

### Reference Example 67

### Methyl 3-(6-(2,4-dichlorophenoxy)pyridin-2-yl)-4-methylbenzoate

The titled compound was obtained in solid form using 2-chloro-6-(2,4-dichlorophenoxy)pyridine obtained in Reference Example 9 and methyl 4-methyl-3-(4,4,5,5-tetramethyl-1,3,2-dioxaborolan-2-yl)benzoate obtained in Reference Example 65 in the same manner as in Reference Example 66. Yield: 82%.
¹H-NMR (CDCl₃ ) δ: 2.24 (3H, s), 3.90 (3H, s), 6.97 (1H, d, J = 8.3 Hz), 7.13 - 7.27 (4H, m), 7.45 (1H, d, J = 2.4 Hz), 7.81 (1H, t, J = 7.9 Hz), 7.89 (1H, dd, J = 7.9, 1.7 Hz), 8.02 (1H, d, J = 1.7 Hz).

### Reference Example 68

### Ethyl 3-(6-(2,4-dichlorophenoxy)-4-(trifluoromethyl)pyridin-2-yl)benzoate

The titled compound was obtained in the form of an oily substance using 2-chloro-6-(2,4-dichlorophenoxy)-4-(trifluoromethyl)pyridine obtained in Reference Example 59 in the same manner as in Reference Example 66. Yield: 75%.
¹H-NMR (CDCl₃ ) δ: 1.42 (3H, t, J = 7.2 Hz), 4.41 (2H, q, J = 7.2 Hz), 7.17 - 7.27 (2H, m), 7.31 - 7.37 (1H, m), 7.44 - 7.55 (2H, m), 7.72 (1H, s), 7.95 - 8.01 (1H, m), 8.05 - 8.11 (1H, m), 8.46 - 8.48 (1H, m).

### Reference Example 69

### Ethyl 3-(6-(2,4-dichlorophenoxy)pyridin-2-yl)-5-fluorobenzoate

A DMF (25 mL) solution of ethyl 3-bromo-5-fluorobenzoate (1.89 g, 7.65 mmol) obtained in Reference Example 61, bispinacolatodiboron (2.33 g, 9.18 mmol), 1,1-bins-(diphenylphosphino)-ferrocene palladium dichloride (312 mg, 0.382 mmol), and potassium acetate (2.25 g, 22.9 mmol) was stirred over night at 80°C in a nitrogen atmosphere. Water was poured into the reaction solution, and the product was extracted with ethyl acetate. The extract was washed with water, dried over anhydrous magnesium sulfate, and concentrated at reduced pressure through a small amount of silica gel to give ethyl 3-fluoro-5-(4,4,5,5-tetramethyl-1,3,2-dioxaborolan-2-yl)benzoate in the form of a crude product. Tetrakis(triphenylphosphine)palladium (0) (606 mg, 0.525 mmol) was added at room temperature in a nitrogen atmosphere to a 1,2-dimethoxyethane (24 mL) solution of the above compound (1.93 g), 2-chloro-6-(2,4-dichlorophenoxy)pyridine (1.20 g, 4.37 mmol) obtained in Reference Example 9, and 2 N sodium carbonate aqueous solution (8.7 mL), and the mixture was stirred at 100°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:19) to give 1.28 g of the titled compound (yield 72%) in solid form.
¹H-NMR (CDCl₃ ) δ: 1.37 - 1.44 (3H, m), 4.35 - 4.44 (2H, m), 6.98 (1H, d, J = 8.1 Hz), 7.20 - 7.28 (1H, m), 7.29 - 7.36 (1H, m), 7.49 - 7.57 (2H, m), 7.65 - 7.73 (2H, m), 7.82 (1H, t, J = 8.1 Hz), 8.25 - 8.31 (1H, m).

### Reference Example 70

### Ethyl 3-(6-(2,4-dichlorophenoxy)pyridin-2-yl)-4-fluorobenzoate

The titled compound was obtained in the form of an oily substance using ethyl 3-bromo-4-fluorobenzoate obtained in Reference Example 62 and 2-chloro-6-(2,4-dichlorophenoxy)pyridine obtained in Reference Example 9 in the same manner as in Reference Example 69. Yield: 14%.
¹H-NMR (CDCl₃ ) δ: 1.39 (3H, t, J = 7.0 Hz), 4.36 (2H, q, J = 7.0 Hz), 6.95 - 7.02 (1H, m), 7.11 - 7.19 (1H, m), 7.22 - 7.34 (2H, m), 7.51 (1H, d, J = 2.3 Hz), 7.58 - 7.64 (1H, m), 7.81 (1H, t, J = 7.8 Hz), 7.98 - 8.05 (1H, m), 8.44 (1H, dd, J = 7.7, 2.3 Hz).

### Reference Example 71

### Ethyl 3-(6-(2,4-dichlorophenoxy)pyridin-2-yl)-2-fluorobenzoate

The titled compound was obtained in the form of an oily substance using ethyl 3-bromo-2-fluorobenzoate obtained in Reference Example 63 and 2-chloro-6-(2,4-dichlorophenoxy)pyridine obtained in Reference Example 9 in the same manner as in Reference Example 69. Yield: 16%.
¹H-NMR (CDCl₃ ) δ: 1.40 (3H, t, J = 7.2 Hz), 4.40 (2H, q, J = 7.2 Hz), 6.96 (1H, d, J = 8.3 Hz), 7.15 - 7.25 (2H, m), 7.26 - 7.31 (1H, m), 7.50 (1H, d, J = 2.3 Hz), 7.63 (1H, dd, J = 7.6, 2.3 Hz), 7.80 (1H, t, J = 8.3 Hz), 7.84 - 7.93 (2H, m).

### Reference Example 72

### Ethyl 5-(6-(2,4-dichlorophenoxy)pyridin-2-yl)-2-fluorobenzoate

The titled compound was obtained in the form of an oily substance using ethyl 5-bromo-2-fluorobenzoate obtained in Reference Example 64 and 2-chloro-6-(2,4-dichlorophenoxy)pyridine obtained in Reference Example 9 in the same manner as in Reference Example 69. Yield: 28%.
¹H-NMR (CDCl₃ ) δ: 1.37 - 1.45 (3H, m), 4.34 - 4.47 (2H, m), 6.93 (1H, d, J = 8.3 Hz), 7.07 - 7.37 (3H, m), 7.45 - 7.54 (2H, m), 7.80 (1H, t, J = 7.8 Hz), 7.91 - 8.00 (1H, m), 8.34 - 8.41 (1H, m).

### Reference Example 73

### Ethyl 6-(2,4-dichlorophenoxy)-2,3'-bipyridine-5'-carboxylate

The titled compound was obtained in the form of an oily substance using ethyl 5-bromonicotinate and 2-chloro-6-(2,4-dichlorophenoxy)pyridine obtained in Reference Example 59 in the same manner as in Reference Example 69. Yield: 42%.
¹H-NMR (CDCl₃ ) δ: 1.38 - 1.47 (3H, m), 4.43 (2H, q, J = 7.1 Hz), 7.02 (1H, d, J = 8.1 Hz), 7.19 - 7.37 (2H, m), 7.43 - 7.61 (2H, m), 7.81 - 7.89 (1H, m), 8.65 (1H, t, J = 2.2 Hz), 9.18 (2H, d, J = 2.2 Hz).

### Reference Example 74

### Methyl 1-(2-((2-(3,4-dimethoxyphenyl)ethyl)amino)pyrimidin-4-yl)-1H-indole-5-carboxylate

Sodium hydride (148 mg, 3.69 mmol) was added in a nitrogen atmosphere to a DMF (5 mL) solution of methyl 1H-indole-5-carboxylate (647 mg, 3.69 mmol), the mixture was stirred for 15 min at room temperature, and a DMF (5 ml) solution of 2,4-dichloropyrimidine (500 mg, 3.36 mmol) was then added in the form of drops, and the mixture was stirred for 1 hour at room temperature. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:9) to give methyl 1-(2-chloropyrimidin-4-yl)-1H-indole-5-carboxylate in the form of a crude product. An n-butanol (2.8 mL) solution of the above compound (240 mg), 3,4-dimethoxyphenethylamine (0.283 ml, 1.67 mmol), and N-ethyl diisopropylamine (0.292 ml, 1.67 mmol) was stirred for 3 hours at 130°C. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:1) to give 157 mg of the titled compound (yield 11 %) in solid form.
¹H-NMR (CDCl₃ ) δ: 2.94 (2H, t, J = 7.0 Hz), 3.79 (2H, q, J = 7.0 Hz), 3.86 (3H, s), 3.87 (3H, s), 3.95 (3H, s), 5.31 (1H, br s), 6.69 (1H, d, J = 5.7 Hz), 6.75 - 6.84 (4H, m), 7.75 (1H, d, J = 3.6 Hz), 8.00 (1H, dd, J = 8.9, 1.5 Hz), 8.29 - 8.39 (2H, m), 8.51 (1H, d, J = 8.8 Hz).

### Reference Example 75

### Ethyl 3-(6-((2,4-dichlorophenyl)amino)pyridin-2-yl)benzoate

A toluene (20 mL) solution of 2,4-dichloropyridine (1.00 g, 6.71 mmol), 2,4-dichloroaniline (1.20 g, 7.38 mmol), sodium tert-butoxide (968 mg, 10.1 mmol), 4,5-bis(diphenylphosphino)-9,9-dimethyl xanthene (233 mg, 0.403 mmol), and tris(dibenzylideneacetone)dipalladium (0) (123 mg, 0.134 mmol) was stirred at 100°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:19) to give 6-chloro-N-(2,4-dichlorophenyl)pyridine-2-amine in the form of a crude product. Tetrakis(triphenylphosphine)palladium (0) (862 mg, 0.746 mmol) was added at room temperature in a nitrogen atmosphere to a 1,2-dimethoxyethane (35 mL) solution of the above compound (1.71 g), (3-(ethoxycarbonyl)-phenyl)boronic acid (1.33 g, 6.84 mmol), and 2 N sodium carbonate aqueous solution (12.4 mL), and the mixture was stirred at 100°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:9) to give 1.41 g of the titled compound (yield 54%) in solid form.
¹H-NMR (CDCl₃ ) δ: 1.44 (3H, t, J = 7.2 Hz), 4.43 (2H, q, J = 7.2 Hz), 6.78 (1H, d, J = 8.1 Hz), 6.92 (1H, s), 7.24 - 7.31 (1H, m), 7.34 - 7.43 (2H, m), 7.51 - 7.58 (1H, m), 7.66 (1H, t, J = 8.1 Hz), 8.05 - 8.12 (1H, m), 8.18 - 8.24 (1H, m), 8.39 (1H, t, J = 8.1 Hz), 8.67 (1H, t, J = 1.7 Hz).

### Reference Example 76

### Ethyl 3-(6-(2,4-dichlorophenoxy)-3-(trifluoromethyl)pyridin-2-yl)benzoate

A DMF (15 mL) solution of 2,6-dichloro-3-(trifluoromethyl)pyridine (1.00 g, 4.63 mmol), 2,4-dichlorophenol (792 mg, 4.86 mmol), and potassium carbonate (704 mg, 5.09 mmol) was stirred at 120°C for 3 hours. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:99) to give 2-chloro-6-(2,4-dichlorophenoxy)-3-(trifluoromethyl)pyridine in the form of a crude product. Tetrakis(triphenylphosphine)palladium (0) (474 mg, 0.410 mmol) was added at room temperature in a nitrogen atmosphere to a 1,2-dimethoxyethane (20 mL) solution of the above compound (1.17 g), (3-(ethoxycarbonyl)-phenyl)boronic acid (729 mg, 3.76 mmol), and 2 N sodium carbonate aqueous solution (6.8 mL), and the mixture was stirred at 100°C over night. Water was poured into the reaction solution, and the product was extracted with ethyl acetate. The extract was washed with water and dried over anhydrous magnesium sulfate, and the solvent was distilled off at reduced pressure. The residue was purified by silica gel column chromatography (ethyl acetate:hexane = 1:19) to give 788 mg of the titled compound (yield 51%) in the form of an oily substance.
¹H-NMR (CDCl₃ ) δ: 1.39 (3H, t, J = 7.1 Hz), 4.28 (2H, q, J = 7.1 Hz), 7.04 (1H, d, J = 8.5 Hz), 7.14 - 7.20 (1H, m), 7.22 - 7.29 (1H, m), 7.41 - 7.50 (2H, m), 7.56 - 7.64 (1H, m), 8.04 - 8.14 (3H, m).

### Reference Example 77

### 3-(6-((2,4-Dichlorophenyl)thio)pyridin-2-yl)benzoic acid

1 N sodium hydroxide aqueous solution (5.6 mL) was added at room temperature to a tetrahydrofuran (14 mL)-methanol (7 mL) solution of ethyl 3-(6-((2,4-dichlorophenyl)thio)pyridin-2-yl)benzoate (1.13 g, 2.79 mmol) obtained in Reference Example 66, and the mixture was stirred over night. Water was poured into the reaction solution, the pH was adjusted to between 2 and 3 with 1 N hydrochloric acid aqueous solution, and the product was extracted with ethyl acetate. The extract was washed with water and dried over anhydrous magnesium sulfate, and the solvent was distilled off at reduced pressure. Diethyl ether was added to the residue, and 708 mg of the titled compound (yield 67%) was filtered off in solid form.
¹H-NMR (DMSO-d₆) δ: 7.12 (1H, dd, J = 6.6, 1.9 Hz), 7.51 - 7.62 (2H, m), 7.76 - 7.90 (3H, m), 7.95 - 8.10 (2H, m), 8.16 (1H, d, J = 7.7 Hz), 8.47 - 8.52 (1H, m).

### Reference Example 78

### 1-(2-((2-(3,4-dimethoxyphenyl)ethyl)amino)pyrimidin-4-yl)-1H-indole-5-carboxylic acid

The titled compound was obtained in solid form using methyl 1-(2-((2-(3,4-dimethoxyphenyl)ethyl)amino)pyrimidin-4-yl)-1H-indole-5-carboxylate obtained in Reference Example 74 in the same manner as in Reference Example 77. Yield: 90%.
¹H-NMR (DMSO-d₆) δ: 2.85 - 2.98 (2H, m), 3.63 - 3.82 (8H, m), 6.77 - 7.09 (4H, m), 7.30 (1H, d, J = 6.2 Hz), 7.90 (1H, dd, J = 8.9, 1.5 Hz), 8.26 - 8.33 (2H, m), 8.36 - 8.49 (1H, m), 8.55 - 8.76 (1H, m), 8.81 - 9.04 (1H, m).

### Reference Example 79

### 3-(6-((2,4-Dichlorophenyl)amino)pyridin-2-yl)benzoic acid

The titled compound was obtained in solid form using ethyl 3-(6-((2,4-dichlorophenyl)amino)pyridin-2-yl)benzoate obtained in Reference Example 75 in the same manner as in Reference Example 77. Yield: 90%.
¹H-NMR (DMSO-d₆) δ: 7.04 (1H, d, J = 8.1 Hz), 7.37 (1H, dd, J = 8.9, 2.4 Hz), 7.47 (1H, d, J = 7.3 Hz), 7.55 - 7.65 (2H, m), 7.73 (1H, t, J = 8.1 Hz), 7.92 - 7.99 (1H, m), 8.23 (1H, d, J = 8.9 Hz), 8.60 (1H, t, J = 1.6 Hz), 8.67 (1H, s).

### Reference Example 80

### 3-(6-(2,4-dichlorophenoxy)pyridin-2-yl)-4-methylbenzoic acid

The titled compound was obtained in solid form using methyl 3-(6-(2,4-dichlorophenoxy)pyridin-2-yl)-4-methylbenzoate obtained in Reference Example 67 in the same manner as in Reference Example 77. Yield: 76%.
¹H-NMR (DMSO-d₆) δ: 2.17 (3H, s), 7.18 (1H, d, J = 8.1 Hz), 7.32 - 7.43 (3H, m), 7.44 - 7.51 (1H, m), 7.76 (1H, t, J = 2.4 Hz), 7.79 - 7.89 (2H, m), 7.97 - 8.05 (1H, m).

### Reference Example 81

### 3-(6-(2,4-Dichlorophenoxy)pyridin-2-yl)-5-fluorobenzoic acid

The titled compound was obtained in solid form using ethyl 3-(6-(2,4-dichlorophenoxy)pyridin-2-yl)-5-fluorobenzoate obtained in Reference Example 69 in the same manner as in Reference Example 77. Yield: 85%.
¹H-NMR (DMSO-d₆) δ: 7.17 (1H, d, J = 8.1 Hz), 7.43 - 7.58 (2H, m), 7.62 - 7.71 (1H, m), 7.83 (1H, d, J = 2.4 Hz), 7.85 - 7.91 (2H, m), 8.03 (1H, t, J = 8.1 Hz), 8.21 - 8.28 (1H, m).

### Reference Example 82

### 3-(6-(2,4-Dichlorophenoxy)pyridin-2-yl)-4-fluorobenzoic acid

The titled compound was obtained in solid form using ethyl 3-(6-(2,4-dichlorophenoxy)pyridin-2-yl)-4-fluorobenzoate obtained in Reference Example 70 in the same manner as in Reference Example 77. Yield: 82%.
¹H-NMR (DMSO-d₆) δ: 7.18 (1H, d, J = 8.1 Hz), 7.37 - 7.53 (3H, m), 7.63 (1H, dd, J = 7.3, 1.9 Hz), 7.78 (1H, d, J = 1.9 Hz), 7.94 - 8.07 (2H, m), 8.24 (1H, dd, J = 7.3, 1.9 Hz).

### Reference Example 83

### 3-(6-(2,4-Dichlorophenoxy)pyridin-2-yl)-2-fluorobenzoic acid

The titled compound was obtained in solid form using ethyl 3-(6-(2,4-dichlorophenoxy)pyridin-2-yl)-2-fluorobenzoate obtained in Reference Example 71 in the same manner as in Reference Example 77. Yield: 85%.
¹H-NMR (DMSO-d₆ ) δ: 7.19 (1H, d, J = 8.1 Hz), 7.34 (1H, t, J = 7.7Hz), 7.42 - 7.53 (2H, m), 7.59 (1H, dd, J = 7.7, 2.2 Hz), 7.68 - 7.76 (1H, m), 7.80 (1H, d, J = 2.2 Hz), 7.82 - 7.90 (1H, m), 8.03 (1H, t, J = 7.7 Hz).

### Reference Example 84

### 5-(6-(2,4-Dichlorophenoxy)pyridin-2-yl)-2-fluorobenzoic acid

The titled compound was obtained in solid form using ethyl 5-(6-(2,4-dichlorophenoxy)pyridin-2-yl)-2-fluorobenzoate obtained in Reference Example 72 in the same manner as in Reference Example 77. Yield: 83%.
¹H-NMR (DMSO-d₆) δ: 7.11 (1H, d, J = 8.1 Hz), 7.34 - 7.48 (2H, m), 7.49 - 7.55 (1H, m), 7.75 - 7.82 (2H, m), 7.96 - 8.11 (2H, m), 8.31 (1H, dd, J = 7.2, 2.4 Hz).

### Reference Example 85

### 3-(6-(2,4-dichlorophenoxy)-3-(trifluoromethyl)pyridin-2-yl)benzoic acid

The titled compound was obtained in solid form using ethyl 3-(6-(2,4-dichlorophenoxy)-3-(trifluoromethyl)pyridin-2-yl)benzoate obtained in Reference Example 76 in the same manner as in Reference Example 77. Yield: 93%.
¹H-NMR (DMSO-d₆) δ: 7.35 - 7.42 (1H, m), 7.45 - 7.53 (2H, m), 7.54 - 7.62 (2H, m), 7.78 (1H, d, J = 1.3 Hz), 7.93 (1H, s), 7.97 - 8.04 (1H, m), 8.40 (1H, d, J = 8.7 Hz).

### Reference Example 86

### 6-(2,4-Dichlorophenoxy)-2,3'-bipyridine-5'-carboxylic acid

The titled compound was obtained in solid form using ethyl 6-(2,4-dichlorophenoxy)-2,3'-bipyridine-5'-carboxylate obtained in Reference Example 73 in the same manner as in Reference Example 77. Yield: 34%.
¹H-NMR (DMSO-d₆) δ: 7.20 (1H, d, J = 7.7 Hz), 7.41 - 7.58 (2H, m), 7.80 - 7.97 (2H, m), 8.05 (1H, t J = 7.7 Hz), 8.59 (1H, t, J = 2.1 Hz), 9.05 (1H, d, J = 1.9 Hz), 9.20 (1H, d, J = 1.9 Hz).

### Reference Example 87

### 3-(6-(2,4-dichlorophenoxy)-4-(trifluoromethyl)pyridin-2-yl)benzoic acid

The titled compound was obtained in solid form using ethyl 3-(6-(2,4-dichlorophenoxy)-4-(trifluoromethyl)pyridin-2-yl)benzoate obtained in Reference Example 68 in the same manner as in Reference Example 77. Yield: 55%.
¹H-NMR (DMSO-d₆) δ: 7.50 - 7.63 (4H, m), 7.85 (1H, dd, J = 2.0, 0.8 Hz), 7.96 - 8.02 (1H, m), 8.13 - 8.20 (2H, m), 8.43 (1H, t, J = 1.5 Hz).

### Reference Example 88

### 6-Bromo-N-(2,4-dichlorobenzyl)pyridine-2-amine

A DMF (15 mL) solution of 2,6-dibromopyridine (2.37 g, 10.0 mmol), triphenylphosphine (524 mg, 2.00 mmol), bis(triphenylphosphine)palladium (II) dichloride (140 mg, 0.20 mmol), and potassium carbonate (3.04 g, 22.0 mmol) was stirred for 10 min at room temperature in a nitrogen atmosphere, 2,4-dichlorobenzylamine (1.76 g, 10.0 mmol) was then added, and the mixture was reacted for 23 hours at 110°C. Water was added to the reaction solution, and the product was extracted with ethyl acetate. The combined organic layers were washed with water and saturated brine, dried over anhydrous sodium sulfate, and concentrated at reduced pressure, and the residue was purified by NH silica gel column chromatography (hexane-ethyl acetate 10:0 → 9:1), and was crystallized from hexane-ethyl acetate and purified to give 903 mg of the titled compound (yield: 27%).
¹H-NMR (CDCl₃) δ : 4.55 (2H, d, J = 6.3 Hz), 5.12 (1H, br.s.), 6.23 (1H, d, J = 8.2 Hz), 6.76 (1H, d, J = 7.4 Hz), 7.14 - 7.28 (2H, m), 7.33 - 7.42 (2H, m)

### Reference Example 89

6-Bromo-N-{2-[3-(trifluoromethyl)phenyl]ethyl}pyridine-2-amine The titled compound was obtained using 2,6-dibromopyridine and 2-[3-(trifluoromethyl)phenyl]ethanamine in the same manner as in Reference Example 88. ¹H-NMR (CDCl₃ ) δ : 2.98 (2H, t, J = 7.0 Hz), 3.52 - 3.63 (2H, m), 4.61 (1H, br.s.), 6.28 (1H, d, J = 7.7 Hz), 6.74 (1H, d, J = 7.4 Hz), 7.20 - 7.30 (1H, m), 7.38 - 7.54 (4H, m)

### Reference Example 90

### Ethyl 3- {6-[(2,4-dichlorobenzyl)amino]pyridin-2-yl}benzoate

The titled compound was obtained using 6-bromo-N-(2,4-dichlorobenzyl)pyridine-2-amine obtained in Reference Example 88 and (3-(ethoxycarbonyl)-phenyl)boronic acid in the same manner as in Reference Example 66. ¹H-NMR (CDCl₃ ) δ : 1.42 (3H, t, J = 7.0 Hz), 4.42 (2H, q, J = 7.1 Hz), 4.68 (2H, d, J = 6.3 Hz), 5.09 (1H, t, J = 6.0 Hz), 6.34 (1H, d, J = 8.2 Hz), 7.13 (1H, d, J = 7.4 Hz), 7.19 (1H, dd, J = 8.2, 1.9 Hz), 7.40 (1H, d, J = 2.2 Hz), 7.43 - 7.55 (3H, m), 8.05 (1H, dt, J = 7.7, 1.5 Hz), 8.15 (1H, dd, J = 7.6, 1.5 Hz), 8.63 (1H, t, J = 1.5 Hz)

### Reference Example 91 1

### Ethyl 3-[6-({2-[3-(trifluoromethyl)phenyl]ethyl}amino)pyridin-2-yl]benzoate

The titled compound was obtained using 6-bromo-N-{2-[3-(trifluoromethyl)phenyl]ethyl}pyridine-2-amine obtained in Reference Example 89 and (3-(ethoxycarbonyl)-phenyl)boronic acid in the same manner as in Reference Example 66. ¹H-NMR (CDCl₃) δ : 1.41 (3H, t, J = 7.1 Hz), 3.05 (2H, t, J = 7.1 Hz), 3.63 - 3.78 (2H, m), 4.41 (2H, q, J = 7.1 Hz), 4.64 (1H, t, J = 5.8 Hz), 6.36 (1H, d, J = 8.2 Hz), 7.12 (1H, d, J = 7.4 Hz), 7.37 - 7.55 (6H, m), 8.05 (1H, dt, J = 8.2, 1.1 Hz), 8.21 (1H, ddd, J = 7.8, 1.6, 1.5 Hz), 8.64 (1H, t, J = 1.8 Hz)

### Reference Example 92

### 3-{6-[(2,4-Dichlorobenzyl)amino]pyridin-2-yl}benzoic acid

The titled compound was obtained in solid form using ethyl 3-{6-[(2,4-dichlorobenzyl)amino]pyridin-2-yl)benzoate obtained in Reference Example 90 in the same manner as in Reference Example 77. ¹H-NMR (DMSO-d₆) δ : 4.60 (2H, d, J = 5.8 Hz), 6.54 (1H, d, J = 8.2 Hz), 7.12 (1H, d, J = 7.4 Hz), 7.35 (2H, dd, J = 8.4, 2.1 Hz), 7.41 - 7.55 (3H, m), 7.57 (1H, d, J = 1.9 Hz), 7.89 (1H, dd, J = 7.7, 1.1 Hz), 8.11 (1H, d, J = 6.6 Hz), 8.49 (1H, d, J = 1.4 Hz), 12.95 (1H, br.s.)

### Reference Example 93

3-[6-({2-[3-(Trifluoromethyl)phenyl]ethyl}amino)pyridin-2-yl]benzoic acid The titled compound was obtained in solid form using ethyl 3-[6-({2-[3-(trifluoromethyl)phenyl]ethyl}amino)pyridin-2-yl)benzoate obtained in Reference Example 91 in the same manner as in Reference Example 66. ¹H-NMR (CDCl₃) δ : 3.15 (2H, t, J = 7.3 Hz), 3.66 (2H, t, J = 7.4 Hz), 6.47 (1H, d, J = 8.5 Hz), 7.03 (1H, d, J = 7.1 Hz), 7.36 - 7.66 (6H, m), 7.96 (1H, d, J = 7.7 Hz), 8.21 (1H, d, J = 7.7 Hz), 9.12 (1H, s)

### Working Example 1

### N-(3-(2-(2-(3,4-dimethoxyphenyl)ethylamino)pyrimidin-4-yl)phenyl)acetamide

Acetic anhydride (45µL, 0.48 mmol) was added to a pyridine (3 mL) solution of (4-(3-aminophenyl)pyrimidin-2-yl)-(2-(3,4-dimethoxyphenyl)ethyl)amine (0.15 g, 0.43 mmol) synthesized in Reference Example 3, and the mixture was stirred for 90 min at room temperature. Ethyl acetate was added to the reaction solution, the solution was washed with water and with saturated brine, and concentrated. The residue was purified by silica gel column chromatography (hexane-ethyl acetate 10:0 → 0:10) and was recrystallized from ethyl acetate-hexane to give 0.11 g (yield 65%) of the titled compound in the form of crystals.
Melting point: 146 - 148°C.
¹H-NMR (CDCl₃) δ : 2.21 (3H, s), 2.91 (2H, t, J = 6.9 Hz), 3.73-3.79 (2H, m), 3.86 (6H, s), 5.20 (1H, br t, J = 6.0 Hz), 6.78-6.81 (3H, m), 6.96 (1H, d, J = 5.4 Hz), 7.37 (1H, br s), 7.42 (1H, t, J = 8.1 Hz), 7.72- 7.75 (2H, m), 8.08 (1H, br s), 8.58 (1H, d, J = 5.1 Hz).

### Working Example 2

### N-(3-(2-(2-(3,4-dimethoxyphenyl)ethylamino)pyrimidin-4-yl)phenyl)-2-methoxyacetamide

Methoxyacetic acid (46 mg, 0.51 mmol), WSC (99 mg, 0.52 mmol), and HOBt (70 mg, 0.52 mmol) were added to a DMF (3 mL) solution of (4-(3-aminophenyl)pyrimidin-2-yl)-(2-(3,4-dimethoxyphenyl)ethyl)amine (0.15 g, 0.43 mmol) synthesized in Reference Example 3, and the mixture was stirred for 16 hours at room temperature. Ethyl acetate was added to the reaction solution, the solution was washed with water and with saturated brine, and concentrated. The residue was purified by silica gel column chromatography (hexane-ethyl acetate 10:0 → 0:10) and was recrystallized from ethyl acetate-hexane to give 0.14 g (yield 77%) of the titled compound in the form of crystals. Melting point: 140 - 141°C.
¹H-NMR (CDCl₃) δ: 2.92 (2H, t, J = 6.9 Hz), 3.53 (3H, s), 3.74-3.80 (2H, m), 3.87 (6H, s), 4.05 (2H,s), 5.22 (1H, br t, J = 5.7 Hz), 6.78 (1H, s), 6.82 (2H, s), 6.98 (1H, d, J = 5.1 Hz), 7.44 (1H, d, J = 8.1 Hz), 7.76- 7.80 (2H, m), 8.19 (1H, br s), 8.33-8.36 (2H, m).

### Working Example 3

### N-(3-(2-(2-(3,4-dimethoxyphenyl)ethylamino)pyrimidin-4-yl)phenyl)benzamide

The titled compound was obtained from (4-(3-aminophenyl)pyrimidin-2-yl)-(2-(3,4-dimethoxyphenyl)ethyl)amine synthesized in Reference Example 3 and benzoic acid in the same manner as in Working Example 2. Yield: 64%. Melting point: 168 - 170°C (ethyl acetate-hexane).
¹H-NMR (CDCl₃) δ : 2.92 (2H, t, J = 6.9 Hz), 3.74-3.81 (2H, m), 3.85 (6H, s), 5.21 (1H, br t), 6.79-6.81 (3H, m), 7.01 (1H, d, J = 5.1 Hz), 7.46- 7.61 (4H, m), 7.79- 7.92 (5H, m), 8.26 (1H, s), 8.35 (1H, d, J = 5.1 Hz).

### Working Example 4

### Tert-butyl ((3-(2-(2-(3,4-dimethoxyphenyl)ethylamino)pyrimidin-4-yl)phenylcarbamoyl)methyl)carbamate ester

The titled compound was obtained from (4-(3-aminophenyl)pyrimidin-2-yl)-(2-(3,4-dimethoxyphenyl)ethyl)amine synthesized in Reference Example 3 and N-Boc-glycine in the same manner as in Working Example 2. Yield: 76%.
¹H-NMR (CDCl₃) δ : 1.49 (9H, s), 2.91 (2H, t, J = 6.6 Hz), 3.73-3.78 (2H, m), 3.68 (6H, s), 3.96 (2H, d, J = 6.0 Hz), 5.21 - 5.23 (2H, m), 6.78-6.82 (3H, m), 6.96 (1H, d, J = 5.4 Hz), 7.42 (1H, t, J = 8.1 Hz), 7.70 (1H, d, J = 7.8 Hz), 7.77 (1H, d, J = 7.8 Hz), 8.12 (1H, s), 8.19 (1H, br s), 8.33 (1H, d, J = 5.1 Hz).

### Working Example 5

### N-(3-(2-((2-(3,4-dimethoxyphenyl)ethyl)amino)pyrimidin-4-yl)phenyl)-2-methylpropanamide

The titled compound was obtained from (4-(3-aminophenyl)pyrimidin-2-yl)-(2-(3,4-dimethoxyphenyl)ethyl)amine synthesized in Reference Example 3 and iso-butyric anhydride in the same manner as in Working Example 1. Yield: 81 %. Melting point: 146 - 147°C (ethyl acetate-hexane).
¹H-NMR (CDCl₃) δ : 1.28 (6H, d, J = 6.6 Hz), 2.54 (1H, m), 2.91 (2H, t, J = 6.9 Hz), 3.76 (2H, td, J = 5.4, 6.9 Hz), 3.86 (6H, s), 5.21 (1H, t, J = 5.4 Hz), 6.78-6.82 (3H, m), 6.97 (1H, d, J = 5.4 Hz), 7.32 (1H, br s), 7.41 (1H, t, J = 7.8 Hz), 7.72- 7.74 (2H, m), 8.15 (1H, s), 8.33 (1H, d, J = 5.1 Hz).

### Working Example 6

### 2-Amino-N-(3-(2-(2-(3,4-dimethoxyphenyl)ethylamino)pyrimidin-4-yl)phenyl)acetamide hydrochloride

4 N hydrogen chloride-ethyl acetate solution (2 mL) was added to an ethyl acetate 5 mL) solution of tert-butyl ((3-(2-(2-(3,4-dimethoxyphenyl)ethylamino)pyrimidin-4-yl)phenylcarbamoyl)methyl)carbamate ester (200 mg, 0.39 mmol) synthesized in Working Example 4, and the mixture was stirred for 16 hours at room temperature. The precipitated crystals were filtered off and washed with ethyl acetate to give 188 mg of the titled compound (yield: quantitative). Melting point: 224 - 226°C.
¹H-NMR (DMSO-d₆) δ : 2.87 (2H, t, J = 6.9 Hz), 3.69 (6H, s), 3.73 (2H, s), 3.82-3.86 (2H, m), 6.79-6.91 (3H, m), 7.28 (1H, br s), 7.54 (1H, d, J = 8.1 Hz), 7.88 (2H, br s), 8.31 (4H, br), 8.45 (2H, d, J = 5.7 Hz), 11.07 (1H, s).

### Working Example 7

### N-(3-(2-((2-(3,4-dimethoxyphenyl)ethyl)amino)pyrimidin-4-yl)phenyl)tetrahydrofuran-3-carboxamide

The titled compound was obtained from (4-(3-aminophenyl)pyrimidin-2-yl)-(2-(3,4-dimethoxyphenyl)ethyl)amine synthesized in Reference Example 3 and tetrahydrofuran-3-carboxylic acid in the same manner as in Working Example 2. Yield: 76%. Melting point: 146 - 147°C (ethyl acetate-hexane).
¹H-NMR (CDCl₃) δ : 2.29 (2H, q, J = 7.2 Hz), 2.91 (2H, t, J = 6.9 Hz), 3.06 (1H, m), 3.73-4.15 (6H, m), 3.86 (6H, s), 5.24 (1H, t, J = 5.7 Hz), 6.78-6.84 (3H, m), 6.97 (1H, d, J = 5.4 Hz), 7.42 (1H, t, J = 7.8 Hz), 7.63 (1H, br s), 7.71 - 7.77 (2H, m), 8.12 (1H, s), 8.33 (1H, d, J = 5.4 Hz).

### Working Example 8

### Tert-butyl (4-((3-(2-((2-(3,4-dimethoxyphenyl)ethyl)amino)pyrimidin-4-yl)phenyl)amino)-4-oxobutyl)carbamate

The titled compound was obtained from (4-(3-aminophenyl)pyrimidin-2-yl)-(2-(3,4-dimethoxyphenyl)ethyl)amine synthesized in Reference Example 3 and 4-(tert-butoxycarbonylamino)butyric acid in the same manner as in Working Example 2. Yield: 79%. Melting point: 154 - 157°C (ethyl acetate-hexane).
¹H-NMR (CDCl₃) δ : 1.47 (9H, s), 1.86-1.94 (2H, m), 2.42 (2H, t, J = 6.6 Hz), 2.91 (2H, t, J = 6.6 Hz), 3.25-3.31 (2H, m), 3.77 (2H, q, J = 6.3 Hz), 3.87 (6H, s), 4.75 (1H, br t), 5.20 (1H, br), 6.78-6.81 (3H, m), 6.98 (1H, d, J = 5.1 Hz), 7.42 (1H, t, J = 7.8 Hz), 7.76- 7.78 (2H, m), 8.28-8.30 (2H, m), 8.91 (1H, br s).

### Working Example 9

### N-(3-(2-((2-(3,4-dimethoxyphenyl)ethyl)amino)pyrimidin-4-yl)phenyl)pentanamide

The titled compound was obtained from (4-(3-aminophenyl)pyrimidin-2-yl)-(2-(3,4-dimethoxyphenyl)ethyl)amine synthesized in Reference Example 3 and valeric anhydride in the same manner as in Working Example 1. Yield: 73%. Melting point: 136 - 137°C (ethanol-hexane).
¹H-NMR (CDCl₃) δ : 0.96 (3H, t, J = 7.2 Hz), 1.39-1.47 (2H, m), 1.69-1.79 (2H, m), 2.40 (2H, t, J = 7.5 Hz), 2.91 (2H, t, J = 6.9 Hz), 3.73-3.80 (2H, m), 3.87 (6H, s), 5.17 (1H, t, J = 5.7 Hz), 6.78-6.82 (3H, m), 6.98 (1H, d, J = 5.1 Hz), 7.24- 7.26 (1H, m), 7.42 (1H, t, J = 7.8 Hz), 7.73 (2H, br s), 8.12 (1H, s), 8.33 (1H, d, J = 5.1 Hz).

### Working Example 10

### N-(3-(2-(2-(3,4-dimethoxyphenyl)ethylamino)pyrimidin-4-yl)phenyl)-3-phenylpropionamide

The titled compound was obtained from (4-(3-aminophenyl)pyrimidin-2-yl)-(2-(3,4-dimethoxyphenyl)ethyl)amine synthesized in Reference Example 3 and 3-phenylpropionic acid in the same manner as in Working Example 2. Yield: 77%. Melting point: 155 - 156°C (ethanol-hexane).
¹H-NMR (CDCl₃) δ : 2.69 (2H, t, J = 7.8 Hz), 2.90 (2H, t, J = 6.9 Hz), 3.08 (2H, t, J = 7.8 Hz), 3.72-3.79 (2H, m), 3.85 (3H, s), 3.86 (3H, s), 5.19 (1H, t, J = 5.7 Hz), 6.78-6.81 (3H, m), 6.95 (1H, d, J = 5.1 Hz), 7.18- 7.33 (6H, m), 7.40 (1H, t, J = 7.8 Hz), 7.65 (1H, d, J = 8.1 Hz), 7.74 (1H, d, J = 7.8 Hz), 8.03 (1H, s), 8.32 (1H, d, J = 2.1 Hz).

### Working Example 11

### 2-Acetylamino-N-(3-(2-(2-(3,4-dimethoxyphenyl)ethylamino)pyrimidin-4-yl)phenyl)acetamide

The titled compound was obtained from (4-(3-aminophenyl)pyrimidin-2-yl)-(2-(3,4-dimethoxyphenyl)ethyl)amine synthesized in Reference Example 3 and N-acetylglycine in the same manner as in Working Example 2. Yield: 45%. Melting point: 177 - 178°C (ethyl acetate-hexane).
¹H-NMR (DMSO-d₆) δ : 1.87 (3H, s), 2.82 (2H, t, J = 7.5 Hz), 3.55-3.58 (2H, m), 3.71 (3H, s), 3.72 (3H, s), 3.89 (2H, d, J = 6.0 Hz), 6.78 (1H, d, J = 8.1 Hz), 6.85-6.88 (2H, m), 7.02 (1H, d, J = 5.1 Hz), 7.23 (1H, t, J = 5.1 Hz), 7.44 (1H, t, J = 7.8 Hz), 7.70 - 7.76 (2H, m), 8.23 (1H, t, J = 6.0 Hz), 8.33-8.36 (2H, m), 10.12 (1H, s).

### Working Example 12

### N-(3-(2-(2-(3,4-dimethoxyphenyl)ethylamino)pyrimidin-4-yl)phenyl)-3-methoxybenzamide

The titled compound was obtained from (4-(3-aminophenyl)pyrimidin-2-yl)-(2-(3,4-dimethoxyphenyl)ethyl)amine synthesized in Reference Example 3 and 3-methoxybenzoic acid in the same manner as in Working Example 2. Yield: 80%. Melting point: 142 - 143°C (ethyl acetate-hexane).
¹H-NMR (CDCl₃) δ : 2.92 (2H, t, J = 7.2 Hz), 3.74-3.81 (2H, m), 3.85 (6H, s), 3.88 (3H, s), 5.30 (1H, br t), 6.78-6.81 (3H, m), 7.01 (1H, d, J = 5.1 Hz), 7.08-7.12 (1H, m), 7.37-7.50 (4H, m), 7.78-7.87 (2H, m), 8.97 (1H, s), 8.26 (1H, s), 8.34 (1H, d, J = 5.4 Hz).

### Working Example 13

### N-(3-(2-((2-(3,4-dimethoxyphenyl)ethyl)amino)pyrimidin-4-yl)phenyl)-3-furamide

The titled compound was obtained from (4-(3-aminophenyl)pyrimidin-2-yl)-(2-(3,4-dimethoxyphenyl)ethyl)amine synthesized in Reference Example 3 and 3-furancarboxylic acid in the same manner as in Working Example 2. Yield: 79%. Melting point: 151 - 152°C (ethyl acetate-hexane).
¹H-NMR (CDCl₃) δ : 2.91 (2H, t, J = 6.9 Hz), 3.75-3.77 (2H, m), 3.84 (6H, s), 5.39 (1H, br s), 6.77-6.80 (4H, m), 6.95 (1H, d, J = 5.4 Hz), 7.41 - 7.50 (2H, m), 7.75- 7.85 (3H, m), 8.09 (1H, s), 8.15 (1H, t, J = 1.8 Hz), 8.32 (1H, d, J = 5.4 Hz).

### Working Example 14

### Tert-butyl (3-((3-(2-((2-(3,4-dimethoxyphenyl)ethyl)amino)pyrimidin-4-yl)phenyl)amino)-3-oxopropyl)carbamate

The titled compound was obtained from (4-(3-aminophenyl)pyrimidin-2-yl)-(2-(3,4-dimethoxyphenyl)ethyl)amine synthesized in Reference Example 3 and N-Boc-β-alanine in the same manner as in Working Example 2. Yield: 85%. Melting point: 138 - 141°C (ethyl acetate-hexane).
¹H-NMR (CDCl₃) δ : 1.44 (9H, s), 2.64 (2H, t, J = 5.7 Hz), 2.91 (2H, t, J = 6.9 Hz), 3.52 (2H, q, J = 6.3 Hz), 3.77 (2H, q, J = 6.6 Hz), 3.86 (6H, s), 5.19-5.23 (2H, m), 6.78-6.82 (3H, m), 6.96 (1H, d, J = 5.4 Hz), 7.42 (1H, t, J = 8.1 Hz), 7.70 - 7.78 (3H, m), 8.15 (1H, s), 8.33 (1H, d, J = 5.1 Hz).

### Working Example 15

### N-(3-(2-((2-(3,4-dimethoxyphenyl)ethyl)amino)pyrimidin-4-yl)phenyl)-β-alaninamide hydrochloride

The titled compound was obtained from tert-butyl (3-((3-(2-((2-(3,4-dimethoxyphenyl)ethyl)amino)pyrimidin-4-yl)phenyl)amino)-3-oxopropyl)carbamate synthesized in Working Example 14 in the same manner as in Working Example 6. Yield: 98%. Melting point: 136 - 138°C (methanol-ethyl acetate).
¹H-NMR (DMSO-d₆) δ : 2.73-2.88 (4H, m), 3.07-3.13 (2H, m), 3.64-3.73 (8H, m), 6.79-6.89 (3H, m), 7.22 (1H, br s), 7.50 (1H, t, J = 7.8 Hz), 7.82 (2H, br s), 7.97 (4H, br s), 8.42-8.46 (2H, m), 10.53 (1H, s).

### Working Example 16

### N-(3-(2-(2-(3,4-dimethoxyphenyl)ethylamino)pyrimidin-4-yl)phenyl)-4-dimethylaminobutyramide

The titled compound was obtained from (4-(3-aminophenyl)pyrimidin-2-yl)-(2-(3,4-dimethoxyphenyl)ethyl)amine synthesized in Reference Example 3 and N,N-dimethyl-4-aminobutyric acid in the same manner as in Working Example 2. Yield: 72%. Melting point: 121 - 123°C (ethyl acetate-diisopropyl ether).
¹H-NMR (CDCl₃) δ : 1.86-1.92 (2H, m), 2.32 (6H, s), 2.43-2.55 (4H, m), 2.90 (2H, t, J = 6.9 Hz), 3.72-3.79 (2H, m), 3.86 (6H, s), 5.23 (1H, t, J = 5.7 Hz), 6.77-6.81 (3H, m), 6.97 (1H, d, J = 5.1 Hz), 7.40 (1H, t, J = 7.8 Hz), 7.72 (2H, d, J = 7.5 Hz), 8.17 (1H, s), 8.32 (1H, d, J = 5.1 Hz), 10.14 (1H, s).

### Working Example 17

### Tert-butyl (5-((3-(2-((2-(3,4-dimethoxyphenyl)ethyl)amino)pyrimidin-4-yl)phenyl)amino)-5-oxopentyl)carbamate

The titled compound was obtained from (4-(3-aminophenyl)pyrimidin-2-yl)-(2-(3,4-dimethoxyphenyl)ethyl)amine synthesized in Reference Example 3 and 5-(tert-butoxycarbonylamino)valeric acid in the same manner as in Working Example 2. Yield: 73%. Melting point: 143 - 144°C (ethyl acetate-diisopropyl ether).
¹H-NMR (CDCl₃) δ : 1.43 (9H, s), 1.53-1.60 (2H, m), 1.73-1.83 (2H, m), 2.43 (2H, t, J = 7.2 Hz), 2.91 (2H, t, J = 6.6 Hz), 3.16-3.20 (2H, brq), 3.76 (2H, q, J = 6.9 Hz), 3.89 (6H, s), 4.67 (1H, br s), 5.29 (1H, t, J = 5.7 Hz), 6.78-6.82 (3H, m), 6.97 (1H, d, J = 5.1 I Hz), 7.41 (1H, t, J = 7.8 Hz), 7.71 - 7.76 (3H, m), 7.16 (1H, s), 8.33 (1H, d, J = 5.4 Hz).

### Working Example 18

### 4-Bromo-N-(3-(2-((2-(3,4-dimethoxyphenyl)ethyl)amino)pyrimidin-4-yl)phenyl)butanamide

The titled compound was obtained from (4-(3-aminophenyl)pyrimidin-2-yl)-(2-(3,4-dimethoxyphenyl)ethyl)amine synthesized in Reference Example 3 and 4-bromobutyric acid in the same manner as in Working Example 2. Yield: 17%. Melting point: 191 - 193°C (ethyl acetate-hexane).
¹H-NMR (CDCl₃) δ : 2.18-2.26 (2H, m), 2.60 (2H, t, J = 7.2 Hz), 2.90 (2H, t, J = 6.9 Hz), 3.68 (2H, t, J = 6.0 Hz), 3.73-3.79 (2H, m), 3.86 (6H, s), 5.28 (1H, t, J = 5.4 Hz), 6.78-6.81 (3H, m), 6.96 (1H, d, J = 5.4 Hz), 7.39-7.48 (2H, m), 7.71 - 7.76 (2H, s), 8.12 (1H, s), 8.32 (1H, d, J = 5.1 Hz).

### Working Example 19

### N-(3-(2-((2-(3,4-dimethoxyphenyl)ethyl)amino)pyrimidin-4-yl)phenyl)-4-pyrrolidin-1-yl butanamide

Pyrrolidine (1 mL) was added to an N-methyl pyrrolidone (10 mL) solution of 4-bromo-N-(3-(2-((2-(3,4-dimethoxyphenyl)ethyl)amino)pyrimidin-4-yl)phenyl)butanamide synthesized in Working Example 18 (200 mg, 0.4 mmol), and the mixture was heated to 80°C for 16 hours. Ethyl acetate was added to the reaction solution, and the solution was washed with water and with saturated brine, dried, and concentrated. The residue was purified by basic silica gel column chromatography (ethyl acetate → ethyl acetate-methanol 95:5) and recrystallized from ethyl acetate-diisopropyl ether to give 118 mg of the titled compound (yield 60%). Melting point: 121 - 122°C.
¹H-NMR (CDCl₃) δ : 1.84-1.96 (6H, m), 2.52-2.65 (8H, m), 2.90 (2H, t, J = 6.9 Hz), 3.72-3.78 (2H, m), 3.86 (6H, s), 5.20 (1H, t, J = 5.7 Hz), 6.77-6.83 (3H, m), 6.96 (1H, d, J = 5.4 Hz), 7.40 (1H, t, J = 7.8 Hz), 7.70 - 7.75 (2H, m), 8.10 (1H, s), 8.32 (1H, d, J = 5.1 Hz), 10.03 (1H, s).

### Working Example 20

### Tert-butyl 4-(((3-(2-((2-(3,4-dimethoxyphenyl)ethyl)amino)pyrimidin-4-yl)phenyl)amino)carbonyl)piperidine-1-carboxylate

The titled compound was obtained from (4-(3-aminophenyl)pyrimidin-2-yl)-(2-(3,4-dimethoxyphenyl)ethyl)amine synthesized in Reference Example 3 and 1-(tert-butoxycarbonyl)piperidine-4-carboxylic acid in the same manner as in Working Example 2. Yield: 66%. Melting point: 117 - 118°C (ethyl acetate-diisopropyl ether).
¹H-NMR (CDCl₃) δ : 1.47 (9H, s), 1.70 - 1.92 (4H, m), 2.36-2.44 (1H, m), 2.78 (2H, br t, J = 11.4 Hz), 2.90 (2H, t, J = 6.9 Hz), 3.75 (2H, q, J = 6.6 Hz), 3.86 (6H, s), 4.16-4.20 (2H, br), 5.23 (1H, t, J = 5.7 Hz), 6.78-6.81 (3H, m), 6.96 (1H, d, J = 5.1 Hz), 7.39- 7.47 (2H, m), 7.72- 7.76 (2H, m), 8.13 (1H, s), 8.32 (1H, d, J = 5.1 Hz).

### Working Example 21

### 5-Amino-N-(3-(2-((2-(3,4-dimethoxyphenyl)ethyl)amino)pyrimidin-4-yl)phenyl)pentanamide hydrochloride

The titled compound was obtained from tert-butyl (5-((3-(2-((2-(3,4-dimethoxyphenyl)ethyl)amino)pyrimidin-4-yl)phenyl)amino)-5-oxopentyl)carbamate synthesized in Working Example 17 in the same manner as in Working Example 6. Yield: 65%. Melting point: 115 - 117°C (methanol-ethyl acetate).
¹H-NMR (DMSO-d₆) δ : 1.18-1.12 (4H, br s), 2.38-2.41 (2H, m), 2.80 - 2.89 (4H, m), 3.45-3.73 (8H, m), 6.82-6.91 (3H, m), 7.26 (1H, br s), 7.42- 7.56 (1H, m), 7.82- 7.93 (5H, m), 8.20 (1H, br s), 8.42-8.50 (2H, m), 10.36 (1H, s).

### Working Example 22

### N-(3-(2-((2-(3,4-dimethoxyphenyl)ethyl)amino)pyrimidin-4-yl)phenyl)piperidine-4-carboxamide hydrochloride

The titled compound was obtained from tert-butyl 4-(((3-(2-((2-(3,4-dimethoxyphenyl)ethyl)amino)pyrimidin-4-yl)phenyl)amino)carbonyl)piperidine-1-carboxylate synthesized in Working Example 20 in the same manner as in Working Example 6. Yield: quantitative. Melting point: 178 - 179°C (methanol-ethyl acetate).
¹H-NMR (DMSO-d₆) δ : 1.81 - 2.02 (4H, m), 2.69-2.76 (1H, m), 2.84-2.97 (4H, m), 3.31 - 3.43 (2H, m), 3.69-3.73 (8H, m), 6.80 - 6.91 (3H, m), 7.28 (1H, br s), 7.50 (1H, t, J = 8.1 Hz), 7.83 (2H, br s), 8.31 (1H, br), 8,43 (1H, d, J = 6.0 Hz), 8.53 (1H, s), 8.81 - 8.83 (1H, brd), 9.11 (1H, br s), 10.49 (1H, s).

### Working Example 23

### N-(3-(2-((2-(3,4-dimethoxyphenyl)ethyl)amino)pyrimidin-4-yl)phenyl)-4-morpholin-4-yl butanamide

The titled compound was obtained from 4-bromo-N-(3-(2-((2-(3,4-dimethoxyphenyl)ethyl)amino)pyrimidin-4-yl)phenyl)butanamide synthesized in Working Example 18 and morpholine in the same manner as in Working Example 19. Yield: 49%. Melting point: 109 - 110°C (ethyl acetate-diisopropyl ether).
¹H-NMR (CDCl₃) δ: 1.89-1.97 (2H, m), 2.45-2.50 (8H, m), 2.90 (2H, t, J = 6.9 Hz), 3.72-3.78 (6H, m), 3.86 (6H, s), 5.29 (1H, br t), 6.69-6.81 (3H, m), 6.97 (1H, d, J = 5.4 Hz), 7.41 (1H, t, J = 7.8 Hz), 7.71 - 7.79 (2H, m), 8.16 (1H, s), 8.33 (1H, d, J = 5.1 Hz), 8.60 (1H, s).

### Working Example 24

### Tert-butyl (3-((3-(2-((2-(3,4-dimethoxyphenyl)ethyl)amino)pyrimidin-4-yl)phenyl)amino)-1-methyl-3-oxopropyl)carbamate

The titled compound was obtained from (4-(3-aminophenyl)pyrimidin-2-yl)-(2-(3,4-dimethoxyphenyl)ethyl)amine synthesized in Reference Example 3 and 3-((tert-butoxycarbonyl)amino)butanoic acid in the same manner as in Working Example 2. Yield: 73%. Melting point: 138 - 140°C (ethyl acetate-hexane).
¹H-NMR (CDCl₃) δ : 2.38 (6H, s), 2.53 (2H, dd, J = 6.0, 4.2 Hz), 2.68 (2H, dd, J = 6.0, 5.1 Hz), 1.31 (3H, d, J = 6.6 Hz), 1.43 (9H, s), 2.62 (1H, d, J = 5.7 Hz), 2.91 (2H, t, J = 6.6 Hz), 3.76 (1H, m), 3.86 (6H, s), 4.10 (1H, m), 5.06 (1H, br d, J = 6.9 Hz), 5.21 (1H, m), 6.78 (1H, s), 6.81 (2H, s), 7.40 (1H, t, J = 8.1 Hz), 7.67- 7.80 (2H, m), 8.18 (br s), 8.32 (1H, d, J = 5.4 Hz).

### Working Example 25

### N-(3-(2-((2-(3,4-Dimethoxyphenyl)ethyl)amino)pyrimidin-4-yl)phenyl)-2-pyrrolidin-2-yl acetamide hydrochloride

WSC (200 mg, 1.1 mmol) and HOBt (142 mg, 1.1 mmol) were added while cooled on ice to a DMF (6.0 mL) solution of Boc-L-β-homoproline (200 mg, 0.87 mmol) and (4-(3-aminophenyl)pyrimidin-2-yl)-(2-(3,4-dimethoxyphenyl)ethyl)amine (305 mg, 0.87 mmol) synthesized in Reference Example 3. The mixture was stirred for another 12 hours at room temperature, and the solvent was then distilled off at reduced pressure. The residue was diluted with ethyl acetate, washed with water and with saturated brine, and dried over magnesium sulfate, and the solvent was then distilled off at reduced pressure. The residue was purified by silica gel column chromatography (ethyl acetate) to give 415 mg of tert-butyl (2-(2-((3-(2-((2-(3,4-dimethoxyphenyl)ethyl)amino)pyrimidin-4-yl)phenyl)amino)-2-oxoethyl)pyrrolidin-1-carboxylate (yield: 85%). This compound (400 mg, 0.71 mmol) was dissolved in chloroform (5.0 mL), and 4 N hydrogen chloride-ethyl acetate solution (1.5 mL) was added at room temperature. The mixture was stirred for another 5 hours at room temperature, the solvent was then distilled off at reduced pressure, diethyl ether was added to the residue, and the precipitated solids were filtered off to give 345 mg of the titled compound (yield: 97%). Amorphous powder.
¹H-NMR (DMSO-d₆) δ: 1.55-1.73 (1H, m), 1.78-2.00 (3H, m), 2.05 - 2.21 (1H, m), 2.80 - 2.99 (4H, m), 3.11 - 3.35 (2H, m), 3.60 - 3.90 (8H, m), 6.78-6.89 (2H, m), 6.90 (1H, s), 7.24 (1H, br s), 7.51 (1H, t, J = 8.1 Hz), 7.85 (2H, br s), 8.15 (1H, br s), 8.43 (1H, s), 8.45 (1H, s), 9.08 (1H, br s), 9.24 (1H, br s), 10.63 (1H, s).

### Working Example 26

### N-(3-(2-((2-(3,4-Dimethoxyphenyl)ethyl)amino)pyrimidin-4-yl)phenyl)-3-(dimethylamino)butanamide hydrochloride

Sodium cyanoborohydride (160 mg, 2.5 mmol) was added at room temperature to a mixture of 3-amino-N-(3-(2-((2-(3,4-dimethoxyphenyl)ethyl)amino)pyrimidin-4-yl)phenyl)butanamide hydrochloride (300 mg, 0.64 mmol) synthesized in Working Example 25, 37% formalin aqueous solution (155 mg, 1.9 mmol), triethylamine (115 µL, 0.83 mmol), acetic acid (0.3 mL), and methanol (6.0 mL), and the mixture was stirred for another 4 hours at room temperature. The solvent was distilled off at reduced pressure, saturated aqueous sodium bicarbonate was added to the residue, and the product was extracted with chloroform. The chloroform extract solution was washed with water and with saturated brine, and was dried over magnesium sulfate, and the solvent was then distilled off at reduced pressure. The residue was purified by silica gel column chromatography (basic silica gel, ethyl acetate). The resulting oily substance was dissolved in methylene chloride (3.0 mL), and a 4 N hydrogen chloride-ethyl acetate solution (1.0 mL) was added. The mixture was stirred for 10 min at room temperature, and the solvent was then distilled off at reduced pressure. Ethyl acetate (3.0 mL) was added to the residue, and the precipitated solids were filtered off to give 130 mg of the titled compound (yield: 41%). Amorphous powder.
¹H-NMR (DMSO-d₆) δ : 1.31 (3H, d, J = 6.6 Hz), 2.65-2.79 (7H, m), 2.80 - 2.91 (2H, m), 3.10 (1H, dd, J = 15.0, 3.6 Hz), 3.50 - 3.90 (9H, m), 6.75-6.88 (2H, m), 6.91 (1H, s), 7.29 (1H, br s), 7.52 (1H, t, J = 8.1 Hz), 7.85 (2H, br s), 8.30 (1H, br s), 8.44 (1H, d, J = 5.7 Hz), 8.51 (1H, br s).

### Working Example 27

### 3-Amino-N-(3-(2-((2-(3,4-dimethoxyphenyl)ethyl)amino)pyrimidin-4-yl)phenyl)butanamide hydrochloride

4 N hydrogen chloride-ethyl acetate solution (3 mL) was added to a chloroform (10 mL) solution of tert-butyl (3-((3-(2-((2-(3,4-dimethoxyphenyl)ethyl)amino)pyrimidin-4-yl)phenyl)-1-methyl-3-oxopropyl)carbamate (1.0 g, 1.86 mmol) synthesized in Working Example 24, and the mixture was stirred for 5 hours at room temperature. The solvent was distilled off at reduced pressure, and the residue was then washed with diethyl ether to give 790 mg of the titled compound (yield: 73%). Amorphous powder.
¹H-NMR (DMSO-d₆) δ : 1.27 (3H, d, J = 6.6 Hz), 2.65-2.91 (4H, m), 3.55-3.69 (3H, m), 3.70 (3H, s), 3.73 (3H, s), 6.75-6.88 (2H. m), 6.91 (1H, br s), 7.27 (1H, br s), 7.51 (1H, t, J = 8.1 Hz), 7.85 (2H, br s), 8.05-8.30 (4H, m), 8.45 (1H, d, J = 6.0 Hz), 8.50 (1H, br s), 10.65 (1H, br s).

### Working Example 28

### N-(3-(2-((2-(3,4-dimethoxyphenyl)ethyl)amino)pyrimidin-4-yl)phenyl)-2-(1-methylpyrrolidin-2-yl)acetamide hydrochloride

Sodium cyanoborohydride (50 mg, 0.80 mmol) was added at room temperature to a mixture of N-(3-(2-((2-(3,4-dimethoxyphenyl)ethyl)amino)pyrimidin-4-yl)phenyl)-2-pyrrolidin-2-ylacetamide hydrochloride (100 mg, 0.20 mmol) synthesized in Working Example 27, 37% formalin aqueous solution (49 mg, 0.60 mmol), triethylamine (36 µL, 0.26 mmol), acetic acid (0.1 mL), and methanol (2.0 ml), and the mixture was stirred for another 4 hours at room temperature. The solvent was distilled off at reduced pressure, saturated aqueous sodium bicarbonate was added to the residue, and the product was extracted with chloroform. The chloroform extract solution was washed with water and with saturated brine, and was dried over magnesium sulfate, and the solvent was then distilled off at reduced pressure. The residue was purified by silica gel column chromatography (basic silica gel, ethyl acetate). The resulting oily substance was dissolved in methylene chloride (3.0 mL), and a 4 N hydrogen chloride-ethyl acetate solution (1.0 mL) was added. The mixture was stirred for 10 min at room temperature, and the solvent was then distilled off at reduced pressure. Ethyl acetate (3.0 mL) was added to the residue, and the precipitated solids were filtered off to give 40 mg of the titled compound (yield: 39%). Amorphous powder.
¹H-NMR (DMSO-d₆) δ : 1.68 - 1.81 (1H, m), 1.85 - 2.10 (3H, m), 2.20 - 2.40 (1H, m), 2.79 - 2.95 (5H, m), 2.98 - 3.22 (2H, m), 3.45 - 3.80 (8H, m), 6.75-6.95 (3H, m), 7.23 (1H, br s), 7.50 (1H, t, J = 8.1 Hz), 7.73 - 7.94 (2H, br s), 8.08 (1H, br s), 8.48 - 8.55 (2H, m).

### Working Example 29

### N-(3-(2-((2-(3,4-dimethoxyphenyl)ethyl)amino)pyrimidin-4-yl)phenyl)-N³,N³-dimethyl-β-alaninamide

WSC (356 mg, 1.85 mmol) and HOBt (250 mg, 1.85 mmol) were added, in that order, while cooled on ice to a DMF (8 mL) solution of 4-(3-aminophenyl)-N-(2-(3,4-dimethoxyphenyl)ethyl)pyrimidine-2-amine (500 mg, 1.43 mmol) synthesized in Reference Example 3, and N,N-dimethyl-β-alanine (263 mg, 1.71 mmol) and the mixture was then stirred for another 13 hours at room temperature. The reaction solution was concentrated at reduced pressure, saturated aqueous sodium bicarbonate was added to the residue, and the product was extracted with ethyl acetate. The ethyl acetate extract solution was washed with water and with saturated brine, was dried over magnesium sulfate, and was then concentrated at reduced pressure. The residue was purified by silica gel column chromatography (basic silica gel, ethyl acetate:hexane = 1:1 → ethyl acetate) to give the titled compound (400 mg, yield: 62%). Melting point: 107 - 108°C (ethyl acetate-hexane).
¹H-NMR (CDCl₃) δ : 2.38 (6H, s), 2.53 (2H, dd, J = 6.0, 4.2 Hz), 2.68 (2H, dd, J = 6.0, 5.1 Hz), 2.92 (2H, t, J = 6.9 Hz), 3.76 (2H, q, J = 6.9 Hz), 3.86 (6H, s), 5.20 (1H, t, J = 5.7 Hz), 6.77 - 6.85 (3H, m), 6.98 (1H, d, J = 5.1 Hz), 7.40 (1H, t, J = 8.1 Hz), 7.65 (1H, dd, J = 7.2, 1.2 Hz), 7.74 (1H, d, J = 7.8 Hz), 8.16 (1H, br s), 8.33 (1H, d, J = 5.1 Hz), 11.06 (1H, br s).

### Working Example 30

### N-(3-(2-((2-(3,4-Dimethoxyphenyl)ethyl)amino)pyrimidin-4-yl)phenyl)-N³,N³-dimethyl-β-alaninamide hydrochloride

4 N hydrogen chloride-ethyl acetate solution (194 µL, 7.78 mmol) was added at room temperature to a methylene chloride (4 mL) solution of N-(3-(2-((2-(3,4-dimethoxyphenyl)ethyl)amino)pyrimidin-4-yl)phenyl)-N³,N³-dimethyl-β-alaninamide (350 mg, 0.78 mmol) synthesized in Working Example 29. The mixture was stirred for another 10 min at room temperature, and the solvent was then distilled off at reduced pressure to give 290 mg of the titled compound (yield: 77%). Amorphous powder.
¹H-NMR (CDCl₃) δ : 2.41 (6H, s), 2.91 (4H, t, J = 6.6 Hz), 3.10 - 3.19 (2H, m), 3.70 - 3.80 (2H, m), 3.86 (6H, s), 5.70 - 5.82 (1H, m), 6.75-6.87 (3H, m), 6.97 (1H, d, J = 5.4 Hz), 7.35 - 7.48 (1H, m), 7.68- 7.81 (2H, m), 8.26 (1H, br s), 8.31 (1H, d, J = 5.1 Hz), 10.28 (1H, br s).

### Working Example 31

### N-(3-(2-(2-(3,4-Dimethoxy-phenyl)ethylamino)pyrimidin-4-yl)phenyl)-4-(piperidin-1-yl)butanamide

The titled compound was obtained from 4-bromo-N-(3-(2-(2-(3,4-dimethoxyphenyl)ethylamino)pyrimidin-4-yl)phenyl)butanamide and piperidine in the same manner as in Working Example 19. Yield: 59%. Melting point: 111 - 112°C (ethyl acetate-diisopropyl ether).
¹H-NMR (CDCl₃) δ : 1.46 - 1.49 (2H, m), 1.60 - 1.67 (4H, m), 1.87 - 1.95 (2H, m), 2.42 - 2.50 (8H, m), 2.91 (2H, t, J = 6.9 Hz), 3.73 - 3.79 (2H, m), 3.87 (6H, s), 5.17 (1H, t, J = 5.7 Hz), 6.78 -6.81 (3H, m), 6.98 (1H, d, J = 5.1 Hz), 7.41 (1H, t, J = 7.8 Hz), 7.72 - 7.75 (2H, m), 8.14 (1H, s), 8.32 (1H, d, J = 5.1 Hz), 9.48 (1H, s).

### Working Example 32

### N-(3-(2-(2-(3,4-dimethoxy-phenyl)ethylamino)pyrimidin-4-yl)phenyl)-4-(piperidin-1-yl)butanamide hydrochloride

N-(3-(2-(2-(3,4-Dimethoxy-phenyl)ethylamino)pyrimidin-4-yl)phenyl)-4-(piperidin-1-yl)butanamide (72 mg, 0.14 mmol) synthesized in Working Example 31 was dissolved in ethyl acetate-methanol (10:1, 22 mL), 4 N hydrogen chloride-ethyl acetate solution (36 µL, 0.14 mmol) was added, and the mixture was concentrated to give the titled compound. Yield: 84%. Amorphous powder.
¹H-NMR (DMSO-d₆) δ : 1.30 - 1.45 (1H, m), 1.67 - 1.77 (5H, m), 1.99 - 2.05 (2H, m), 2.47 (2H, t, J = 7.0Hz), 2.81 - 2.85 (4H, m), 3.00 - 3.08 (2H, m), 3.37 - 3.47 (2H, m), 3.53 - 3.65 (2H, m), 3.71 (3H, s), 3.73 (3H, s), 6.77-6.88 (3H, m), 7.01 (1H, d, J = 5.1 Hz), 7.22 (1H, br s), 7.43 (1H, t, J = 7.9 Hz), 7.75 (2H, brd, J = 7.9Hz), 8.37 (2H, br s), 10.12 (1H, br s), 10.32 (1H, s).

### Working Example 33

### 4-Amino-N-(3-(2-((2-(3,4-dimethoxyphenyl)ethyl)amino)pyrimidin-4-yl)phenyl)butanamide hydrochloride

The titled compound was obtained from tert-butyl (4-((3-(2-((2-(3,4-dimethoxyphenyl)ethyl)amino)pyrimidin-4-yl)phenyl)amino)-4-oxobutyl)carbamate synthesized in Working Example 8 in the same manner as in Working Example 6. Yield: quantitative; melting point: 141 - 143°C (methanol-ethyl acetate).
¹H-NMR (DMSO-d₆) δ : 1.86 - 1.93 (2H, m), 2.48 - 2.51 (2H, m), 2.82 - 2.89 (4H, m), 3.69 - 3.73 (8H, m), 6.80 - 6.91 (3H, m), 7.28 (1H, br s), 7.50 (1H, t, J = 7.8 Hz), 7.83 (2H, br s), 8.04 (3H, br s), 8.31 (1H, br s), 8.44 (1H, d, J = 6.0 Hz), 8.50 (1H, br s), 10.47 (1H, s).

### Working Example 34

### (3-(2-(2-(3,4-dimethoxyphenyl)ethylamino)pyrimidin-4-yl)-N-(3-dimethylaminopropyl)benzamide

The titled compound was obtained from 3-(2-(2-(3,4-dimethoxyphenyl)ethylamino)pyrimidin-4-yl)benzoic acid synthesized in Reference Example 6 and N,N-dimethyl-1,3-propane diamine in the same manner as in Working Example 2. Yield: 58%. Melting point: 110 - 111°C (ethyl acetate-diisopropyl ether).
¹H-NMR (CDCl₃) δ : 1.74-1.82 (2H, m), 2.29 (6H, s), 2.51 (2H, t, J = 5.7 Hz), 2.91 (2H, t, J = 6.9 Hz), 3.60 (2H, q, J = 5.7 Hz), 3.77 (2H, q, J = 6.9 Hz), 3.87 (6H, s), 5.20 (1H, t, J = 5.7 Hz), 6.78 - 6.82 (3H, m), 7.01 (1H, d, J = 5.4 Hz), 7.52 (1H, t, J = 7.5 Hz), 7.85 (1H, d, J = 7.8 Hz), 8.16 (1H, brd, J = 7.8 Hz), 8.36 (1H, d, J = 5.4 Hz), 8.42 (1H, s), 8.65 (1H, s).

### Working Example 35

### Tert-butyl (3-((3-(2-((2-(3,4-dimethoxyphenyl)ethyl)amino)pyrimidin-4-yl)benzoyl) amino)propyl)carbamate

The titled compound was obtained from 3-(2-(2-(3,4-dimethoxyphenyl)ethylamino)pyrimidin-4-yl)benzoic acid synthesized in Reference Example 6 and N-(tert-butoxycarbonyl)-1,3-diaminopropane in the same manner as in Working Example 2. Yield: 85%. Melting point: 152 - 153°C (ethyl acetate-diisopropyl ether).
¹H-NMR (CDCl₃) δ : 1.46 (9H, s), 1.62 - 1.78 (2H, m), 2.92 (2H, t, J = 6.9 Hz), 3.28 (2H, q, J = 6.3 Hz), 3.54 (2H, q, J = 6.0 Hz), 3.77 (2H, q, J = 6.3 Hz), 3.87 (6H, s), 4.88 (1H, br t), 5.21 (1H, t, J = 5.4 Hz), 6.78-6.82 (3H, m), 7.11 (1H, d, J = 5.1 Hz), 7.49 (1H, br s), 7.55 (1H, t, J = 7.8 Hz), 7.97 (1H, d, J = 7.5 Hz), 8.23 (1H, brd, J = 7.8 Hz), 8.35 (1H, t, J = 5.1 Hz), 8.53 (1H, s).

### Working Example 36

### N-(3-Aminopropyl)-3-(2-(2-(3,4-dimethoxyphenyl)ethylamino)pyrimidin-4-yl)benzamide dihydrochloride

The titled compound was obtained from tert-butyl (3-((3-(2-((2-(3,4-dimethoxyphenyl)ethyl)amino)pyrimidin-4-yl)benzoyl) amino)propyl)carbamate synthesized in Working Example 35 in the same manner as in Working Example 6. Yield: quantitative; melting point: 167 - 168°C (methanol-ethyl acetate).
¹H-NMR (DMSO-d₆) δ: 1.83-1.87 (2H, m), 2.83-2.88 (4H, m), 3.35-3.41 (2H, m), 3.66-3.73 (8H, m), 6.79-6.89 (4H, m), 7.43 (1H, br s), 7.66 (1H, t, J = 7.8 Hz), 7.94 (4H, br s), 8.07 (1H, d, J = 7.8 Hz), 8.31 - 8.33 (1H, m), 8.46 (1H, d, J = 5.7 Hz), 8.65 (1H, s), 8.96 (1H, br s).

### Working Example 37

### 3-(2-(2-(3,4-Dimethoxyphenyl)ethylamino)pyrimidin-4-yl)-N-(3-dimethylaminopropyl)-N-methylbenzamide hydrochloride

The titled compound was obtained from 3-(2-(2-(3,4-dimethoxyphenyl)ethylamino)pyrimidin-4-yl)benzoic acid and N,N,N'-trimethyl-1,3-propanediamine in the same manner as in Working Example 2. Yield: 81 %. Melting point: 121 - 123°C (ethanol-ethyl acetate).
¹H-NMR (DMSO-d₆) δ : 1.99-2.03 (2H, br s), 2.51 - 3.27 (13H, m), 3.55-3.37 (10H, m), 6.78 - 6.90 (3H, m), 7.46 (1H, br s), 7.64 - 7.66 (2H, m), 8.22 - 8.25 (3H, m), 8.47 (1H, d, J = 5.7 Hz), 10.51 (1H, br s).

### Working Example 38

### 3-(2-(2-(3,4-Dimethoxyphenyl)ethylamino)pyrimidin-4-yl)-N-(3-pyrrolidin-1-ylpropyl)benzamide

The titled compound was obtained from 3-(2-(2-(3,4-dimethoxyphenyl)ethylamino)pyrimidin-4-yl)benzoic acid and 1-(3-aminopropyl)pyrrolidine in the same manner as in Working Example 2. Yield: 52%. Melting point: 109 - 110°C (ethyl acetate-diisopropyl ether).
¹H-NMR (CDCl₃) δ : 1.71 - 1.84 (6H, m), 2.55 (4H, br t), 2.71 (2H, t, J = 5.7 Hz), 2.91 (2H, t, J = 6.9 Hz), 3.61 (2H, q, J = 5.7 Hz), 3.77 (2H, q, J = 6.9 Hz), 3.87 (6H, s), 5.23 (1H, t, J = 5.7 Hz), 6.78 - 6.82 (3H, m), 7.02 (1H, d, J = 5.1 Hz), 7.50 (1H, t, J = 7.8 Hz), 7.81 (1H, d, J = 7.8 Hz), 8.15 (1H, brd, J = 7.5 Hz), 8.36 (1H, d, J = 5.1 Hz), 8.44 (1H, s), 8.85 (1H, br s).

### Working Example 39

### 3-(6-(2-(3,4-Dimethoxyphenyl)ethoxy)pyridin-2-yl)-N-(2-pyrrolidin-1-ylethyl)benzamide

The titled compound was obtained using 3-(6-(2-(3,4-dimethoxyphenyl)ethoxy)pyridin-2-yl)benzoic acid obtained in Reference Example 23 and 2-pyrrolidin-1-ylethanamine in the same manner as in Working Example 2.
Yield: 45%; melting point: 126 - 127°C (ethyl acetate-hexane).
¹H-NMR (CDCl₃) δ : 1.73 - 1.82 (4H, m), 2.47 - 2.61 (4H, m), 2.70 (2H, t, J = 6.0 Hz), 3.09 (2H, t, J = 6.9 Hz), 3.57 (2H, q, J = 6.0 Hz), 3.86 (3H, s), 3.87 (3H, s), 4.63 (2H, t, J = 6.9 Hz), 6.70 (1H, d, J = 7.8 Hz), 6.81 - 6.88 (4H, m), 7.38 (1H, d, J = 7.2 Hz), 7.50 (1H, t, J = 7.5 Hz), 7.64 (1H, t, J = 8.1 Hz), 7.76 (1H, d, J = 8.4 Hz), 8.16 (1H, d, J = 8.4 Hz), 8.42 (1H, t, J = 1.5 Hz).

### Working Example 40

### 3'-(2-(3,4-Dimethoxyphenyl)ethoxy)-N-(2-pyrrolidin-1-ylethyl)biphenyl-3-carboxamide

The titled compound was obtained using 3'-(2-(3,4-dimethoxyphenyl)ethoxy)biphenyl-3-carboxylic acid obtained in Reference Example 24 and 2-pyrrolidin-1-ylethanamine in the same manner as in Working Example 2. Yield: 30%. Melting point: 101 - 102°C. (Ethyl acetate-hexane).
¹H-NMR (CDCl₃) δ : 1.75 - 1.82 (4H, m), 2.52 - 2.61 (4H, m), 2.72 (2H, t, J = 6.0 Hz), 3.08 (2H, t, J = 6.9 Hz), 3.58 (2H, q, J = 6.0 Hz), 3.87 (3H, s), 3.89 (3H, s), 4.22 (2H, t, J = 6.9 Hz), 5.22 (1H, brs), 6.86 -6.94 (5H, m), 7.12 - 7.15 (1H, m), 7.20 (1H, d, J = 8.4 Hz), 7.36 (1H, t, J = 7.8 Hz), 7.48 (1H, t, J = 7.8 Hz), 7.70 (1H, dt, J = 7.8, 1.2 Hz), 8.01 (1H, d, J = 1.2 Hz).

### Working Example 41

### 3-(6-(2-(3,4-Dimethoxyphenyl)ethoxy)pyridin-2-yl)-N-(tetrahydro-2H-pyran-4-yl)benzamide

The titled compound was obtained using 3-(6-(2-(3,4-dimethoxyphenyl)ethoxy)pyridin-2-yl)benzoic acid obtained in Reference Example 23 and tetrahydro-2H-pyran-4-amine in the same manner as in Working Example 2. Yield: 68%. Melting point: 172 - 173°C. (Ethyl acetate-hexane).
¹H-NMR (CDCl₃) δ : 1.50 - 1.64 (2H, m), 1.95-2.07 (2H, m), 3.09 (2H, t, J = 6.9 Hz), 3.49 - 3.59 (2H, m), 3.86 (6H, s), 3.97 - 4.04 (2H, m), 4.18 - 4.32 (1H, m), 4.62 (2H, t, J = 6.9 Hz), 6.03 (1H, d, J = 8.1 Hz), 6.71 (1H, d, J = 8.1 Hz), 6.79 - 6.85 (3H, m), 7.38 (1H, d, J = 7.2 Hz), 7.50 (1H, t, J = 7.8 Hz), 7.65 (1H, t, J = 7.8 Hz), 7.73 (1H, d, J = 8.4 Hz), 8.16 (1H, d, J = 8.1 Hz), 8.38 (1H, s).

### Working Example 42

### 3-(2-(2-(3,4-Dimethoxyphenyl)ethoxy)pyridin-4-yl)-N-(2-pyrrolidin-1-ylethyl)benzamide

The titled compound was obtained using 3-(2-(2-(3,4-dimethoxyphenyl)ethoxy)pyridin-4-yl)benzoic acid obtained in Reference Example 33 and 2-pyrrolidin-1-ylethanamine in the same manner as in Working Example 2. Yield: 50%. Melting point: 116 - 117°C. (Ethyl acetate-hexane).
¹H-NMR (CDCl₃) δ : 1.70 - 1.82 (4H, m), 2.50 - 2.64 (4H, m), 2.72 (2H, t, J = 6.0 Hz), 3.06 (2H, t, J = 7.2 Hz), 3.58 (2H, q, J = 6.0 Hz), 3.86 (3H, s), 3.88 (3H, s), 4.55 (2H, t, J = 7.2 Hz), 6.77 - 6.98 (5H, m), 7.12 (1H, d, J = 6.9 Hz), 7.50 (1H, t, J = 7.5 Hz), 7.71 (1H, t, J = 8.7 Hz), 7.79 (1H, d, J = 7.5 Hz), 8.06 (1H, s), 8.20 (1H, d, J = 6.0 Hz).

### Working Example 43

### 3-(4-(2-(3,4-Dimethoxyphenyl)ethoxy)pyridin-2-yl)-N-(2-pyrrolidin-1-ylethyl)benzamide

The titled compound was obtained using 3-(4-(2-(3,4-dimethoxyphenyl)ethoxy)pyridin-2-yl)benzoic acid obtained in Reference Example 31 and 2-pyrrolidin-1-ylethanamine in the same manner as in Working Example 2. Yield: 77%. Melting point: 114 - 115°C. (Ethyl acetate-hexane).
¹H-NMR (CDCl₃) δ : 1.70 - 1.82 (4H, m), 2.52 - 2.62 (4H, m), 2.71 (2H, t, J = 6.0 Hz), 3.09 (2H, t, J = 6.9 Hz), 3.58 (2H, q, J = 6.0 Hz), 3.87(3H, s), 3.89 (3H, s), 4.27 (2H, t, J = 6.9 Hz), 6.75 - 6.90 (5H, m), 7.27 (1H, d, J = 2.4 Hz), 7.52 (1H, t, J = 8.1 Hz), 7.81 (1H, dd, J = 7.8, 1.2 Hz), 8.10 (1H, dd, J = 7.8, 1.2 Hz), 8.34 (1H, t, J = 1.5 Hz), 8.49 (1H, d, J = 6.0 Hz).

### Working Example 44

### 3-(5-(2-(3,4-Dimethoxyphenyl)ethoxy)pyridin-3-yl)-N-(2-pyrrolidin-1-ylethyl)benzamide

The titled compound was obtained using 3-(5-(2-(3,4-dimethoxyphenyl)ethoxy)pyridin-3-yl)benzoic acid obtained in Reference Example 32 and 2-pyrrolidin-1-ylethanamine in the same manner as in Working Example 2. Yield: 77%. Melting point: 112 - 113°C. (Ethyl acetate-hexane).
¹H-NMR (CDCl₃) δ : 1.72 - 1.8 (4H, m), 2.50 - 2.62 (4H, m), 2.72 (2H, t, J = 6.0 Hz), 3.09 (2H, t, J = 6.9 Hz), 3.57 (2H, q, J = 6.0 Hz), 3.87(3H, s), 3.89 (3H, s), 4.27 (2H, t, J = 6.9 Hz), 6.80 - 6.91 (4H, m), 7.23 - 7.39 (1H, m), 7.52 (1H, t, J = 7.5 Hz), 7.68 (1H, dd, J = 6.6, 1.5 Hz), 7.74 (1H, d, J = 7.5 Hz), 8.00 - 8.02 (1H, m), 8.31 (1H, d, J = 2.7 Hz), 8.45 (1H, d, J = 1.5 Hz).

### Working Example 45

### 3-(4-(2-(3,4-Dimethoxyphenyl)ethoxy)pyrimidin-2-yl)-N-(2-pyrrolidin-1-ylethyl)benzamide

The titled compound was obtained using 3-(4-(2-(3,4-dimethoxyphenyl)ethoxy)pyrimidin-2-yl)benzoic acid obtained in Reference Example 22 and 2-pyrrolidin-1-ylethanamine in the same manner as in Working Example 2. Yield: 47%. Melting point: 147 - 148°C. (Ethyl acetate-hexane).
¹H-NMR (CDCl₃) δ : 1.73 - 1.82 (4H, m), 2.50 - 2.61 (4H, m), 2.71 (2H, t, J = 6.0 Hz), 3.09 (2H, t, J = 6.9 Hz), 3.58 (2H, q, J = 6.0 Hz), 3.86 (3H, s), 3.88 (3H, s), 4.70 (2H, t, J = 6.9 Hz), 6.63 (1H, d, J = 5.7 Hz), 6.80 - 6.91 (4H, m), 7.53 (1H, t, J = 7.5 Hz), 7.91 (1H, t, J = 7.5 Hz), 8.47 - 8.55 (2H, m), 8.78 (1H, s).

### Working Example 46

### 3-(2-(2-(3,4-Dimethoxyphenyl)ethoxy)pyrimidin-4-yl)-N-(2-pyrrolidin-1-ylethyl)benzamide

The titled compound was obtained using 3-(2-(2-(3,4-dimethoxyphenyl)ethoxy)pyrimidin-4-yl)benzoic acid obtained in Reference Example 21 and 2-pyrrolidin-1-ylethanamine in the same manner as in Working Example 2. Yield: 24%. Melting point: 86 - 87°C. (Ethyl acetate-hexane).
¹H-NMR (CDCl₃) δ : 1.75 - 1.83 (4H, m), 2.50 - 2.63 (4H, m), 2.72 (2H, t, J = 6.0 Hz), 3.13 (2H, t, J = 7.2 Hz), 3.58 (2H, q, J = 6.0 Hz), 3.85 (3H, s), 3.88 (3H, s), 4.65 (2H, t, J = 7.2 Hz), 6.78 - 6.89 (4H, m), 7.40 (1H, d, J = 5.4 Hz), 7.55 (1H, t, J = 7.5 Hz), 7.88 (1H, d, J = 7.8 Hz), 8.22 (1H, d, J = 7.8 Hz), 8.48 (1H, s), 8.56 (1H, d, J = 5.4 Hz).

### Working Example 47

### 3-(6-(2-(3,4-Dimethoxyphenyl)ethoxy)pyridin-2-yl)-N-ethylbenzamide

The titled compound was obtained using 3-(6-(2-(3,4-dimethoxyphenyl)ethoxy)pyridin-2-yl)benzoic acid obtained in Reference Example 23 and ethylamine in the same manner as in Working Example 2. Yield: 72%. Melting point: 137 - 138°C. (Ethyl acetate-hexane).
¹H-NMR (CDCl₃) δ : 1.26 (3H, t, J = 8.1 Hz), 3.09 (2H, t, J = 7.2 Hz), 3.45-3.57 (2H, m), 3.86(3H, s), 3.87 (3H, s), 4.63 (2H, t, J = 7.2 Hz), 6.16 (1H, br s), 6.70 (1H, d, J = 8.1 Hz), 6.81 - 6.89 (3H, m), 7.37 (1H, d, J = 7.5 Hz), 7.49 (1H, t, J = 7.8 Hz), 7.64 (1H, t, J = 7.8 Hz), 7.74 (1H, dt, J = 7.5, 1.5 Hz), 8.15 (1H, dt, J = 7.5, 1.5 Hz), 8.38 (1H, t, J = 1.8 Hz).

### Working Example 48

### N-Cyclopropyl-3-(6-(2-(3,4-dimethoxyphenyl)ethoxy)pyridin-2-yl)benzamide

The titled compound was obtained using 3-(6-(2-(3,4-dimethoxyphenyl)ethoxy)pyridin-2-yl)benzoic acid obtained in Reference Example 23 and cyclopropanamine in the same manner as in Working Example 2. Yield: 76%. Melting point: 163 - 164°C. (Ethyl acetate-hexane).
¹H-NMR (CDCl₃) δ : 0.59 - 0.66 (2H, m), 0.84 - 0.91 (2H, m), 2.82 - 3.00 (1H, m), 3.09 (2H, t, J = 7.2 Hz), 3.86 (3H, s), 3.87 (3H, s), 4.62 (2H, t, J = 7.2 Hz), 6.27 (1H, br s), 6.70 (1H, d, J = 8.4 Hz), 6.82 - 6.88 (3H, m), 7.37 (1H, d, J = 7.5 Hz), 7.48 (1H, t, J = 8.1 Hz), 7.64 (1H, t, J = 7.8 Hz), 7.71 (1H, d, J = 7.5 Hz), 8.15 (1H, dd, J = 6.3, 0.9 Hz), 8.35 (1H, s).

### Working Example 49

### (3-(6-(2-(3,4-Dimethoxyphenyl)ethoxy)pyridin-2-yl)benzamide

A mixture of 2-chloro-6-(2-(3,4-dimethoxyphenyl)ethoxy)pyridine (300 mg, 1.02 mmol) obtained in Reference Example 7, (3-carbamoylphenyl)boronic acid (203 mg, 1.23 mmol), and tetrakis(triphenylphosphine)palladium (0) (59 mg, 0.051 mmol) in 2 N sodium carbonate aqueous solution (10 mL)-1,2-dimethoxyethane (10 mL) was reacted for 16 hours at 90°C in a nitrogen atmosphere. Water was added to the reaction solution, and the product was extracted with ethyl acetate. The combined organic layers were washed with saturated brine, then dried over anhydrous sodium sulfate, and then concentrated at reduced pressure, and the residue was purified by silica gel column chromatography (ethyl acetate-hexane 1:1) to give 240 mg of the titled compound (yield 62%). Melting point: 141 - 142°C.
¹H-NMR (CDCl₃) δ : 3.09 (2H, t, J = 7.2 Hz), 3.86 (3H, s), 3.87 (3H, s), 4.63 (2H, t, J = 7.2 Hz), 5.80 (1H, br s), 6.20 (1H, br s), 6.71 (1H, d, J = 8.4 Hz), 6.79 - 6.89 (3H, m), 7.38 (1H, d, J = 7.2 Hz), 7.52 (1H, t, J = 7.8 Hz), 7.64 (1H, t, J = 7.8 Hz), 7.81 (1H, d, J = 7.5 Hz), 8.19 (1H, d, J = 7.8 Hz), 8.45 (1H, s).

### Working Example 50

### 3-(6-(2-(3,4-Dimethoxyphenyl)ethoxy)pyridin-2-yl)-N-prop-2-yn-1-ylbenzamide

WSC (123 mg, 0.64 mmol) and HOBt (86.5 mg, 0.64 mmol) were added a DMF (3 mL) solution of 3-(6-(2-(3,4-dimethoxyphenyl)ethoxy)pyridin-2-yl)benzoic acid (200 mg, 0.53 mmol) obtained in Reference Example 23 and prop-2-yn-1-amine (0.0439 mL, 0.64 mmol), and the mixture was stirred for 16 hours at room temperature. The addition of saturated aqueous sodium bicarbonate to the reaction solution was followed by extraction with ethyl acetate. The extract was washed with water and dried over anhydrous magnesium sulfate, and the solvent was then distilled off at reduced pressure. The residue was purified by silica gel column chromatography and recrystallized from ethyl acetate-hexane to give 195 mg of the titled compound (yield: 88%). Melting point: 142 - 143°C (ethyl acetate-hexane).
¹H-NMR (DMSO-d₆):δ 3.03 (2H, t, J = 7.0 Hz), 3.15 (1H, t, J = 2.5 Hz), 3.72 (3H, s), 3.74 (3H, s), 4.10 (2H, dd, J = 5.5, 2.5 Hz), 4.57 (2H, t, J = 7.0 Hz), 6.81 (1H, d, J = 8.0 Hz), 6.83 - 6.91 (2H, m), 6.95 (1H, d, J = 1.5 Hz), 7.59 (1H, t, J = 8.0 Hz), 7.63 (1H, d, J = 7.2 Hz), 7.79 - 7.86 (1H, m), 7.90 (1H, d, J = 8.0 Hz), 8.25 (1H, d, J = 8.0 Hz), 8.53 (1H, s), 9.07 (1H, t, J = 5.7 Hz).

### Working Example 51

### 2,2,2-Trichloroethyl 3-(6-(2-(3,4-dimethoxyphenyl)ethoxy)pyridin-2-yl)phenyl)carbamate

The titled compound was synthesized using 3-(6-(2-(3,4-dimethoxyphenyl)ethoxy)pyridin-2-yl)aniline obtained in Reference Example 19 in the same manner as in Reference Example 60. Yield: 93%. Melting point: 91 - 92°C (ethyl acetate).
¹H-NMR (CDCl₃):δ 3.09 (2H, t, J = 7.2 Hz), 3.87 (3H, s), 3.87 (3H, s), 4.63 (2H, t, J = 7.2 Hz), 4.85 (2H, s), 6.69 (1H, d, J = 8.3 Hz), 6.81 - 6.90 (3H, m), 6.98 (1H, br s), 7.33 (1H, d, J = 7.6 Hz), 7.42 (1H, t, J = 8.0 Hz), 7.55 (1H, d, J = 7.2 Hz), 7.59- 7.66 (1H, m), 7.76 (1H, d, J = 8.0 Hz), 8.03 (1H, s).

### Working Example 52

### 1-3-(6-(2-(3,4-Dimethoxyphenyl)ethoxy)pyridin-2-yl)phenyl)-3-(6-fluoropyridin-3-yl)urea

A DMSO (3 mL) solution of 3-(6-(2-(3,4-dimethoxyphenyl)ethoxy)pyridin-2-yl)aniline (200 mg, 0.57 mmol) obtained in Reference Example 19, 2,2,2-trichloroethyl (6-fluoropyridin-3-yl)carbamate (196 mg, 0.68 mmol) obtained in Reference Example 60, and diisopropylethylamine (0.118 mL, 0.68 mol) was heated and stirred for 14 hours at 70°C. The addition of water to the reaction solution was followed by extraction with ethyl acetate. The extract was washed with water and saturated brine, and was dried over anhydrous magnesium sulfate, and the solvent was then distilled off at reduced pressure. The residue was purified by silica gel column chromatography and recrystallized from ethyl acetate-hexane to give 160 mg of the titled compound (yield: 57%). Melting point: 97 - 98°C (ethyl acetate-hexane).
¹H-NMR (DMSO-d₆):δ 3.03 (2H, t, J = 7.2 Hz), 3.70 (3H, s), 3.73 (3H, s), 4.56 (2H, t, J = 7.2 Hz), 6.77 (1H, d, J = 8.0 Hz), 6.86 (2H, s), 6.94 (1H, s), 7.13 (1H, dd, J = 8.9, 3.2 Hz), 7.39 (1H, t, J = 8.0 Hz), 7.48 (1H, d, J = 7.6 Hz), 7.54 (1H, d, J = 9.1 Hz), 7.68 (1H, d, J = 7.6 Hz), 7.74 - 7.83 (1H, m), 8.03 - 8.11 (1H, m), 8.17 (1H, s), 8.29 (1H, s), 8.95 (2H, d, J = 5.7 Hz).

### Working Example 53

### 1-3-(6-(2-(3,4-Dimethoxyphenyl)ethoxy)pyridin-2-yl)phenyl)-3-(2-(dimethylamino)ethyl)urea

The titled compound was synthesized using 2,2,2-trichloroethyl 3-(6-(2-(3,4-dimethoxyphenyl)ethoxy)pyridin-2-yl)phenyl)carbamate obtained in Working Example 51 and N,N-dimethylethane-1,2-diamine in the same manner as in Working Example 52. Yield: 67%. Melting point: 93 - 122°C (ethyl acetate-hexane).
¹H-NMR (DMSO-d₆):δ 2.17 (6H, s), 2.33 (2H, t, J = 6.1 Hz), 3.02 (2H, t, J = 7.0 Hz), 3.19 (2H, q, J = 5.9 Hz), 3.72 (3H, s), 3.74 (3H, s), 4.55 (2H, t, J = 7.0 Hz), 6.11 (1H, t, J = 5.1 Hz), 6.75 (1H, d, J = 8.3 Hz), 6.87 (2H, d, J = 2.3 Hz), 6.94 (1H, s), 7.31 (1H, t, J = 8.0 Hz), 7.43 (1H, d, J = 7.2 Hz), 7.49 (1H, d, J = 8.3 Hz), 7.56 (1H, d, J = 7.6 Hz), 7.77 (1H, t, J = 8.0 Hz), 8.05 (1H, s), 8.77 (1H, s).

### Working Example 54

### 3-3-(6-(2-(3,4-Dimethoxyphenyl)ethoxy)pyridin-2-yl)phenyl)-1-(2-(dimethylamino)ethyl)-1-methyl urea

The titled compound was synthesized using 2,2,2-trichloroethyl 3-(6-(2-(3,4-dimethoxyphenyl)ethoxy)pyridin-2-yl)phenyl)carbamate obtained in Working Example 51 and N,N,N'-trimethylethane-1,2-diamine in the same manner as in Working Example 52. Yield: 70%. Melting point: 119 - 120°C (ethyl acetate-hexane).
¹H-NMR (DMSO-d₆):δ 2.19 (6H, s), 2.43 (2H, t, J = 5.7 Hz), 2.93 (3H, s), 3.01 (2H, t, J = 7.0 Hz), 3.38 (2H, t, J = 5.7 Hz), 3.72 (3H, s), 3.73 (3H, s), 4.54 (2H, t, J = 7.2 Hz), 6.75 (1H, d, J = 8.0 Hz), 6.81 - 6.90 (2H, m), 6.94 (1H, s), 7.32 (1H, t, J = 7.8 Hz), 7.44 (1H, d, J = 7.2 Hz), 7.51 (1H, d, J = 8.3 Hz), 7.60 (1H, d, J = 7.6 Hz), 7.77 (1H, t, J = 7.8 Hz), 8.02 (1H, s), 9.38 (1H, br s).

### Working Example 55

### 3-(6-(2-(2,4-Dichlorophenyl)ethoxy)pyridin-2-yl)-N-pyridin-2-ylbenzamide

2-Aminopyridine (80 mg, 0.85 mmol), HATU (323 mg, 0.85 mmol), and diisopropylethylamine (0.148 mL, 0.85 mmol) were added to a DMF (3 mL) solution of 3-(6-(2-(2,4-dichlorophenyl)ethoxy)pyridin-2-yl)benzoic acid (0.30 g, 0.77 mmol) obtained in Reference Example 28, and the mixture was stirred for 16 hours at 80°C. Ethyl acetate was added to the reaction solution, the solution was washed with water and with saturated brine, and concentrated. The residue was purified by silica gel column chromatography (hexane-ethyl acetate = 80:20 → 30:70) and was recrystallized from ethyl acetate-hexane to give 61 mg of the titled compound in the form of crystals (yield 17%). Melting point: 113 - 115°C.
¹H-NMR (CDCl₃) δ: 3.25 (2H, t, J = 7.2 Hz), 4.68 (2H, t, J = 7.2 Hz), 6.70 (1H, d, J = 8.1 Hz), 7.05 - 7.19 (2H, m), 7.21 - 7.42 (3H, m), 7.61 (1H, t, J = 7.2 Hz), 7.68 (1H, t, J = 7.2 Hz), 7.78 (1H, t, J = 7.8 Hz), 7.92 (1H, d, J = 7.8 Hz), 8.19 - 8.32 (2H, m), 8.41 (1H, d, J = 9.0 Hz), 8.55 (1H, s), 8.65 (1H, br s).

### Working Example 56

### 3-(6-(2-(2,4-Dichlorophenyl)ethoxy)pyridin-2-yl)-N-(2-pyrrolidin-1-ylethyl)benzamide

The titled compound was obtained using 3-(6-(2-(2,4-dichlorophenyl)ethoxy)pyridin-2-yl)benzoic acid obtained in Reference Example 28 in the same manner as in Working Example 2. Yield: 55%. Melting point: 115 - 116°C. (Ethyl acetate-hexane).
¹H-NMR (CDCl₃) δ : 1.72-1.85 (4H, m), 2.50 - 2.62 (4H, m), 2.72 (2H, t, J = 6.0 Hz), 3.24 (2H, t, J = 6.3 Hz), 3.59 (2H, q, J = 6.0 Hz), 4.66 (2H, t, J = 6.9 Hz), 6.68 (1H, d, J = 8.1 Hz), 6.89 (1H, br s), 7.17 (1H, dd, J = 8.4, 2.1 Hz), 7.25 - 7.29 (1H, m), 7.33 - 7.38 (2H, m), 7.51 (1H, t, J = 7.8 Hz), 7.64 (1H, t, J = 8.4 Hz), 7.77 (1H, d, J = 8.4 Hz), 8.14 (1H, d, J = 6.9 Hz), 8.42 (1H, s).

### Working Example 57

### 3-(6-((2,4-Dichlorobenzyl)oxy)pyridin-2-yl)-N-pyridin-2-ylbenzamide

The titled compound was obtained using 3-(6-((2,4-dichlorobenzyl)oxy)pyridin-2-yl)benzoic acid obtained in Reference Example 30 and 2-aminopyridine in the same manner as in Working Example 55. Yield: 12%. Melting point: 133 - 134°C. (Ethyl acetate-hexane).
¹H-NMR (CDCl₃) δ : 5.60 (2H, s), 6.82 (1H, d, J = 8.4 Hz), 7.00 - 7.10 (1H, m), 7.20 - 7.30 (1H, m), 7.28 - 7.80 (6H, m), 7.90 - 7.96 (1H, m), 8.17 - 8.22 (1H, m), 8.29 - 8.33 (1H, m), 8.46 (1H, d, J = 8.4 Hz), 8.54 (1H, t, J = 2.1 Hz), 8.69 (1H, br s).

### Working Example 58

### 3-(6-((2,4-Dichlorobenzyl)oxy)pyridin-2-yl)-N-(2-pyrrolidin-1-ylethyl)benzamide

The titled compound was obtained using 3-(6-((2,4-dichlorobenzyl)oxy)pyridin-2-yl)benzoic acid obtained in Reference Example 30 and 2-pyrrolidin-1-ylethanamine in the same manner as in Working Example 2. Yield: 42%. Melting point: 96 - 97°C. (Ethyl acetate-hexane).
¹H-NMR (CDCl₃) δ : 1.70 - 1.82 (4H, m), 2.48-2.62 (4H, m), 2.73 (2H, t, J = 6.0 Hz), 3.59 (2H, q, J = 6.0 Hz), 5.58 (2H, s), 6.76 - 6.90 (2H, m), 7.20 - 7.30 (1H, m), 7.41 - 7.58 (4H, m), 7.64 - 7.79 (2H, m), 8.13 (1H, dt, J = 8.4, 1.8 Hz), 8.41 (1H, t, J = 1.8 Hz).

### Working Example 59

### N-(2-Hydroxyethyl)-3-(6-(2-(3-methylphenyl)ethoxy)pyridin-2-yl)benzamide

The titled compound was obtained using 3-(6-(2-(3-methylphenyl)ethoxy)pyridin-2-yl)benzoic acid obtained in Reference Example 27 and 2-aminoethanol in the same manner as in Working Example 2. Yield: 68%. Melting point: 97 - 98°C. (Ethyl acetate-hexane).
¹H-NMR (CDCl₃) δ : 2.35 (3H, s), 2.42 - 2.62 (1H, m), 3.11 (2H, t, J = 7.2 Hz), 3.64 (2H, t, J = 5.4 Hz), 3.77 - 3.85 (2H, m), 4.62 (2H, t, J = 7.2 Hz), 6.67-6.80 (2H, m), 7.05 (1H, d, J = 7.5 Hz), 7.09 - 7.14 (2H, m), 7.17- 7.24 (1H, m), 7.36 (1H, d, J = 7.2 Hz), 7.50 (1H, t, J = 7.5 Hz), 7.63 (1H, d, J = 7.8 Hz), 7.98 (1H, d, J = 7.2 Hz), 8.16 (1H, d, J = 7.2 Hz), 8.40 (1H, s).

### Working Example 60

### 3-(6-(2-(3-Methylphenyl)ethoxy)pyridin-2-yl)-N-(2-methylpyridin-4-yl)benzamide

The titled compound was obtained using 3-(6-(2-(3-methylphenyl)ethoxy)pyridin-2-yl)benzoic acid obtained in Reference Example 27 and 2-methylpyridine-4-amine in the same manner as in Working Example 55. Yield: 36%. Melting point: 134 - 135°C. (Ethyl acetate-hexane).
¹H-NMR (CDCl₃) δ : 2.31 (3H, s), 2.51 (3H, s), 3.10 (2H, t, J = 6.9 Hz), 4.61 (2H, t, J = 6.9 Hz), 6.71 (1H, d, J = 7.5 Hz), 7.02 (1H, d, J = 7.2 Hz), 7.10 - 7.25 (3H, m), 7.30 - 7.34 (2H, m), 7.49 - 7.55 (2H, m), 7.62 (1H, t, J = 7.8 Hz), 7.82 (1H, d, J = 6.9 Hz), 8.18 (1H, d, J = 7.8 Hz), 8.31 (1H, s), 8.38 (1H, d, J = 5.1 Hz), 8.45 (1H, s).

### Working Example 61

### 3-(6-(2-(3-Fluorophenyl)ethoxy)pyridin-2-yl)-N-1,3-thiazol-2-ylbenzamide

The titled compound was obtained using 3-(6-(2-(3-fluorophenyl)ethoxy)pyridin-2-yl)benzoic acid obtained in Reference Example 26 and 1,3-thiazole-2-amine in the same manner as in Working Example 55. Yield: 62%. Melting point: 115 - 116°C. (Ethyl acetate-hexane).
¹H-NMR (CDCl₃) δ : 3.09 (2H, t, J = 6.0 Hz), 4.56 (2H, t, J = 6.9 Hz), 6.70 (1H, d, J = 8.4 Hz), 6.81 - 6.90 (2H, m), 6.94 - 7.02 (2H, m), 7.06 (1H, d, J = 7.8 Hz), 7.16 - 7.26 (1H, m), 7.32 (1H, d, J = 6.9 Hz), 7.56 - 7.65 (2H, m), 8.01 (1H, d, J = 7.8 Hz), 8.30 (1H, d, J = 7.5 Hz), 8.64 (1H, s), 12.9 (1H, s).

### Working Example 62

### 3-(6-(2-(3-Fluorophenyl)ethoxy)pyridin-2-yl)-N-(2-hydroxyethyl)benzamide

The titled compound was obtained using 3-(6-(2-(3-fluorophenyl)ethoxy)pyridin-2-yl)benzoic acid obtained in Reference Example 26 and 2-aminoethanol in the same manner as in Working Example 2. Yield: 32%. Melting point: 85 - 86°C. (Ethyl acetate-hexane).
¹H-NMR (CDCl₃) δ : 2.60 (1H, br s), 3.14 (2H, t, J = 6.6 Hz), 3.65 (2H, t, J = 5.1 Hz), 3.84 (2H, t, J = 5.1 Hz), 4.64 (2H, t, J = 6.6 Hz), 6.67 - 6.81 (2H, m), 6.88 - 6.96 (1H, m), 7.03 (1H, d, J = 10.2 Hz), 7.08 (1H, d, J = 8.1 Hz), 7.21 - 7.30 (1H, m), 7.36 (1H, d, J = 7.8 Hz), 7.49 (1H, d, J = 7.5 Hz), 7.63 (1H, d, J = 7.5 Hz), 7.77 (1H, d, J = 8.4 Hz), 8.14 (1H, d, J = 7.2 Hz), 8.41 (1H, s).

### Working Example 63

### N-(2-Cyanoethyl)-3-(6-(2-(3-fluorophenyl)ethoxy)pyridin-2-yl)benzamide

The titled compound was synthesized using 3-(6-(2-(3-fluorophenyl)ethoxy)pyridin-2-yl)benzoic acid obtained in Reference Example 26 and 3-aminopropanenitrile in the same manner as in Working Example 2. Yield: 73%. Melting point: 142 - 143°C (ethyl acetate-hexane).
¹H-NMR (DMSO-d₆):δ 2.81 (2H, t, J = 6.5 Hz), 3.13 (2H, t, J = 6.8 Hz), 3.54 (2H, q, J = 6.4 Hz), 4.63 (2H, t, J = 6.8 Hz), 6.80 (1H, d, J = 7.9 Hz), 7.01 - 7.12 (1H, m), 7.15 - 7.25 (2H, m), 7.28 - 7.41 (1H, m), 7.56 - 7.68 (2H, m), 7.83 (1H, t, J = 7.8 Hz), 7.90 (1H, d, J = 8.1 Hz), 8.26 (1H, d, J = 7.9 Hz), 8.54 (1H, s), 8.99 (1H, t, J = 5.6 Hz).

### Working Example 64

### N-(2-Cyanoethyl)-3-(6-(2-(3-(trifluoromethyl)phenyl)ethoxy)pyridin-2-yl)benzamide

The titled compound was obtained using 3-(6-(2-(3-(trifluoromethyl)phenyl)ethoxy)pyridin-2-yl)benzoic acid obtained in Reference Example 25 and 3-aminopropanenitrile in the same manner as in Working Example 2. Yield: 75%. Melting point: 104 - 105°C. (Ethyl acetate-hexane).
¹H-NMR (CDCl₃) δ : 2.77 (2H, t, J = 6.0 Hz), 3.20 (2H, t, J = 6.9 Hz), 3.75 (2H, q, J = 6.0 Hz), 4.67 (2H, t, J = 6.9 Hz), 6.60 - 6.72 (2H, m), 7.34 - 7.59 (6H, m), 7.65 (1H, t, J = 7.8 Hz), 7.76 (1H, d, J = 6.9 Hz), 8.17 (1H, d, J = 7.8 Hz), 8.42 (1H, s).

### Working Example 65

### N-(2-Hydroxyethyl)-3-(6-(2-(3-(trifluoromethyl)phenyl)ethoxy)pyridin-2-yl)benzamide

The titled compound was obtained using 3-(6-(2-(3-(trifluoromethyl)phenyl)ethoxy)pyridin-2-yl)benzoic acid obtained in Reference Example 25 and 2-aminoethanol in the same manner as in Working Example 2. Yield: 21%. Melting point: 106 - 107°C. (Ethyl acetate-hexane).
¹H-NMR (CDCl₃) δ : 2.55 (1H, br s), 3.20 (2H, t, J = 6.9 Hz), 3.66 (2H, t, J = 5.1 Hz), 3.78 - 3.90 (2H, m), 4.66 (2H, t, J = 6.9 Hz), 6.64 - 6.79 (2H, m), 7.35 - 7.67 (7H, m), 7.76 (1H, d, J = 7.2 Hz), 8.15 (1H, d, J = 7.5 Hz), 8.41 (1H, s).

### Working Example 66

### N-(2-Methoxyethyl)-3-(6-(2-(3-methoxyphenyl)ethoxy)pyridin-2-yl)benzamide

The titled compound was obtained using 3-(6-(2-(3-methoxyphenyl)ethoxy)pyridin-2-yl)benzoic acid obtained in Reference Example 29 and 2-methoxyethanamine in the same manner as in Working Example 2. Yield: 34%. Melting point: 70 - 71°C. (Ethyl acetate-hexane).
¹H-NMR (CDCl₃) δ : 3.12 (2H, t, J = 6.9 Hz), 3.37 (3H, s), 3.58 (2H, t, J = 4.8 Hz), 3.68 (2H, t, J = 4.8 Hz), 3.79 (3H, s), 4.65 (2H, t, J = 6.9 Hz), 6.50 - 6.97 (5H, m), 7.23 (1H, t, J = 8.1 Hz), 7.37 (1H, d, J = 7.2 Hz), 7.50 (1H, t, J = 7.8 Hz), 7.63 (1H, t, J = 7.8 Hz), 7.77 (1H, d, J = 7.5 Hz), 8.16 (1H, d, J = 8.4 Hz), 8.39 (1H, s).

### Working Example 67

### 3-(6-(2-(3-Methoxyphenyl)ethoxy)pyridin-2-yl)-N-(tetrahydro-2H-pyran-4-yl)benzamide

The titled compound was obtained using 3-(6-(2-(3-methoxyphenyl)ethoxy)pyridin-2-yl)benzoic acid obtained in Reference Example 29 and tetrahydro-2H-pyran-4-amine in the same manner as in Working Example 2. Yield: 76%. Melting point: 136 - 137°C. (Ethyl acetate-hexane).
¹H-NMR (CDCl₃) δ : 1.50 - 1.64 (2H, m), 1.97 - 2.07 (2H, m), 3.13 (2H, t, J = 7.2 Hz), 3.54 (2H, t, J = 11.7 Hz), 3.79 (3H, s), 3.95-4.05 (2H, m), 4.15-4.30 (1H, m), 4.65 (2H, t, J = 6.6 Hz), 6.05 (1H, d, J = 8.1 Hz), 6.71 (1H, d, J = 7.2 Hz), 6.74 - 6.80 (1H, m), 6.86 (1H, s), 6.90 (1H, d, J = 7.5 Hz), 7.20 - 7.25 (1H, m), 7.37 (1H, d, J = 8.1 Hz), 7.51 (1H, t, J = 7.5 Hz), 7.61 - 7.68 (1H, m), 7.71 - 7.77 (1H, m), 8.13-8.18 (1H, m), 8.27 (1H, s).

### Working Example 68

### 3-(6-(2-(3-Methoxyphenyl)ethoxy)pyridin-2-yl)-N-methylbenzamide

The titled compound was obtained using 3-(6-(2-(3-methoxyphenyl)ethoxy)pyridin-2-yl)benzoic acid obtained in Reference Example 29 and methylamine in the same manner as in Working Example 2. Yield: 19%. Melting point: 90 - 91 °C. (Ethyl acetate-hexane).
¹H-NMR (CDCl₃) δ : 3.04 (3H, d, J = 4.8 Hz), 3.13 (2H, t, J = 7.2 Hz), 3.80 (3H, s), 4.65 (2H, t, J = 7.2 Hz), 6.18 (1H, br s), 6.70 (1H, d, J = 7.5 Hz), 6.78 (1H, d, J = 7.8 Hz), 6.87 (1H, s), 6.91 (1H, d, J = 8.1 Hz), 7.23 (1H, d, J = 7.5 Hz), 7.37 (1H, d, J = 8.1 Hz), 7.50 (1H, d, J = 7.8 Hz), 7.64 (1H, t, J = 7.8 Hz), 7.76 (1H, d, J = 7.8 Hz), 8.15 (1H, d, J = 7.5 Hz), 8.37 (1H, s).

### Working Example 69

### N-(2-Hydroxyethyl)-3-(6-(2-(3-methoxyphenyl)ethoxy)pyridin-2-yl)benzamide

The titled compound was obtained using 3-(6-(2-(3-methoxyphenyl)ethoxy)pyridin-2-yl)benzoic acid obtained in Reference Example 29 and 2-aminoethanol in the same manner as in Working Example 2. Yield: 18%. Melting point: 95 - 96°C. (Ethyl acetate-hexane).
¹H-NMR (CDCl₃) δ : 2.65 (1H, br s), 3.12 (2H, t, J = 7.2 Hz), 3.64 (2H, q, J = 6.0 Hz), 3.72-3.85 (5H, m), 4.64 (2H, t, J = 7.2 Hz), 6.67-6.94 (5H, m), 7.24 (1H, d, J = 8.1 Hz), 7.36 (1H, d, J = 7.5 Hz), 7.49 (1H, t, J = 7.5 Hz), 7.63 (1H, t, J = 7.5 Hz), 7.79 (1H, d, J = 7.8 Hz), 8.14 (1H, d, J = 7.8 Hz), 8.41 (1H, s).

### Working Example 70

### 3-(6-(2-(3-Methoxyphenyl)ethoxy)pyridin-2-yl)-N-(2-pyrrolidin-1-ylethyl)benzamide

The titled compound was obtained using 3-(6-(2-(3-methoxyphenyl)ethoxy)pyridin-2-yl)benzoic acid obtained in Reference Example 29 and 2-pyrrolidin-1-ylethanamine in the same manner as in Working Example 2. Yield: 49%. Melting point: 95 - 96°C. (Ethyl acetate-hexane).
¹H-NMR (CDCl₃) δ : 1.70 - 1.89 (4H, m), 2.43 - 2.62 (4H, m), 2.71 (2H, t, J = 6.0 Hz), 3.12 (2H, t, J = 7.2 Hz), 3.58 (2H, q, J = 6.0 Hz), 3.80 (3H, s), 4.65 (2H, t, J = 7.2 Hz), 6.70 (1H, d, J = 8.4 Hz), 6.77 (1H, dd, J = 8.4, 2.4 Hz), 6.85-6.93 (3H, m), 7.22 (1H, d, J = 8.1 Hz), 7.38 (1H, d, J = 7.2 Hz), 7.50 (1H, t, J = 7.8 Hz), 7.64 (1H, t, J = 8.1 Hz), 7.77 (1H, d, J = 7.5 Hz), 8.16 (1H, d, J = 7.8 Hz), 8.41 (1H, s).

### Working Example 71

### N-(2-Cyanoethyl)-3-(6-(2-(3-methoxyphenyl)ethoxy)pyridin-2-yl)benzamide

The titled compound was synthesized using 3-(6-(2-(3-methoxyphenyl)ethoxy)pyridin-2-yl)benzoic acid obtained in Reference Example 29 and 3-aminopropanenitrile in the same manner as in Working Example 2. Yield: 90%. Melting point: 107 - 108°C (ethyl acetate-hexane).
¹H-NMR (DMSO-d₆):δ 2.81 (2H, t, J = 6.6 Hz), 3.07 (2H, t, J = 7.0 Hz), 3.53 (2H, q, J = 6.4 Hz), 3.74 (3H, s), 4.60 (2H, t, J = 7.0 Hz), 6.77-6.83 (2H, m), 6.89 - 6.96 (2H, m), 7.23 (1H, t, J = 8.1 Hz), 7.56 - 7.65 (2H, m), 7.79 - 7.86 (1H, m), 7.89 (1H, d, J = 8.0 Hz), 8.25 (1H, d, J = 8.0 Hz), 8.53 (1H, t, J = 1.5 Hz), 8.98 (1H, t, J = 5.7 Hz).

### Working Example 72

### Tert-butyl (2-((3-(6-(2-(3-methoxyphenyl)ethoxy)pyridin-2-yl)benzoyl)amino)ethyl)carbamate

WSC (130 mg, 0.68 mmol) and HOBt (91.8 mg, 0.68 mmol) were added to a DMF (2 mL) solution of 3-(6-(2-(3-methoxyphenyl)ethoxy)pyridin-2-yl)benzoic acid (200 mg, 0.57 mmol) obtained in Reference Example 29 and tert-butyl (2-aminoethyl)carbamate (0.108 mL, 0.68 mmol), and the mixture was stirred for 3 hours at room temperature. The addition of saturated aqueous sodium bicarbonate to the reaction solution was followed by extraction with ethyl acetate. The extract was washed with water and dried over anhydrous magnesium sulfate, and the solvent was then distilled off at reduced pressure. The residue was purified by silica gel column chromatography to give 230 g of the titled compound (yield: 82%). Melting point: 128 - 129°C (ethyl acetate).
¹H-NMR (CDCl₃):δ 1.41 (9H, s), 3.13 (2H, t, J = 7.0 Hz), 3.42 (2H, q, J = 5.6 Hz), 3.55-3.63 (2H, m), 3.80 (3H, s), 4.67 (2H, t, J = 7.2 Hz), 4.99 (1H, br s), 6.70 (1H, d, J = 8.3 Hz), 6.78 (1H, dd, J = 8.0, 2.3 Hz), 6.86-6.90 (1H, m), 6.92 (1H, d, J = 7.6 Hz), 7.22 (1H, d, J = 8.0 Hz), 7.31 (1H, br s), 7.43 (1H, d, J = 7.6 Hz), 7.51 (1H, t, J = 7.8 Hz), 7.59 - 7.68 (1H, m), 7.83 (1H, d, J = 7.6 Hz), 8.21 (1H, d, J = 8.0 Hz), 8.45 (1H, s) .

### Working Example 73

### 3-(6-(2-(3-Methoxyphenyl)ethoxy)pyridin-2-yl)-N-(2-methylpyridin-4-yl)benzamide

The titled compound was obtained using 3-(6-(2-(3-methoxyphenyl)ethoxy)pyridin-2-yl)benzoic acid obtained in Reference Example 29 and 2-methylpyridine-4-amine in the same manner as in Working Example 55. Yield: 44%. Melting point: 125 - 126°C. (Ethyl acetate-hexane).
¹H-NMR (CDCl₃) δ : 2.56 (3H, s), 3.13 (2H, t, J = 7.2 Hz), 3.76 (3H, s), 4.66 (2H, t, J = 7.2 Hz), 6.70 - 6.78 (2H, m), 6.87 - 6.92 (2H, m), 7.20 (1H, t, J = 7.8 Hz), 7.33 (1H, dd, J = 6.0, 1.8 Hz), 7.38 (1H, d, J = 7.5 Hz), 7.53 (1H, d, J = 1.8 Hz), 7.57 (1H, d, J = 7.8 Hz), 7.66 (1H, t, J = 7.8 Hz), 7.86 (1H, d, J = 7.5 Hz), 8.04 (1H, br s), 8.20 (1H, d, J = 7.8 Hz), 8.42 (1H, d, J = 5.7 Hz), 8.48 (1H, s).

### Working Example 74

### 3-(6-(2-(3-Methoxyphenyl)ethoxy)pyridin-2-yl)-N-1,3-thiazol-2-ylbenzamide

The titled compound was obtained using 3-(6-(2-(3-methoxyphenyl)ethoxy)pyridin-2-yl)benzoic acid obtained in Reference Example 29 and 1,3-thiazole-2-amine in the same manner as in Working Example 55. Yield: 69%. Melting point: 123 - 125°C. (Ethyl acetate-hexane).
¹H-NMR (CDCl₃) δ : 3.09 (2H, t, J = 7.2 Hz), 3.76 (3H, s), 4.59 (2H, t, J = 7.2 Hz), 6.69 - 6.79 (2H, m), 6.86 (2H, d, J = 2.4 Hz), 6.90 (1H, d, J = 7.5 Hz), 7.05 (1H, br s), 7.19 (1H, t, J = 7.8 Hz), 7.32 (1H, d, J = 7.8 Hz), 7.56 - 7.66 (2H, m), 7.98 (1H, d, J = 7.5 Hz), 8.31 (1H, d, J = 7.5 Hz), 8.61 (1H, s), 1H unconfirmed.

### Working Example 75

### N-(6-Fluoropyridin-3-yl)-3-(6-(2-(3-methoxyphenyl)ethoxy)pyridin-2-yl)benzamide

The titled compound was obtained using 3-(6-(2-(3-methoxyphenyl)ethoxy)pyridin-2-yl)benzoic acid obtained in Reference Example 29 and 6-fluoropyridine-3-amine in the same manner as in Working Example 55. Yield: 68%. Melting point: 127 - 128°C. (Ethyl acetate-hexane).
¹H-NMR (CDCl₃) δ : 3.13 (2H, t, J = 6.9 Hz), 3.77 (3H, s), 4.66 (2H, t, J = 6.9 Hz), 6.70 - 6.78 (2H, m), 6.86 - 6.93 (2H, m), 6.97 (1H, dd, J = 8.7, 2.2 Hz), 7.21 (1H, t, J = 7.8 Hz), 7.38 (1H, d, J = 7.2 Hz), 7.58 (1H, t, J = 7.8 Hz), 7.66 (1H, t, J = 7.8 Hz), 7.88 (1H, t, J = 7.8 Hz), 8.00 (1H, br s), 8.16 - 8.22 (1H, m), 8.30 - 8.35 (2H, m), 8.49 (1H, t, J = 1.5 Hz).

### Working Example 76

### 3-(6-(2-(3-Methoxyphenyl)ethoxy)pyridin-2-yl)-N-pyridin-2-ylbenzamide

The titled compound was obtained using 3-(6-(2-(3-methoxyphenyl)ethoxy)pyridin-2-yl)benzoic acid obtained in Reference Example 29 and 2-aminopyridine in the same manner as in Working Example 55. Yield: 71 %. Oily substance.
¹H-NMR (CDCl₃) δ : 3.13 (2H, t, J = 7.2 Hz), 3.79 (3H, s), 4.67 (2H, t, J = 7.2 Hz), 6.69 - 6.79 (2H, m), 6.87 (1H, s), 6.92 (1H, d, J = 7.5 Hz), 7.05-7.12 (1H, m), 7.21 (1H, t, J = 8.4 Hz), 7.40 (1H, d, J = 7.8 Hz), 7.58 (1H, t, J = 7.8 Hz), 7.65 (1H, t, J = 8.4 Hz), 7.78 (1H, t, J = 7.8 Hz), 7.92 (1H, d, J = 7.5 Hz), 8.25 (1H, d, J = 6.9 Hz), 8.30 (1H, d, J = 4.2 Hz), 8.42 (1H, d, J = 8.7 Hz), 8.55 (1H, s), 8.77 (1H, s).

### Working Example 77

### 3-(6-(2-(3-Methoxyphenyl)ethoxy)pyridin-2-yl)-N-(3-methylisothiazol-5-yl)benzamide

The titled compound was obtained using 3-(6-(2-(3-methoxyphenyl)ethoxy)pyridin-2-yl)benzoic acid obtained in Reference Example 29 and 3-methylisothiazole-5-amine in the same manner as in Working Example 55. Yield: 25%; melting point: 46 - 47°C (ethyl acetate-hexane).
¹H-NMR (DMSO-d₆) δ: 2.37 (3H, s), 3.08 (2H, t, J = 7.1 Hz), 3.73 (3H, s), 4.62 (2H, t, J = 7.1 Hz), 6.75 - 6.87 (2H, m), 6.89 - 6.98 (3H, m), 7.16 - 7.26 (1H, m), 7.65 - 7.76 (2H, m), 7.86 (1H, t, J = 7.8 Hz), 8.08 (1H, d, J = 7.8 Hz), 8.35 (1H, d, J = 7.8 Hz), 8.71 (1H, s), 12.37 (1H, s).

### Working Example 78

### 3-(6-(2-(3-Methoxyphenyl)ethoxy)pyridin-2-yl)-N-1,3,4-thiadiazol-2-ylbenzamide

The titled compound was obtained using 3-(6-(2-(3-methoxyphenyl)ethoxy)pyridin-2-yl)benzoic acid obtained in Reference Example 29 and 1,3,4-thiadiazole-2-amine in the same manner as in Working Example 55. Yield: 65%; melting point: 141 - 142°C (ethyl acetate-hexane).
¹H-NMR (DMSO-d₆) δ: 3.08 (2H, t, J = 6.9 Hz), 3.73 (3H, s), 4.62 (2H, t, J = 6.9 Hz ), 6.75 - 6.87 (2H, m), 6.88 - 6.98 (2H, m), 7.22 (1H, t, J = 7.9 Hz), 7.64 - 7.77 (2H, m), 7.86 (1H, t, J = 7.9 Hz), 8.16 (1H, d, J = 7.9 Hz), 8.39 (1H, d, J = 7.9 Hz), 8.82 (1H, s), 9.26 (1H, s), 13.2 (1H, br s).

### Working Example 79

### 3-(6-(2-(3-Methoxyphenyl)ethoxy)pyridin-2-yl)-N-(3-methyl-1H-pyrazol-5-yl)benzamide

The titled compound was obtained using 3-(6-(2-(3-methoxyphenyl)ethoxy)pyridin-2-yl)benzoic acid obtained in Reference Example 29 and 3-methyl-1H-pyrazole-5-amine in the same manner as in Working Example 55. Yield: 36%; melting point: 112 - 113°C (ethyl acetate-hexane).
¹H-NMR (DMSO-d₆) δ: 2.25 (3H, s), 3.08 (2H, t, J = 7.0 Hz), 3.73 (3H, s), 4.61 (2H, t, J = 7.0 Hz ), 6.44 (1H, br s), 6.73 - 6.84 (2H, m), 6.86 - 6.97 (2H, m), 7.22 (1H, t, J = 8.0 Hz), 7.60 (1H, t, J = 7.7 Hz), 7.67 - 7.76 (1H, m), 7.83 (1H, t, J = 7.7 Hz), 8.03 (1H, d, J = 7.7 Hz), 8.28 (1H, d, J = 8.0 Hz), 8.66 (1H, s), 10.86 (1H, s), 12.13 (1H, br s).

### Working Example 80

### N-(1,3-Dimethyl-1H-pyrazol-5-yl)-3-(6-(2-(3-methoxyphenyl)ethoxy)pyridin-2-yl)benzamide

The titled compound was obtained using 3-(6-(2-(3-methoxyphenyl)ethoxy)pyridin-2-yl)benzoic acid obtained in Reference Example 29 and 1,3-dimethyl-1H-pyrazole-5-amine in the same manner as in Working Example 55. Yield: 68%; melting point: 141 - 142°C (ethyl acetate-hexane).
¹H-NMR (DMSO-d₆) δ: 2.14 (3H, s), 3.08 (2H, t, J = 7.0 Hz), 3.61 (3H, s), 3.73 (3H, s), 4.61 (2H, t, J = 7.0 Hz ), 6.06 (1H, s), 6.74 - 6.85 (2H, m), 6.87 - 6.96 (2H, m), 7.16 - 7.26 (1H, m), 7.60 - 7.71 (2H, m), 7.84 (1H, t, J = 7.8 Hz), 8.00 (1H, d, J = 7.8 Hz), 8.32 (1H, d, J = 7.8 Hz), 8.64 (1H, s), 10.39 (1H, s).

### Working Example 81

### N-(2-Aminoethyl)-3-(6-(2-(3-methoxyphenyl)ethoxy)pyridin-2-yl)benzamide hydrochloride

4 N hydrogen chloride/ethyl acetate were added to an ethyl acetate (10 mL) solution of tert-butyl (2-((3-(6-(2-(3-methoxyphenyl)ethoxy)pyridin-2-yl)benzoyl)amino)ethyl)carbamate (220 mg, 0.45 mmol) obtained in Working Example 72, and the mixture was stirred for 5 hours at 30°C. The reaction solution was concentrated and recrystallized from methanol-diethyl ether to give 100 mg of the titled compound (yield: 52%). Melting point: 161 - 162°C (methanol-diethyl ether).
¹H-NMR (DMSO-d₆):δ 2.95 - 3.13 (4H, m), 3.57 (2H, q, J = 5.8 Hz), 3.74 (3H, s), 4.60 (2H, t, J = 7.0 Hz), 6.80 (2H, d, J = 8.0 Hz), 6.89 - 6.97 (2H, m), 7.23 (1H, t, J = 8.0 Hz), 7.60 (1H, t, J = 7.8 Hz), 7.68 (1H, d, J = 7.6 Hz), 7.82 (1H, t, J = 7.8 Hz), 7.96 (1H, d, J = 7.6 Hz), 8.06 (3H, br s), 8.26 (1H, d, J = 8.0 Hz), 8.59 (1H, s), 8.92 (1H, t, J = 5.1 Hz).

### Working Example 82

### 3-(6-(3,4-Dichlorophenoxy)pyridin-2-yl)-N-(2-pyrrolidin-1-ylethyl)benzamide

The titled compound was obtained using 3-(6-(3,4-dichlorophenoxy)pyridin-2-yl)benzoic acid obtained in Reference Example 41 and 2-pyrrolidin-1-ylethanamine in the same manner as in Working Example 2. Yield: 64%. Melting point: 114 - 115°C. (Ethyl acetate-hexane).
¹H-NMR (CDCl₃) δ : 1.70 - 1.90 (4H, m), 2.46-2.62 (4H, m), 2.71 (2H, t, J = 6.0 Hz), 3.57 (2H, q, J = 6.0 Hz), 6.78 (1H, br s), 6.88 (1H, d, J = 8.1 Hz), 7.00 (1H, dd, J = 9.0, 3.0 Hz), 7.37 - 7.51 (3H, m), 7.58 (1H, d, J = 6.9 Hz), 7.73 - 7.80 (2H, m), 7.98 - 8.03 (1H, m), 8.29 (1H, s).

### Working Example 83

### 3-(6-(2,3-Dichlorophenoxy)pyridin-2-yl)-N-(2-pyrrolidin-1-ylethyl)benzamide

The titled compound was obtained using 3-(6-(2,3-dichlorophenoxy)pyridin-2-yl)benzoic acid obtained in Reference Example 40 and 2-pyrrolidin-1-ylethanamine in the same manner as in Working Example 2. Yield: 64%. Melting point: 118 - 119°C. (Ethyl acetate-hexane).
¹H-NMR (CDCl₃) δ : 1.70 - 1.89 (4H, m), 2.42 - 2.62 (4H, m), 2.71 (2H, t, J = 6.0 Hz), 3.56 (2H, q, J = 6.0 Hz), 6.73 (1H, br s), 6.91 (1H, d, J = 8.4 Hz), 7.18-7.31 (2H, m), 7.37 (1H, dd, J = 7.8, 2.1 Hz), 7.44 (1H, t, J = 7.8 Hz), 7.55 (1H, t, J = 7.5 Hz), 7.75 (1H, d, J = 8.1 Hz), 7.80 (1H, t, J = 7.8 Hz), 7.93 (1H, d, J = 8.1 Hz), 8.23 (1H, s).

### Working Example 84

### 3-(6-(3,5-Dichlorophenoxy)pyridin-2-yl)-N-(2-pyrrolidin-1-ylethyl)benzamide

The titled compound was obtained using 3-(6-(3,5-dichlorophenoxy)pyridin-2-yl)benzoic acid obtained in Reference Example 39 and 2-pyrrolidin-1-ylethanamine in the same manner as in Working Example 2. Yield: 56%. Melting point: 111 - 112°C. (Ethyl acetate-hexane).
¹H-NMR (CDCl₃) δ : 1.70 - 1.90 (4H, m), 2.47 - 2.61 (4H, m), 2.71 (2H, t, J = 6.0 Hz), 3.57 (2H, q, J = 6.0 Hz), 6.77 (1H, br s), 6.89 (1H, d, J = 7.8 Hz), 7.15-7.22 (3H, m), 7.48 (1H, t, J = 7.5 Hz), 7.60 (1H, d, J = 7.5 Hz), 7.76 - 7.85 (2H, m), 8.02 (1H, d, J = 7.8 Hz), 8.29 (1H, s).

### Working Example 85

### 3-(6-(2,4-Dichlorophenoxy)pyridin-2-yl)-N-(2-pyrrolidin-1-ylethyl)benzamide

The titled compound was obtained using 3-(6-(2,4-dichlorophenoxy)pyridin-2-yl)benzoic acid obtained in Reference Example 38 and 2-pyrrolidin-1-ylethanamine in the same manner as in Working Example 2. Yield: 33%. Melting point: 125 - 126°C. (Ethyl acetate-hexane).
¹H-NMR (CDCl₃) δ : 1.74 - 1.83 (4H, m), 2.51 - 2.61 (4H, m), 2.71 (2H, t, J = 6.3 Hz), 3.57 (2H, q, J = 6.3 Hz), 6.76 (1H, br s), 6.90 (1H, d, J = 8.1 Hz), 7.23-7.32 (2H, m), 7.40 - 7.56 (3H, m), 7.71 - 7.82 (2H, m), 7.93 (1H, dd, J = 6.6, 1.5 Hz), 8.24 (1H, t, J = 1.5 Hz).

### Working Example 86

### 3-(6-(2,4-Dichlorophenoxy)pyridin-2-yl)-N-(tetrahydro-2H-pyran-4-yl)benzamide

The titled compound was obtained using 3-(6-(2,4-dichlorophenoxy)pyridin-2-yl)benzoic acid obtained in Reference Example 38 and tetrahydro-2H-pyran-4-amine in the same manner as in Working Example 2. Yield: 74%. Melting point: 193 - 194°C. (Ethyl acetate-hexane).
¹H-NMR (CDCl₃) δ : 1.47 - 1.65 (2H, m), 1.96 - 2.07 (2H, m), 3.50 - 3.59 (2H, m), 3.96 - 4.05 (2H, m), 4.09 - 4.27 (1H, m), 5.96 (1H, t, J = 7.8 Hz), 6.92 (1H, d, J = 8.4 Hz), 7.21 - 7.33 (2H, m), 7.42 - 7.56 (3H, m), 7.76 - 7.83 (2H, m), 7.94 (1H, d, J = 7.8 Hz), 8.15 (1H, s).

### Working Example 87

### 3-(6-(3,5-Dichlorophenoxy)pyridin-2-yl)-N-(2-hydroxyethyl)benzamide

The titled compound was obtained using 3-(6-(3,5-dichlorophenoxy)pyridin-2-yl)benzoic acid obtained in Reference Example 39 and 2-aminoethanol in the same manner as in Working Example 2. Yield: 15%. Melting point: 163 - 164°C. (Ethyl acetate-hexane).
¹H-NMR (CDCl₃) δ : 2.60 - 2.70 (1H, m), 3.65 (2H, q, J = 5.4 Hz), 3.85 (2H, q, J = 5.4 Hz), 6.60 - 6.70 (1H, m), 6.90 (1H, d, J = 8.4 Hz), 7.17 - 7.26 (3H, m), 7.50 (1H, t, J = 7.5 Hz), 7.58 (1H, d, J = 7.2 Hz), 7.77 - 7.86 (2H, m), 8.02 (1H, d, J = 7.8 Hz), 8.27 (1H, s).

### Working Example 88

### 3-(6-(3,5-Dichlorophenoxy)pyridin-2-yl)-N-(3-hydroxypropyl)benzamide

3-aminopropan-1-ol (90 mg, 1.2 mmol) and DMTMM (324 mg, 1.1 mmol) were added to a 3-methanol (5 mL) solution of 3-(6-(3,5-dichlorophenoxy)pyridin-2-yl)benzoic acid (0.355 g, 0.97 mmol) obtained in Reference Example 39, and the mixture was stirred for 3 hours at room temperature. The solvent was distilled off at reduced pressure, ethyl acetate was added to the residue, the solution was washed with water and with saturated brine, and concentrated. The residue was purified by silica gel column chromatography (hexane-ethyl acetate 80:20 → 0:100) and was recrystallized from ethyl acetate-hexane to give 0.27 g of the titled compound (yield 81%). Melting point: 101 - 102°C. (Ethyl acetate-hexane).
¹H-NMR (CDCl₃) δ : 1.81 (2H, quint, J = 5.7 Hz), 3.20 (1H, br s), 3.65 (2H, q, J = 5.7 Hz), 3.71 (2H, q, J = 5.4 Hz), 6.65 (1H, br s), 6.90 (1H, d, J = 8.1 Hz), 7.18 - 7.24 (3H, m), 7.50 (1H, t, J = 7.5 Hz), 7.59 (1H, d, J = 7.5 Hz), 7.78 - 7.87 (2H, m), 8.02 (1H, d, J = 7.8 Hz), 8.27 (1H, t, J = 1.8 Hz).

### Working Example 89

### 3-(6-(3,5-Dichlorophenoxy)pyridin-2-yl)-N-(2,3-dihydroxypropyl)benzamide

The titled compound was obtained using 3-(6-(3,5-dichlorophenoxy)pyridin-2-yl)benzoic acid obtained in Reference Example 39 and 3-aminopropane-1,2-diol in the same manner as in Working Example 88. Yield: 60%. Melting point: 124 - 126°C. (Ethyl acetate-hexane).
¹ H-NMR (CDCl₃ ) δ : 1.89 (2H, br s), 3.58 - 3.71 (4H, m), 3.81 - 3.92 (1H, m), 6.73 (1H, br s), 6.90 (1H, d, J = 8.1 Hz), 7.17 - 7.24 (3H, m), 7.51 (1H, t, J = 7.8 Hz), 7.59 (1H, d, J = 7.5 Hz), 7.78 - 7.86 (2H, m), 8.03 (1H, d, J = 7.8 Hz), 8.28 (1H, t, J = 1.5 Hz).

### Working Example 90

### 3-(6-(3,5-Dichlorophenoxy)pyridin-2-yl)-N-(6-fluoropyridin-3-yl)benzamide

The titled compound was obtained using 3-(6-(3,5-dichlorophenoxy)pyridin-2-yl)benzoic acid obtained in Reference Example 39 and 6-fluoropyridine-3-amine in the same manner as in Working Example 55. Yield: 50%. Melting point: 179 - 180°C. (Ethyl acetate-hexane).
¹H-NMR (CDCl₃) δ : 6.92 (1H, d, J = 8.4 Hz), 6.98 (1H, dd, J = 8.4, 1.8 Hz), 7.18 (2H, d, J = 1.8 Hz), 7.20 (1H, t, J = 1.8 Hz), 7.53 - 7.63 (2H, m), 7.84 (1H, dt, J = 7.8, 0.9 Hz), 7.94 (1H, d, J = 7.5 Hz), 8.01 (1H, br s), 8.07 (1H, d, J = 8.1 Hz), 8.26 - 8.40 (3H, m).

### Working Example 91

### 3-(6-(3,5-Dichlorophenoxy)pyridin-2-yl)-N-(2-hydroxy-1,1-dimethylethyl)benzamide

The titled compound was obtained using 3-(6-(3,5-dichlorophenoxy)pyridin-2-yl)benzoic acid obtained in Reference Example 39 and 2-amino-2-methylpropan-1-ol in the same manner as in Working Example 88. Yield: 39%. Melting point: 155 - 156°C. (Ethyl acetate-hexane).
¹H-NMR (CDCl₃) δ : 1.42 (6H, s), 3.71 (2H, d, J = 5.7 Hz), 4.72 (1H, t, J = 5.7 Hz), 6.17 (1H, br s), 6.91 (1H, d, J = 8.1 Hz), 7.16 (2H, d, J = 1.5 Hz), 7.21 (1H, t, J = 1.5 Hz), 7.49 (1H, t, J = 7.5 Hz), 7.58 (1H, d, J = 7.5 Hz), 7.74 - 7.85 (2H, m), 8.01 (1H, d, J = 7.8 Hz), 8.19 (1H, t, J = 1.8 Hz).

### Working Example 92

### 3-(6-(3,5-Dichlorophenoxy)pyridin-2-yl)-N-(2-hydroxy-1-(hydroxymethyl)ethyl)benzamide

The titled compound was obtained using 3-(6-(3,5-dichlorophenoxy)pyridin-2-yl)benzoic acid obtained in Reference Example 39 and 2-aminopropane-1,3-diol in the same manner as in Working Example 88. Yield: 61%. Melting point: 150 - 151°C. (Ethyl acetate-hexane).
¹H-NMR (CDCl₃) δ : 3.53 (4H, t, J = 5.7 Hz), 3.90 - 4.07 (1H, m), 4.67 (2H, t, J = 5.7 Hz), 7.09 (1H, d, J = 7.8 Hz), 7.40 (2H, d, J = 2.1 Hz), 7.47 - 7.56 (2H, m), 7.85 - 7.91 (2H, m), 7.96 - 8.11 (3H, m), 8.38 (1H, s).

### Working Example 93

### N-((1S)-2-Cyano-1-methylethyl)-3-(6-(3,5-dichlorophenoxy)pyridin-2-yl)benzamide

The titled compound was synthesized using 3-(6-(3,5-dichlorophenoxy)pyridin-2-yl)benzoic acid obtained in Reference Example 39 and (3S)-3-aminobutanenitrile in the same manner as in Working Example 2. Yield: 65%. Melting point: 160 - 161°C (ethyl acetate-hexane).
¹H-NMR (CDCl₃):δ 1.49 (3H, d, J = 6.8 Hz), 2.63 - 2.73 1H, m), 2.93 (1H, dd, J = 16.8, 5.9 Hz), 4.38 - 4.53 (1H, m), 6.19 (1H, d, J = 7.2 Hz), 6.91 (1H, d, J = 8.0 Hz), 7.18 (2H, d, J = 1.9 Hz), 7.23 (1H, t, J = 1.9 Hz), 7.52 (1H, t, J = 7.8 Hz), 7.60 (1H, d, J = 7.6 Hz), 7.78 - 7.87 (2H, m), 8.05 (1H, d, J = 8.0 Hz), 8.24 (1H, s).

### Working Example 94

### N-((1R)-2-Cyano-1-methylethyl)-3-(6-(3,5-dichlorophenoxy)pyridin-2-yl)benzamide

The titled compound was synthesized using 3-(6-(3,5-dichlorophenoxy)pyridin-2-yl)benzoic acid obtained in Reference Example 39 and (3R)-3-aminobutanenitrile in the same manner as in Working Example 2. Yield: 79%. Melting point: 149 - 150°C (ethyl acetate-hexane).
¹H-NMR (CDCl₃):δ 1.49 (3H, d, J = 7.2 Hz), 2.68 ( H, dd, J = 16.7, 4.2 Hz), 2.93 (1H, dd, J = 16.7, 6.1 Hz), 4.40 - 4.53 (1H, m), 6.19 (1H, d, J = 6.8 Hz), 6.91 (1H, d, J = 8.0 Hz), 7.18 (2H, d, J = 1.9 Hz), 7.23 (1H, t, J = 1.7 Hz), 7.52 (1H, t, J = 7.8 Hz), 7.60 (1H, d, J = 7.6 Hz), 7.78 - 7.87 (2H, m), 8.05 (1H, d, J = 8.0 Hz), 8.24 (1H, s).

### Working Example 95

### 3-(6-(3,5-Dichlorophenoxy)pyridin-2-yl)-N-(2-(methylthio)ethyl)benzamide

The titled compound was obtained using methyl 3-(6-(3,5-dichlorophenoxy)pyridin-2-yl)benzoic acid obtained in Reference Example 30 in the same manner as in Working Example 2. Yield: 86%. Melting point: 123 - 124°C. (Ethyl acetate-hexane).
¹H-NMR (CDCl₃) δ : 2.15 (3H, s), 2.77 (2H, t, J = 6.3 Hz), 3.69 (2H, q, J = 6.3 Hz), 6.60 (1H, br s), 6.90 (1H, d, J = 7.8 Hz), 7.18 (2H, d, J = 1.8 Hz), 7.22 (1H, t, J = 1.8 Hz), 7.50 (1H, t, J = 7.8 Hz), 7.59 (1H, dd, J = 7.8, 0.6 Hz), 7.78 - 7.86 (2H, m), 7.99 - 8.05 (1H, m), 8.27 (1H, t, J = 1.8 Hz).

### Working Example 96

### 3-(6-(3,5-Dichlorophenoxy)pyridin-2-yl)-N-(2-(methylsulfmyl)ethyl)benzamide

Meta-chloroperbenzoic acid (70% 114 mg, 0.462 mmol) was added at 0°C to a methylene chloride (5 mL) solution of 3-(6-(3,5-dichlorophenoxy)pyridin-2-yl)-N-(2-(methylthio)ethyl)benzamide (200 mg, 0.462 mmol) obtained in Working Example 95, and the mixture was stirred for 1 hour at 0°C. Saturated aqueous sodium bicarbonate was added to the reaction solution, and the mixture was extracted with methylene chloride. The extract was washed with water, dried over anhydrous magnesium sulfate, and then passed through a small amount of silica gel to distill the solvent off at reduced pressure. The resulting residue was crystallized from ethyl acetate-hexane to give 120 mg of the titled compound in the form of crystals (yield 58%). Melting point: 158 - 159°C.
¹H-NMR (CDCl₃) δ : 2.67 (3H, s), 2.83 - 2.97 (1H, m), 3.13- 3.23 (1H, m), 4.02 (2H, q, J = 5.7 Hz), 6.89 (1H, d, J = 7.8 Hz), 7.15 - 7.18 (2H, m), 7.22 (1H, t, J = 2.1 Hz), 7.39 (1H, br s), 7.48 (1H, t, J = 7.8 Hz), 7.59 (1H, d, J = 7.5 Hz), 7.77 - 7.84 (2H, m), 8.04 (1H, d, J = 7.8 Hz), 8.29 (1H, t, J = 2.1 Hz).

### Working Example 97

### 3-(6-(2,4-Dichlorophenoxy)pyridin-2-yl)-N-(3-hydroxy-3-methylbutyl)benzamide

The titled compound was obtained using 3-(6-(2,4-dichlorophenoxy)pyridin-2-yl)benzoic acid obtained in Reference Example 38 and 4-amino-2-methylbutan-2-ol in the same manner as in Working Example 2. Yield: 55%. Melting point: 139 - 140°C (ethyl acetate-hexane).
¹H-NMR (DMSO-d₆) δ: 1.15 (6H, s), 1.60 - 1.70 (2H, m), 3.28 - 3.41 (2H, m), 4.35 (1H, s), 7.11 (1H, d, J = 8.0 Hz), 7.41 - 7.57 (3H, m), 7.75 - 7.84 (3H, m), 7.89 (1H, d, J = 8.0 Hz), 8.02 (1H, t, J = 8.0 Hz), 8.29 (1H, s), 8.46 (1H, t, J = 5.3 Hz).

### Working Example 98

### N-(Cyanomethyl)-3-(6-(2,4-dichlorophenoxy)pyridin-2-yl)benzamide

The titled compound was obtained using 3-(6-(2,4-dichlorophenoxy)pyridin-2-yl)benzoic acid obtained in Reference Example 38 and aminoacetonitrile in the same manner as in Working Example 2. Yield: 81 %. Melting point: 165 - 166°C (tetrahydrofuran-hexane).
¹H-NMR (DMSO-d₆) δ: 4.35 (2H, d, J = 5.7 Hz), 7.12 (1H, d, J = 8.3 Hz), 7.42 - 7.60 (3H, m), 7.78 - 7.91 (3H, m), 7.93 - 8.07 (2H, m), 8.37 (1H, s), 9.30 (1H, t, J = 5.7 Hz).

### Working Example 99

### 3-(6-(2,4-Dichlorophenoxy)pyridin-2-yl)-N-(3,3,3-trifluoropropyl)benzamide

The titled compound was obtained using 3-(6-(2,4-dichlorophenoxy)pyridin-2-yl)benzoic acid obtained in Reference Example 38 and 3,3,3-trifluoropropan-1-amine in the same manner as in Working Example 2. Yield: 50%; melting point: 169 - 170°C (ethyl acetate-hexane).
¹H-NMR (DMSO-d₆) δ: 2.50 - 2.66 ( 2H, m), 3.46 - 3.56 (2H, m), 7.11 (1H, d, J = 8.0 Hz), 7.41 - 7.58 (3H, m), 7.76 - 7.86 (3H, m), 7.93 (1H, d, J = 8.0 Hz), 8.02 (1H, t, J = 8.0 Hz), 8.30 (1H, s), 8.76 (1H, t, J = 5.5 Hz).

### Working Example 100

### N-(2-Cyanoethyl)-3-(6-((2,4-dichlorophenyl)thio)pyridin-2-yl)benzamide

The titled compound was obtained using 3-(6-((2,4-dichlorophenyl)thio)pyridin-2-yl)benzoic acid obtained in Reference Example 77 and 3-aminopropanenitrile in the same manner as in Working Example 2. Yield: 56%. Melting point: 161 - 162°C (ethyl acetate-hexane).
¹H-NMR (DMSO-d₆) δ: 2.81 (2H, t, J = 6.5 Hz), 3.54 (2H, q, J = 6.5 Hz), 7.03 - 7.11 (1H, m), 7.52 - 7.61(2H, m), 7.76 - 7.93 (5H, m), 8.02 - 8.10 (1H, m), 8.41 (1H, br s), 8.91 - 9.00 (1H, m).

### Working Example 101

### 3-(6-(2,4-Dichlorophenyl)thio)pyridin-2-yl)-N-(2-hydroxyethyl)benzamide

The titled compound was obtained using 3-(6-((2,4-dichlorophenyl)thio)pyridin-2-yl)benzoic acid obtained in Reference Example 77 and 2-aminoethanol in the same manner as in Working Example 2. Yield: 62%. Melting point: 117 - 118°C (tetrahydrofuran-hexane).
¹H-NMR (DMSO-d₆) δ: 3.32 - 3.41 (2H, m), 3.54 (2H, q, J = 5.9 Hz), 4.75 (1H, t, J = 5.9 Hz), 7.07 (1H, dd, J = 6.7, 1.8 Hz), 7.49 - 7.61 (2H, m), 7.75 - 7.93 (5H, m), 7.99 - 8.07 (1H, m), 8.41 (1H, br s), 8.51 - 8.61 (1H, m).

### Working Example 102

### 1-(2-((2-(3,4-Dimethoxyphenyl)ethyl)amino)pyrimidin-4-yl)-N-(2-(dimethylamino)ethyl)-1H-indole-5-carboxamide

The titled compound was obtained using 1-(2-((2-(3,4-dimethoxyphenyl)ethyl)amino)pyrimidin-4-yl)-1H-indole-5-carboxylic acid obtained in Reference Example 78 and N,N-dimethylethane-1,2-diamine in the same manner as in Working Example 2. Yield: 50%. Melting point: 140 - 141°C (ethyl acetate-hexane).
¹H-NMR (DMSO-d₆) δ: 2.19 (6H, s), 2.42 (2H, t, J = 6.8 Hz), 2.81 - 2.91 (2H, m), 3.34 - 3.43 (2H, m), 3.54 - 3.66 (2H, m), 3.71 (6H, s), 6.74 - 6.91 (4H, m), 6.98 (1H, d, J = 5.7 Hz), 7.55 (1H, br s), 7.76 (1H, dd, J = 8.9, 1.7 Hz), 8.11 - 8.18 (2H, m), 8.29 - 8.40 (2H, m), 8.60 - 8.92 (1H, m).

### Working Example 103

### N-(2-Cyanoethyl)-3-(6-((2,4-dichlorophenyl)amino)pyridin-2-yl)benzamide

The titled compound was obtained using 3-(6-((2,4-dichlorophenyl)amino)pyridin-2-yl)benzoic acid obtained in Reference Example 79 and 3-aminopropanenitrile in the same manner as in Working Example 2. Yield: 88%. Melting point: 137 - 138°C (ethyl acetate-hexane).
¹H-NMR (DMSO-d₆) δ: 2.81 (2H, t, J = 6.4 Hz), 3.54 (2H, q, J = 6.4 Hz), 7.02 - 7.08 (1H, m), 7.37 - 7.51(2H, m), 7.54 - 7.65 (2H, m), 7.70 - 7.79 (1H, m), 7.87 (1H, d, J = 7.7 Hz), 8.16 (1H, d, J = 7.7 Hz), 8.26 (1H, d, J = 8.9 Hz), 8.46 (1H, s), 8.64 (1H, s), 8.95 (1H, t, J = 5.7 Hz).

### Working Example 104

### 3-(6-((2,4-Dichlorophenyl)amino)pyridin-2-yl)-N-(2-hydroxyethyl)benzamide

The titled compound was obtained using 3-(6-((2,4-dichlorophenyl)amino)pyridin-2-yl)benzoic acid obtained in Reference Example 79 and 2-aminoethanol in the same manner as in Working Example 2. Yield: 71 %. Melting point: 131 - 132°C (tetrahydrofuran-hexane).
¹H-NMR (DMSO-d₆) δ: 3.33 - 3.41 ( 2H, m), 3.54 (2H, q, J = 5.9 Hz), 4.75 (1H, t, J = 5.9 Hz), 7.04 (1H, d, J = 8.3 Hz), 7.41 (1H, dd, J = 8.9, 2.5 Hz), 7.47 - 7.58 (2H, m), 7.63 (1H, d, J = 2.5 Hz), 7.75 (1H, t, J = 8.3 Hz), 7.86 (1H, d, J = 7.9 Hz ), 8.13 (1H, d, J = 7.9 Hz ), 8.23 - 8.30 (1H, m), 8.45 (1H, s), 8.54 (1H, t, J = 5.6 Hz), 8.63 (1H, s).

### Working Example 105

### N-(2-Cyanoethyl)-3-(6-(2,4-dichlorophenoxy)pyridin-2-yl)-4-methylbenzamide

The titled compound was obtained using 3-(6-(2,4-dichlorophenoxy)pyridin-2-yl)-4-methylbenzoic acid obtained in Reference Example 80 and 3-aminopropanenitrile in the same manner as in Working Example 2. Yield: 58%. Melting point: 139 - 140°C (ethyl acetate-hexane).
¹H-NMR (DMSO-d₆) δ: 2.13 (3H, s), 2.76 (2H, t, J = 6.5 Hz ), 3.48 (2H, q, J = 6.5 Hz), 7.18 (1H, d, J = 8.1 Hz), 7.32 (1H, d, J = 8.1 Hz), 7.36 - 7.42 (2H, m), 7.44 - 7.50 (1H, m), 7.72 - 7.79 (2H, m), 7.83 (1H, d, J = 1.7 Hz ), 8.03 (1H, t, J = 7.9 Hz ), 8.83 (1H, t, J = 5.6 Hz).

### Working Example 106

### 3-(6-(2,4-Dichlorophenoxy)pyridin-2-yl)-N-(2-hydroxyethyl)-4-methylbenzamide

The titled compound was obtained using 3-(6-(2,4-dichlorophenoxy)pyridin-2-yl)-4-methylbenzoic acid obtained in Reference Example 80 and 2-aminoethanol in the same manner as in Working Example 2. Yield: 58%. Melting point: 159 - 160°C (ethyl acetate-hexane).
¹H-NMR (DMSO-d₆) δ: 2.11 (3H, s), 3.26 - 3.35 (2H, m), 3.49 (2H, q, J = 6.0 Hz ), 4.70 (1H, t, J = 6.0 Hz), 7.17 (1H, d, J = 8.1 Hz ), 7.29 (1H, d, J = 8.1 Hz), 7.36 - 7.42 (2H, m), 7.44 - 7.50 (1H, m), 7.72 - 7.78 (2H, m), 7.83 (1H, d, J = 1.7 Hz ), 8.03 (1H, t, J = 7.8 Hz ), 8.43 (1H, t, J = 5.6 Hz).

### Working Example 107

### N-(2-Cyanoethyl)-3-(6-(2,4-dichlorophenoxy)pyridin-2-yl)-5-fluorobenzamide

The titled compound was obtained using 3-(6-(2,4-dichlorophenoxy)pyridin-2-yl)-5-fluorobenzoic acid obtained in Reference Example 81 and 3-aminopropanenitrile in the same manner as in Working Example 2. Yield: 32%. Melting point: 173 - 174°C (ethyl acetate-hexane).
¹H-NMR (DMSO-d₆) δ: 2.79 (2H, t, J = 6.4 Hz ), 3.52 (2H, q, J = 6.4 Hz), 7.16 (1H, d, J = 8.0 Hz ), 7.44 - 7.57 (2H, m), 7.62 - 7.74 (2H, m), 7.80 - 7.89 (2H, m), 8.05 (1H, t, J = 8.0 Hz), 8.23 (1H, s), 9.02 (1H, t, J = 5.5 Hz).

### Working Example 108

### 3-(6-(2,4-Dichlorophenoxy)pyridin-2-yl)-5-fluoro-N-(2-hydroxyethyl)benzamide

The titled compound was obtained using 3-(6-(2,4-dichlorophenoxy)pyridin-2-yl)-5-fluorobenzoic acid obtained in Reference Example 81 and 2-aminoethanol in the same manner as in Working Example 2. Yield: 69%. Melting point: 166 - 167°C (tetrahydrofuran-hexane).
¹H-NMR (DMSO-d₆) δ: 3.30 - 3.39 (2H, m), 3.53 (2H, q, J = 5.9 Hz), 4.75 (1H, t, J = 5.9 Hz), 7.15 (1H, d, J = 8.0 Hz ), 7.45 - 7.57 (2H, m), 7.66 (2H, d, J = 10.2 Hz), 7.81 - 7.91 (2H, m), 8.05 (1H, t, J = 8.0 Hz ), 8.24 (1H, s), 8.63 (1H, t, J = 5.7 Hz).

### Working Example 109

### N-(2-Cyanoethyl)-3-(6-(2,4-dichlorophenoxy)pyridin-2-yl)-4-fluorobenzamide

The titled compound was obtained using 3-(6-(2,4-dichlorophenoxy)pyridin-2-yl)-4-fluorobenzoic acid obtained in Reference Example 82 and 3-aminopropanenitrile in the same manner as in Working Example 2. Yield: 82%. Melting point: 192 - 193°C (tetrahydrofuran-hexane).
¹H-NMR (DMSO-d₆) δ: 2.77 (2H, t, J = 6.4 Hz), 3.44 - 3.54 (2H, m), 7.16 (1H, d, J = 8.0 Hz ), 7.38 - 7.52 (3H, m), 7.60 (1H, dd, J = 7.6, 1.5 Hz), 7.78 (1H, d, J = 2.2 Hz), 7.86 - 7.94 (1H, m), 8.04 (1H, t, J = 7.6 Hz), 8.13 (1H, dd, J = 7.6, 2.2 Hz), 8.88 (1H, t, J = 5.5 Hz).

### Working Example 110

### 3-(6-(2,4-Dichlorophenoxy)pyridin-2-yl)-4-fluoro-N-(2-hydroxyethyl)benzamide

The titled compound was obtained using 3-(6-(2,4-dichlorophenoxy)pyridin-2-yl)-4-fluorobenzoic acid obtained in Reference Example 82 and 2-aminoethanol in the same manner as in Working Example 2. Yield: 62%. Melting point: 176 - 177°C (tetrahydrofuran-hexane).
¹H-NMR (DMSO-d₆) δ: 3.26 - 3.37 (2H, m), 3.51 (2H, q, J = 6.2 Hz), 4.72 (1H, t, J = 6.2 Hz), 7.15 (1H, d, J = 8.3 Hz ), 7.34 - 7.52 (3H, m), 7.60 (1H, dd, J = 7.6, 1.5 Hz), 7.78 (1H, d, J = 2.2 Hz), 7.86 - 7.95 (1H, m), 8.03 (1H, t, J = 7.6 Hz ), 8.13 (1H, dd, J = 7.6, 2.2 Hz), 8.46 (1H, t, J = 5.3Hz).

### Working Example 111

### N-(2-Cyanoethyl)-3-(6-(2,4-dichlorophenoxy)pyridin-2-yl)-2-fluorobenzamide

The titled compound was obtained using 3-(6-(2,4-dichlorophenoxy)pyridin-2-yl)-2-fluorobenzoic acid obtained in Reference Example 83 and 3-aminopropanenitrile in the same manner as in Working Example 2. Yield: 76%. Melting point: 151 - 152°C (ethyl acetate-hexane).
¹H-NMR (DMSO-d₆) δ: 2.77 (2H, t, J = 6.6 Hz), 3.50 (2H, q, J = 6.6 Hz), 7.19 (1H, d, J = 8.0 Hz ), 7.33 (1H, t, J = 7.8 Hz), 7.42 - 7.53 (2H, m), 7.55 - 7.67 (3H, m), 7.80 (1H, d, J = 2.7 Hz), 8.03 (1H, t, J = 7.8 Hz), 8.77 (1H, t, J = 5.7 Hz).

### Working Example 112

### 3-(6-(2,4-Dichlorophenoxy)pyridin-2-yl)-2-fluoro-N-(2-hydroxyethyl)benzamide

The titled compound was obtained using 3-(6-(2,4-dichlorophenoxy)pyridin-2-yl)-2-fluorobenzoic acid obtained in Reference Example 83 and 2-aminoethanol in the same manner as in Working Example 2. Yield: 62%; melting point: 162 - 163°C (tetrahydrofuran-hexane).
¹H-NMR (DMSO-d₆) δ: 3.27 - 3.37 (2H, m), 3.50 (2H, q, J = 5.8 Hz), 4.72 (1H, t, J = 5.8 Hz), 7.18 (1H, d, J = 8.3 Hz ), 7.26 - 7.33 (1H, m), 7.41 - 7.53 (2H, m), 7.54 - 7.64 (3H, m), 7.80 (1H, d, J = 2.3 Hz), 8.03 (1H, t, J = 8.0 Hz), 8.33 (1H, t, J = 5.3Hz).

### Working Example 113

### N-(2-Cyanoethyl)-5-(6-(2,4-dichlorophenoxy)pyridin-2-yl)-2-fluorobenzamide

The titled compound was obtained using 5-(6-(2,4-dichlorophenoxy)pyridin-2-yl)-2-fluorobenzoic acid obtained in Reference Example 84 and 3-aminopropanenitrile in the same manner as in Working Example 2. Yield: 72%. Melting point: 165 - 166°C (tetrahydrofuran-hexane).
¹H-NMR (DMSO-d₆) δ: 2.77 (2H, t, J = 6.6 Hz), 3.50 (2H, q, J = 6.6 Hz), 7.10 (1H, d, J = 8.0 Hz ), 7.33 - 7.56 (3H, m), 7.72 - 7.83 (2H, m), 7.90 - 8.03 (2H, m), 8.07 (1H, dd, J = 6.8, 2.3 Hz), 8.73 (1H, br s).

### Working Example 114

### 5-(6-(2,4-Dichlorophenoxy)pyridin-2-yl)-2-fluoro-N-(2-hydroxyethyl)benzamide

The titled compound was obtained using 5-(6-(2,4-dichlorophenoxy)pyridin-2-yl)-2-fluorobenzoic acid obtained in Reference Example 84 and 2-aminoethanol in the same manner as in Working Example 2. Yield: 61%. Melting point: 142 - 143°C (tetrahydrofuran-hexane).
¹H-NMR (DMSO-d₆) δ: 3.28 - 3.37 (2H, m), 3.51 (2H, q, J = 5.8 Hz), 4.73 (1H, t, J = 5.8 Hz), 7.09 (1H, d, J = 8.3 Hz ), 7.29 - 7.39 (1H, m), 7.41 - 7.56 (2H, m), 7.74 - 7.83 (2H, m), 7.87 - 7.94 (1H, m), 7.99 (1H, t, J = 8.0 Hz), 8.07 (1H, dd, J = 6.8, 2.3 Hz), 8.29 (1H, br s).

### Working Example 115

### N-(2-Cyanoethyl)-3-(6-(2,4-dichlorophenoxy)-3-(trifluoromethyl)pyridin-2-yl)benzamide

The titled compound was obtained using 3-(6-(2,4-dichlorophenoxy)-3-(trifluoromethyl)pyridin-2-yl)benzoic acid obtained in Reference Example 85 and 3-aminopropanenitrile in the same manner as in Working Example 2. Yield: 61%. Melting point: 155 - 156°C (ethyl acetate-hexane).
¹H-NMR (DMSO-d₆) δ: 2.77 (2H, t, J = 6.5 Hz), 3.49 (2H, q, J = 6.5 Hz), 7.37 (1H, d, J = 8.7 Hz ), 7.46 - 7.60 (4H, m), 7.78 (1H, t, J = 1.4 Hz), 7.85 (1H, s), 7.89 - 7.96 (1H, m), 8.40 (1H, d, J = 8.7 Hz), 8.90 (1H, t, J = 5.6 Hz).

### Working Example 116

### 3-(6-(2,4-Dichlorophenoxy)-3-(trifluoromethyl)pyridin-2-yl)-N-(2-hydroxyethyl)benzamide

The titled compound was obtained using 3-(6-(2,4-dichlorophenoxy)-3-(trifluoromethyl)pyridin-2-yl)benzoic acid obtained in Reference Example 85 and 2-aminoethanol in the same manner as in Working Example 2. Yield: 47%. Melting point: 130 - 131°C (ethyl acetate-hexane).
¹H-NMR (DMSO-d₆) δ: 3.27 - 3.37 (2H, m), 3.50 (2H, q, J = 6.0 Hz), 4.71 (1H, t, J = 6.0 Hz), 7.37 (1H, d, J = 8.7 Hz ), 7.43 - 7.57 (4H, m), 7.78 (1H, s), 7.84 (1H, s), 7.93 (1H, d, J = 7.7 Hz), 8.39 (1H, d, J = 8.7 Hz), 8.48 (1H, t, J = 5.5Hz).

### Working Example 117

### N-(2-Cyanoethyl)-6-(2,4-dichlorophenoxy)-2,3'-bipyridine-5'-carboxamide

The titled compound was obtained using 6-(2,4-dichlorophenoxy)-2,3'-bipyridine-5'-carboxylic acid obtained in Reference Example 86 and 3-aminopropanenitrile in the same manner as in Working Example 2. Yield: 43%. Melting point: 152 - 153°C (ethyl acetate-hexane).
¹H-NMR (DMSO-d₆) δ: 2.80 (2H, t, J = 6.4 Hz), 3.54 (2H, q, J = 6.4 Hz), 7.19 (1H, d, J = 7.9 Hz ), 7.45 - 7.58 (2H, m), 7.83 (1H, d, J = 2.4 Hz), 7.90 (1H, d, J = 7.5 Hz), 8.03 - 8.11 (1H, m), 8.60 (1H, t, J = 2.2 Hz), 9.00 (1H, d, J = 2.2 Hz), 9.08 (1H, d, J = 2.2 Hz), 9.13 (1H, t, J = 5.5 Hz).

### Working Example 118

### 6-(2,4-dichlorophenoxy)-N-(2-hydroxyethyl)-2,3'-bipyridine-5'-carboxamide

The titled compound was obtained using 6-(2,4-dichlorophenoxy)-2,3'-bipyridine-5'-carboxylic acid obtained in Reference Example 86 and 2-aminoethanol in the same manner as in Working Example 2. Yield: 43%. Melting point: 180 - 181°C (tetrahydrofuran-hexane).
¹H-NMR (DMSO-d₆) δ: 3.32 - 3.40 (2H, m), 3.54 (2H, q, J = 5.8 Hz), 4.77 (1H, t, J = 5.8 Hz), 7.18 (1H, d, J = 8.1 Hz ), 7.45 - 7.58 (2H, m), 7.83 (1H, d, J = 2.3 Hz), 7.90 (1H, d, J = 7.5 Hz), 8.07 (1H, t, J = 7.5 Hz), 8.61 (1H, t, J = 2.3 Hz), 8.74 (1H, t, J = 5.4Hz), 8.97 - 9.06 (2H, m).

### Working Example 119

### N-(2-Cyanoethyl)-3-(6-(2,4-dichlorophenoxy)-4-(trifluoromethyl)pyridin-2-yl)benzamide

The titled compound was obtained using 3-(6-(2,4-dichlorophenoxy)-4-(trifluoromethyl)pyridin-2-yl)benzoic acid obtained in Reference Example 87 and 3-aminopropanenitrile in the same manner as in Working Example 2. Yield: 78%. Melting point: 231 - 232°C (tetrahydrofuran-hexane).
¹H-NMR (DMSO-d₆) δ: 2.80 (2H, t, J = 6.5 Hz), 3.53 (2H, q, J = 6.5 Hz), 7.53 - 7.63 (4H, m), 7.86 (1H, d, J = 1.3 Hz), 7.91 (1H, d, J = 7.9 Hz), 7.98 (1H, d, J = 7.9 Hz), 8.14 (1H, s), 8.39 (1H, s), 8.95 (1H, t, J = 5.6 Hz).

### Working Example 120

### 3-(6-(2,4-Dichlorophenoxy)-4-(trifluoromethyl)pyridin-2-yl)-N-(2-hydroxyethyl)benzamide

The titled compound was obtained using 3-(6-(2,4-dichlorophenoxy)-4-(trifluoromethyl)pyridin-2-yl)benzoic acid obtained in Reference Example 87 and 2-aminoethanol in the same manner as in Working Example 2. Yield: 84%. Melting point: 197 - 198°C (tetrahydrofuran-hexane).
¹H-NMR (DMSO-d₆) δ: 3.32 - 3.41 (2H, m), 3.54 (2H, q, J = 5.8 Hz), 4.77 (1H, t, J = 5.8 Hz), 7.48 - 7.62 (4H, m), 7.86 (1H, d, J = 1.1 Hz), 7.88 - 7.96 (2H, m), 8.16 (1H, s), 8.40 (1H, s), 8.57 (1H, t, J = 5.6Hz).

### Working Example 121

### N-(2-Cyanoethyl)-3-(6-(3,5-dichlorophenoxy)pyridin-2-yl)benzamide

The titled compound was synthesized using 3-(6-(3,5-dichlorophenoxy)pyridin-2-yl)benzoic acid obtained in Reference Example 39 and 3-aminopropanenitrile in the same manner as in Working Example 2. Yield: 60%. Melting point: 171 - 172°C (THF-diethyl ether).
¹H-NMR (DMSO-d₆):δ 1.98-2.10 (2H, m), 2.81 (2H, t, J = 6.4 Hz), 3.46 (2H, br s), 3.61 (3H, s), 5.68 (1H, d, J = 2.7 Hz), 6.24 (1H, dd, J = 8.3 Hz and 2.7 Hz), 6.93 (1H, d, J = 8.0 Hz), 7.24 - 7.26 (2H, m), 7.49 (1H, s).

### Working Example 122

### N-(2-Cyanoethyl)-3-(6-(2,4-dimethylphenoxy)pyridin-2-yl)benzamide

Tetrakistriphenylphosphine palladium (0) (58.9 mg, 0.051 mmol) was added to a mixture of 2-chloro-6-(2,4-dimethylphenoxy)pyridine (300 mg, 1.28 mmol) obtained in Reference Example 44, N-(2-cyanoethyl)-3-(4,4,5,5-tetramethyl-1,3,2-dioxaborolan-2-yl)benzamide (462 mg, 1.54 mmol) obtained in Reference Example 43, 2 N sodium carbonate aqueous solution (2.56 mL), and DME (7 mL), and the mixture was heated and stirred for 18 hours to 90°C in a nitrogen atmosphere. Saturated aqueous sodium bicarbonate was added to the reaction solution, and the product was extracted with ethyl acetate. The extract was dried over anhydrous magnesium sulfate, and the solvent was then distilled off at reduced pressure. The residue was purified by silica gel column chromatography to give 126 mg of the titled compound (yield: 27%). Melting point: 114 - 115°C (ethyl acetate-hexane).
¹H-NMR (CDCl₃):δ 2.19 (3H, s), 2.37 (3H, s), 2.77 (2H, t, J = 6.2 Hz), 3.73 (2H, q, J = 6.2 Hz), 6.66 (1H, d, J = 8.0 Hz), 6.71 (1H, br s), 6.98- 7.16 (3H, m), 7.46 - 7.55 (2H, m), 7.68 - 7.76 (1H, m), 7.85 (1H, d, J = 8.3 Hz), 8.07 (1H, d, J = 8.0 Hz), 8.34 (1H, s).

### Working Example 123

### N-(2-Cyanoethyl)-3-(6-(2,4-difluorophenoxy)pyridin-2-yl)benzamide

The titled compound was synthesized using 2-chloro-6-(2,4-difluorophenoxy)pyridine obtained in Reference Example 45 in the same manner as in Working Example 122. Yield: 45%. Melting point: 133 - 1324°C (ethyl acetate-hexane).
¹H-NMR (CDCl₃):δ 2.76 (2H, t, J = 6.2 Hz), 3.72 (2H, q, J = 6.2 Hz), 6.57 (1H, br s), 6.90 - 7.08 (2H, m), 6.94 (1H, d, J = 8.3 Hz), 7.22 - 7.32 (1H, m), 7.47 (1H, t, J = 7.8 Hz), 7.54 (1H, d, J = 7.2 Hz), 7.74 - 7.84 (2H, m), 7.98 (1H, d, J = 8.0 Hz), 8.21 (1H, s).

### Working Example 124

### 3-(6-(4-chlorophenoxy)pyridin-2-yl)-N-(2-cyanoethyl)benzamide

The titled compound was synthesized using 2-chloro-6-(4-chlorophenoxy)pyridine obtained in Reference Example 46 in the same manner as in Working Example 122. Yield: 44%. Melting point: 143 - 144°C (ethyl acetate-hexane).
¹H-NMR (CDCl₃):δ 2.77 (2H, t, J = 6.3 Hz), 3.74 (2H, q, J = 6.2 Hz), 6.62 (1H, br s), 6.86 (1H, d, J = 8.3 Hz), 7.18 (2H, d, J = 8.9 Hz), 7.41 (2H, d, J = 8.9 Hz), 7.47 - 7.60 (2H, m), 7.75 - 7.87 (2H, m), 8.02 - 8.08 (1H, m), 8.28 (1H, s).

### Working Example 125

### 3-(6-(3-Chlorophenoxy)pyridin-2-yl)-N-(2-cyanoethyl)benzamide

The titled compound was synthesized using 2-chloro-6-(3-chlorophenoxy)pyridine obtained in Reference Example 47 in the same manner as in Working Example 122. Yield: 41%. Melting point: 139 - 140°C (ethyl acetate-hexane).
¹H-NMR (CDCl₃):δ 2.77 (2H, t, J = 6.2 Hz), 3.74 (2H, q, J = 6.4 Hz), 6.66 (1H, br s), 6.88 (1H, d, J = 8.0 Hz), 7.12 (1H, dd, J = 8.7 Hz and 1.9 Hz), 7.20 - 7.25 (1H, m), 7.31 (1H, t, J = 2.1 Hz), 7.37 (1H, t, J = 8.1 Hz), 7.51 (1H, t, J = 7.8 Hz), 7.56 (1H, d, J = 7.6 Hz), 7.77 - 7.88 (2H, m), 8.06 (1H, d, J = 8.0 Hz), 8.29 (1H, s).

### Working Example 126

### 3-(6-(2-Chlorophenoxy)pyridin-2-yl)-N-(2-cyanoethyl)benzamide

The titled compound was synthesized using 2-chloro-6-(2-chlorophenoxy)pyridine obtained in Reference Example 48 in the same manner as in Working Example 122. Yield: 51%. Melting point: 82 - 83°C (ethyl acetate-hexane).
¹H-NMR (CDCl₃):δ 2.75 (2H, t, J = 6.4 Hz), 3.71 (2H, q, J = 6.4 Hz), 6.64 (1H, t, J = 5.5 Hz), 6.87 (1H, d, J = 8.0 Hz), 7.20 - 7.27 (1H, m), 7.27 - 7.40 (2H, m), 7.46 (1H, t, J = 7.8 Hz), 7.53 (2H, d, J = 8.0 Hz), 7.79 (2H, t, J = 8.0 Hz), 8.00 (1H, d, J = 8.0 Hz), 8.24 (1H, s).

### Working Example 127

### N-(2-Cyanoethyl)-3-(6-(3-trifluoromethyl)phenoxy)pyridin-2-yl)benzamide

The titled compound was synthesized using 2-chloro-6-(3-trifluoromethyl)phenoxy)pyridine in the same manner as in Working Example 122. Yield: 33%. Melting point: 136 - 137°C (ethyl acetate-hexane).
¹H-NMR (CDCl₃):δ 2.76 (2H, t, J = 6.4 Hz), 3.72 (2H, q, J = 6.1 Hz), 6.56 (1H, br s), 6.93 (1H, d, J = 8.3 Hz), 7.42 (1H, d, J = 8.0 Hz), 7.45 - 7.62 (5H, m), 7.83 (2H, t, J = 7.8 Hz), 8.03 (1H, d, J = 8.0 Hz), 8.23 (1H, s).

### Working Example 128

### N-(2-Cyanoethyl)-3-(6-(3-methoxyphenoxy)pyridin-2-yl)benzamide

The titled compound was synthesized using 2-chloro-6-(3-methoxyphenoxy)pyridine obtained in Reference Example 49 in the same manner as in Working Example 122. Yield: 17%. Melting point: 85 - 86°C (ethyl acetate-hexane).
¹H-NMR (CDCl₃):δ 2.77 (2H, t, J = 6.2 Hz), 3.73 (2H, q, J = 6.4 Hz), 3.82 (3H, s), 6.68 (1H, br s), 6.77 - 6.84 (4H, m), 7.29 - 7.37 (1H, m), 7.47-7.57 (2H, m), 7.77 (1H, t, J = 7.8 Hz), 7.84 (1H, d, J = 8.0 Hz), 8.09 (1H, d, J = 8.0 Hz), 8.34 (1H, s).

### Working Example 129

### N-(2-Cyanoethyl)-3-(6-(cyclohexyloxy)pyridin-2-yl)benzamide

The titled compound was synthesized using 2-chloro-6-(cyclohexyloxy)pyridine obtained in Reference Example 50 in the same manner as in Working Example 122. Yield: 72%. Melting point: 131 - 132°C (ethyl acetate-hexane).
¹H-NMR (CDCl₃):δ 1.24 - 1.69 (6H, m), 1.77 - 1.90 (2H, m), 2.05 - 2.16 (2H, m), 2.78 (2H, t, J = 6.2 Hz), 3.76 (2H, q, J = 6.1 Hz), 5.12 - 5.25 (1H, m), 6.65 - 6.77 (2H, m), 7.35 (1H, d, J = 6.8 Hz), 7.54 (1H, t, J = 7.8 Hz), 7.59 - 7.68 (1H, m), 7.78 (1H, d, J = 8.3 Hz), 8.19 (1H, d, J = 8.0 Hz), 8.41 (1H, s).

### Working Example 130

### N-(2-Cyanoethyl)-3-(6-(1H-indol-5-yloxy)pyridin-2-yl)benzamide

The titled compound was synthesized using 5-((6-chloropyridin-2-yl)oxy)-1H-indole obtained in Reference Example 51 in the same manner as in Working Example 122. Yield: 77%. Melting point: 150 - 151°C (ethyl acetate-hexane).
¹H-NMR (CDCl₃):δ 2.69 (2H, t, J = 6.5 Hz), 3.63 (2H, q, J = 6.2 Hz), 6.54 (1H, br s.), 6.58 (1H, br s), 6.76 (1H, d, J = 8.3 Hz), 7.08 (1H, dd, J = 8.8 Hz and 2.0 Hz), 7.30 (1H, t, J = 2.6 Hz), 7.41 - 7.54 (4H, m), 7.71 (1H, t, J = 7.9 Hz), 7.86 (1H, d, J = 7.9 Hz), 8.08 (1H, d, J = 7.5 Hz), 8.31 (1H, s), 8.37 (1H, br s).

### Working Example 131

### N-(2-Cyanoethyl)-3-(6-(2,3-dihydro-1H-inden-5-yloxy)pyridin-2-yl)benzamide

The titled compound was synthesized 2-chloro-6-(2,3-dihydro-1H-inden-5-yloxy)pyridine obtained in Reference Example 52 in the same manner as in Working Example 122. Yield: 55%. Melting point: 122 - 123°C (ethyl acetate-hexane).
¹H-NMR (CDCl₃):δ 2.05-2.24 (2H, m), 2.77 (2H, t, J = 6.4 Hz), 2.93 (4H, t, J = 7.4 Hz), 3.73 (2H, q, J = 6.3 Hz), 6.74 (2H, d, J = 8.1 Hz), 6.98 (1H, d, J = 8.1 Hz), 7.06 (1H, s), 7.22 - 7.29 (1H, m), 7.47 - 7.57 (2H, m), 7.73 (1H, t, J = 7.9 Hz), 7.84 (1H, d, J = 7.7 Hz), 8.11 (1H, d, J = 7.9 Hz), 8.37 (1 H, s).

### Working Example 132

### 3-(6-(3-Acetylphenoxy)pyridin-2-yl)-N-(2-cyanoethyl)benzamide

The titled compound was synthesized using 1-(3-((6-chloropyridin-2-yl)oxy)phenyl)ethanone obtained in Reference Example 53 in the same manner as in Working Example 122. Yield: 50%. Melting point: 140 - 141°C (ethyl acetate-hexane).
¹H-NMR (CDCl₃):δ 2.68 (3H, s), 2.78 (2H, t, J = 6.5 Hz), 3.76 (2H, q, J = 6.4 Hz), 6.94 (1H, d, J = 7.5 Hz), 7.18 (1H, br s), 7.38 - 7.46 (1H, m), 7.47 - 7.58 (3H, m), 7.76 - 7.86 (2H, m), 7.91 - 8.03 (2H, m), 8.20 - 8.25 (1H, m), 8.32 (1H, t, J = 1.6 Hz).

### Working Example 133

### N-(2-Cyanoethyl)-3-(6-(3-(dimethylamino)phenoxy)pyridin-2-yl)benzamide

The titled compound was synthesized using (3-((6-chloropyridin-2-yl)oxy)-N,N-dimethylaniline obtained in Reference Example 54 in the same manner as in Working Example 122. Yield: 68%. Melting point: 95 - 96°C (ethyl acetate-hexane).
¹H-NMR (CDCl₃):δ 2.77 (2H, t, J = 6.4 Hz), 2.98 (6H, s), 3.74 (2H, q, J = 6.4 Hz), 6.58 (3H, br s), 6.78 (2H, d, J = 8.3 Hz), 7.23 - 7.34 (1H, m), 7.52 (2H, t, J = 7.1 Hz), 7.74 (1H, t, J = 7.8 Hz), 7.87 (1H, d, J = 7.5 Hz), 8.11 (1H, d, J = 7.0 Hz), 8.38 (1H, t, J = 1.7 Hz).

### Working Example 134

### 3-(6-((5-Chloropyridin-3-yl)oxy)pyridin-2-yl)-N-(2-cyanoethyl)benzamide

The titled compound was synthesized using 2-chloro-6-((5-chloropyridin-3-yl)oxy)pyridine obtained in Reference Example 55 in the same manner as in Working Example 122. Yield: 45%. Melting point: 143 - 144°C (ethyl acetate-hexane).
¹H-NMR (CDCl₃):δ 2.78 (2H, t, J = 6.2 Hz), 3.75 (2H, q, J = 6.4 Hz), 6.64 (1H, br s), 6.98 (1H, d, J = 8.3 Hz), 7.51 (1H, t, J = 7.8 Hz), 7.61 (1H, d, J = 7.6 Hz), 7.70 (1H, t, J = 2.3 Hz), 7.78 - 7.90 (2H, m), 8.02 (1H, d, J = 8.0 Hz), 8.26 (1H, s), 8.46 (1H, d, J = 2.3 Hz), 8.54 (1H, d, J = 2.3 Hz).

### Working Example 135

### N-(2-Cyanoethyl)-3-(6-(2,5-dichlorophenoxy)pyridin-2-yl)benzamide

The titled compound was synthesized using 2-chloro-6-(2,5-dichlorophenoxy)pyridine obtained in Reference Example 56 in the same manner as in Working Example 122. Yield: 27%. Melting point: 149 - 150°C (ethyl acetate-hexane).
¹H-NMR (CDCl₃):δ 2.77 (2H, t, J = 6.3 Hz), 3.73 (2H, q, J = 6.2 Hz), 6.61 (1H, br s), 6.94 (1H, d, J = 8.1 Hz), 7.22 (1H, dd, J = 8.6 Hz and 2.4 Hz), 7.37 (1H, d, J = 2.4 Hz), 7.42 - 7.52 (2H, m), 7.56 (1H, d, J = 7.5 Hz), 7.78 - 7.87 (2H, m), 7.99 (1H, d, J = 7.7 Hz), 8.23 (1H, s).

### Working Examples 136 - 166

The compounds of Working Examples 136 through 166 were synthesized by reacting ethyl 3-(2-chloropyrimidin-4-yl)benzoate obtained in Reference Example 4 with various amines and 2-pyrrolidin-1-ylethanamine in the same manner as in Reference Example 5, Reference Example 6, and Working Example 2.

### Working Examples 167 - 233

The compounds of Working Examples 167 through 234 were synthesized by reacting 3-(6-(2-(3,4-dimethoxyphenyl)ethoxy)pyridin-2-yl)benzoic acid obtained in Reference Example 23 with various amines in the same manner as in Working Example 2.

### Working Examples 234 - 324

The compounds of Working Examples 234 through 324 were synthesized by reacting 3-(6-(2-(3,4-dimethoxyphenyl)ethoxy)pyridin-2-yl)aniline obtained in Reference Example 19 with various carboxylic acids in the same manner as in Working Example 2.

### Working Example 325

3-(6-((2,4-Dichlorobenzyl)amino)pyridine-2-yl)-N-(2-hydroxyethyl)benzamide The titled compound was obtained using 3-(6-((2-(3-(trifluoromethyl)phenyl)ethyl)amino)pyridin-2-yl)benzoic acid obtained in Reference Example 93 and 2-aminoethanol in the same manner as in Working Example 2. Melting point: 173 - 174°C.
¹H-NMR (DMSO-d₆) δ : 3.52 (2H, q, J = 5.9 Hz), 4.63 (2H, d, J = 5.8 Hz), 4.71 (1H, t, J = 5.6 Hz), 6.54 (1H, d, J = 8.2 Hz), 7.16 (1H, d, J = 7.1 Hz), 7.27 (1H, t, J = 5.2 Hz), 7.32 - 7.40 (1H, m), 7.41 - 7.55 (3H, m), 7.58 (1H, d, J = 2.2 Hz), 7.80 (1H, d, J = 8.0 Hz), 8.04 (1H, d, J = 7.7 Hz), 8.38 (1H, s), 8.48 (1H, t, J = 5.2 Hz) 2H unconfirmed

### Working Example 326

### N-(2-hydroxyethyl)-3-(6-((2-(3-(trifluoromethyl)phenyl)ethyl)amino)pyridine-2-yl)benzamide

The titled compound was obtained using 3-(6-((2,4-dichlorobenzyl)amino)pyridin-2-yl)benzoic acid obtained in Reference Example 92 and 2-aminoethanol in the same manner as in Working Example 2.
Melting point: 112 - 113°C.
¹H-NMR (CDCl₃) δ : 3.05 (2H, t, J = 7.0 Hz), 3.60 - 3.75 (4H, m), 3.85 (2H, d, J = 5.5 Hz), 4.69 (1H, br. s.), 6.38 (1H, d, J = 8.0 Hz), 6.71 (1H, br. s.), 7.11 (1H, d, J = 7.4 Hz), 7.40 - 7.56 (6H, m), 7.72 - 7.83 (1H, m), 8.12 (1H, dd, J = 7.8, 1.2 Hz), 8.39 (1H, t, J = 1.8 Hz)

### Working Example 327

3-{6-[(2,4-Dichlorobenzyl)(ethyl)amino]pyridin-2-yl}-N-(2-hydroxyethyl)benzamide 3-{6-[(2,4-dichlorobenzyl)amino]pyridin-2-yl}-N-(2-hydroxyethyl)benzamide (150 mg, 0.36 mmol) obtained in Working Example 325 was reacted with an ethanol (4 mL) solution of acetaldehyde (0.65 mL) and acetic acid (108 mg, 1.8 mmol) for 15 min at 50°C, and sodium cyanotrihydroborate (28.3 mg, 0.45 mmol) was then added, and the mixture was reacted for another hour. To bring the reaction to conclusion, acetaldehyde (0.65 mL) and sodium cyanotrihydroborate (28.3 mg, 0.45 mmol) were added, and the mixture was reacted for one hour and then concentrated at reduced pressure. Ethyl acetate was added to the resulting residue, which was washed with saturated sodium bicarbonate aqueous solution and then dried over anhydrous sodium sulfate. The solvent was distilled off at reduced pressure, and the resulting residue was purified by NH silica gel column chromatography (hexane-ethyl acetate 1:1 → 1:9) and recrystallized from ethyl acetate-hexane to give 35.2 mg of the titled compound in the form of crystals (yield: 22%). Melting point: 118 - 119°C.
¹H-NMR (CDCl₃) δ : 1.29 (3H, t, J = 7.0 Hz), 2.56 (1H, t, J = 5.1 Hz), 3.60 - 3.76 (4H, m), 3.86 (2H, q, J = 4.8 Hz), 4.84 (2H, s), 6.44 (1H, d, J = 8.8 Hz), 6.54 (1H, br.s.), 7.08 - 7.16 (3H, m), 7.42 (1H, s), 7.44 - 7.57 (2H, m), 7.79 (1H, d, J = 7.7 Hz), 8.08 (1H, d, J = 7.7 Hz), 8.29 (1H, s).
Table 1 shows the chemical formulas of the compounds synthesized in Working Examples 1 through 327 ("Working Example" is abbreviated as "Ex." in the table).
Table 2 also shows the measured results for the LCMS of the compounds synthesized in Working Examples 136 through 324 ("Working Example No." is abbreviated as "Ex. No." in the table).

### [Table 1]

**[Table 1-1]**

| | | |
|---|---|---|
| Ex.1 | Ex.2 | Ex.3 |
| 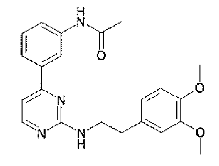 | 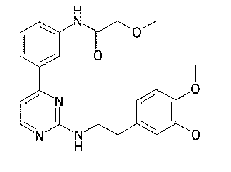 | 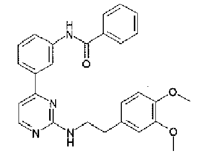 |
| Ex.4 | Ex.5 | Ex.6 |
| 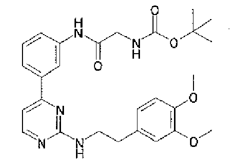 | 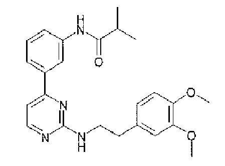 | 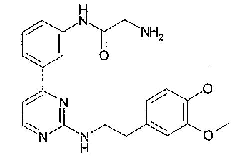 |
| Ex.7 | Ex.8 | Ex.9 |
| 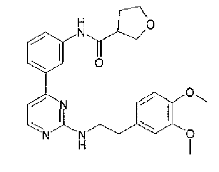 | 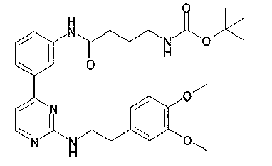 | 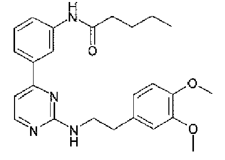 |
| Ex.10 | Ex.11 | Ex.12 |
| 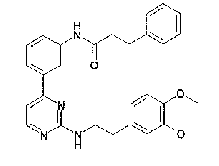 | 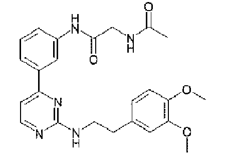 | 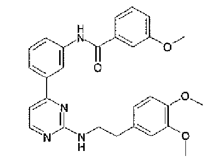 |
| Ex.13 | Ex.14 | Ex.15 |
| 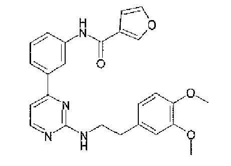 | 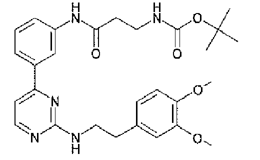 | 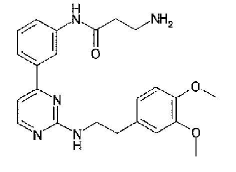 |
| Ex.16 | Ex.17 | Ex.18 |
| 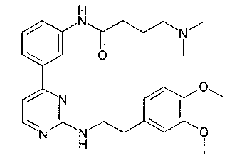 | 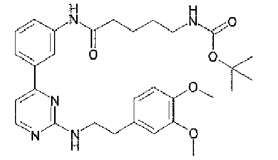 | 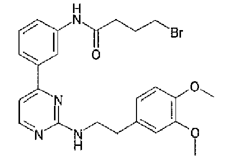 |

**[Table 1-2]**

| | | |
|---|---|---|
| Ex.19 | Ex.20 | Ex.21 |
| 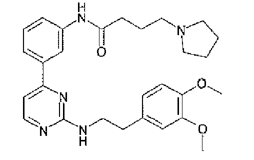 | 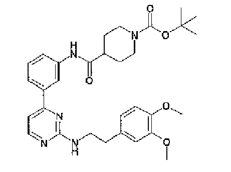 | 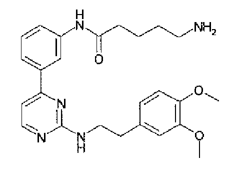 |
| Ex.22 | Ex.23 | Ex.24 |
| 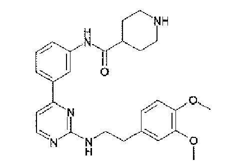 | 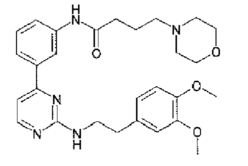 | 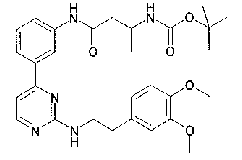 |
| Ex.25 | Ex.26 | Ex.27 |
| 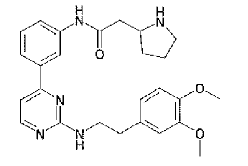 | 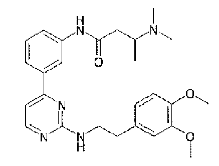 | 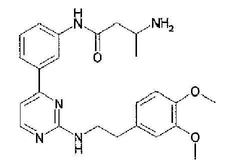 |
| Ex.28 | Ex.29 | Ex.30 |
| 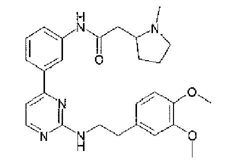 | 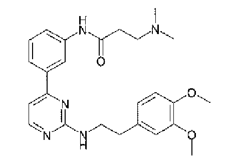 | 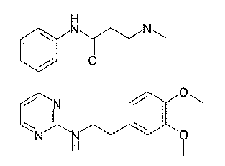 |
| Ex.31 | Ex.32 | Ex.33 |
| 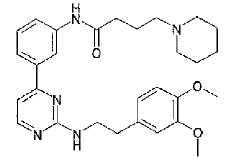 | 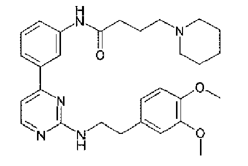 | 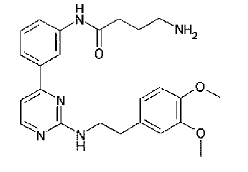 |
| Ex.34 | Ex.35 | Ex.36 |
| 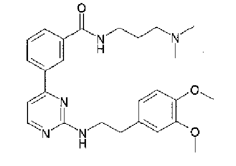 | 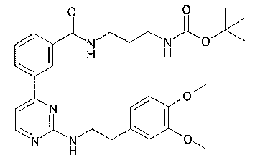 | 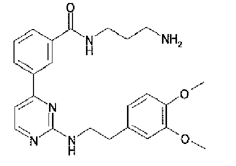 |

**[Table 1-3]**

| | | |
|---|---|---|
| Ex.37 | Ex.38 | Ex.39 |
| 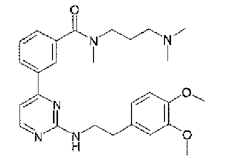 | 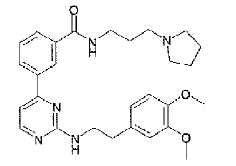 | 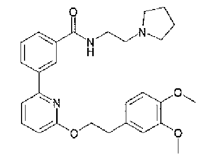 |
| Ex.40 | Ex.41 | Ex.42 |
| 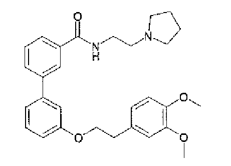 | 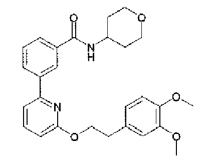 | 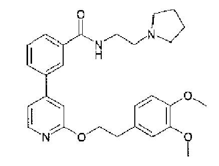 |
| Ex.43 | Ex.44 | Ex.45 |
| 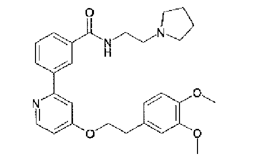 | 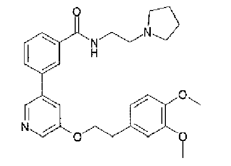 | 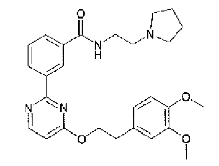 |
| Ex.46 | Ex.47 | Ex.48 |
| 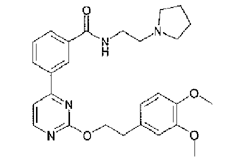 | 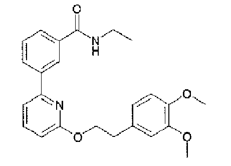 | 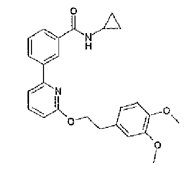 |
| Ex.49 | Ex.50 | Ex.51 |
| 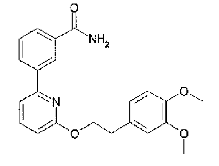 | 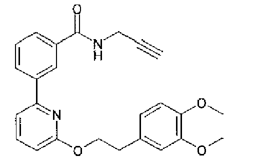 | 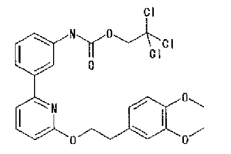 |
| Ex.52 | Ex.53 | Ex.54 |
| 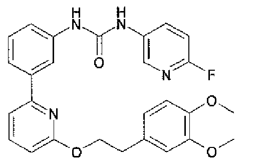 | 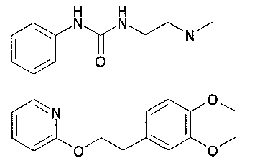 | 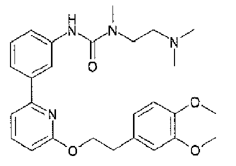 |

**[Table 1-4]**

| | | |
|---|---|---|
| Ex.55 | Ex.56 | Ex.57 |
| 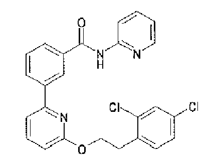 | 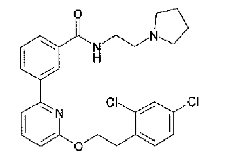 | 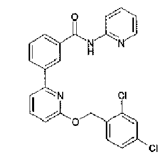 |
| Ex.58 | Ex.59 | Ex.60 |
| 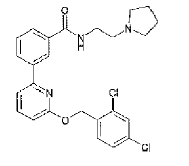 | 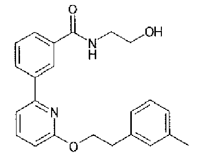 | 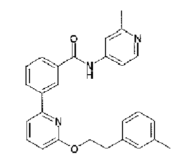 |
| Ex.61 | Ex.62 | Ex.63 |
| 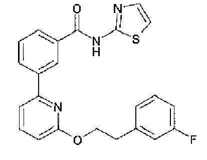 | 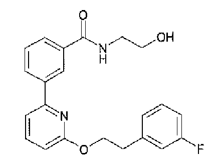 | 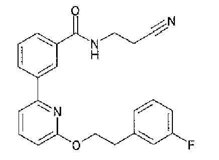 |
| Ex.64 | Ex.65 | Ex.66 |
| 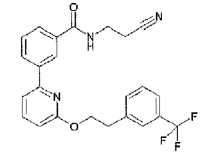 | 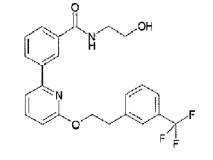 | 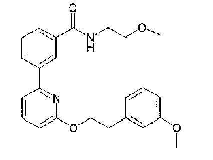 |
| Ex.67 | Ex.68 | Ex.69 |
| 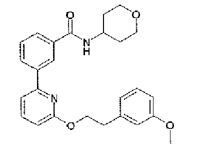 | 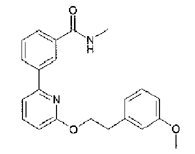 | 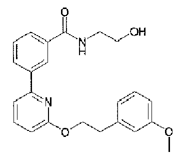 |
| Ex.70 | Ex.71 | Ex.72 |
| 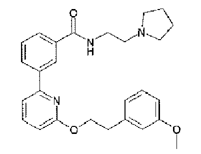 | 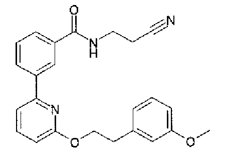 | 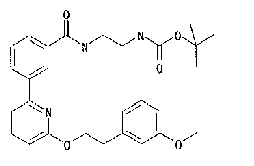 |

**[Table 1-5]**

| | | |
|---|---|---|
| Ex.73 | Ex.74 | Ex.75 |
| 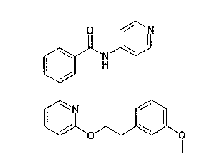 | 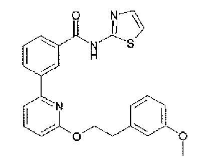 | 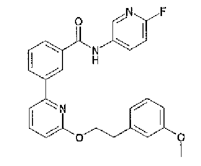 |
| Ex.76 | Ex.77 | Ex.78 |
| 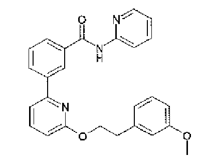 | 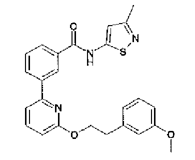 | 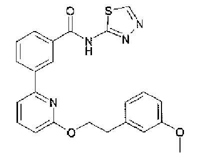 |
| Ex.79 | Ex.80 | Ex.81 |
| 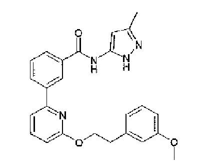 | 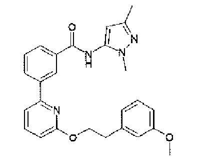 | 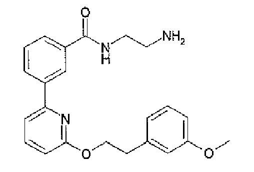 |
| Ex.82 | Ex.83 | Ex.84 |
| 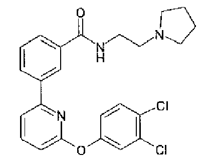 | 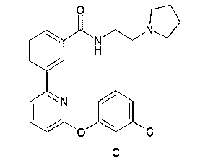 | 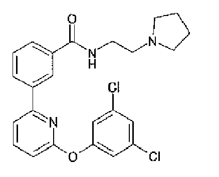 |
| Ex.85 | Ex.86 | Ex.87 |
| 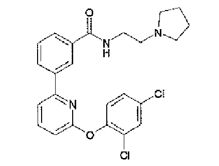 | 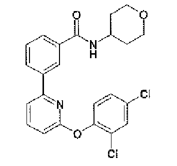 | 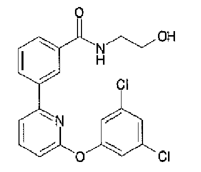 |
| Ex.88 | Ex.89 | Ex.90 |
| 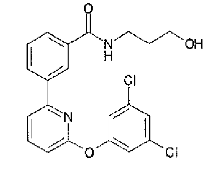 | 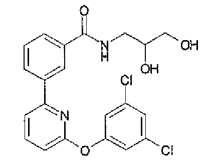 | 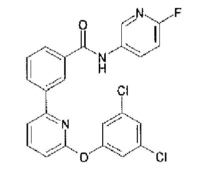 |

**[Table 1-6]**

| | | |
|---|---|---|
| Ex.91 | Ex.92 | Ex.93 |
| 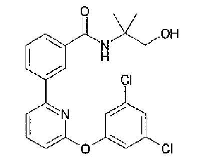 | 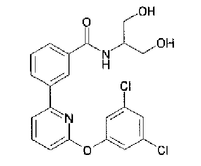 | 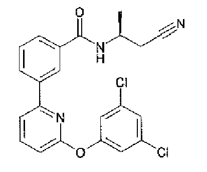 |
| Ex.94 | Ex.95 | Ex.96 |
| 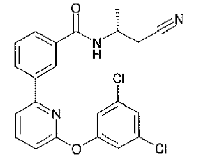 | 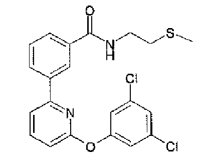 | 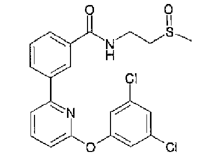 |
| Ex.97 | Ex.98 | Ex.99 |
| 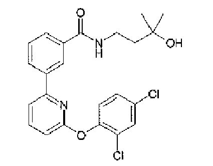 | 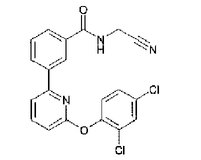 | 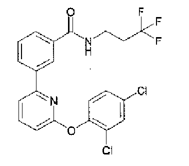 |
| Ex.100 | Ex.101 | Ex.102 |
| 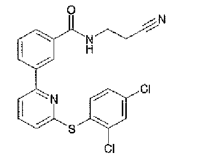 | 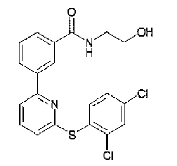 | 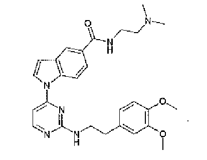 |
| Ex.103 | Ex.104 | Ex.105 |
| 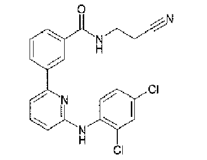 | 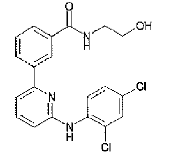 | 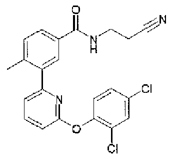 |
| Ex.106 | Ex.107 | Ex.108 |
| 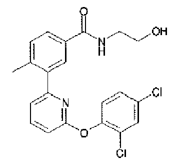 | 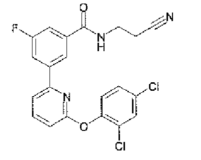 | 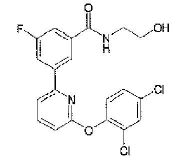 |

**[Table 1-7]**

| | | |
|---|---|---|
| Ex.109 | Ex.110 | Ex.111 |
| 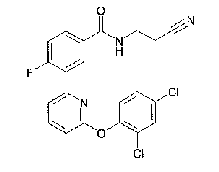 | 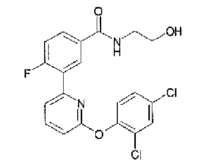 | 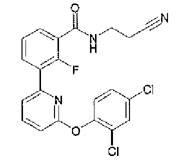 |
| Ex.112 | Ex.113 | Ex.114 |
| 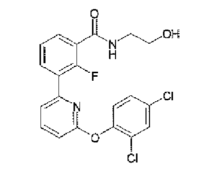 | 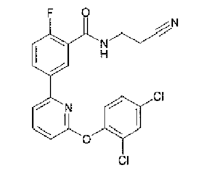 | 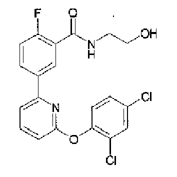 |
| Ex.115 | Ex.116 | Ex.117 |
| 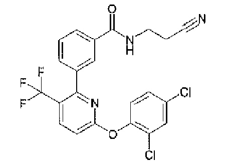 | 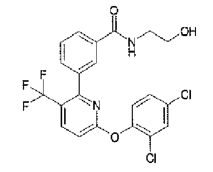 | 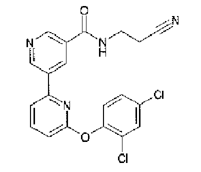 |
| Ex.118 | Ex.119 | Ex.120 |
| 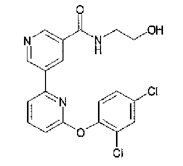 | 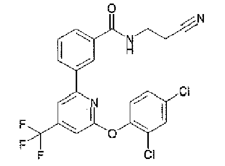 | 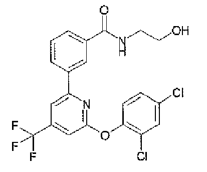 |
| Ex.121 | Ex.122 | Ex.123 |
| 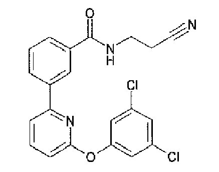 | 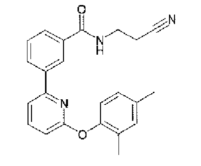 | 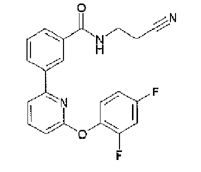 |
| Ex.124 | Ex.125 | Ex.126 |
| 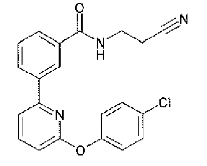 | 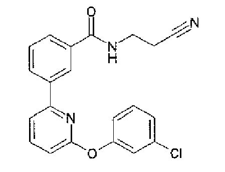 | 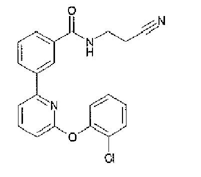 |

**[Table 1-8]**

| | | |
|---|---|---|
| Ex.127 | Ex.128 | Ex.129 |
| 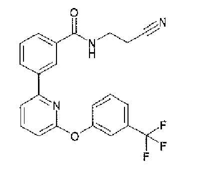 | 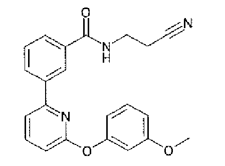 | 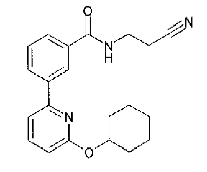 |
| Ex.130 | Ex.131 | Ex.32 |
| 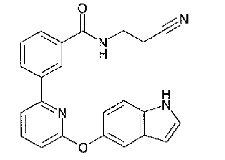 | 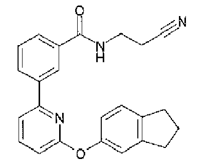 | 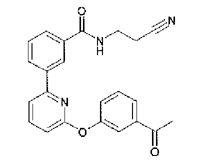 |
| Ex.133 | Ex.134 | Ex.135 |
| 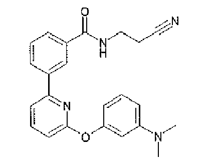 | 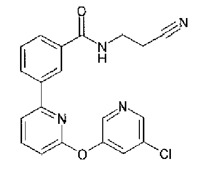 | 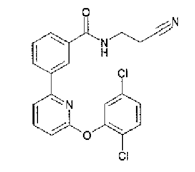 |
| Ex.136 | Ex.137 | Ex.138 |
| 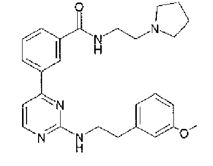 | 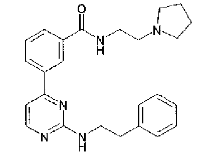 | 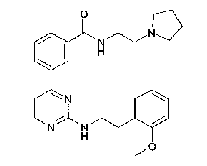 |
| Ex.139 | Ex.140 | Ex.141 |
| 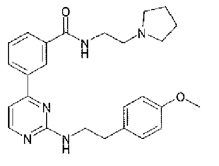 | 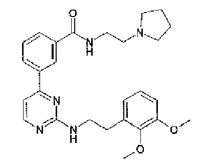 | 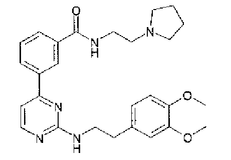 |
| Ex.142 | Ex.143 | Ex.144 |
| 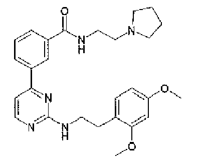 | 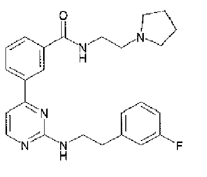 | 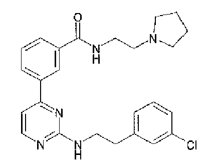 |

**[Table 1-9]**

| | | |
|---|---|---|
| Ex.145 | Ex.146 | Ex.147 |
| 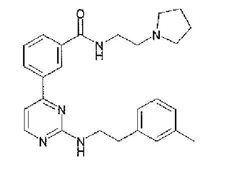 | 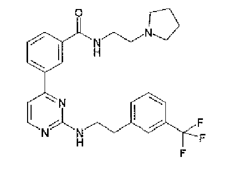 | 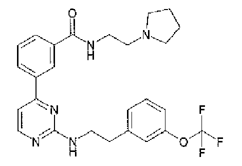 |
| Ex.148 | Ex.149 | Ex.150 |
| 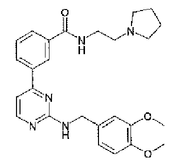 | 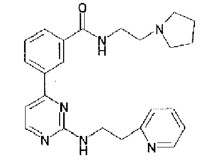 | 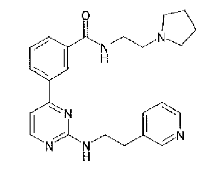 |
| Ex.151 | Ex.152 | Ex.153 |
| 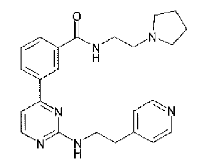 | 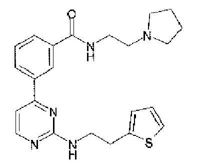 | 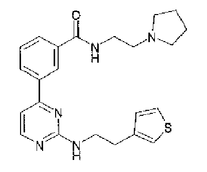 |
| Ex.154 | Ex.155 | Ex.156 |
| 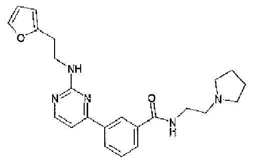 | 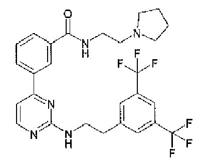 | 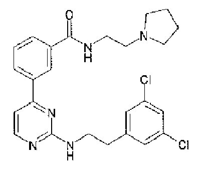 |
| Ex.157 | Ex.158 | Ex.159 |
| 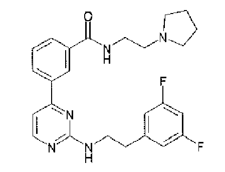 | 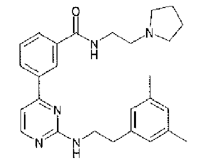 | 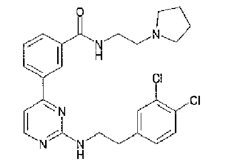 |
| Ex.160 | Ex.161 | Ex.162 |
| 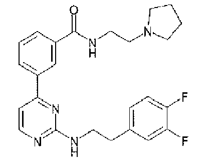 | 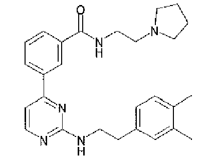 | 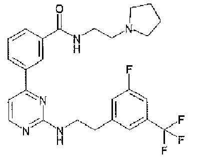 |

**[Table 1-10]**

| | | |
|---|---|---|
| Ex.163 | Ex.164 | Ex.165 |
| 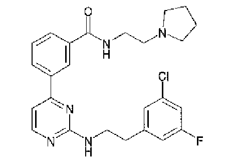 | 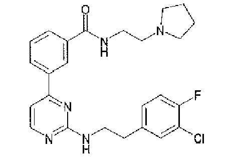 | 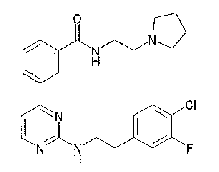 |
| Ex.166 | Ex.167 | Ex.168 |
| 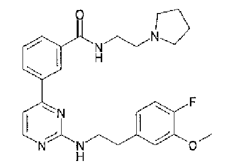 | 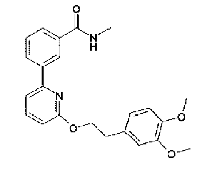 | 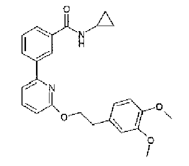 |
| Ex.169 | Ex.170 | Ex.171 |
| 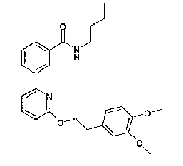 | 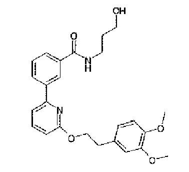 | 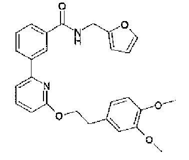 |
| Ex.172 | Ex.173 | Ex.174 |
| 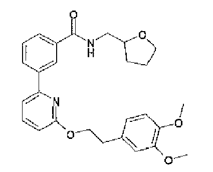 | 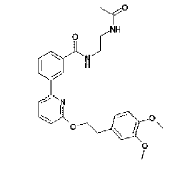 | 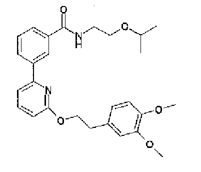 |
| Ex.175 | Ex.176 | Ex.177 |
| 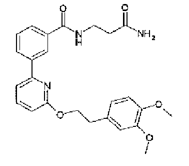 | 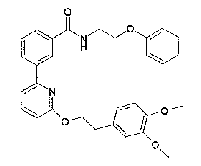 | 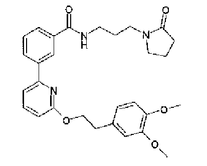 |
| Ex.178 | Ex.179 | Ex.180 |
| 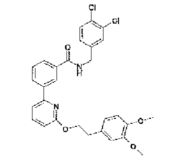 | 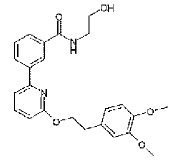 | 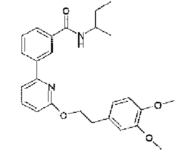 |

**[Table 1-11]**

| | | |
|---|---|---|
| Ex.181 | Ex.182 | Ex.183 |
| 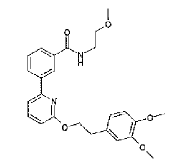 | 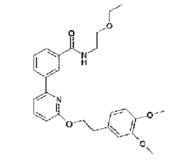 | 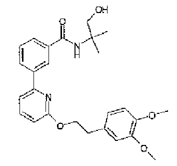 |
| Ex.184 | Ex.185 | Ex.186 |
| 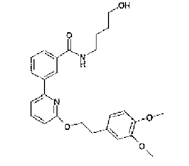 | 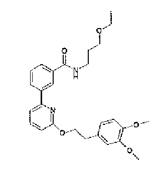 | 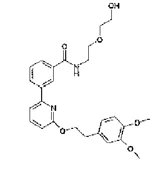 |
| Ex.187 | Ex.18B | Ex.189 |
| 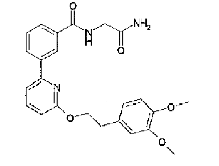 | 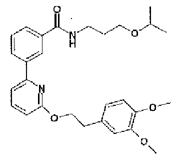 | 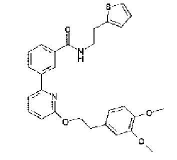 |
| Ex.190 | Ex.191 | Ex.192 |
| 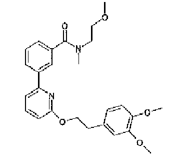 | 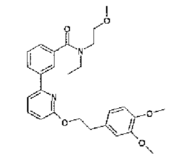 | 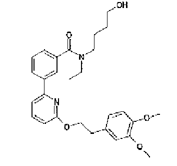 |
| Ex.193 | Ex.194 | Ex.195 |
| 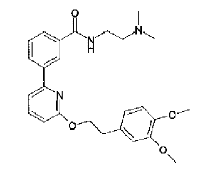 | 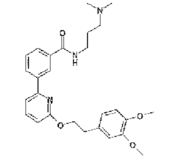 | 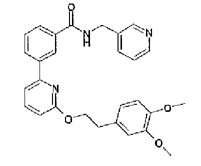 |
| Ex.196 | Ex.197 | Ex.198 |
| 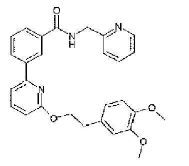 | 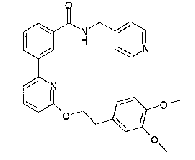 | 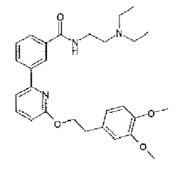 |

**[Table 1-12]**

| | | |
|---|---|---|
| Ex.199 | Ex.200 | Ex.201 |
| 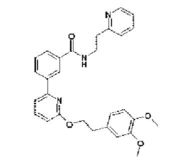 | 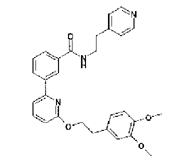 | 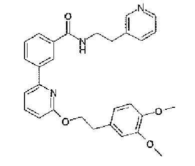 |
| Ex.202 | Ex.203 | Ex.204 |
| 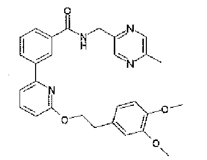 | 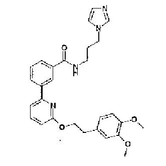 | 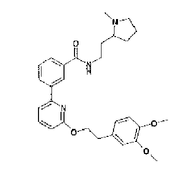 |
| Ex.205 | Ex.206 | Ex.207 |
| 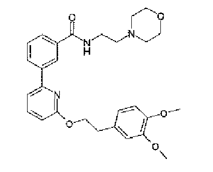 | 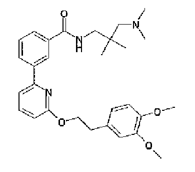 | 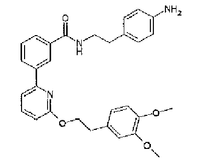 |
| Ex.208 | Ex.209 | Ex.210 |
| 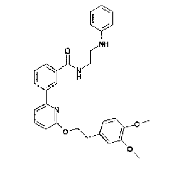 | 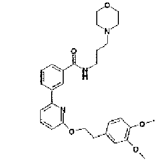 | 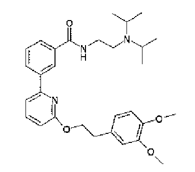 |
| Ex.211 | Ex.212 | Ex.213 |
| 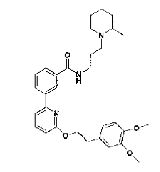 | 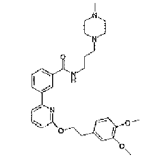 | 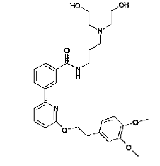 |
| Ex.294 | Ex.215 | Ex.216 |
| 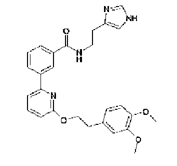 | 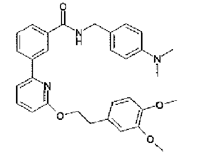 | 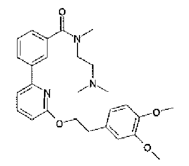 |

**[Table 1-13]**

| | | |
|---|---|---|
| Ex.217 | Ex.218 | Ex.219 |
| 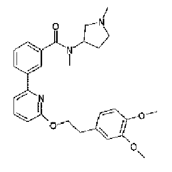 | 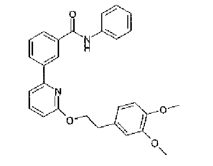 | 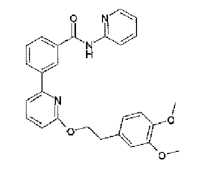 |
| Ex.220 | Ex.221 | Ex,222 |
| 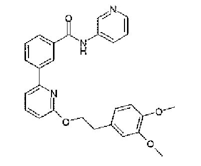 | 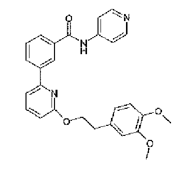 | 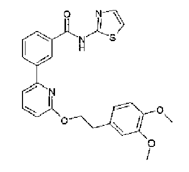 |
| Ex.223 | Ex.224 | Ex.225 |
| 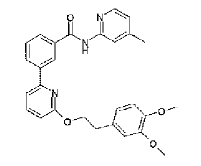 | 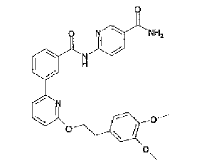 | 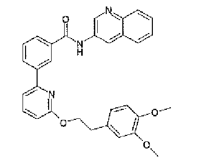 |
| Ex.226 | Ex.227 | Ex.228 |
| 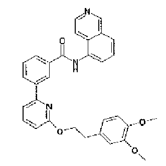 | 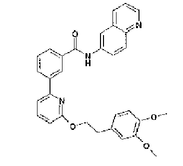 | 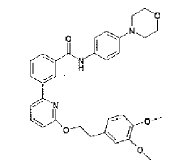 |
| Ex.229 | Ex.230 | Ex.231 |
| 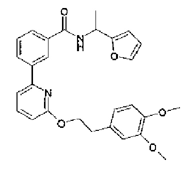 | 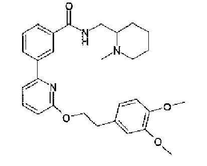 | 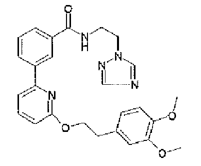 |
| Ex.232 | Ex.233 | Ex.234 |
| 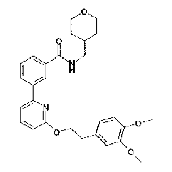 | 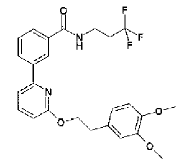 | 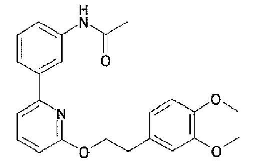 |

**[Table 1-14]**

| | | |
|---|---|---|
| Ex.235 | Ex.236 | Ex.237 |
| 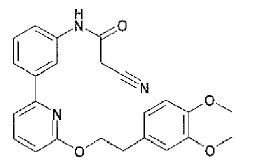 | 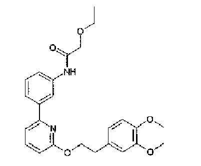 | 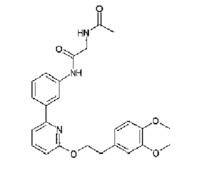 |
| Ex.238 | Ex.239 | Ex.240 |
| 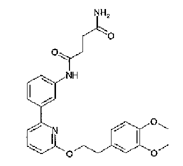 | 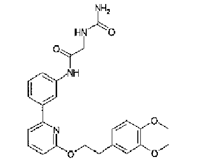 | 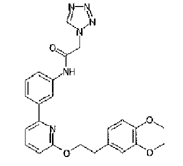 |
| Ex.241 | Ex.242 | Ex.243 |
| 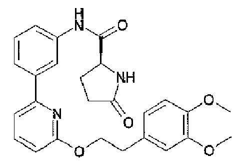 | 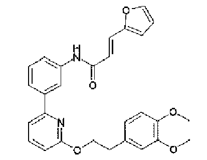 | 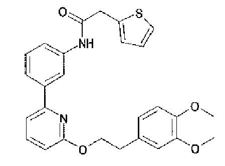 |
| Ex.244 | Ex.245 | Ex.246 |
| 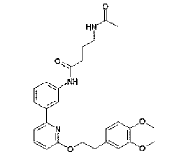 | 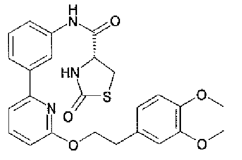 | 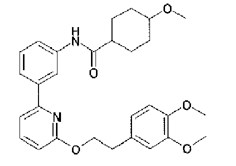 |
| Ex.247 | Ex.248 | Ex.249 |
| 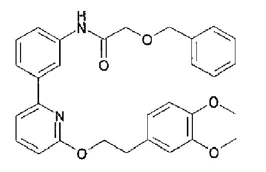 | 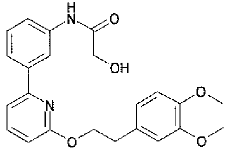 | 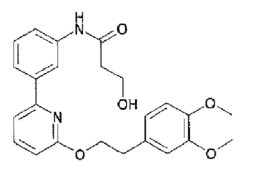 |
| Ex.250 | Ex.251 | Ex.252 |
| 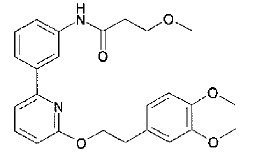 | 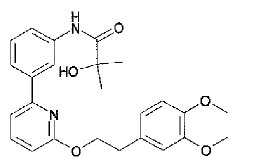 | 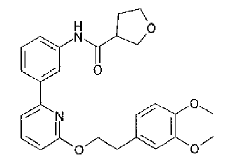 |

**[Table 1-15]**

| | | |
|---|---|---|
| Ex.253 | Ex.254 | Ex.255 |
| 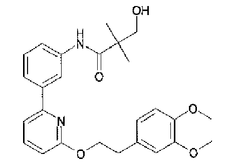 | 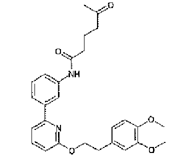 | 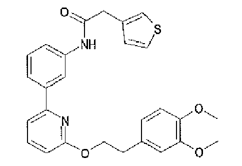 |
| Ex.256 | Ex.257 | Ex.258 |
| 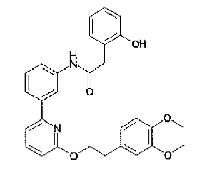 | 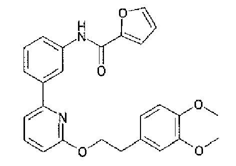 | 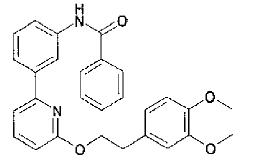 |
| Ex.259 | Ex.260 | Ex.261 |
| 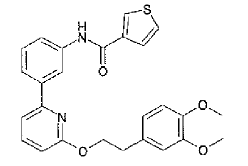 | 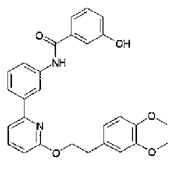 | 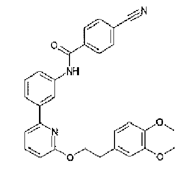 |
| Ex.262 | Ex.263 | Ex.264 |
| 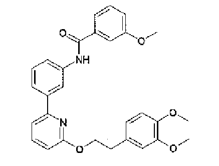 | 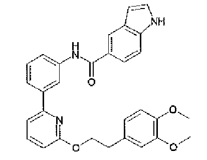 | 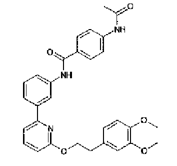 |
| Ex.265 | Ex.266 | Ex.267 |
| 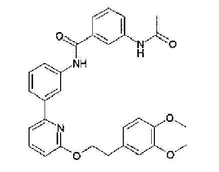 | 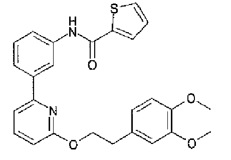 | 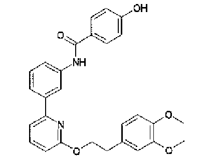 |
| Ex.268 | Ex.269 | Ex.270 |
| 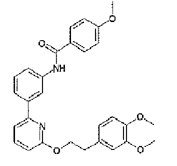 | 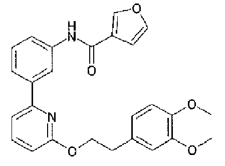 | 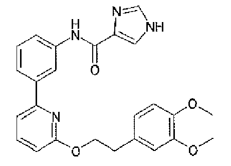 |

**[Table 1-16]**

| | | |
|---|---|---|
| Ex.271 | Ex.272 | Ex.273 |
| 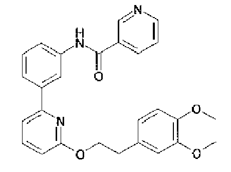 | 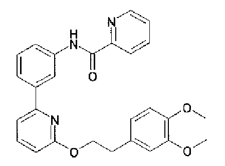 | 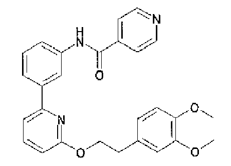 |
| Ex.274 | Ex.275 | Ex.276 |
| 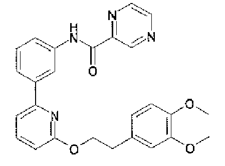 | 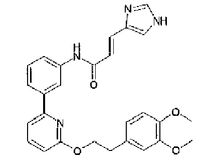 | 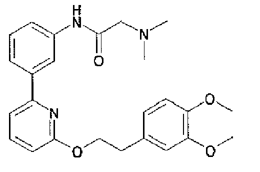 |
| Ex.277 | Ex.278 | Ex.279 |
| 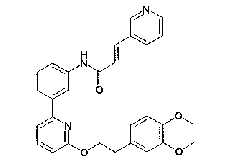 | 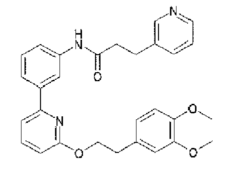 | 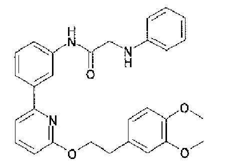 |
| Ex.280 | Ex.281 | Ex.282 |
| 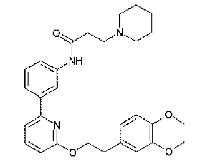 | 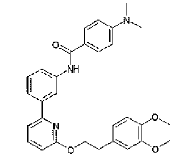 | 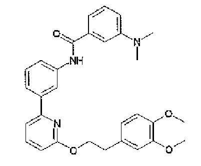 |
| Ex.283 | Ex.284 | Ex.285 |
| 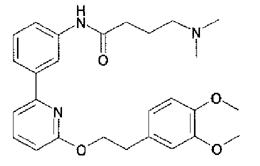 | 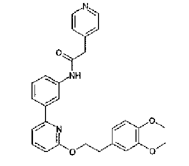 | 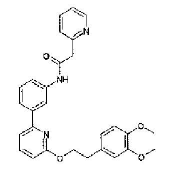 |
| Ex.286 | Ex.287 | Ex.288 |
| 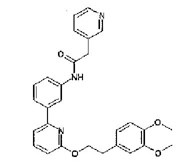 | 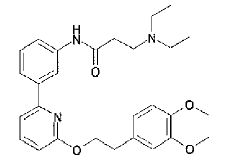 | 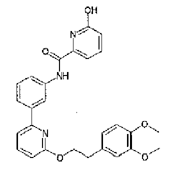 |

**[Table 1-17]**

| | | |
|---|---|---|
| Ex.289 | Ex.290 | Ex.291 |
| 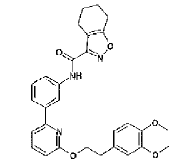 | 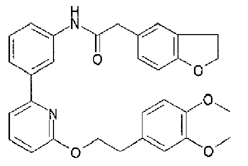 | 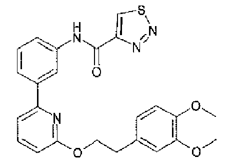 |
| Ex.292 | Ex.293 | Ex.294 |
| 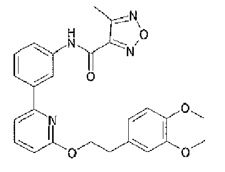 | 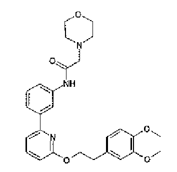 | 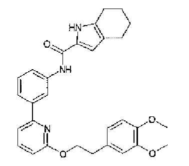 |
| Ex.295 | Ex.296 | Ex.297 |
| 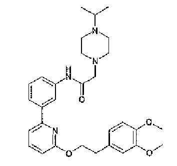 | 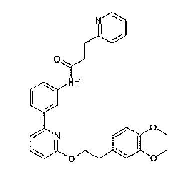 | 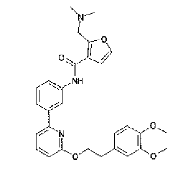 |
| Ex.298 | Ex.299 | Ex.300 |
| 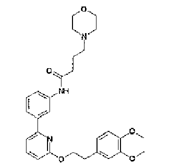 | 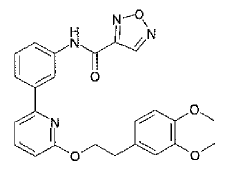 | 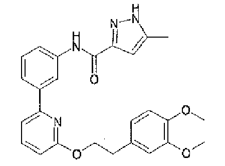 |
| Ex.301 | Ex.302 | Ex.303 |
| 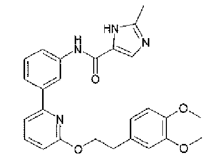 | 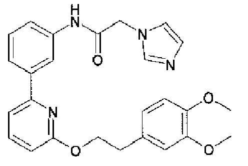 | 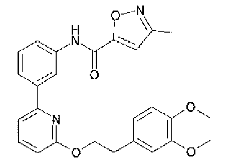 |
| Ex.304 | Ex.305 | Ex.306 |
| 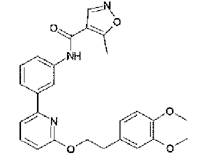 | 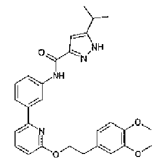 | 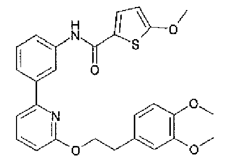 |

**[Table 1-18]**

| | | |
|---|---|---|
| Ex.307 | Ex.308 | Ex.309 |
| 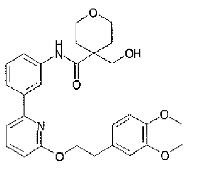 | 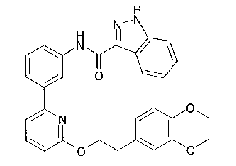 | 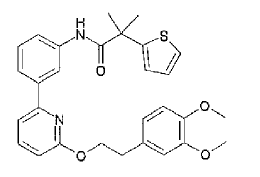 |
| Ex.310 | Ex.311 | Ex.312 |
| 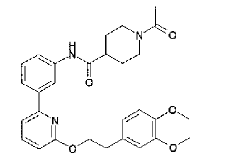 | 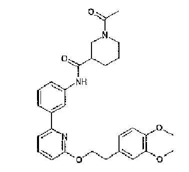 | 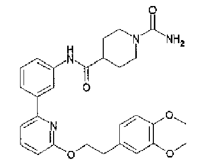 |
| Ex.313 | Ex.314 | Ex.315 |
| 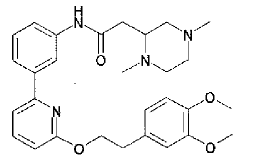 | 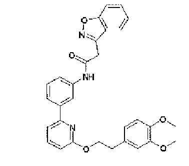 | 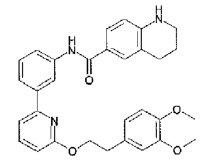 |
| Ex.316 | Ex.317 | Ex.318 |
| 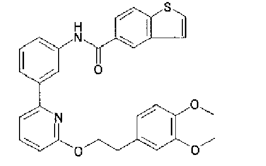 | 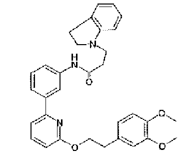 | 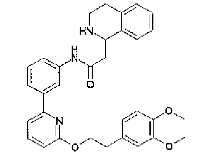 |
| Ex.319 | Ex.320 | Ex.321 |
| 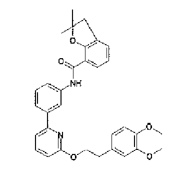 | 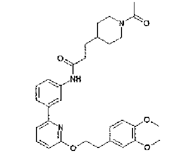 | 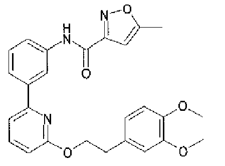 |
| Ex.322 | Ex.323 | Ex.324 |
| 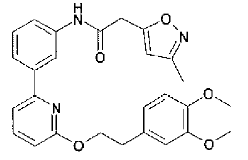 | 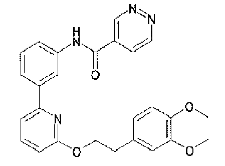 | 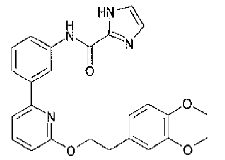 |

**[Table 1-19]**

| | | |
|---|---|---|
| Ex.325 | Ex.326 | Ex.327 |
| 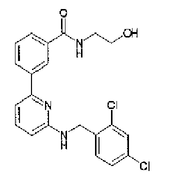 | 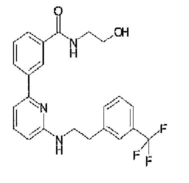 | 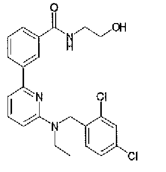 |

**[Table 2-1]**

| Ex. No. | **LCMS** | Ex. No. | **LCMS** |
|---|---|---|---|
| **136** | **446** | **146** | **484** |
| **137** | **416** | **147** | **500** |
| **138** | **446** | **148** | **462** |
| **139** | **446** | **149** | **417** |
| **140** | **476** | **150** | **417** |
| **141** | **476** | **151** | **417** |
| **142** | **476** | **152** | **422** |
| **143** | **434** | **153** | **422** |
| **144** | **450** | **154** | **406** |
| **145** | **430** | **155** | **552** |

**[Table 2-2]**

| Ex. No. | **LCMS** | Ex. No. | LCMS |
|---|---|---|---|
| **156** | **485** | **162** | **502** |
| **157** | **452** | **163** | **467** |
| **158** | **444** | **164** | **468** |
| **159** | **484** | **165** | **468** |
| **160** | **452** | **166** | **464** |
| **161** | **444** | | |

**[Table 2-3]**

| Ex. No. | LCMS | Ex. No. | LCMS |
|---|---|---|---|
| 167 | 393 | 178 | 537 |
| 168 | 419 | 179 | 423 |
| 169 | 435 | 180 | 435 |
| 170 | 437 | 181 | 437 |
| 171 | 459 | 182 | 451 |
| 172 | 463 | 183 | 451 |
| 173 | 464 | 184 | 451 |
| 174 | 465 | 185 | 465 |
| 175 | 450 | 186 | 467 |
| 176 | 499 | 187 | 436 |
| 177 | 504 | 188 | 479 |

**[Table 2-4]**

| Ex. No. | LCMS | Ex. No. | LCMS |
|---|---|---|---|
| 189 | 489 | 201 | 484 |
| 190 | 451 | 202 | 485 |
| 191 | 465 | 203 | 487 |
| 192 | 479 | 204 | 490 |
| 193 | 450 | 205 | 492 |
| 194 | 465 | 206 | 492 |
| 195 | 470 | 207 | 498 |
| 196 | 470 | 208 | 498 |
| 197 | 478 | 209 | 506 |
| 198 | 484 | 210 | 506 |
| 199 | 165 | 211 | 518 |
| 200 | 484 | 212 | 519 |

**[Table 2-5]**

| Ex. No. | LCMS | Ex.No. | LCMS |
|---|---|---|---|
| 213 | 524 | 225 | 506 |
| 214 | 473 | 226 | 506 |
| 215 | 512 | 227 | 506 |
| 216 | 464 | 228 | 540 |
| 217 | 476 | 229 | 473 |
| 218 | 455 | 230 | 490 |
| 219 | 456 | 231 | 474 |
| 220 | 456 | 232 | 477 |
| 221 | 456 | 233 | 475 |
| 222 | 462 | | |
| 223 | 470 | | |
| 224 | 499 | | |

**[Table 2-6]**

| Ex. No. | LCMS | Ex. No. | LCMS |
|---|---|---|---|
| 234 | 393 | 247 | 499 |
| 235 | 418 | 248 | 409 |
| 236 | 437 | 249 | 423 |
| 237 | 450 | 250 | 437 |
| 238 | 450 | 251 | 437 |
| 239 | 451 | 252 | 449 |
| 240 | 461 | 253 | 451 |
| 241 | 462 | 254 | 463 |
| 242 | 471 | 255 | 475 |
| 243 | 475 | 256 | 485 |
| 244 | 478 | 257 | 445 |
| 245 | 480 | 258 | 455 |
| 246 | 491 | 259 | 461 |

**[Table 2-7]**

| Ex. No. | LCMS | Ex. No. | LCMS |
|---|---|---|---|
| 260 | 471 | 276 | 436 |
| 261 | 480 | 277 | 481 |
| 262 | 485 | 278 | 484 |
| 263 | 494 | 279 | 484 |
| 264 | 512 | 280 | 490 |
| 265 | 512 | 281 | 498 |
| 266 | 461 | 282 | 498 |
| 267 | 471 | 283 | 464 |
| 268 | 485 | 284 | 470 |
| 269 | 445 | 285 | 470 |
| 270 | 445 | 286 | 470 |
| 271 | 456 | 287 | 478 |
| 272 | 456 | 288 | 472 |
| 273 | 456 | 289 | 500 |
| 274 | 457 | 290 | 511 |
| 275 | 471 | 291 | 463 |

**[Table 2-8]**

| Ex. No. | LCMS | Ex. No. | LCMS |
|---|---|---|---|
| 292 | 461 | 309 | 503 |
| 293 | 478 | 310 | 504 |
| 294 | 498 | 311 | 504 |
| 295 | 519 | 312 | 505 |
| 296 | 484 | 313 | 505 |
| 297 | 502 | 314 | 510 |
| 298 | 506 | 315 | 510 |
| 299 | 447 | 316 | 511 |
| 300 | 459 | 317 | 524 |
| 301 | 459 | 318 | 524 |
| 302 | 459 | 319 | 525 |
| 303 | 460 | 320 | 532 |
| 304 | 460 | 321 | 460 |
| 305 | 487 | 322 | 474 |
| 306 | 491 | 323 | 457 |
| 307 | 493 | 324 | 445 |
| 308 | 495 | | |

### Preparation Example 1

### Preparation Example 1

| | |
|---|---|
| (1) Compound of Working Example 1 | 10.0 g |
| (2) Lactose | 70.0 g |
| (3) Corn starch | 50.0 g |
| (4) Soluble starch | 7.0 g |
| (5) Magnesium stearate | 3.0 g |

The compound of Working Example 1 (10.0 g) and magnesium stearate (3.0 g) were granulated in a 70 mL aqueous solution of soluble starch (7.0 g as soluble starch), and the granules were then dried and mixed with the lactose (70.0 g) and corn starch (50.0 g) (the lactose, corn starch, soluble starch, and magnesium stearate were all compliant with the Japanese Pharmacopoeia, Fourteenth Edition). The mixture was compressed to obtain tablets.

### Test Example 1

### Increased intracellular cAMP levels in human GPR52 expression CHO cells

1×10⁴ human GPR52 expression CHO (dhfr-) cells were incubated with 1 µM test compound for 30 min at 37°C in 30 µL assay buffer (HBSS (containing Ca²⁺ and Mg²⁺), 0.5% BSA, 100 µM IBMX, 100 µM Ro 20-1724, 5mM HEPES (pH 7.55)) using an OptiPlate-384 (by PerkinElmer). The intracellular cAMP level was then assayed with an EnVision (by PerkinElmer) according to the protocol of the AlphaScreen cAMP Assay Kit (by PerkinElmer). The GPR52 agonist activity was calculated, assuming the intracellular cAMP level to be 100% in the presence of 1 µM of 3-(6-(2-(3,4-dimethoxyphenyl)ethoxy)pyridin-2-yl)-N-(2-pyrrolidin-1-ylethyl)benzamide obtained in Reference Example 39 and assuming the intracellular cAMP level to be 0% when DMSO was added instead of test compound.
The results are shown in Table 3.

### [Table 3]

**[Table 3-1]**

| Working Example No. | GPR52 agonist activity (%) |
|---|---|
| Working Example 1 | 69 |
| Working Example 12 | 47 |
| Working Example 19 | 85 |
| Working Example 22 | 80 |
| Working Example 25 | 71 |
| Working Example 28 | 74 |
| Working Example 29 | 85 |
| Working Example 31 | 98 |
| Working Example 37 | 92 |
| Working Example 39 | 106 |
| Working Example 40 | 87 |
| Working Example 41 | 56 |
| Working Example 42 | 71 |
| Working Example 44 | 68 |
| Working Example 45 | 85 |
| Working Example 46 | 99 |
| Working Example 49 | 55 |
| Working Example 53 | 113 |
| Working Example 59 | 84 |
| Working Example 61 | 43 |
| Working Example 65 | 64 |
| Working Example 67 | 48 |
| Working Example 69 | 65 |
| Working Example 75 | 60 |
| Working Example 76 | 52 |
| Working Example 82 | 64 |
| Working Example 83 | 97 |
| Working Example 87 | 79 |
| Working Example 88 | 62 |
| Working Example 91 | 61 |
| Working Example 94 | 58 |
| Working Example 96 | 66 |

**[Table 3-2]**

| | |
|---|---|
| Working Example 101 | 53 |
| Working Example 102 | 89 |
| Working Example 113 | 77 |
| Working Example 121 | 51 |
| Working Example 125 | 55 |
| Working Example 134 | 48 |
| Working Example 138 | 104 |
| Working Example 141 | 113 |
| Working Example 143 | 72 |
| Working Example 146 | 135 |
| Working Example 152 | 64 |
| Working Example 164 | 107 |
| Working Example 165 | 68 |
| Working Example 166 | 109 |
| Working Example 167 | 68 |
| Working Example 170 | 79 |
| Working Example 175 | 110 |
| Working Example 201 | 85 |
| Working Example 203 | 97 |
| Working Example 208 | 67 |
| Working Example 219 | 94 |
| Working Example 227 | 65 |
| Working Example 234 | 67 |
| Working Example 245 | 58 |
| Working Example 302 | 57 |
| Working Example 318 | 82 |

### [Industrial Applicability]

The compounds of the present invention have GPR52 agonist activity and are useful as agents for preventing and treating schizophrenia or the like.

## Claims

1. A compound represented by formula (I): 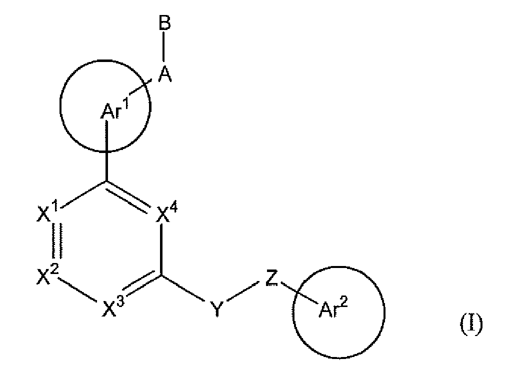 wherein
A represents -(CH₂)ₙ-CO-NR^{a}- (n is an integer of 0 to 3) or -NR^{a}-CO-,
B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
X¹, X², X³, and X⁴ represent the same or different -CR^{x}=, or -N=,
Y represents -O-, -S-, -S(O)-, -S(O)₂-, or -NR^{y}-,
Z represents a bond, methylene, or ethylene,
Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, providing that 1-{2-[(3-chlorobenzyl)oxy]-6-methylpyridin-4-yl}-2-methyl-1H-imidazole-4-carboxamide, tert-butyl {5-[6-(3-chlorophenoxy)pyrazin-2-yl]pyridin-3-yl}carbamate, 5-(6-[3-(trifluoromethyl)phenoxy]pyridin-2-yl)-1,3,4-oxadiazole-2-carboxamide, and N-hydroxy-5-{6-[methyl(2-phenylethyl)amino]pyridin-2-yl}thiophene-2-carboxamide are excluded,
or a salt thereof.

2. The compound according to claim 1, wherein A is -CO-NH- or NH-CO-.

3. The compound according to claim 1, wherein
B is a
(1) hydrogen atom,
(2) C₁₋₆ alkyl group which may be substituted with one or more substituents selected from
(a) halogen atoms,
(b) hydroxy group,
(c) cyano group,
(d) amino group which may be mono- or di-substituted with substituents selected from optionally hydroxy-substituted C₁₋₆ alkyl groups, C₆₋₁₀ aryl groups, C₁₋₆ alkoxy-carbonyl groups, C₁₋₆ alkyl-carbonyl groups, and carbamoyl groups,
(e) C₆₋₁₀ aryl groups which may be substituted with 1 to 3 substituents selected from halogen atoms, hydroxy group, and amino group,
(f) C₁₋₆ alkylsulfanyl groups,
(g) C₁₋₆ alkylsulfinyl groups,
(h) C₁₋₆ alkylsulfonyl groups,
(i) C₁₋₆ alkoxy groups,
(j) C₆₋₁₀ aryloxy groups,
(k) C₇₋₁₃ aralkyloxy groups,
(l) five- to ten-membered heterocyclic groups which may be substituted with one or more substituents selected from C₁₋₆ alkyl groups, C₁₋₆ alkyl-carbonyl groups, and oxo group, and
(m) carbamoyl,
(3) C₂₋₆ alkenyl group which may be substituted with a five- to six-membered heterocyclic group,
(4) C₃₋₁₀ cycloalkyl group,
(5) C₆-₁₀ aryl group which may be substituted with one or more substituents selected from
(a) hydroxy group,
(b) cyano group,
(c) C₁₋₆ alkoxy groups,
(d) mono- or di-C₁₋₆ alkyl-amino groups, and
(e) C₁₋₆ alkyl-earbonylamino groups,
(6) amino group which may be mono- or di-substituted with substituents selected from
(a) C₁₋₆ alkyl groups which may be substituted with mono- or di-C₁₋₆ alkylamino groups, and
(b) five- to ten-membered heterocyclic groups which may be substituted with one or more substituents selected from halogen atoms and oxo group, or
(7) five- to ten-membered heterocyclic group which may be substituted with one or more substituents selected from
(a) halogen atoms,
(b) C₁₋₆ alkyl groups which may be substituted with one or more substituents selected from hydroxy group and mono- or di-C₁₋₆ alkylamino groups,
(c) C₁₋₆ alkoxy groups,
(d) C₁₋₆ alkyl-carbonyl groups,
(e) C₁₋₆ alkoxy-carbonyl groups, and
(f) carbamoyl groups.

4. The compound according to claim 1, wherein one or two of X¹, X², X³, and X⁴ are -N=.

5. The compound according to claim 1, wherein Y is -O-.

6. The compound according to claim 1, wherein Ar¹ is a benzene ring or indole ring which may be substituted with one or more substituents selected from halogen atoms and optionally halogenated C₁₋₆ alkyl groups.

7. The compound according to claim 1, wherein R^{x} is a hydrogen atom or optionally halogenated C₁₋₆ alkyl group.

8. The compound according to claim 1, wherein
A is -CO-NH- or NH-CO-,
B is a
(1) hydrogen atom,
(2) C₁₋₆ alkyl group which may be substituted with one or more substituents selected from
(a) halogen atoms,
(b) hydroxy group,
(c) cyano group,
(d) amino group which may be mono- or di-substituted with substituents selected from optionally hydroxy-substituted C₁₋₆ alkyl groups, C₆₋₁₀ aryl groups, C₁₋₆ alkoxy-carbonyl groups, C₁₋₆ alkyl-carbonyl groups, and carbamoyl groups,
(e) C₆₋₁₀ aryl groups which may be substituted with 1 to 3 substituents selected from halogen atoms, hydroxy group, and amino group,
(f) C₁₋₆ alkylsulfanyl groups,
(g) C₁₋₆ alkylsulfinyl groups,
(h) C₁₋₆ alkylsulfonyl groups,
(i) C₁₋₆ alkoxy groups,
(j) C₆₋₁₀ aryloxy groups,
(k) C₇₋₁₃ aralkyloxy groups,
(l) five- to ten-membered heterocyclic groups which may be substituted with one or more substituents selected from C₁₋₆ alkyl groups, C₁₋₆ alkyl-carbonyl groups, and oxo group, and
(m) carbamoyl,
(3) C₂₋₆ alkenyl group which may be substituted with a five- to six-membered heterocyclic group,
(4) C₃₋₁₀ cycloalkyl group,
(5) C₆₋₁₀ aryl group which may be substituted with one or more substituents selected from
(a) hydroxy group,
(b) cyano group,
(c) C₁₋₆ alkoxy groups,
(d) mono- or di-C₁₋₆ alkyl-amino groups, and
(e) C₁₋₆ alkyl-carbonylamino groups,
(6) amino group which may be mono- or di-substituted with substituents selected from
(a) C₁₋₆ alkyl groups which may be substituted with mono- or di-C₁₋₆ alkylamino groups, and
(b) five- to ten-membered heterocyclic groups which may be substituted with one or more substituents selected from halogen atoms and oxo group, or
(7) five- to ten-membered heterocyclic group which may be substituted with one or more substituents selected from
(a) halogen atoms,
(b) C₁₋₆ alkyl groups which may be substituted with one or more substituents selected from hydroxy group and mono- or di-C₁₋₆ alkylamino groups,
(c) C₁₋₆ alkoxy groups,
(d) C₁₋₆ alkyl-carbonyl groups,
(e) C₁₋₆ alkoxy-carbonyl groups, and
(f) carbamoyl groups;
X¹, X², X³, and X⁴ are the same or different -CR^{x}=, or -N=, and one or two of X¹, X²,
X³, and X⁴ are -N=,
Y is -O-,
Ar¹ is a benzene ring or indole ring which may be substituted with one or more substituents selected from halogen atoms and optionally halogenated C₁₋₆ alkyl groups,
Ar² is a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
R^{x} is a hydrogen atom or optionally halogenated C₁₋₆ alkyl group.

9. The compound according to claim 1, selected from:
4-amino-N-(3-(2-((2-(3,4-dimethoxyphenyl)ethyl)amino)pyrimidin-4-yl)phenyl)butanamide hydrochloride,
N-(3-(2-((2-(3,4-dimethoxyphenyl)ethyl)amino)pyrimidin-4-yl)phenyl)-N³,N³-dimethyl-β-alaninamide hydrochloride,
3-(6-(2-(3,4-dimethoxyphenyl)ethoxy)pyridin-2-yl)-N-(2-pyrrolidin-1-ylethyl)benzamide,
N-(2-hydroxyethyl)-3-(6-(2-(3-(trifluoromethyl)phenyl)ethoxy)pyridin-2-yl)benzamide,
N-(2-hydroxyethyl)-3-(6-(2-(3-methoxyphenyl)ethoxy)pyridin-2-yl)benzamide,
3-(6-(3,5-dichlorophenoxy)pyridin-2-yl)-N-(2-hydroxyethyl)benzamide,
N-(2-aminoethyl)-3-(6-(2-(3-methoxyphenyl)ethoxy)pyridin-2-yl)benzamide hydrochloride,
3-(6-(2,4-dichlorophenoxy)pyridin-2-yl)-N-(2-pyrrolidin-1-yl)benzamide,
3-(6-((2,4-dichlorophenyl)thio)pyridin-2-yl)-N-(2-hydroxyethyl)benzamide,
N-(2-pyrrolidin-1-ylethyl)-3-[2-({2-[3-(trifluoromethyl)phenyl]ethyl}amino)pyrimidin-4-yl]benzamide,
N-cyclopropyl-3-{6-[2-(3,4-dimethoxyphenyl)ethoxy]pyridin-2-yl}benzamide,
N-(3-(6-(2-(3,4-dimethoxyphenyl)ethoxy)pyridin-2-yl)phenyl)acetamide,
3-(6-(2-(3,4-dimethoxyphenyl)ethoxy)pyridin-2-yl)-N-(pyridin-3-ylmethyl)benzamide,
3-(6-(3,5-dichlorophenoxy)pyridin-2-yl)-N-(2-(methylsulfinyl)ethyl)benzamide, and
3-(6-((2,4-dichlorophenyl)amino)pyridin-2-yl)-N-(2-hydroxyethyl)benzamide.

10. A prodrug of the compound according to claim 1.

11. A pharmaceutical agent comprising the compound according to claim 1 or the prodrug according to claim 10.

12. A GPR52 activating agent comprising a compound represented by formula (I): 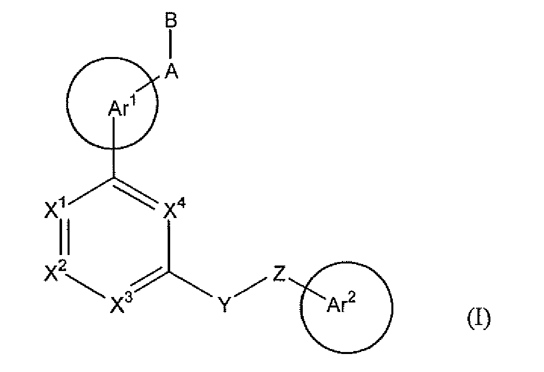 wherein
A represents -(CH₂)ₙ-CO-NR^{a}- (n is an integer of 0 to 3) or -NR^{a}-CO-,
B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
X¹, X², X³, and X⁴ represent the same or different -CR^{x}=, or -N=,
Y represents -O-, -S-, -S(O)-, -S(O)₂-, or -NR^{y}-,
Z represents a bond, methylene, or ethylene,
Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, or a salt or prodrug thereof.

13. The GPR52 activating agent according to claim 12, which is an agent for preventing or treating schizophrenia.

14. A method for preventing or treating diseases involving GPR52 activity in a mammal, comprising administering a compound represented by formula (I): 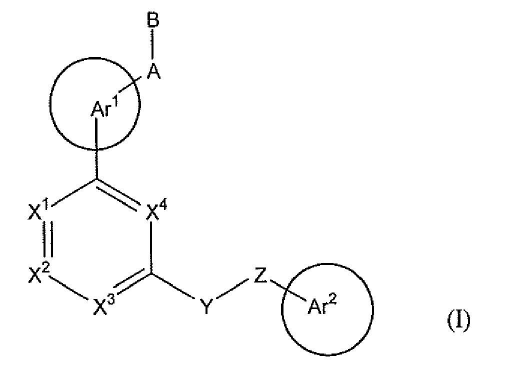 wherein
A represents -(CH₂)ₙ-CO-NR^{a}- (n is an integer of 0 to 3) or -NR^{a}-CO-,
B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
X¹, X², X³, and X⁴ represent the same or different -CR^{x}=, or -N=,
Y represents -O-, -S-, -S(O)-, -S(O)₂-, or -NR^{y}-,
Z represents a bond, methylene, or ethylene,
Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
R^{a}, R^{b}, R^{x}, and R^{y} represent the same or different hydrogen atom, halogen atoms,
optionally halogenated C₁₋₆ alkyl groups, or optionally halogenated C₁₋₆ alkoxy groups,
or a salt or prodrug thereof, to the mammal.

15. The method according to claim 14, wherein the disease involving GPR52 activity is schizophrenia.

16. The use of a compound represented by formula (I): 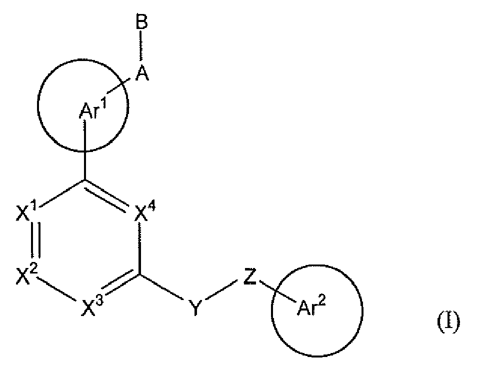 wherein
A represents -(CH₂)ₙ-CO-NR^{a}- (n is an integer of 0 to 3) or -NR^{a}-CO-,
B represents a hydrogen atom, halogen atom, cyano group, hydroxy group, -O-R^{b}, -S-R^{b}, -S(O)-R^{b}, optionally substituted C₁₋₁₄ hydrocarbon group, optionally substituted five- to ten-membered heterocyclic group, optionally substituted amino group, or acyl group,
X¹, X², X³, and X⁴ represent the same or different -CR^{x}=, or -N=,
Y represents -O-, -S-, -S(O)-, -S(O)₂-, or -NR^{y}-,
Z represents a bond, methylene, or ethylene,
Ar¹ represents a five- to ten-membered aromatic ring (except for thiazole) which may be substituted with one or more substituents selected from halogen atoms, optionally halogenated C₁₋₆ alkyl groups, and optionally halogenated C₁₋₆ alkoxy groups,
Ar² represents a five- to six-membered aromatic ring which may be substituted with one or more substituents selected from halogen atoms, optionally halogenated C₁₋₆ alkyl groups, and optionally halogenated C₁₋₆ alkoxy groups, and which may be condensed with an optionally substituted five- to six-membered ring, and
R^{a}, R^{b}, R^{x}, and R^{y} represent the same or different hydrogen atom, halogen atoms, optionally halogenated C₁₋₆ alkyl groups, or optionally halogenated C₁₋₆ alkoxy groups, or a salt or prodrug thereof, for the manufacture of a GPR52 activating agent.

17. The use according to claim 16, wherein the GPR52 activating agent is as an agent for preventing or treating schizophrenia.
